(19) Europäisches Patentamt
European Patent Office
Office européen des brevets

(11) EP 4 772 511 A1

(12) EUROPEAN PATENT APPLICATION
published in accordance with Art. 153(4) EPC

(43) Date of publication:
**08.07.2026 Bulletin 2026/28**

(21) Application number: **24858775.0**

(22) Date of filing: **02.09.2024**

(51) International Patent Classification (IPC):
***C07D 487/08*** *(2006.01)* ***C07D 487/04*** *(2006.01)*
***C07D 403/14*** *(2006.01)* ***C07D 403/12*** *(2006.01)*
***C07D 403/08*** *(2006.01)* ***C07D 413/14*** *(2006.01)*
***C07D 401/14*** *(2006.01)* ***C07D 498/14*** *(2006.01)*
***C07D 405/14*** *(2006.01)* ***A61K 31/506*** *(2006.01)*
***A61K 31/501*** *(2006.01)* ***A61K 31/502*** *(2006.01)*
***A61P 35/00*** *(2006.01)*

(52) Cooperative Patent Classification (CPC):
**A61K 31/496; A61K 31/501; A61K 31/502; A61K 31/506; A61K 31/553; A61P 29/00; A61P 31/14; A61P 31/20; A61P 35/00; A61P 37/06; C07D 401/12; C07D 401/14; C07D 403/08; C07D 403/12; C07D 403/14;** (Cont.)

(86) International application number:
**PCT/CN2024/116240**

(87) International publication number:
**WO 2025/045246 (06.03.2025 Gazette 2025/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.09.2023 CN 202311125694**
**30.10.2023 CN 202311423018**

(71) Applicant: **Chongqing Pharscin Innobio Co., Ltd.**
**Chongqing 401121 (CN)**

(72) Inventors:
- **LIU, Xiaohui**
**Chongqing 402460 (CN)**
- **ZHANG, Wei**
**Chongqing 402460 (CN)**
- **WANG, Ying**
**Chongqing 402460 (CN)**
- **ZHANG, Qi**
**Chongqing 402460 (CN)**
- **JIANG, Shiyi**
**Chongqing 402460 (CN)**
- **FENG, Xuerong**
**Chongqing 402460 (CN)**
- **TIAN, Shijie**
**Chongqing 402460 (CN)**
- **YANG, Xia**
**Chongqing 402460 (CN)**

(74) Representative: **Elzaburu S.L.P.**
**Edificio Torre de Cristal**
**Paseo de la Castellana 259 C, planta 28**
**28046 Madrid (ES)**

(54) **PARP7 INHIBITOR AND USE THEREOF**

(57) The present disclosure relates to a compound as shown in formula (I) having PARP7 inhibitory activity, a pharmaceutically acceptable salt, stereoisomer, solvate, or prodrug thereof, and its use and preparation method.

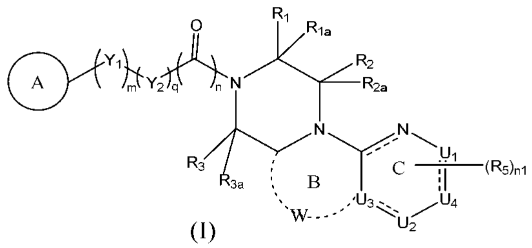

(I)

EP 4 772 511 A1

(52) Cooperative Patent Classification (CPC): (Cont.)
**C07D 405/14; C07D 413/14; C07D 471/04;
C07D 471/14; C07D 487/04; C07D 487/08;
C07D 498/14**

## Description

This application claims priority to Chinese Patent Application No. 202311125694.7, filed on September 1, 2023, and Chinese Patent Application No. 202311423018.8, filed on October 30, 2023, the entire contents of which are incorporated herein by reference.

## Technical Field

The present disclosure relates to the field of medicine, and particularly to a compound with PARP7 inhibitory activity, its use, and preparation method.

## Background

Poly-ADP-ribose polymerases (PARPs) are a family of proteins consisting of 17 different members in the human body, which play important roles in different biological processes, including playing important roles in inflammation, viral infection, and tumor cell stress. The PARP family can be divided into three categories according to their catalytic activity: polyPARPs that poly-ADP-ribosylate target proteins (4 members, including PARP1, PARP2, PARP5a, PARP5b), mono-PARPs that mono-ADP-ribosylate target proteins (12 members, including PARP3, PARP4, PARP6, PARP7, PARP8, PARP9, PARP10, PARP11, PARP12, PARP14, PARP15, PARP16), and a PARP with no catalytic activity (1 member, PARP13). Most research on PARPs has focused on polyPARPs (PARP1, 2, 5a, and 5b), with the role of PARP1/2 in DNA damage repair being the most studied. DNA damage refers to a permanent change in the DNA nucleotide sequence that occurs during DNA replication, leading to a change in genetic traits. If DNA damage or abnormal changes in genetic information cannot be corrected, it will affect the function or survival of the cell. The repair of DNA damage is a programmed, orderly, multi-stage, and precise process involving multiple factors, and PARP is an important protein in the DNA repair process, participating in a series of important cellular processes including DNA repair and maintenance of genomic stability. Four PARP inhibitors have been approved as drugs: niraparib and talazoparib target PARP1 and PARP2; olaparib and rucaparib target PARP1, PARP2, and PARP3. Moreover, olaparib (First-in-Class), a P ARP 112 inhibitor developed by AstraZeneca, has become a blockbuster drug with significant anti-cancer efficacy, and its indications can be gradually expanded through combination therapy.

There has been relatively little research on monoPARPs that mono-ADP-ribosylate target proteins. As mentioned above, most members (12) of the PARP family are monoPARPs, and recent research on monoPARPs has found that PARP7 plays an important role in immunity and cancer. Among them, studies suggest that PARP7 is one of the target genes of AHR, PARP7 is part of a negative feedback loop that regulates AHR activity, and AHR can regulate immune function, inflammation, etc., and plays a role in cancer. It can be induced by cancer-related stress, such as harmful chemicals in tobacco. In-depth research on lung cancer, esophageal cancer, and head and neck cancer, which are strongly correlated with smoking, has found amplification and high expression at the gene locus where PARP7 is located. Studies have found that PARP7 is overactive in tumor cells, many cancer cells rely on PARP7 for cell survival, and studies have shown that PARP7 enables cancer cells to evade immune attack from the immune system. Inhibiting PARP7 can effectively inhibit the growth of cancer cells, restore interferon signaling, and reverse immunosuppression. In several cancer models, PARP7 inhibitors have shown durable tumor growth inhibition and restoration of interferon signaling. In summary, PARP7 plays an important role in cancer and immune response, and is a potential ideal cancer target. Due to the lack of PARP7-selective chemical probes in earlier studies, genetic methods were mostly used to reveal the function of PARP7, making it impossible to distinguish between enzymatic activity and the entire protein and its interactions. In addition, the previous lack of mature high-throughput on-target activity screening test methods also hindered the corresponding drug research and development for the PARP7 target. In view of the successive reports of PARP7-related research results in recent years, PARP7 has become a relatively ideal anti-cancer target. Currently, no PARP7 inhibitors have been approved for marketing. RBN-2397, developed by Ribon Therapeutics, is the first compound with strong inhibitory activity and selectivity for PARP7. Therefore, it is of great significance to further research and develop new, safe, and effective PARP7 inhibitors. In addition, as mentioned above, besides playing an important role in neoplasms, PARP7 also plays a key role in diseases such as inflammation and viral infection. Therefore, in addition to their anti-cancer effects, PARP7 inhibitors are also expected to have potential application value in other diseases such as inflammation, immune diseases, and viral infections.

## Summary

An object of the present disclosure is to provide a compound with PARP7 inhibitory activity, or a pharmaceutically acceptable salt, stereoisomer, solvate, or prodrug thereof.

In one aspect, the present invention provides a compound represented by formula (I), a pharmaceutically

acceptable salt, stereoisomer, solvate, or prodrug thereof,

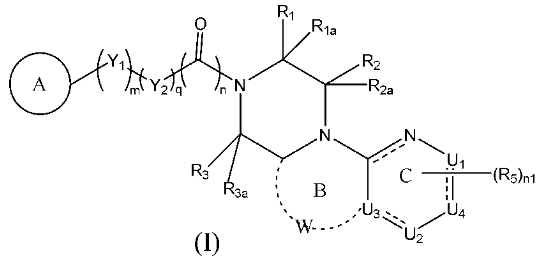

(I)

wherein, in formula (I),
Ring A is selected from

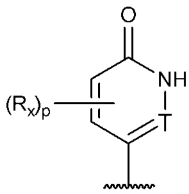 , 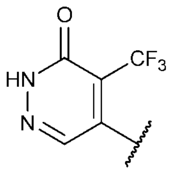 , or 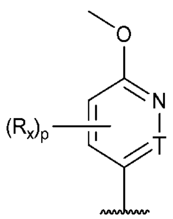 ;

T is selected from CH or N;
$R_x$ is selected from hydrogen, deuterium, halogen, cyano, $C_{1-6}$ alkyl, $C_{3-7}$ cycloalkyl, $C_{1-6}$ alkoxy, $C_{3-7}$ cycloalkoxy, $C_{2-6}$ alkenyl, $C_{2-6}$ alkynyl, $C_{1-6}$ haloalkyl, $-NO_2$, $-NH_2$, wherein the $C_{1-6}$ alkyl, $C_{3-7}$ cycloalkyl, $C_{1-6}$ alkoxy, $C_{3-7}$ cycloalkoxy, $C_{2-6}$ alkenyl, $C_{2-6}$ alkynyl, $C_{1-6}$ haloalkyl are optionally substituted with one or more hydrogen, deuterium, methyl, ethyl, propyl, or isopropyl;
p is selected from 0, 1, or 2;
Ring A is selected from

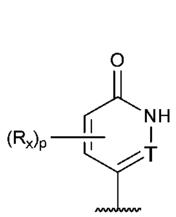 or 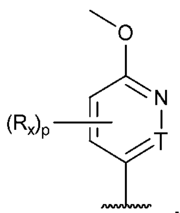 ,

Y is selected from $-C(R_6)(R_7)-$, m is selected from 1, 2, or 3, q is selected from 1, n is selected from 1, wherein $R_6$, $R_7$ are each independently selected from hydrogen, deuterium, $C_{1-3}$ alkyl, or $R_6$, $R_7$ together with the carbon atom to which they are attached form a 3-6 membered cycloalkyl;
or Ring A is selected from

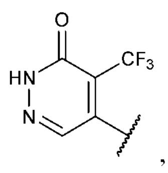 ,

$Y_1$ is selected from $-N(R_6)-C_{1-3}$ alkyl-, m is selected from 1, q is selected from 1, n is selected from 1, wherein $R_6$ is selected from hydrogen or deuterium, any two of $R_1$, $R_2$, $R_3$, $R_4$ are optionally connected to each other to form $-(CH_2)_{n2}-$, wherein $n_2$ is selected from 1, 2, or 3;
$Y_2$ is selected from $-N(R_a)-C_{3-7}$ cycloalkyl-, $-N(R_a)-C_{6-10}$ aryl-, a 3-10 membered nitrogen-containing heterocycloalkyl containing at least one nitrogen atom, or a 7-10 membered nitrogen-containing spirocycloalkyl containing at least one nitrogen atom, wherein $R_a$ is selected from hydrogen, deuterium, or $C_{1-3}$ alkyl, and wherein the $-N(R_a)-C_{3-7}$ cycloalkyl-, $-N(R_a)-C_{6-10}$ aryl-, 3-10 membered nitrogen-containing heterocycloalkyl containing at least one nitrogen atom, 7-10 membered nitrogen-containing spirocycloalkyl containing at least one nitrogen atom, and $C_{1-3}$ alkyl are

optionally substituted with one or more hydrogen, deuterium, methyl, ethyl, propyl, or isopropyl;

Ring B is absent, and the group

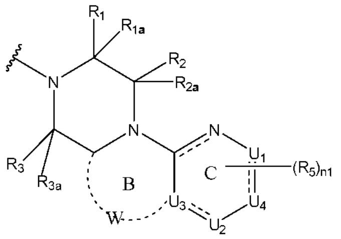 is 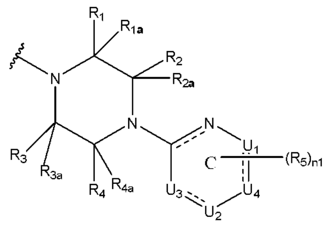 ;

or Ring B is a 5-8 membered saturated or unsaturated heterocycle, wherein the Ring B is optionally substituted with one or more halogen, hydroxyl, $C_{1-6}$ alkyl, or $C_{1-6}$ alkoxy, and wherein the $C_{1-6}$ alkyl and $C_{1-6}$ alkoxy are optionally substituted with one or more hydrogen, deuterium, methyl, ethyl, propyl, or isopropyl;

W is selected from C, N, O, or S;

$U_1$, $U_2$, $U_3$, and $U_4$ are each independently selected from CH, $CH_2$, N, or NH;

the halogen is selected from fluorine, chlorine, or bromine;

$R_1$, $R_{1a}$, $R_2$, $R_{2a}$, $R_3$, $R_{3a}$, $R_4$, $R_{4a}$ are each independently selected from hydrogen, deuterium, halogen, hydroxyl, amino, nitro, cyano, $C_{1-6}$ alkyl, wherein the $C_{1-6}$ alkyl is optionally substituted with one or more halogen, hydroxyl, cyano, carboxyl, or $C_{1-3}$ alkyl, or any two of $R_1$, $R_2$, $R_3$, $R_4$ are optionally connected to each other to form $-(CH_2)_{n2}-$, wherein $n_2$ is selected from 1, 2, or 3, or $R_1$ and $R_{1a}$ together form an oxo, or $R_2$ and $R_{2a}$ together form an oxo, or $R_3$ and $R_{3a}$ together form an oxo, or $R_4$ and $R_{4a}$ together form an oxo;

$n_1$ $R_5$ are the same or different from each other, $n_1$ is selected from 0, 1, 2, or 3, $R_5$ is selected from hydrogen, deuterium, halogen, cyano, $C_{1-6}$ alkyl, $C_{1-6}$ haloalkyl, $C_{3-7}$ cycloalkyl, $C_{1-6}$ alkoxy, $C_{3-7}$ cycloalkoxy, $C_{2-6}$ alkenyl, $C_{2-6}$ alkynyl, $C_{1-3}$ amido, aminoacyl, 4-6 membered cycloaminoacyl, 4-6 membered saturated or unsaturated heterocyclic group, sulfonamido, 4-6 membered heterocycloamino, or two $R_5$ substituents on adjacent carbon atoms of Ring C, together with the carbon atoms to which they are attached, form a 4-6 membered saturated or unsaturated heterocyclic group, wherein the $C_{1-6}$ alkyl, $C_{1-6}$ haloalkyl, $C_{3-7}$ cycloalkyl, $C_{1-6}$ alkoxy, $C_{3-7}$ cycloalkoxy, $C_{2-6}$ alkenyl, $C_{2-6}$ alkynyl, $C_{1-3}$ amido, aminoacyl, 4-6 membered cycloaminoacyl, 4-6 membered saturated or unsaturated heterocyclic group, sulfonamido, and 4-6 membered heterocycloamino are optionally substituted with one or more halogen, hydroxyl, or $C_{2-6}$ ester group.

In another aspect, the present invention provides a compound represented by formula (I'), a pharmaceutically acceptable salt, stereoisomer, solvate, or prodrug thereof,

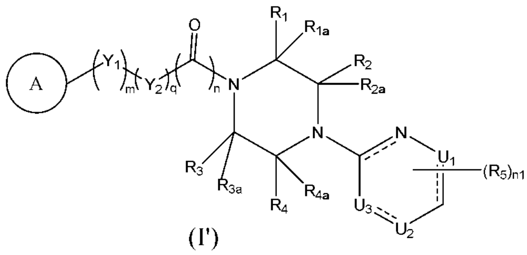

(I')

wherein, in formula (I'),

Ring A is selected from

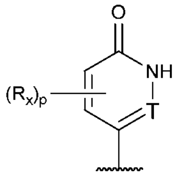 or 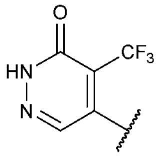 ;

T is selected from CH or N;

$R_x$ is selected from hydrogen, deuterium, halogen, cyano, $C_{1-6}$ alkyl, $C_{1-6}$ alkoxy, $C_{2-6}$ alkenyl, $C_{2-6}$ alkynyl, $C_{1-6}$

haloalkyl, $-NO_2$, or $-NH_2$;

p is selected from 0, 1, or 2;

$Y_2$ is selected from $-N(R_a)-C_{3-7}$ cycloalkyl-, $-N(R_a)-C_{6-10}$ aryl-, a 3-10 membered nitrogen-containing heterocycloalkyl containing at least one nitrogen atom, or a 7-10 membered nitrogen-containing spirocycloalkyl containing at least one nitrogen atom, wherein $R_a$ is selected from hydrogen or deuterium;

$U_1$, $U_2$, and $U_3$ are each independently selected from CH, $CH_2$, N, or NH; the halogen is selected from fluorine, chlorine, or bromine;

$R_1$, $R_{1a}$, $R_2$, $R_{2a}$, $R_3$, $R_{3a}$, $R_4$, $R_{4a}$ are each independently selected from hydrogen, deuterium, halogen, hydroxyl, amino, nitro, cyano, $C_{1-6}$ alkyl, wherein the $C_{1-6}$ alkyl is optionally substituted with one or more halogen, hydroxyl, cyano, carboxyl, or $C_{1-3}$ alkyl, or any two of $R_1$, $R_2$, $R_3$, $R_4$ are optionally connected to each other to form $-(CH_2)_{n2}-$, wherein $n_2$ is selected from 1, 2, or 3, or $R_1$ and $R_{1a}$ together form an oxo, or $R_2$ and $R_{2a}$ together form an oxo, or $R_3$ and $R_{3a}$ together form an oxo, or $R_4$ and $R_{4a}$ together form an oxo;

$n_1$ $R_5$ are the same or different from each other, $n_1$ is selected from 0, 1, 2, or 3, $R_5$ is selected from hydrogen, deuterium, halogen, cyano, $C_{1-6}$ alkyl, $C_{1-6}$ alkoxy, $C_{2-6}$ alkenyl, or $C_{2-6}$ alkynyl, wherein the $C_{1-6}$ alkyl, $C_{1-6}$ alkoxy, $C_{2-6}$ alkenyl, and $C_{2-6}$ alkynyl are optionally substituted with one or more halogen, hydroxyl, $C_{2-6}$ ester group, $C_{1-3}$ amido, or sulfonamido;

the $C_{1-6}$ alkyl, $C_{1-6}$ alkoxy, $C_{2-6}$ alkenyl, $C_{2-6}$ alkynyl, $C_{1-6}$ haloalkyl, $C_{1-3}$ alkyl, $C_{3-7}$ cycloalkyl, $C_{6-10}$ aryl, 3-10 membered nitrogen-containing heterocycloalkyl containing at least one nitrogen atom, 7-10 membered nitrogen-containing spirocycloalkyl containing at least one nitrogen atom, $C_{2-6}$ ester group, and $C_{1-3}$ amido are optionally substituted with one or more hydrogen, deuterium, methyl, ethyl, propyl, or isopropyl;

when Ring A is selected from

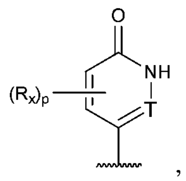

,

$Y_1$ is selected from $-C(R_6)(R_7)-$, m is selected from 0, 1, 2, or 3, q is selected from 0 or 1, n is selected from 0 or 1, and m, n, and q are not 0 at the same time, wherein $R_6$, $R_7$ are each independently selected from hydrogen or deuterium;

when Ring A is selected from

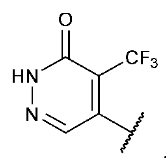

,

$Y_1$ is selected from $-N(R_6)-C_{1-3}$ alkyl-, m is selected from 1, q is selected from 1, n is selected from 1, wherein $R_6$ is selected from hydrogen or deuterium, and any two of $R_1$, $R_2$, $R_3$, $R_4$ are optionally connected to each other to form $-(CH_2)_{n2}-$, wherein $n_2$ is selected from 1, 2, or 3.

In some embodiments,

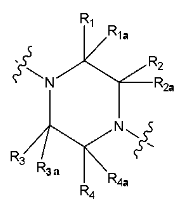

is selected from

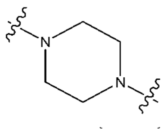, 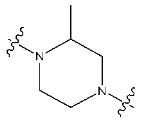, 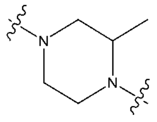,

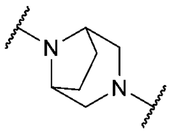, 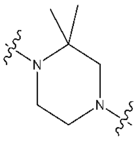, 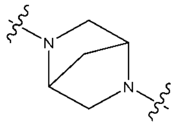, 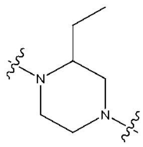, 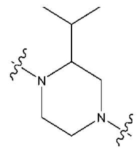, 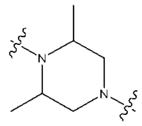, 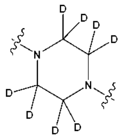,

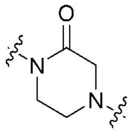, 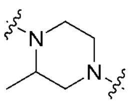, 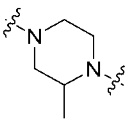, 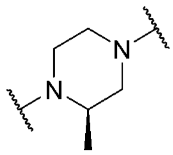, or 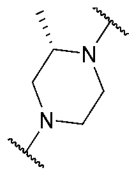;

Preferably,

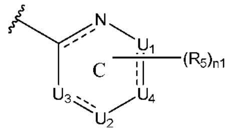

is selected from

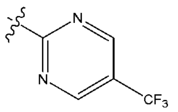, 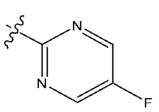, 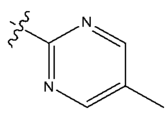,

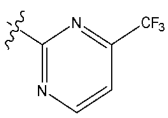, 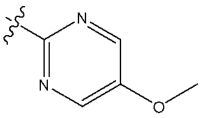, 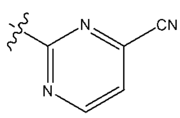, 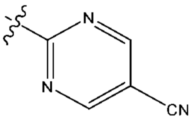, 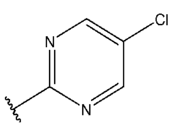, 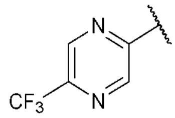,

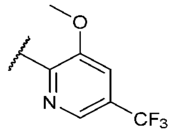, 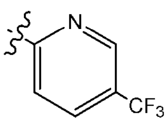 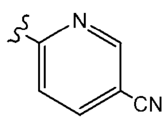 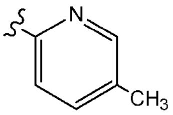, 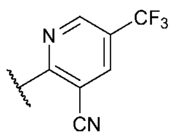, 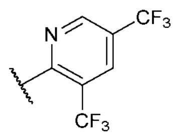,

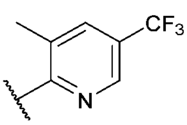, 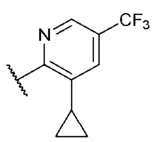, 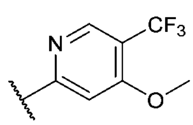, 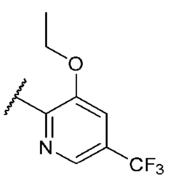, 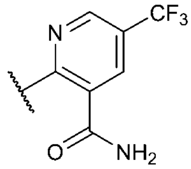, 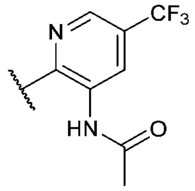,

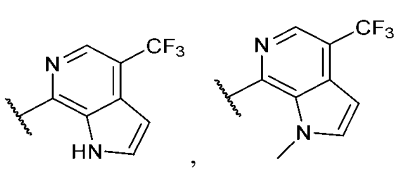, 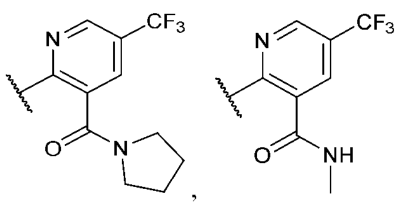, 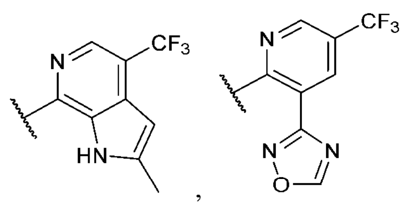,

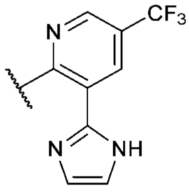, 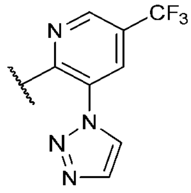, 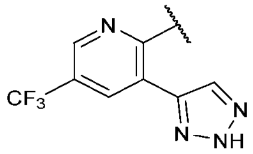, 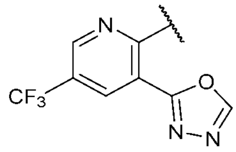, 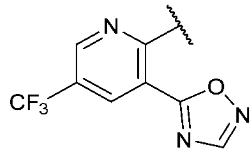,

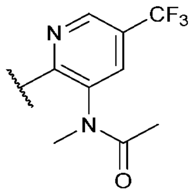, 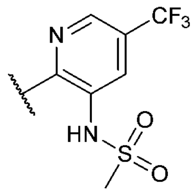, 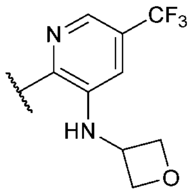, or 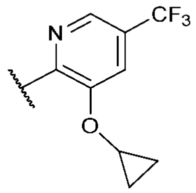;

Preferably, Ring B is present, and

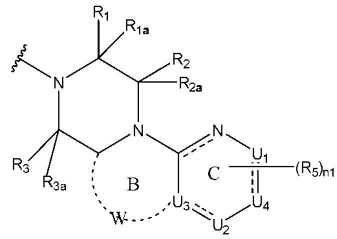

is selected from

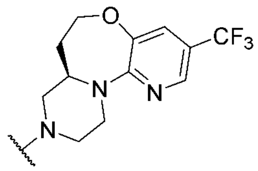,

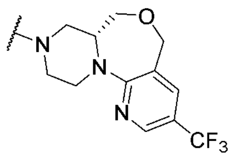, 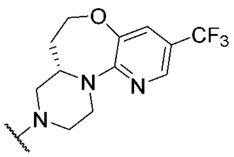, 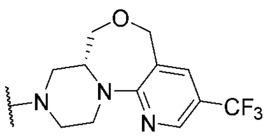, 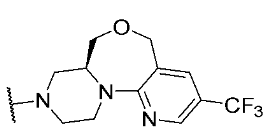, or

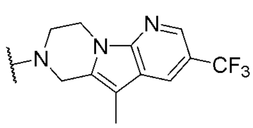;

Preferably, W is selected from C or O.

In some embodiments, Ring A is selected from

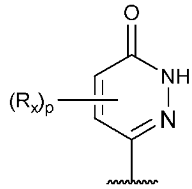, 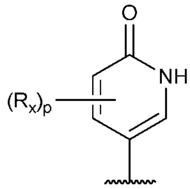, 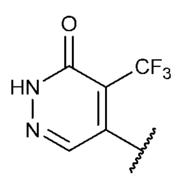,

or

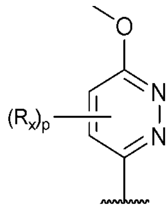

;

Preferably, $R_x$ is selected from hydrogen, deuterium, $C_{1-6}$ alkoxy, $C_{3-7}$ cycloalkoxy, $C_{1-6}$ haloalkyl, wherein the $C_{1-6}$ alkoxy, $C_{3-7}$ cycloalkoxy, $C_{2-6}$ alkenyl, $C_{2-6}$ alkynyl, and $C_{1-6}$ haloalkyl are optionally substituted with one or more hydrogen, deuterium, methyl, ethyl, propyl, or isopropyl;

Preferably, p is selected from 1 or 2;
Preferably, Ring A is selected from

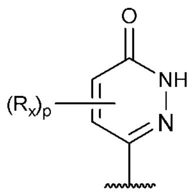

,

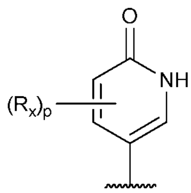

, or

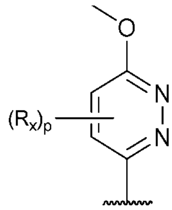

,

$Y_1$ is selected from -C($R_6$) ($R_7$)-, m is selected from 1, q is selected from 1, n is selected from 1, wherein $R_6$, $R_7$ are each independently selected from hydrogen, deuterium, methyl, or $R_6$, $R_7$ together with the carbon atom to which they are attached form a 3-6 membered cycloalkyl;
or Ring A is selected from

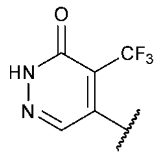

,

$Y_1$ is selected from -N($R_6$)-$C_{1-3}$ alkyl-, m is selected from 1, q is selected from 1, n is selected from 1, wherein $R_6$ is selected from hydrogen or deuterium, and any two of $R_1$, $R_2$, $R_3$, $R_4$ are optionally connected to each other to form $-(CH_2)_{n2}-$, wherein $n_2$ is selected from 1 or 2;
Preferably, $Y_2$ is selected from -N($R_a$)-$C_{3-7}$ cycloalkyl-, -N($R_a$)-$C_{6-10}$ aryl-, a 3-10 membered nitrogen-containing heterocycloalkyl containing at least one nitrogen atom, or a 7-10 membered nitrogen-containing spirocycloalkyl containing at least one nitrogen atom, wherein $R_a$ is selected from hydrogen, deuterium, or $C_{1-3}$ alkyl, and wherein the -N($R_a$)-$C_{3-7}$ cycloalkyl-, -N($R_a$)-$C_{6-10}$ aryl-, 3-10 membered nitrogen-containing heterocycloalkyl containing at least one nitrogen atom, 7-10 membered nitrogen-containing spirocycloalkyl containing at least one nitrogen atom, and $C_{1-3}$ alkyl are optionally substituted with one or more hydrogen, deuterium, methyl, ethyl, propyl, or isopropyl;
Preferably, $U_1$, $U_2$, $U_3$, and $U_4$ are each independently selected from CH, $CH_2$, N, or NH;
Preferably, the halogen is selected from fluorine or chlorine;
Preferably, $R_1$, $R_{1a}$, $R_2$, $R_{2a}$, $R_3$, $R_{3a}$, $R_4$, $R_{4a}$ are each independently selected from hydrogen, deuterium, $C_{1-6}$ alkyl, or any two of $R_1$, $R_2$, $R_3$, $R_4$ are optionally connected to each other to form $-(CH_2)_{n2}-$, wherein $n_2$ is selected from 1 or 2;
Preferably, $n_1$ $R_5$ are the same or different from each other, $n_1$ is selected from 0, 1, 2, or 3, $R_5$ is selected from hydrogen, deuterium, halogen, cyano, $C_{1-6}$ alkyl, $C_{1-6}$ haloalkyl, $C_{3-7}$ cycloalkyl, $C_{1-6}$ alkoxy, $C_{3-7}$ cycloalkoxy, $C_{1-3}$ amido, aminoacyl, 4-6 membered cycloaminoacyl, 4-6 membered saturated or unsaturated heterocyclic group, sulfonamido, 4-6 membered heterocycloamino, or two $R_5$ substituents on adjacent carbon atoms of Ring C, together with the carbon atoms to which they are attached, form a 4-6 membered saturated or unsaturated heterocyclic group, wherein the $C_{1-6}$ alkyl, $C_{1-6}$ haloalkyl, $C_{3-7}$ cycloalkyl, $C_{1-6}$ alkoxy, $C_{3-7}$ cycloalkoxy, $C_{1-3}$ amido, aminoacyl, 4-6 membered cycloaminoacyl, 4-6 membered saturated or unsaturated heterocyclic group, sulfonamido, and 4-6 membered heterocycloamino are optionally substituted with one or more halogen or hydroxyl.

In some embodiments, Ring A is selected from

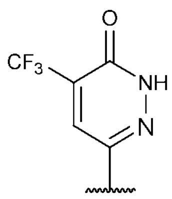, 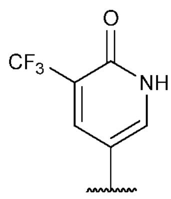, 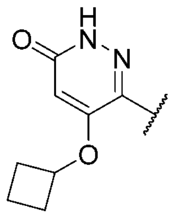,

or

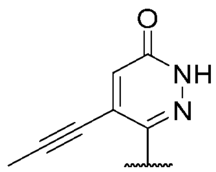,

and $Y_1$ is selected from $-CH_2-$, $-CH(CH_3)-$, $-C(CH_3)_2-$, or

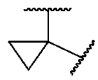;

or Ring A is selected from

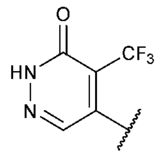,

$Y_1$ is selected from $-NH-CH(CH_3)\ CH_2-$, and any two of $R_1$, $R_2$, $R_3$, $R_4$ are optionally connected to each other to form $-(CH_2)_{n2}-$, wherein $n_2$ is selected from 1 or 2;

Preferably, $Y_2$ is selected from

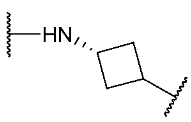, 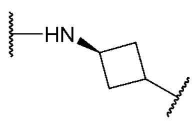, 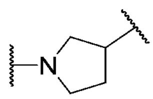, 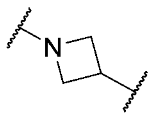,

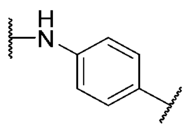, 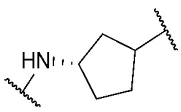, 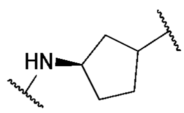, 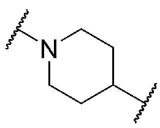, 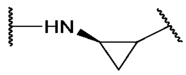, 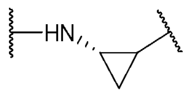,

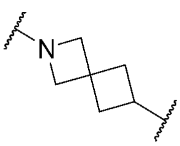, 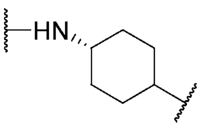, 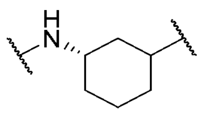, 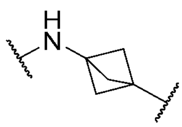, 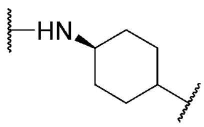,

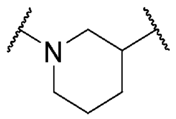 , or 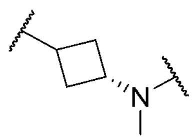 ;

Preferably, Ring B is absent, and the group

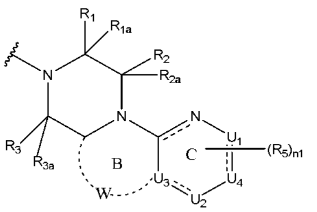

is selected from

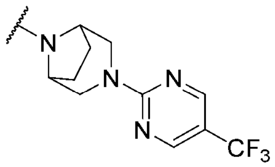 , 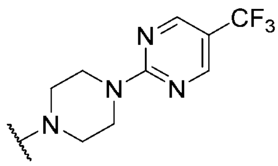 , 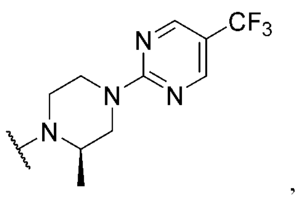 , 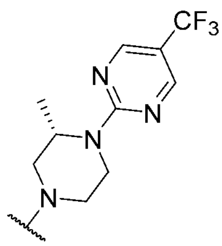 ,

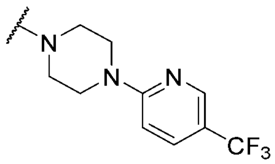 , 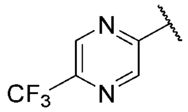 , 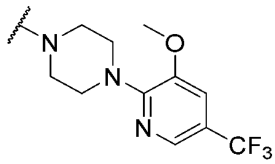 , 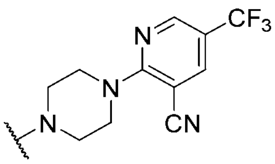 ,

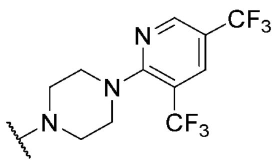 , 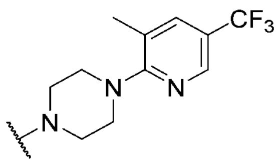 , 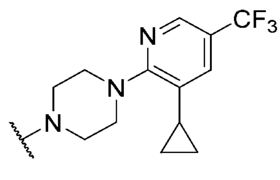 , 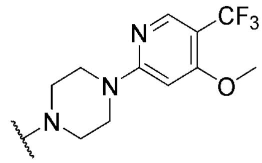 ,

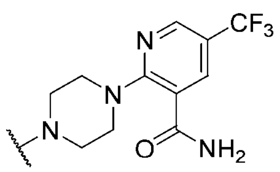 , 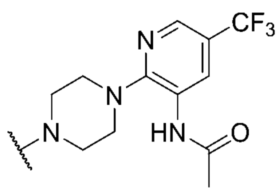 , 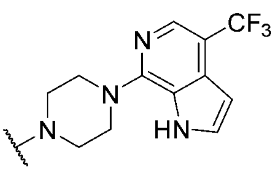 , 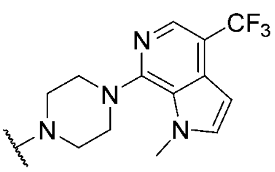 ,

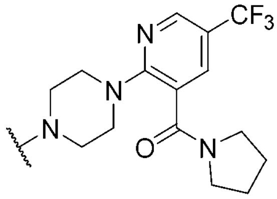 , 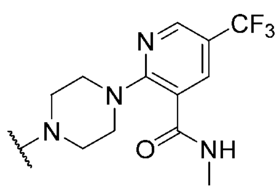 , 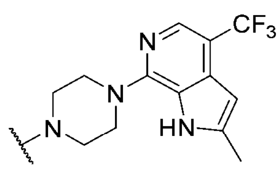 , 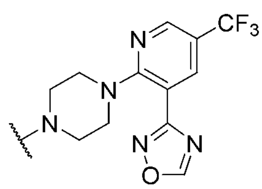 ,

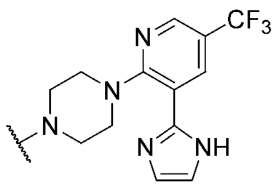, 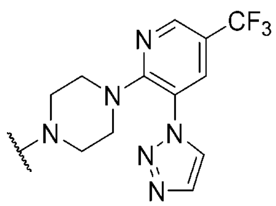, 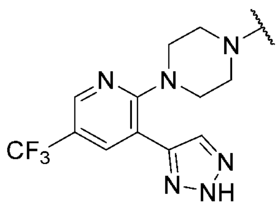, 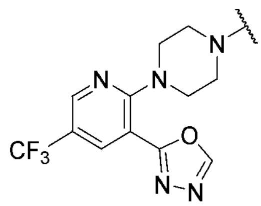,

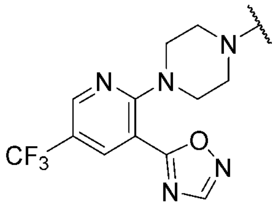, 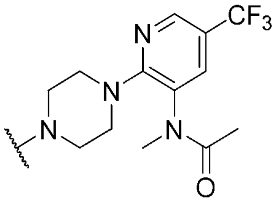, 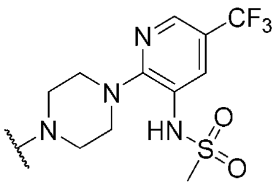, 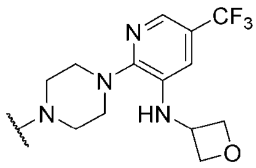, or

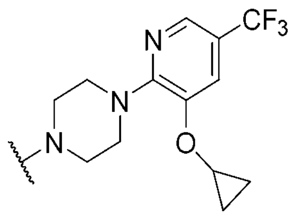;

or Ring B is a 5-8 membered saturated or unsaturated heterocycle, and the group

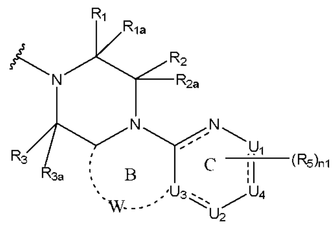

is selected from

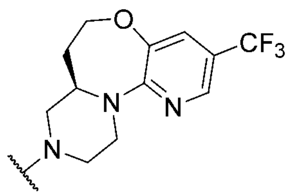, 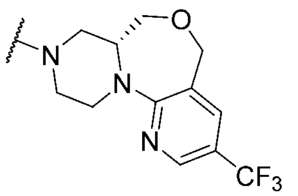, 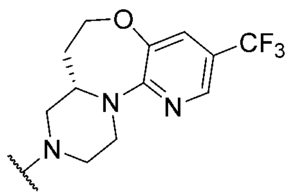,

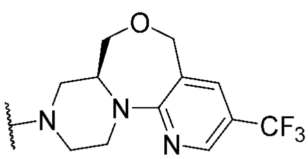, or 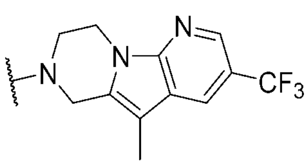.

In some embodiments, the compound, pharmaceutically acceptable salt, stereoisomer, solvate, or prodrug thereof is a compound represented by formula (II), formula (III), formula (IV), or formula (V):

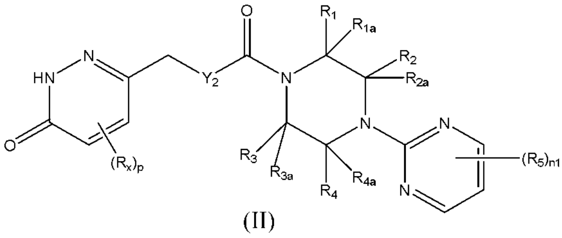

(II)

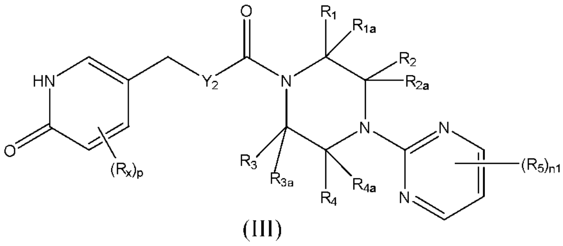

(III)

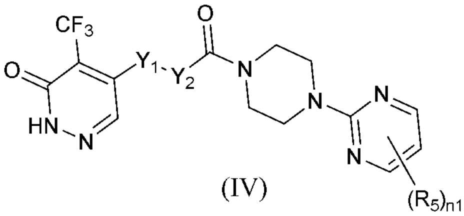

(IV)

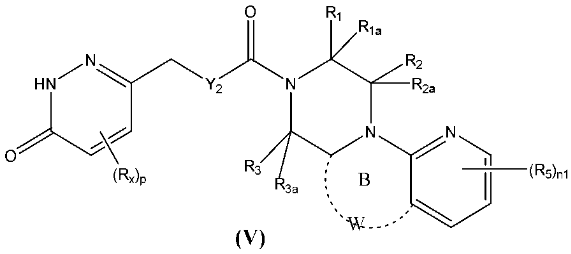

(V)

wherein $R_x$, p, $Y_1$, $Y_2$, $n_1$, $R_1$, $R_{1a}$, $R_2$, $R_{2a}$, $R_3$, $R_{3a}$, $R_4$, $R_{4a}$, $R_5$, W, and Ring B are as defined previously.

In a preferred embodiment, the compound of formula (I), a pharmaceutically acceptable salt, stereoisomer, solvate, or prodrug thereof, wherein the compound is selected from the group consisting of:

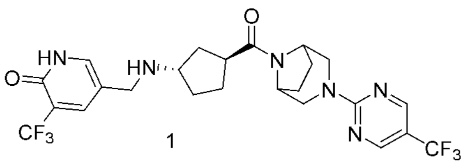

1

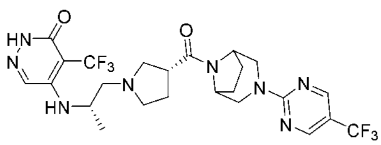

2

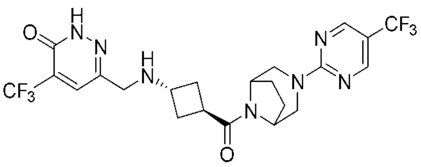

3

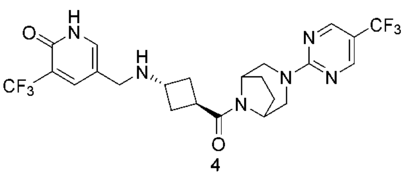

4

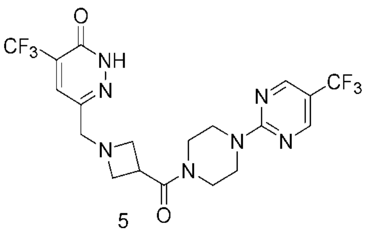

5

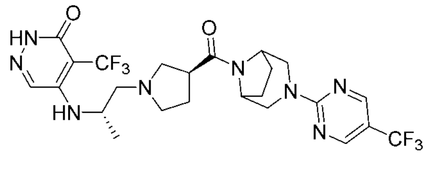

6

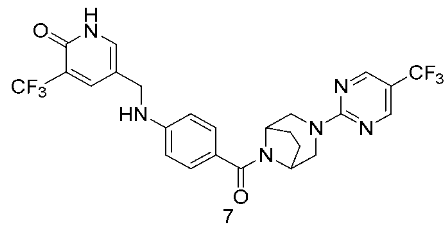

7

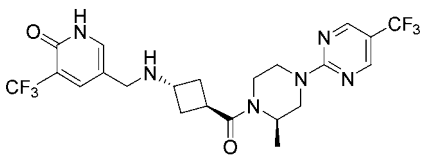

8

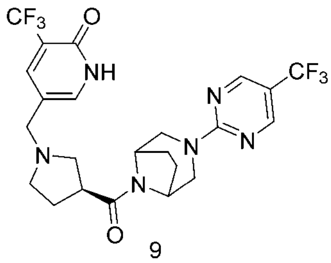

9

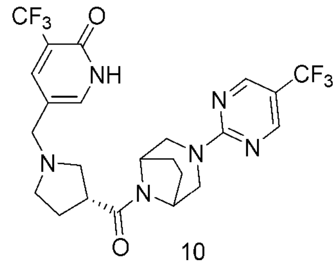

10

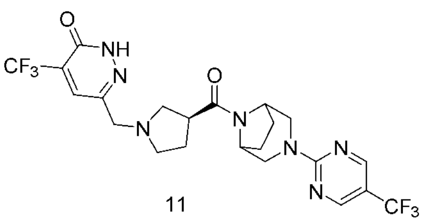

11

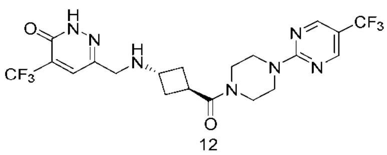

12

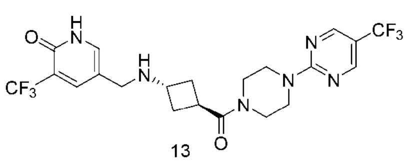

13

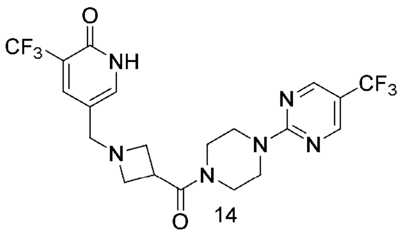

14

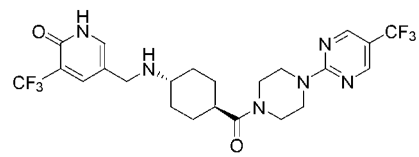

15

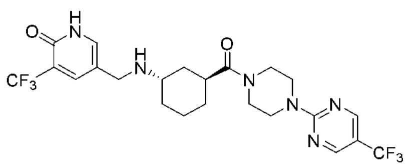

16

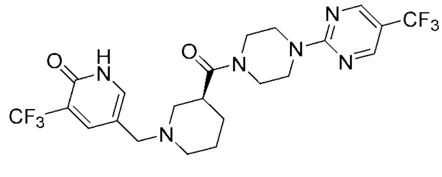

17

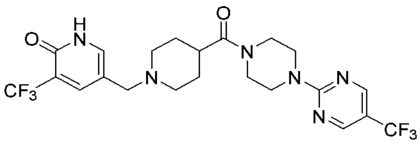

18

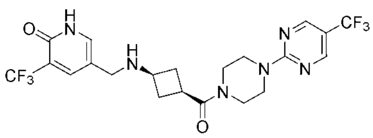
19

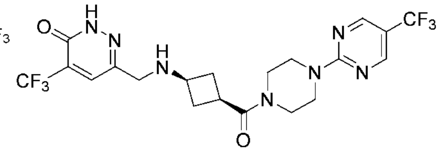
20

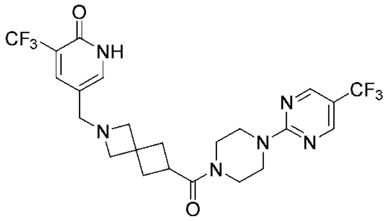
21

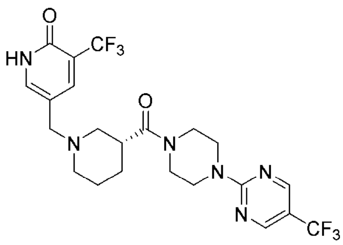
22

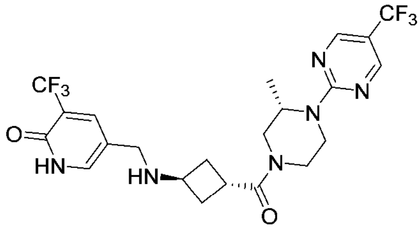
23

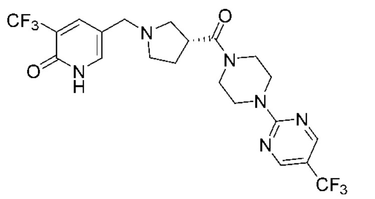
24

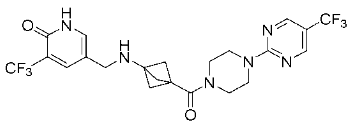
25

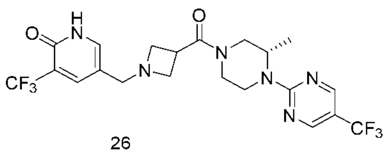
26

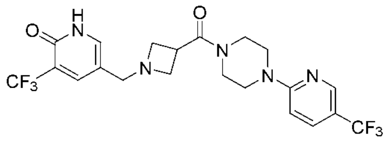
27

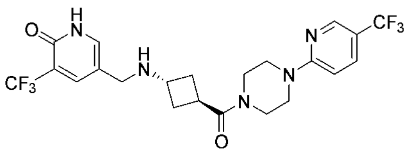
28

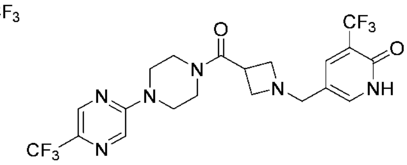
29

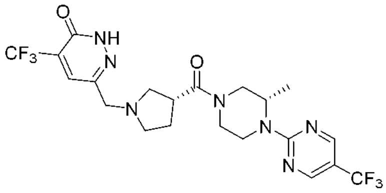
30

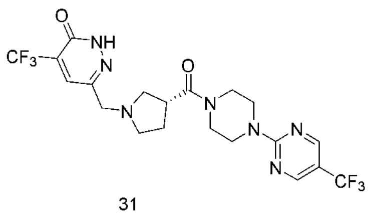
31

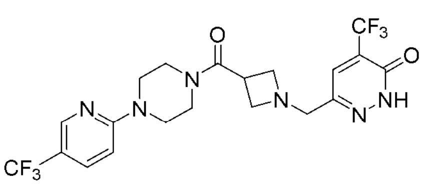
32

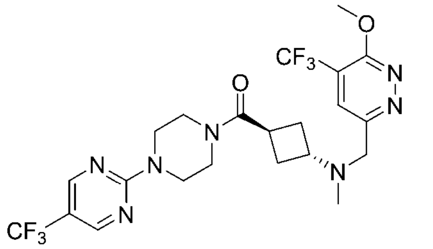
33

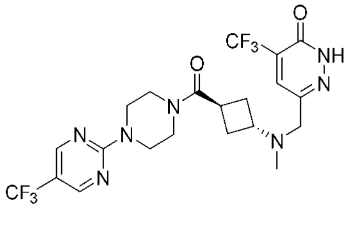
34

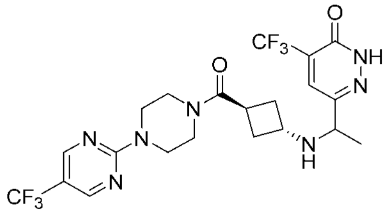
35-P1

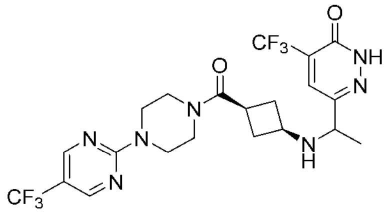
35-P2

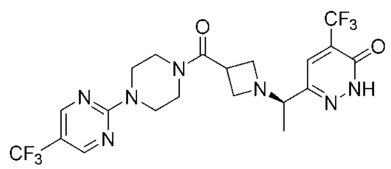
36-P1

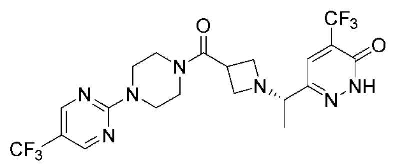
36-P2

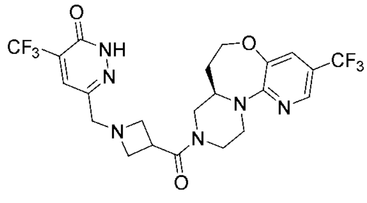
37

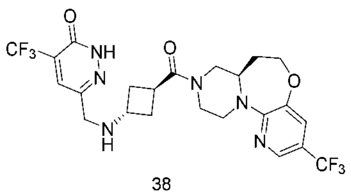
38

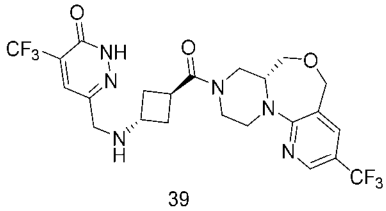
39

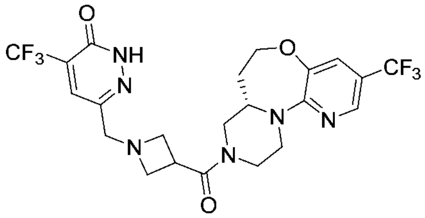
40

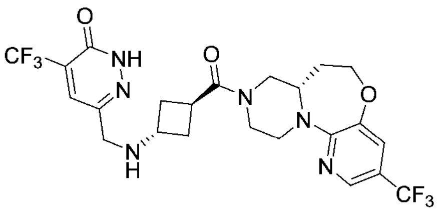
41

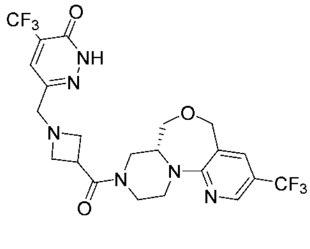
42

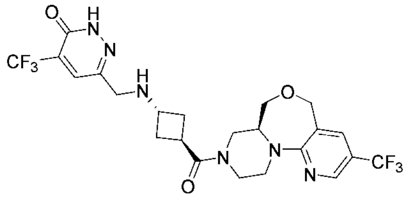
43

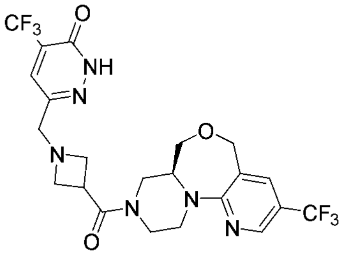
44

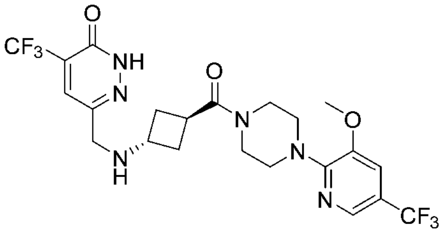
45

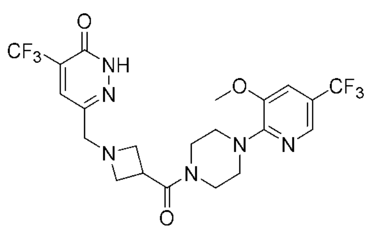
46

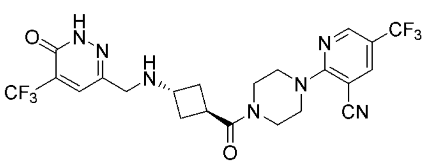
47

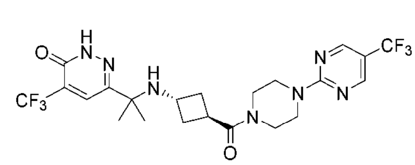
48

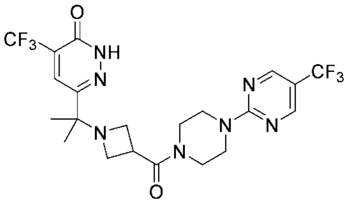
49

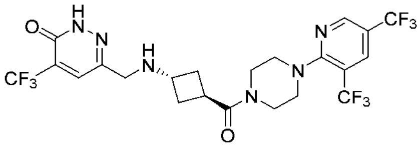
50

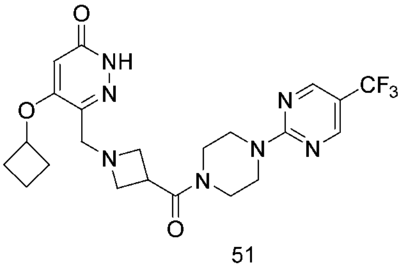
51

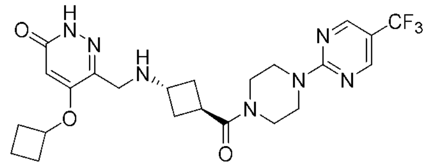

52

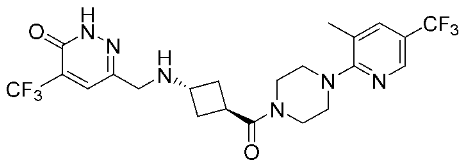

53

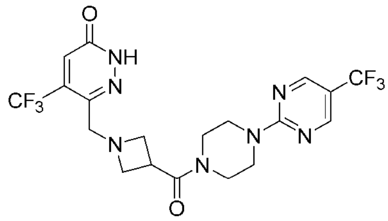

54

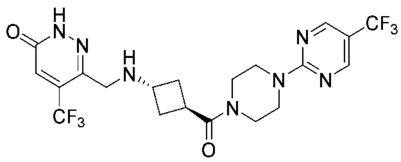

55

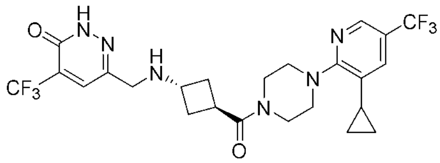

56

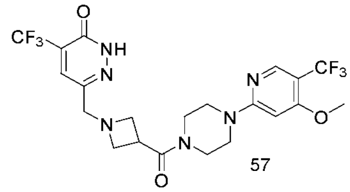

57

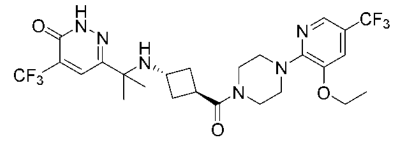

58

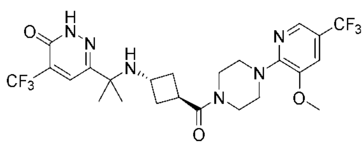

59

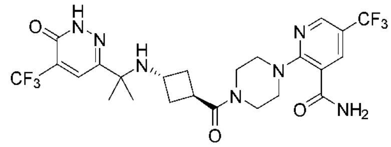

60

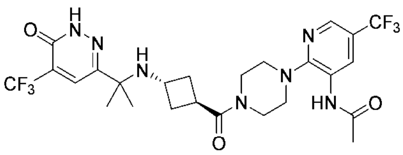

61

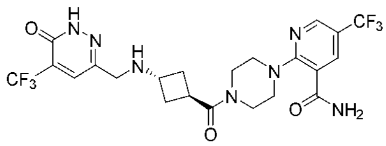

62

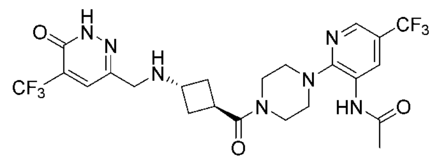

63

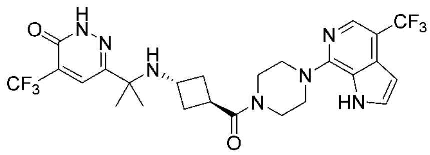

64

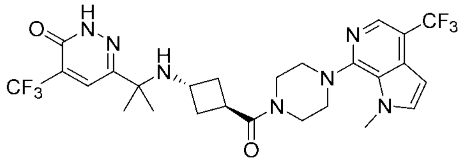

65

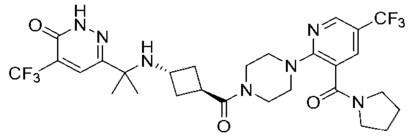

66

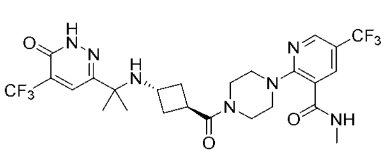
67

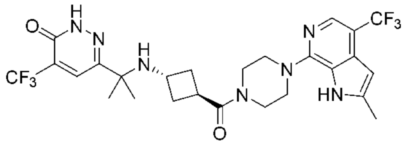
68

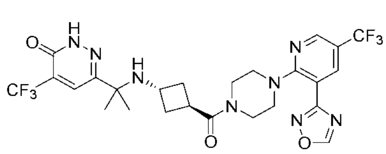
69

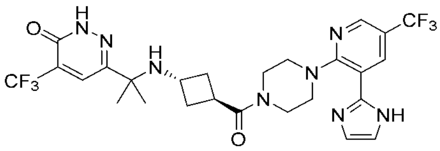
70

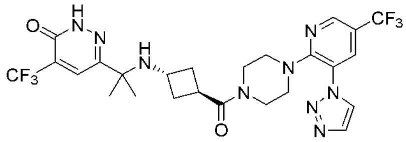
71

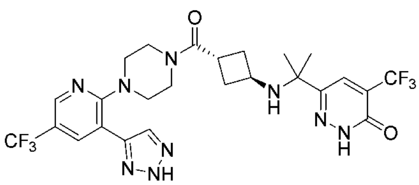
72

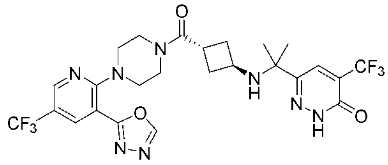
73

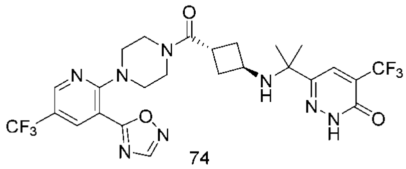
74

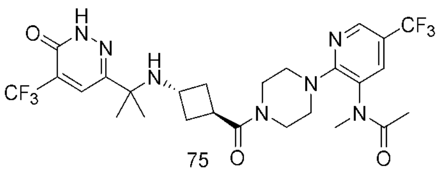
75

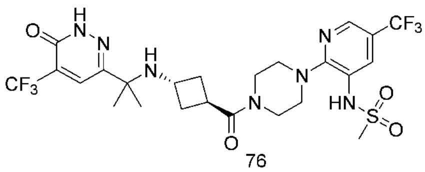
76

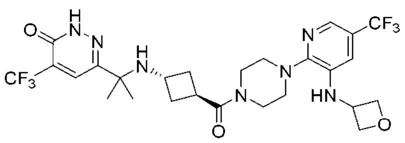
77

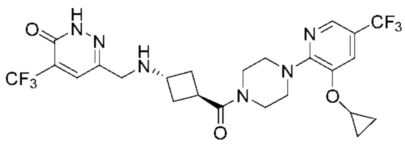
78

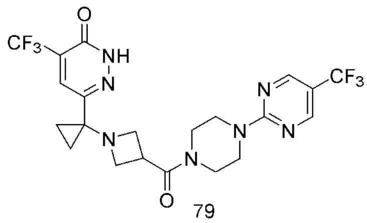
79

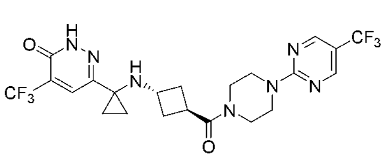
80

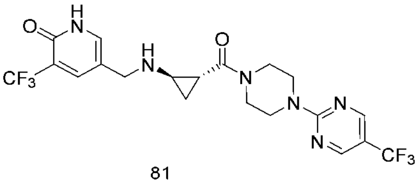
81

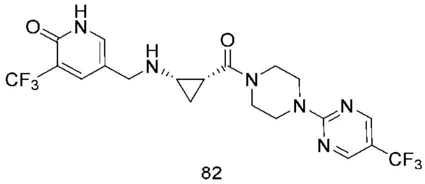
82

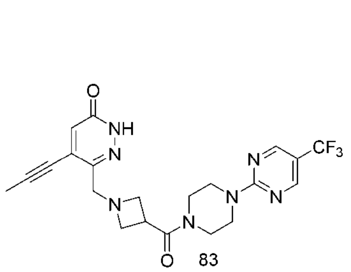
83

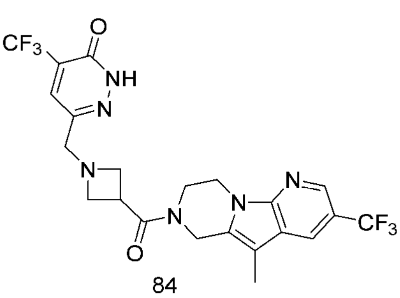
84

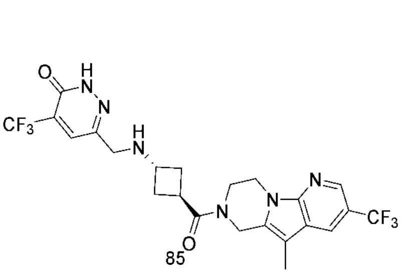
85

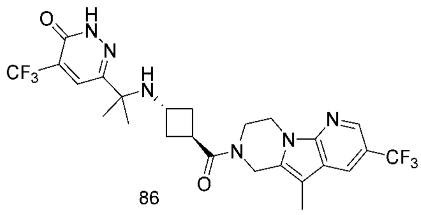

86 .

Another aspect of the present invention provides a pharmaceutical composition, characterized in that the pharmaceutical composition comprises the above-mentioned compound, a pharmaceutically acceptable salt, stereoisomer, solvate, or prodrug thereof, and at least one pharmaceutically acceptable carrier.

Another aspect of the present invention provides a use of the above-mentioned compound or a pharmaceutically acceptable salt, stereoisomer, solvate, prodrug thereof, or the pharmaceutical composition as described above, in the manufacture of a PARP7 inhibitor or for use in treating a PARP7-mediated disease;

preferably, the PARP7-mediated disease includes cancer, immune diseases, inflammation, and viral infection;
preferably, the cancer includes PARP7-amplified solid tumors, small cell lung cancer, squamous cell lung cancer, or advanced non-small cell lung squamous carcinoma;
preferably, the PARP7-amplified solid tumors include esophageal cancer and head and neck cancer.

Another aspect of the present invention provides a method of inhibiting PARP7 in a patient in need thereof, comprising administering to the patient the above-mentioned compound, a pharmaceutically acceptable salt, stereoisomer, solvate, or prodrug thereof, or the pharmaceutical composition as described above.

Another aspect of the present invention provides a method of inhibiting PARP7 in a biological sample, comprising contacting the biological sample with the above-mentioned compound, a pharmaceutically acceptable salt, stereoisomer, solvate, or prodrug thereof, or the pharmaceutical composition as described above. Another aspect of the present invention provides a method for treating a PARP7-mediated condition in a patient in need thereof, comprising administering to the patient the above-mentioned compound, a pharmaceutically acceptable salt, stereoisomer, solvate, or prodrug thereof, or the pharmaceutical composition as described above; preferably, the PARP7-mediated disease includes cancer, immune diseases, inflammation, and viral infection; preferably, the cancer includes PARP7-amplified solid tumors, small cell lung cancer, squamous cell lung cancer, or advanced non-small cell lung squamous carcinoma;

Preferably, the PARP7-amplified solid tumors include esophageal cancer and head and neck cancer;
Preferably, the compound, its pharmaceutically acceptable salt, stereoisomer, solvate, prodrug, or the pharmaceutical composition is administered in an effective amount.

**Detailed Description of Embodiments**

Based on the content of the present disclosure, various other modifications, substitutions, or changes can be made in accordance with common general knowledge and conventional means in the art without departing from the basic technical ideas of the present disclosure.

**I. Definitions**

The compounds of the present disclosure may be asymmetric, for example, having one or more stereoisomers. Unless otherwise indicated, all stereoisomers are included, such as enantiomers and diastereomers. Compounds of the present disclosure containing asymmetrically substituted carbon atoms can be isolated in optically active pure forms or racemic forms. Optically active pure forms can be resolved from racemic mixtures or synthesized by using chiral starting materials or chiral reagents. Racemates, diastereomers, and enantiomers are all included within the scope of the present disclosure.

The compounds of the present disclosure also include tautomeric forms. Tautomeric forms arise from the exchange of a single bond with an adjacent double bond, accompanied by the migration of a proton.

The term "optional" or "optionally" means that the subsequently described event or circumstance may or may not occur, and that the description includes instances where the event or circumstance occurs and instances where it does not. A numerical ranges herein refers to each integer within the given range. For example, "$C_{1-6}$" means that the group can have 1 carbon atom, 2 carbon atoms, 3 carbon atoms, 4 carbon atoms, 5 carbon atoms, or 6 carbon atoms; "$C_{2-6}$" means that the group can have 2 carbon atoms, 3 carbon atoms, 4 carbon atoms, 5 carbon atoms, or 6 carbon atoms.

The term "substituted" means that any one or more hydrogen atoms on a specific atom or group are replaced by a

substituent, provided that the valence of the specific atom or group is normal and the substituted compound is stable. When the substituent is a keto group (i.e., =O), it means that two hydrogen atoms are substituted. Unless otherwise specified, the type and number of substituents can be arbitrary on a chemically achievable basis.

In the present disclosure, when any variable (e.g., $R_n$) occurs more than once in the composition or structure of a compound, its definition in each case is independent. Thus, for example, if a group is substituted by 1-5 R groups, then said group can optionally be substituted by up to 5 R groups, and R in each case has an independent option. Furthermore, combinations of substituents and/or variants thereof are only permissible if such combinations result in stable compounds.

The term "alkyl" refers to a saturated aliphatic hydrocarbon group, which is a straight-chain or branched-chain group containing 1 to 20 carbon atoms, preferably an alkyl group containing 1 to 8 carbon atoms, more preferably an alkyl group containing 1 to 6 carbon atoms, and most preferably an alkyl group containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 2,2-diethylhexyl, 2,2-diethylhexyl, and their various branched isomers, etc. More preferred are lower alkyl groups containing 1 to 6 carbon atoms, and non-limiting embodiments include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. The alkyl group can be substituted or unsubstituted. When substituted, the substituent can be substituted at any available point of attachment. The substituent is preferably one or more of the following groups, independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate group. The present disclosure preferably includes methyl, ethyl, isopropyl, tert-butyl, haloalkyl, deuteroalkyl, alkoxy-substituted alkyl, and hydroxy-substituted alkyl.

The term "alkenyl" refers to an alkyl group as defined above, consisting of at least two carbon atoms and at least one carbon-carbon double bond, for example, vinyl, 1-propenyl, 2-propenyl, 1-, 2-, or 3-butenyl, etc. The alkenyl group can be substituted or unsubstituted. When substituted, the substituent is preferably one or more of the following groups, independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, or heterocycloalkylthio.

The term "alkynyl" refers to (CH≡C-), wherein the alkynyl group can be further substituted with other related groups, for example: alkyl, alkenyl, alkynyl, alkoxy, alkylthio, alkylamino, halogen, mercapto, cyano, nitro, phenyl, cycloalkyl, heterocycloalkyl, aryl, heteroaryl, cycloalkoxy, heterocycloalkoxy, cycloalkylthio, heterocycloalkylthio, carboxyl, or carboxylate group.

The term "cycloalkyl" refers to a saturated monocyclic alkane substituent, wherein the cycloalkyl ring contains at least 3 carbon atoms, preferably 3 to 12 carbon atoms, and more preferably 3 to 6 carbon atoms. Non-limiting examples of cycloalkyl include cyclopropyl, cyclobutyl, cyclopentyl, cyclohexyl, etc.

The term "heterocycloalkyl" refers to a saturated monocyclic hydrocarbon substituent in which one or more ring atoms are heteroatoms selected from nitrogen, oxygen, or $S(O)_m$ (where m is an integer from 0 to 2), but does not include ring moieties of -O-O-, -O-S-, or -S-S-, and the remaining ring atoms are carbon. Non-limiting examples of heterocycloalkyl include pyrrolyl, imidazolyl, tetrahydrofuranyl, tetrahydrothiophenyl, dihydroimidazolyl, dihydrofuranyl, dihydropyrazolyl, dihydropyrrolyl, piperidinyl, piperazinyl, morpholinyl, thiomorpholinyl, etc., preferably pyrrolidinyl, morpholinyl, piperidinyl, cycloheptyl, 1,4-diazepanyl, and piperazinyl.

The heterocyclyl group can be optionally substituted or unsubstituted. When substituted, the substituent is preferably one or more of the following groups, independently selected from alkyl, alkenyl, alkynyl, alkoxy, alkylthio, alkylamino, halogen, mercapto, cyano, nitro, chloro group, cycloalkyl, heterocycloalkyl, aryl, heteroaryl, cycloalkoxy, heterocycloalkoxy, cycloalkylthio, heterocycloalkylthio, oxo, carboxyl, or carboxylate group.

The term "heterocycle" or "heterocyclyl" refers to a saturated or unsaturated heterocyclic group in which one or more ring atoms are heteroatoms selected from nitrogen, oxygen, or $S(O)_m$ (where m is an integer from 0 to 2), but does not include ring moieties of -O-O-, -O-S-, or -S-S-, and the remaining ring atoms are carbon.

The term "aryl" refers to a 6- to 14-membered all-carbon monocyclic or fused polycyclic (i.e., rings that share adjacent pairs of carbon atoms) group with a conjugated π-electron system, preferably 6- to 12-membered, for example, phenyl and naphthyl. More preferably phenyl. The aryl ring can be fused to a heteroaryl, heterocyclyl, or cycloalkyl ring, including benzo 5-10 membered heteroaryl, benzo 3-8 membered cycloalkyl, and benzo 3-8 membered heteroalkyl,

preferably benzo 5-6 membered heteroaryl, benzo 3-6 membered cycloalkyl, and benzo 3-6 membered heteroalkyl, wherein the heterocyclyl is a heterocyclyl group containing 1-3 nitrogen, oxygen, or sulfur atoms; or it may also include a three-membered nitrogen-containing fused ring containing a benzene ring. The aryl group can be substituted or unsubstituted. When substituted, the substituent is preferably one or more of the following groups, independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or carboxylate group.

The term "alkoxy" refers to -O-(alkyl) and -O-(unsubstituted cycloalkyl), wherein alkyl is as defined above. Non-limiting examples of alkoxy include: methoxy, ethoxy, propoxy, butoxy, cyclopropoxy, cyclobutoxy, cyclopentyloxy, cyclohexyloxy. The alkoxy group can be optionally substituted or unsubstituted. When substituted, the substituent is preferably one or more of the following groups, independently selected from alkyl, alkenyl, alkynyl, alkoxy, alkylthio, alkylamino, halogen, mercapto, hydroxyl, nitro, chloro group, cycloalkyl, heterocycloalkyl, aryl, heteroaryl, cycloalkoxy, heterocycloalkoxy, cycloalkylthio, heterocycloalkylthio, carboxyl, or carboxylate group.

The term "spirocycloalkyl" refers to an alicyclic hydrocarbon in which two carbon rings share one carbon atom in the molecule. The shared carbon atom is called a "spiro atom".

The term "amido group" refers to a group formed by replacing the hydroxyl group of the carboxyl group in a carboxylic acid molecule with an amino group or a hydrocarbylamino group [-NHR or -N(R)R].

The term "aminoacyl group" refers to the remaining group after removing the hydroxyl group from an amino acid molecule, which are collectively called aminoacyl groups.

The term "cycloaminoacyl group" refers to the remaining group after removing the hydroxyl group from a cycloamino acid molecule, which are collectively called cycloaminoacyl groups.

The term "sulfonamido group" refers to the remaining group after a hydrogen atom of a sulfonamide is substituted, which are collectively called sulfonamido groups.

The term "heterocycloamino group" refers to an amino group substituted by a saturated or unsaturated heterocyclic group, and the remaining groups are collectively called heterocycloamino groups.

In the present disclosure, "hydroxyl" refers to an -OH group; "halogen" refers to fluorine, chlorine, bromine, or iodine; "amino" refers to $-NH_2$; "cyano" refers to -CN; "nitro" refers to $-NO_2$; "carbonyl" refers to -C(O)-; "carboxyl" refers to -C(O)OH.

All hydrogen atoms described in the present disclosure can be replaced by their isotope deuterium. In the present disclosure, "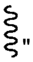" refers to the point of attachment of a chemical bond. In the present disclosure, "----" refers to a bond or group that may or may not be present.

NMP: N-methylpyrrolidone;
EA: ethyl acetate;
Dioxane: dioxane;
SEMCl: 2-(trimethylsilyl)ethoxymethyl chloride;
DMF: N,N-dimethylformamide;
$NH_2NH_2.H_2O$: hydrazine hydrate;
NBS: N-bromosuccinimide;
BPO: dibenzoyl peroxide;
ACN: acetonitrile;
n-BuLi: n-butyllithium;
THF: tetrahydrofuran;
TEA: triethylamine;
t-BuOK: potassium tert-butoxide;
H-Pro-OH: L-proline;
AIBN: azobisisobutyronitrile;
AcOH: acetic acid;
EtOH: ethanol;
Bn: benzyl;
$Pd(dppf)Cl_2$: [1,1'-bis(diphenylphosphino)ferrocene]palladium(II) dichloride;
MeOH: methanol;
Boc: tert-butoxycarbonyl;

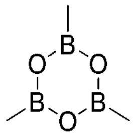:

2,4,6-trimethyl-1,3,5,2,4,6-trimethoxytriborane;

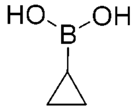

: cyclopropylboronic acid;
DIPEA or DIEA: N,N-diisopropylethylamine;
HATU: *O*-(7-azabenzotriazol-1-yl)-*N,N,N',N'*-tetramethyluronium hexafluorophosphate;
DCM: dichloromethane;
TFA: trifluoroacetic acid;

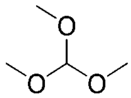

: trimethyl orthoformate;
$NaBH_3CN$: sodium cyanoborohydride;
DETA: diethylenetriamine;
$BF_3Et_2O$: boron trifluoride etherate;
$Cs_2CO_3$: cesium carbonate;
NaOAc: sodium acetate;
MeOH: methanol;
PyBOP: (benzotriazol-1-yloxy)tripyrrolidinophosphonium hexafluorophosphate.

Drug or Pharmaceutical Composition

The term "pharmaceutically acceptable" refers to those compounds, materials, compositions, and/or dosage forms which are, within the scope of sound medical judgment, suitable for use in contact with the tissues of humans and animals without excessive toxicity, irritation, allergic response, or other problems or complications, commensurate with a reasonable benefit/risk ratio.

The term "pharmaceutically acceptable salt" refers to salts that retain the biological effectiveness of the free acids and bases of the specific compound and that do not have biologically undesirable effects. For example, acid (including organic and inorganic acids) addition salts or base (including organic and inorganic bases) addition salts.

The pharmaceutically acceptable salts of the present disclosure can be synthesized from the parent compound containing an acidic or basic moiety by conventional chemical methods. Generally, such salts are prepared by reacting the free acid or base forms of these compounds with a stoichiometric amount of the appropriate base or acid in water or an organic solvent, or a mixture of the two.

The drug or pharmaceutical composition of the present disclosure may be administered orally, topically, parenterally, or mucosally (e.g., buccally, by inhalation, or rectally) in dosage unit formulations containing conventional non-toxic pharmaceutically acceptable carriers. The oral route is generally preferred. The active agent can be administered orally in the form of capsules, tablets, etc. (see Remington: The Science and Practice of Pharmacy, 20th Edition).

For oral administration in the form of tablets or capsules, the active drug component can be combined with non-toxic, pharmaceutically acceptable excipients such as binders (e.g., pregelatinized corn starch, polyvinylpyrrolidone or hydroxypropyl methylcellulose); fillers (e.g., lactose, sucrose, glucose, mannitol, sorbitol and other reducing and non-reducing sugars, microcrystalline cellulose, calcium sulfate or dicalcium phosphate); lubricants (e.g., magnesium stearate, talc or silica, stearic acid, sodium stearyl fumarate, glyceryl behenate, calcium stearate, etc.); disintegrants (e.g., potato starch or sodium starch glycolate); or wetting agents (e.g., sodium lauryl sulfate), colorants and flavoring agents, gelatin, sweeteners, natural and synthetic gums (such as acacia, tragacanth or alginates), buffer salts, carboxymethylcellulose, polyethylene glycols, waxes, etc. For oral administration in liquid form, the drug component can be combined with pharmaceutically acceptable inert carriers (e.g., ethanol, glycerol, water), suspending agents (e.g., sorbitol syrup, cellulose derivatives or hydrogenated edible fats), emulsifying agents (e.g., lecithin or acacia), non-aqueous

carriers (e.g., almond oil, oily esters, ethyl alcohol or fractionated vegetable oils), preservatives (e.g., methyl p-hydroxybenzoate or propyl p-hydroxybenzoate or sorbic acid), etc. Stabilizers such as antioxidants (BHA, BHT, propyl gallate, sodium ascorbate, citric acid) may also be added to stabilize the dosage form.

Tablets containing the active compound can be coated by methods well known in the art. The compositions of the present disclosure comprising a compound of Formula I as an active compound can also be incorporated into beads, microspheres, or microcapsules, for example, constructed from poly(lactic-co-glycolic acid) (PGLA). Liquid drug products for oral administration may take the form of, for example, solutions, syrups, emulsions or suspensions, or they may be presented as a dry product for reconstitution with water or other suitable vehicle before use. Drug products for oral administration may be suitably formulated to give controlled or delayed release of the active compound.

The drug or pharmaceutical composition of the present disclosure can be delivered parenterally, i.e., by intravenous (i.v.),intracerebroventricular (i.c.v.), subcutaneous (s.c.), intraperitoneal (i.p.), intramuscular (i.m.), subdermal (s.d.), or intradermal (i.d.) administration, by direct injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for reconstitution with a suitable vehicle, e.g., sterile pyrogen-free water, before use.

The drug or pharmaceutical composition of the present disclosure may also be formulated for rectal administration, e.g., as suppositories or retention enemas (e.g., containing conventional suppository bases such as cocoa butter or other glycerides).

The term "treatment" includes inhibiting, alleviating, preventing or eliminating one or more symptoms or side effects associated with the disease, condition or disorder being treated.

The use of the terms "reduce", "inhibit", "alleviate", or "decrease" is relative to a control. A person skilled in the art will readily determine the appropriate control for each experiment. For example, a reduced response in a subject or cell treated with a compound is compared to the response in a subject or cell not treated with the compound.

As used herein, the term "effective amount" or "therapeutically effective amount" refers to a dose sufficient to treat, inhibit or alleviate one or more symptoms of the disease state being treated or otherwise provide the desired pharmacological and/or physiological effect. The precise dosage will vary according to a variety of factors, such as subject-dependent variables (e.g., age, immune system health, etc.), the disease or condition, and the treatment being administered. The effect of an effective amount can be compared to a control. These controls are known in the art and discussed herein, and can be, for example, the condition of the subject prior to or in the absence of administration of the drug or drug combination, or in the case of a drug combination, the effect of the combination can be compared to the effect of administering only one drug.

The term "excipient" is used herein to include any other compound that may be included in or on the microparticles that is not a therapeutic or biologically active compound. Thus, an excipient should be pharmaceutically or biologically acceptable or relevant, for example, an excipient is generally non-toxic to the subject. "Excipient" includes a single such compound and is also intended to include multiple compounds.

The term "pharmaceutical composition" means a composition comprising a compound of the present disclosure or a pharmaceutically acceptable salt thereof, and at least one pharmaceutically acceptable ingredient selected from the following, depending on the mode of administration and the nature of the dosage form, including but not limited to: carriers, diluents, adjuvants, excipients, preservatives, fillers, disintegrants, wetting agents, emulsifiers, suspending agents, sweeteners, flavoring agents, fragrance agents, antibacterial agents, antifungal agents, lubricants, dispersing agents, temperature-sensitive materials, temperature-regulating agents, adhesives, stabilizers, suspending aids, etc.

## Uses and Methods of Treatment

The terms "patient", "subject", "individual", and the like are used interchangeably herein and refer to any animal or its cells, whether *in vitro* or *in situ,* that is subject to the methods described herein. In some non-limiting embodiments, the patient, subject, or individual is a human.

According to the methods of the present disclosure, the compound or composition can be administered in any amount and by any route of administration effective for treating or alleviating the severity of a disease associated with PARP.

The present disclosure relates to a method of inhibiting PARP in a biological sample, comprising the step of contacting said biological sample with a compound of the present disclosure or a composition comprising said compound.

The term "biological sample" includes (but is not limited to) cell cultures or extracts thereof; biopsy material obtained from a mammal or extracts thereof; and blood, saliva, urine, feces, semen, tears, or other body fluids or extracts thereof. Inhibition of an enzyme in a biological sample can be used to achieve a variety of purposes known to those skilled in the art. Examples of such purposes include (but are not limited to) bioanalysis, gene expression studies, and biological

target identification.

A method of the present disclosure for inhibiting PARP in a patient, comprising the step of administering to said patient a compound of the present disclosure or a composition comprising said compound.

The provided compounds are PARP inhibitors and thus can be used to treat one or more conditions associated with PARP activity. Thus, in certain embodiments, the present disclosure provides a method for treating a PARP-mediated condition, comprising the step of administering to a patient in need thereof a compound of the present disclosure or a pharmaceutically acceptable composition thereof.

As used herein, the term "PARP-mediated" condition, disease, and/or disorder, as used herein, means any disease or other deleterious condition in which PARP or a mutant thereof is known to play a role. Accordingly, another embodiment of the present disclosure relates to treating or lessening the severity of one or more diseases in which PARP or a mutant thereof is known to play a role.

The present disclosure provides a method for treating one or more conditions, diseases, and/or disorders, wherein the condition, disease, or disorder is a proliferative disease, such as cancer, an inflammatory condition, or a viral infection.

In certain embodiments, the present disclosure provides a method of treating cancer or another proliferative condition, which comprises administering a compound or composition of the present disclosure to a patient with cancer or another proliferative condition. In certain embodiments, the method of treating cancer or another proliferative condition comprises administering a compound and composition of the present disclosure to a mammal. In certain embodiments, the mammal is a human.

As used herein, the terms "inhibiting cancer" and "inhibiting cancer cell proliferation" refer to inhibiting the growth, dissociation, maturation, or survival of cancer cells, and/or causing cancer cell death through cytotoxicity, nutrient depletion, or induction of apoptosis, individually or collectively with other cancer cells. Examples of tissues containing cancer cells whose proliferation is inhibited by the compounds and compositions described herein and to which the methods described herein are applicable include (but are not limited to) breast, prostate, brain, blood, bone marrow, liver, pancreas, epidermis, kidney, colon, ovary, lung, testis, penis, thyroid, parathyroid, pituitary, thymus, retina, uvea, conjunctiva, spleen, head, neck, trachea, gallbladder, rectum, salivary gland, adrenal gland, throat, esophagus, lymph node, sweat gland, sebaceous gland, muscle, heart, and stomach.

Cancers treated by the compounds or compositions of the present disclosure are melanoma, liposarcoma, lung cancer, breast cancer, prostate cancer, leukaemia, renal cancer, oesophageal carcinoma, brain cancer, lymphoma, or colon cancer. In certain embodiments, the cancer is primary effusion lymphoma (PEL).

The compounds of the present disclosure can be used to treat a proliferative disease selected from: benign or malignant tumors or carcinomas of the brain, kidney, liver, adrenal gland, bladder, breast, stomach, gastric neoplasm, ovary, colon, rectum, prostate, pancreas, lung, vagina, cervix, testis, genitourinary tract, esophagus, larynx, skin, bone, or thyroid; sarcoma, glioblastoma, neuroblastoma, multiple myeloma, or gastrointestinal carcinoma (especially colon cancer or colorectal adenoma) or tumors of the neck and head, epidermal hyperproliferation, psoriasis, prostatic hyperplasia, neoplasia, neoplasia of epithelial character, adenoma, adenocarcinoma, keratoacanthoma, epidermoid carcinoma, large cell carcinoma, non-small cell lung cancer, Hodgkin's and non-Hodgkin's lymphoma, breast cancer, follicular carcinoma, undifferentiated carcinoma, papillary carcinoma, seminoma, melanoma, MYD88-driven disorders, DLBCL, ABC DLBCL, IL-1 driven disorders, and smoldering or indolent multiple myeloma or leukaemia.

Cancers described in the present disclosure include (but are not limited to) leukaemia (e.g., acute leukaemia, acute lymphocytic leukaemia, acute myelocytic leukaemia, acute myeloblastic leukaemia, acute promyelocytic leukaemia, acute myelomonocytic leukaemia, acute monocytic leukaemia, acute erythroid leukaemia, chronic leukaemia, chronic myelocytic leukaemia, chronic lymphocytic leukaemia), polycythemia vera, lymphoma (e.g., Hodgkin's disease or non-Hodgkin's disease), Waldenstrom's macroglobulinemia, multiple myeloma, heavy chain disease, and solid tumors, such as sarcomas and carcinomas (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, pancreatic carcinoma, breast cancer, ovarian cancer, prostate cancer, squamous cell carcinoma, basal cell carcinoma, adenocarcinoma, sweat gland carcinoma, sebaceous carcinoma, papillary carcinoma, papillary adenocarcinoma, cystadenocarcinoma, medullary carcinoma, bronchial carcinoma, renal cell carcinoma, hepatoma, bile duct cancer, choriocarcinoma, seminoma, embryonal carcinoma, Wilms' tumor, cervix carcinoma, uterine cancer, testis cancer, lung cancer, small cell lung cancer, bladder cancer, epithelial carcinoma, glioma, astrocytoma, glioblastoma multiforme (GBM, also known as glioblastoma), medulloblastoma, craniopharyngioma, ependymoma, pinealoma, haemangioblastoma, acoustic neuroma, oligodendroglioma, schwannoma, neurofibrosarcoma, meningioma, melanoma, neuroblastoma, and retinoblastoma.

In some specific embodiments, the cancer is glioma, astrocytoma, glioblastoma multiforme (GBM, also known as glioblastoma), medulloblastoma, craniopharyngioma, ependymoma, pinealoma, haemangioblastoma, acoustic neuroma, oligodendroglioma, schwannoma, neurofibrosarcoma, meningioma, melanoma, neuroblastoma, or retinoblastoma.

In some specific embodiments, the cancer is acoustic neuroma, astrocytoma (e.g., Grade I - pilocytic astrocytoma, Grade II - low-grade astrocytoma, Grade III - anaplastic astrocytoma, or Grade IV - glioblastoma (GBM)), chordoma, CNS lymphoma, craniopharyngioma, brain stem glioma, ependymoma, mixed glioma, optic nerve glioma, subependymoma, medulloblastoma, meningioma, metastatic brain tumors, oligodendroglioma, pituitary tumour, primitive neuroectodermal (PNET) tumor, or schwannoma. In some embodiments, the cancer is of a type more common in children than in adults, such as brain stem glioma, craniopharyngioma, ependymoma, juvenile pilocytic astrocytoma (JPA), medulloblastoma, optic nerve glioma, pineal neoplasm, primitive neuroectodermal neoplasm (PNET), or rhabdoid tumour. In some embodiments, the patient is an adult patient. In some embodiments, the patient is a child or pediatric patient.

In another specific embodiment, the cancer includes (but is not limited to): mesothelioma, hepatobiliary (liver and bile duct), bone cancer, pancreatic carcinoma, skin cancer, head or neck cancer, cutaneous or intraocular melanoma, ovarian cancer, colon cancer, rectal cancer, anal region cancer, gastric cancer, gastrointestinal (stomach, colorectal, and duodenal), uterine cancer, fallopian tube cancer, endometrial cancer, cervix carcinoma, vaginal cancer, vulval cancer, Hodgkin's disease, oesophageal carcinoma, small intestine carcinoma, endocrine system cancer, thyroid cancer, parathyroid cancer, adrenal gland cancer, soft tissue sarcoma, urethral cancer, penile cancer, prostate cancer, testis cancer, chronic or acute leukaemia, chronic myeloid leukaemia, lymphocytic lymphoma, bladder cancer, kidney or ureter cancer, renal cell carcinoma, renal pelvis carcinoma, non-Hodgkin's lymphoma, spinal axis tumors, brain stem glioma, pituitary adenoma, adrenocortical carcinoma, gallbladder cancer, multiple myeloma, bile duct cancer, fibrosarcoma, neuroblastoma, retinoblastoma, or a combination of one or more of said cancers.

In some specific embodiments, the cancer is selected from small cell lung cancer, squamous cell lung cancer, advanced non-small cell lung squamous carcinoma, hepatocellular carcinoma, ovarian cancer, ovarian epithelial cancer or fallopian tube cancer; papillary serous cystadenocarcinoma or uterine papillary serous carcinoma (UPSC); prostate cancer; testis cancer; gallbladder cancer; cholangiohepatoma; soft tissue and bone synovial sarcoma; rhabdomyosarcoma; bone sarcoma; chondrosarcoma; Ewing's sarcoma; anaplastic thyroid cancer; adrenocortical adenoma; pancreatic carcinoma; pancreatic ductal carcinoma or pancreatic carcinoma; gastrointestinal/gastric (GIST) cancer; lymphoma; squamous cell carcinoma of head and neck (SCCHN); salivary gland carcinoma; glioma or brain cancer; neurofibromatosis-1 associated malignant peripheral nerve sheath tumor (MPNST); Waldenstrom's macroglobulinemia; or medulloblastoma.

The term "primary tumor" is relative to secondary tumors. A primary tumor refers to a tumor that first appears in a certain site, such as the lung, liver, intestine, head, or skin, and can be called primary lung cancer, primary hepatic cancer, primary intestinal cancer, etc.

The term "inflammatory disease" includes said autoimmune, allergic disorders, and inflammatory disorders, such as those selected from arthritis, ankylosing spondylitis, inflammatory bowel disease, colitis ulcerative, gastritis, pancreatitis, Crohn's disease, coeliac disease, multiple sclerosis, systemic lupus erythematosus, rheumatoid arthritis, rheumatic fever, gout, organ or transplant rejection, acute or chronic graft versus host disease, chronic allograft rejection, Behcet's disease, uveitis, psoriasis, dermatitis, atopic dermatitis, dermatomyositis, myasthenia gravis, Graves' disease, Hashimoto's thyroiditis, Sjogren's syndrome, and blistering disorders (e.g., pemphigus vulgaris), antibody-mediated vasculitis syndromes, including ANCA-associated vasculitis, purpura, and immune complex vasculitis (primary or secondary to cancer or infection). The allergic disorders may be selected in particular from dermatitis contact, coeliac disease, asthma, hypersensitivity to house dust mites, pollen and related allergens, berylliosis. The respiratory disorders may be selected in particular from asthma, bronchitis, chronic obstructive pulmonary disease (COPD), cystic fibrosis, pulmonary oedema, pulmonary embolism, pneumonia, pulmonary sarcoidosis, silicosis, pulmonary fibrosis, respiratory failure, acute respiratory distress syndrome, primary pulmonary arterial hypertension, and emphysema, etc.

The term "viral infection" includes but is not limited to retroviral infection, hepatitis virus infections, COVID-19 coronavirus infection, zika virus infection, dengue virus infection, etc.

## Combination Therapy Methods

The present disclosure provides for combination therapy using a compound as described herein with other therapeutic agents. The term "combination therapy" as used in the present disclosure includes the administration of these agents in a sequential manner, wherein each therapeutic agent is administered at a different time, as well as the administration of these therapeutic agents, or at least two of the agents, substantially concurrently. The sequence of each agent, or substantially concurrent administration, can be affected by any appropriate route, including, but not limited to, the oral route, intravenous route, intramuscular, subcutaneous routes, and direct absorption through mucosal tissues. The agents can be administered by the same route or by different routes. For example, a first agent may be administered orally while a second agent is administered intravenously. In addition, a selected combination agent may be administered by intravenous injection, while other agents of the combination may be administered orally. Alternatively, for example, two or more agents may be administered by intravenous or subcutaneous injection.

## II. Embodiments

The present disclosure is further explained below with reference to the embodiments. The description of specific exemplary embodiments of the present disclosure is for the purpose of illustration and exemplification. These descriptions are not intended to limit the present disclosure to the precise forms disclosed, and it is apparent that many modifications and variations are possible in light of the teachings of the present disclosure. The exemplary embodiments are chosen and described in order to explain the particular principles of the disclosure and their practical application, thereby enabling those skilled in the art to realize and utilize the various exemplary embodiments of the disclosure and various choices and modifications.

The experimental methods used in the following embodiments are conventional methods unless otherwise specified.

The materials, reagents, etc. used in the following embodiments are commercially available unless otherwise specified.

Experimental Instruments and Methods:

Instruments and Reagents:

NMR measurements were performed on an Agilent 400MR DD2 spectrometer, using deuterated dimethyl sulfoxide (DMSO-$d_6$), deuterated methanol ($CD_3OD$), and deuterated chloroform ($CDCl_3$) as solvents, with tetramethylsilane (TMS) as the internal standard. Liquid Chromatography-Mass Spectrometry (LC-MS): Agilent 1260 Infinity II - InfinityLab LC/MSD mass spectrometer. HPLC: Agilent 1260 Infinity II High-Performance Liquid Chromatograph (Sunfire C18 5μm 150 x 4.6 mm column). The reagents used were 2-chloro-5-(trifluoromethyl)pyrimidine, N-methylpyrrolidone, tert-butyl piperazine-1-carboxylate, tert-butyl 3,8-diazabicyclo[3.2.1]octane-8-carboxylate, ethyl acetate, tert-butyl (R)-2-methylpiperazine-1-carboxylate, tert-butyl (S)-3-methylpiperazine-1-carboxylate, hydrogen chloride in dioxane solution, 4,5-dibromopyridin-3(2H)-one, N,N-dimethylformamide, methyl 3,3,3-trifluoro-2-oxopropanoate, etc. The reagents and starting materials were purchased from Shanghai Bide Reagent Co., Ltd., or synthesized by methods known in the art. Unless otherwise specified, all reactions in the present disclosure were carried out under continuous magnetic stirring under dry nitrogen or argon, using dry solvents, and reaction temperatures are in degrees Celsius.

### Intermediates

| No. | Name | Structure | LC-MS ESI $[M+H]^+$ |
|---|---|---|---|
| I-1 | 2-(piperazin-1-yl)-5-(trifluoromethyl)pyrimidine hydrochloride | 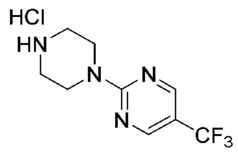 | 233.1 |
| I-2 | 3-(5-(trifluoromethyl)pyrimidin-2-yl)-3,8-diazabicyclo [3.2.1]octane hydrochloride | 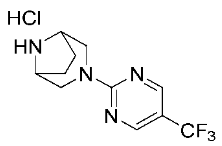 | 259.2 |
| I-3 | (R)-2-(3-methylpiperazin-1-yl)-5-(trifluoromethyl)pyrimidine hydrochloride | 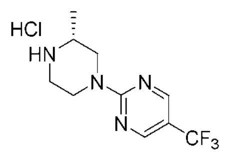 | 247.2 |
| I-4 | (S)-2-(2-methylpiperazin-1-yl)-5-(trifluoromethyl)pyrimidine hydrochloride | 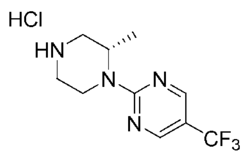 | 247.2 |

(continued)

| No. | Name | Structure | LC-MS ESI [M+H]+ |
|---|---|---|---|
| I-5 | 5-chloro-4-(trifluoromethyl)-2-((2-(trimethylsilyl)ethoxy) methyl)pyridazin-3(2H)-one | 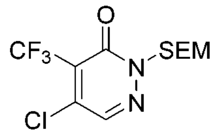 | ESI[2M+Na]+= 679.1 |
| I-6 | 6-oxo-5-(trifluoromethyl)-1,6-dihydropyridazine-3-carbaldehyde | 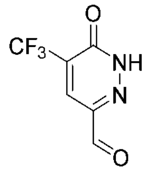 | 193.02 |
| I-7 | 6-oxo-5-(trifluoromethyl)-1,6-dihydropyridine-3-carbaldehyde | 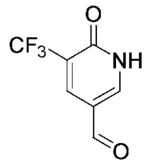 | 192.1 |
| I-8 | 1-(5-(trifluoromethyl)pyridin-2-yl)piperazine hydrochloride | 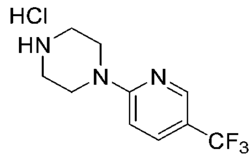 | 232.0 |
| I-9 | 3-(5-(trifluoromethyl)pyrimidin-2-yl)-3,8-diazabicyclo [3.2.1]octane hydrochloride | 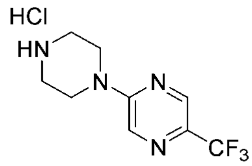 | 233.2 |
| I-10 | (*R*)-3-(trifluoromethyl)-7,7a,8,9,10,11-hexahydro-6*H*-pyrazino[1,2-*d*]pyrido[3,2-*b*][1,4]oxazepine hydrochloride | 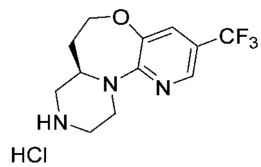 | 274.3 |
| I-11 | (*S*)-3-(trifluoromethyl)-7,7a,8,9,10,11-hexahydro-6*H*-pyrazino[1,2-*d*]pyrido[3,2-*b*][1,4]oxazepine hydrochloride | 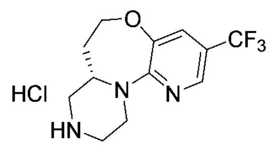 | 274.4 |
| I-12 | (*R*)-3-(trifluoromethyl)-7,7a,8,9,10,11-hexahydro-5*H*-pyrazino[2,1-c]pyrido[2,3-*e*][1,4]oxazepine hydrochloride | 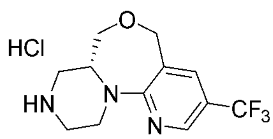 | 274.4 |
| I-13 | (S)-3-(trifluoromethyl)-7,7a,8,9,10,11-hexahydro-6H-pyrazino[1,2-d]pyrido[3,2-b][1,4]oxazepine hydrochloride | 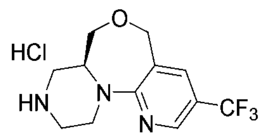 | 274.4 |
| I-14 | 2-(piperazin-1-yl)-5-(trifluoromethyl)nicotinonitrile hydrochloride | 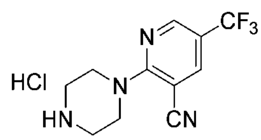 | 257.2 |

(continued)

| No. | Name | Structure | LC-MS ESI $[M+H]^+$ |
|---|---|---|---|
| I-15 | 1-(3-methoxy-5-(trifluoromethyl)pyridin-2-yl)piperazine hydrochloride | 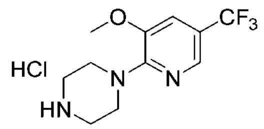 | 262.5 |
| I-16 | 6-(1-bromoethyl)-4-(trifluoromethyl)pyridazin-3(2*H*)-one | 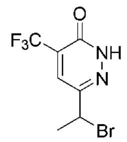 | 298.3, 300.3 |
| I-17 | 6-(2-bromopropan-2-yl)-4-(trifluoromethyl)pyridazin-3(2*H*)-one | 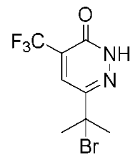 | 285.2, 287.2 |
| I-18 | 6-(bromomethyl)-5-(trifluoromethyl)pyridazin-3(2*H*)-one | 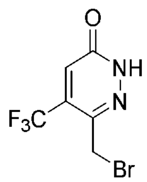 | 256.8, 258.8 |
| I-19 | 6-(bromomethyl)-5-(cyclobutoxy)pyridazin-3(2*H*)-one | 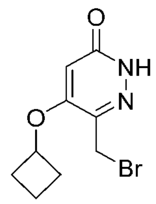 | 259.2, 261.2 |
| I-20 | 1-(3-ethoxy-5-(trifluoromethyl)pyridin-2-yl)piperazine hydrochloride | 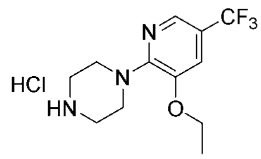 | 276.0 |
| I-21 | 1-(3,5-bis(trifluoromethyl)pyridin-2-yl)piperazine hydrochloride | 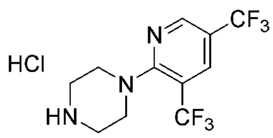 | 300.1 |
| I-22 | 1-(3-methyl-5-(trifluoromethyl)pyridin-2-yl)piperazine | 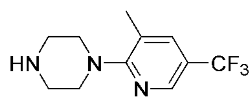 | 246.3 |
| I-23 | 1-(3-cyclopropyl-5-(trifluoromethyl)pyridin-2-yl)piperazine | 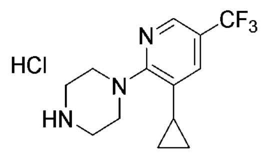 | 272.6 |
| I-24 | 1-(4-methoxy-5-(trifluoromethyl)pyridin-2-yl)piperazine hydrochloride | 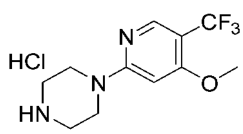 | 262.0 |

(continued)

| No. | Name | Structure | LC-MS ESI [M+H]+ |
|---|---|---|---|
| I-25 | *N*-(2-(piperazin-1-yl)-5-(trifluoromethyl)pyridin-3-yl)acetamide | 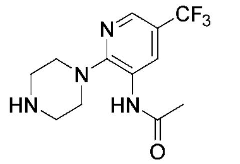 | 289.1 |
| I-26 | 2-(piperazin-1-yl)-5-(trifluoromethyl)nicotinamide hydrochloride | 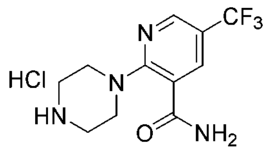 | 275.1 |
| I-27 | 6-(2-chloropropan-2-yl)-4-(trifluoromethyl)pyridazin-3(2H)-one | 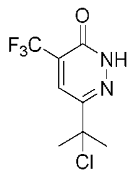 | 241.0, 243.0 |
| I-28 | 7-(piperazin-1-yl)-4-(trifluoromethyl)-1*H*-pyrrolo[2,3-*c*]pyridine hydrochloride | 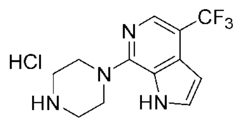 | 271.4 |
| I-29 | 1-methyl-7-(piperazin-1-yl)-4-(trifluoromethyl)-1*H*-pyrrolo [2,3-c]pyridine hydrochloride | 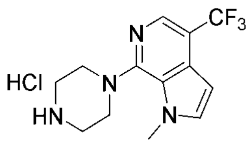 | 285.4 |
| I-30 | (2-(piperazin-1-yl)-5-(trifluoromethyl)pyridin-3-yl)(pyrrolidin-1-yl)methanone hydrochloride | 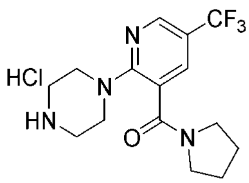 | 329.0 |
| I-31 | *N*-methyl-2-(piperazin-1-yl)-5-(trifluoromethyl)nicotinamide hydrochloride | 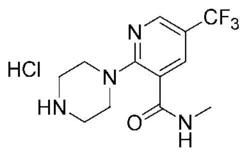 | 289.0 |
| I-32 | 2-methyl-7-(piperazin-1-yl)-4-(trifluoromethyl)-1*H*-pyrrolo [2,3-c]pyridine hydrochloride | 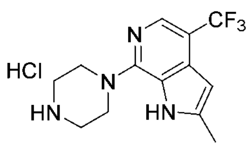 | 285.3 |
| I-33 | 3-(2-(piperazin-1-yl)-5-(trifluoromethyl)pyridin-3-yl)-1,2,4-oxadiazole trifluoroacetate | 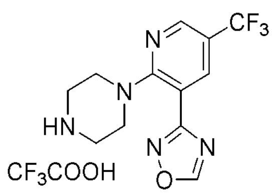 | 300.1 |

**Preparation of Intermediate I-1: 2-(piperazin-1-yl)-5-(trifluoromethyl)pyrimidine hydrochloride (I-1)**

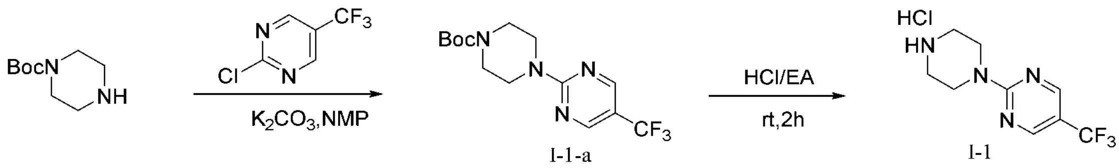


**Step 1: Preparation of tert-butyl 4-(5-(trifluoromethyl)pyrimidin-2-yl)piperazine-1-carboxylate (I-1-a)**

2-chloro-5-(trifluoromethyl)pyrimidine (1825 mg, 10 mmol) was dissolved in *N*-methylpyrrolidone (30 mL), and tert-butyl piperazine-1-carboxylate (1862 mg, 10 mmol) and potassium carbonate (2764 mg, 20 mmol) were added. The mixture was reacted at 80°C for 1 hour. Water (100 mL) was added, and the solid was collected by filtration and dried under vacuum to obtain the target product tert-butyl 4-(5-(trifluoromethyl)pyrimidin-2-yl)piperazine-1-carboxylate (I-1-a, 2.9 g, yield 87%).

**Step 2: Preparation of 2-(piperazin-1-yl)-5-(trifluoromethyl)pyrimidine hydrochloride (I-1)**

tert-butyl 4-(5-(trifluoromethyl)pyrimidin-2-yl)piperazine-1-carboxylate (I-1-a, 332 mg, 1 mmol) was dissolved in 4M hydrogen chloride in ethyl acetate (5 mL) and reacted at room temperature for 2 hours. The reaction mixture was filtered, and the solid was washed with petroleum ether to obtain the target product 2-(piperazin-1-yl)-5-(trifluoromethyl) pyrimidine hydrochloride (I-1, 289 mg, yield 94%). ESI[M+H]$^+$=233.1 $^1$H NMR (400 MHz, DMSO-$d_6$) NMR (400 MHz, DMSO--$d_6$) δ 9.48 (S, 2H), 8.78 (S, 2H), 4.06 (S, 4H), 3.18 (S, 4H).

**Preparation of Intermediate 1-2: 3-(5-(trifluoromethyl)pyrimidin-2-yl)-3,8-diazabicyclo[3.2.1]octane hydrochloride (1-2)**

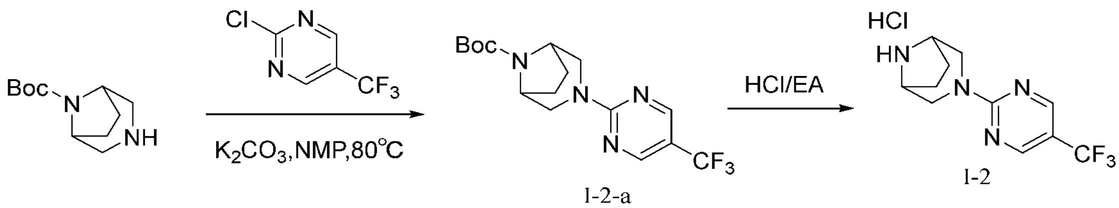


**Step 1: Preparation of tert-butyl 3-(5-(trifluoromethyl)pyrimidin-2-yl)-3,8-diazabicyclo[3.2.1]octane-8-carboxylate (I-2-a)**

tert-butyl 3,8-diazabicyclo[3.2.1]octane-8-carboxylate (200 mg, 0.942 mmol) was dissolved in *N*-methylpyrrolidone (5 mL), and 2-chloro-5-(trifluoromethyl)pyrimidine (172 mg, 0.942 mmol) and potassium carbonate (260 mg, 1.88 mmol) were added. The mixture was reacted at 80°C for 5 hours. Water (20 mL) was added, and the mixture was stirred at 0°C for 30 minutes. The precipitated solid was collected by filtration, washed with water, and dried at 70°C to obtain the target product tert-butyl 3-(5-(trifluoromethyl)pyrimidin-2-yl)-3,8-diazabicyclo[3.2.1]octane-8-carboxylate (I-2-a, 312 mg, yield 92.6%). ESI[M+H-56]$^+$ =303.1

**Step 2: Preparation of 3-(5-(trifluoromethyl)pyrimidin-2-yl)-3,8-diazabicyclo[3.2.1]octane hydrochloride (I-2)**

tert-butyl 3-(5-(trifluoromethyl)pyrimidin-2-yl)-3,8-diazabicyclo[3.2.1]octane-8-carboxylate (I-2-a, 312 mg, 0.871 mmol) was dissolved in ethyl acetate (5 mL), and 4M hydrogen chloride in dioxane solution (10 mL) was added. The mixture was reacted at room temperature for 3 hours. The precipitated solid was collected by filtration and washed with ethyl acetate to obtain the target product 3-(5-(trifluoromethyl)pyrimidin-2-yl)-3,8-diazabicyclo[3.2.1]octane hydrochloride (I-2, 232 mg, yield 90.6%). ESI[M+H]$^+$=259.2

**Preparation of Intermediate I-3: (*R*)-2-(3-methylpiperazin-1-yl)-5-(trifluoromethyl)pyrimidine hydrochloride (1-3)**

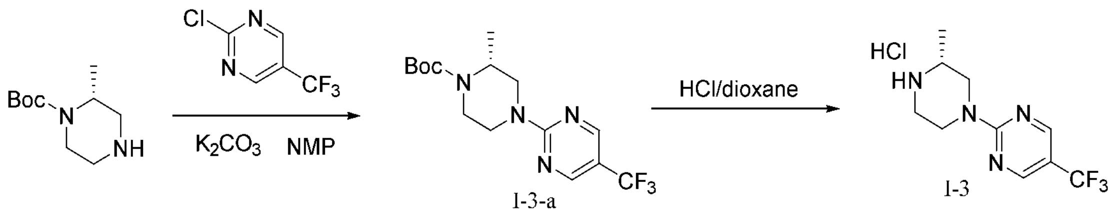


I-3-a

I-3

**Step 1: Preparation of tert-butyl (*R*)-2-methyl-4-(5-(trifluoromethyl)pyrimidin-2-yl)piperazine-1-carboxylate (I-3-a)**

To a solution of tert-butyl (*R*)-2-methylpiperazine-1-carboxylate (100 mg, 0.499 mmol) in N-methylpyrrolidone (10 mL) at room temperature were added potassium carbonate (138 mg, 0.999 mmol) and 2-chloro-5-(trifluoromethyl) pyrimidine (91 mg, 0.499 mmol). The reaction mixture was stirred at 80°C overnight. The reaction was quenched by adding 20 mL of water and extracted with 3×10 mL of ethyl acetate. The combined organic layers were washed with 3×10 mL of water, dried over anhydrous sodium sulfate, and concentrated under reduced pressure to obtain tert-butyl (*R*)-2-methyl-4-(5-(trifluoromethyl)pyrimidin-2-yl)piperazine-1-carboxylate (I-3-a, 110 mg, yield 63.61%). ESI[M+H]$^+$=347.3

**Step 2: Preparation of (*R*)-2-(3-methylpiperazin-1-yl)-5-(trifluoromethyl)pyrimidine hydrochloride (I-3)**

To a solution of tert-butyl (*R*)-2-methyl-4-(5-(trifluoromethyl)pyrimidin-2-yl)piperazine-1-carboxylate (I-3-a, 110 mg, 0.318 mmol) in ethyl acetate (3 mL) at room temperature was added 4M hydrogen chloride in dioxane solution (3 mL). The reaction mixture was stirred at room temperature for 2 hours and then concentrated under reduced pressure to obtain crude (*R*)-2-(3-methylpiperazin-1-yl)-5-(trifluoromethyl)pyrimidine (I-3, 100 mg). ESI[M+H]$^+$=247.2

**Preparation of Intermediate 1-4: (*S*)-2-(2-methylpiperazin-1-yl)-5-(trifluoromethyl)pyrimidine hydrochloride (I-4)**

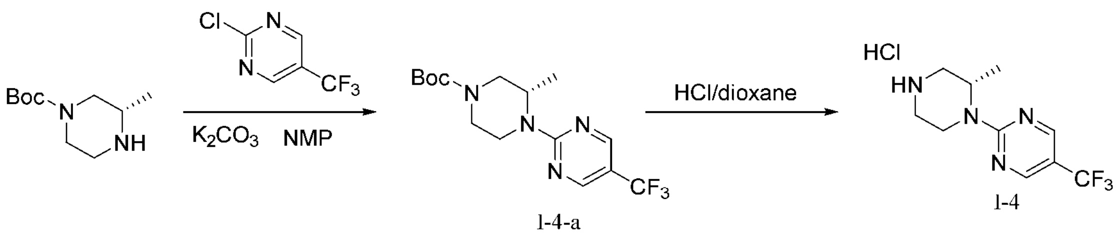


I-4-a

I-4

**Step 1: Preparation of tert-butyl (*S*)-3-methyl-4-(5-(trifluoromethyl)pyrimidin-2-yl)piperazine-1-carboxylate (I-4-a)**

tert-butyl (*S*)-3-methylpiperazine-1-carboxylate (200 mg, 1 mmol) was dissolved in *N*-methylpyrrolidone (5 mL), and 2-chloro-5-(trifluoromethyl)pyrimidine (183 mg, 1 mmol) and potassium carbonate (276 mg, 2 mmol) were added. The mixture was reacted at 100°C for 2 hours. Water (20 mL) was added, and the mixture was stirred at 0°C for 30 minutes. The precipitated solid was collected by filtration, washed with water, and dried at 70°C to obtain the target product tert-butyl (S)-3-methyl-4-(5-(trifluoromethyl)pyrimidin-2-yl)piperazine-1-carboxylate (I-4-a, 276 mg, yield 79.8%). ESI[M+H-56]$^+$ =291.0 ESI[M+H-100]$^+$=247.1

**Step 2: Preparation of (S)-2-(2-methylpiperazin-1-yl)-5-(trifluoromethyl)pyrimidine hydrochloride (1-4)**

tert-butyl (*S*)-3-methyl-4-(5-(trifluoromethyl)pyrimidin-2-yl)piperazine-1-carboxylate (I-4-a, 276 mg, 0.797 mmol) was dissolved in ethyl acetate (5 ml), and 4M hydrogen chloride in dioxane solution (5 ml) was added. The mixture

was reacted at room temperature for 3 hours. The precipitated solid was collected by filtration and washed with ethyl acetate, followed by distillation under reduced pressure to obtain the target product (*S*)-2-(2-methylpiperazin-1-yl)-5-(trifluoromethyl)pyrimidine hydrochloride (I-4, 220 mg, yield 97.8%). ESI[M+H]$^+$=247.2

**Preparation of Intermediate I-5: 5-chloro-4-(trifluoromethyl)-2-((2-(trimethylsilyl)ethoxy)methyl)pyridazin-3(2H)-one (1-5)**

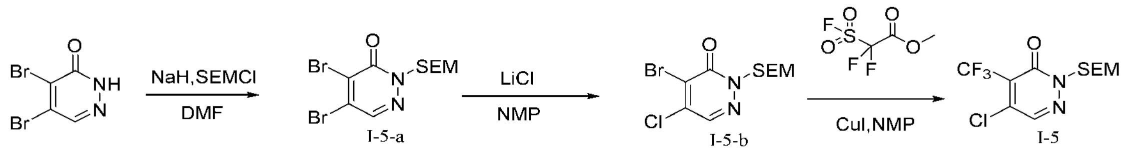


**Step 1: Preparation of 4,5-dibromo-2-((2-(trimethylsilyl)ethoxy)methyl)pyridazin-3(2H)-one (I-5-a)**

Under an ice bath, 4,5-dibromopyridin-3(2*H*)-one (20 g, 78.78 mmol) was dissolved in *N*,*N*-dimethylformamide (200 mL). 60% sodium hydride (4.0 g, 94.54 mmol) was added slowly. After stirring at room temperature for 1 hour, 2-(trimethylsilyl)ethoxymethyl chloride (14.4 g, 86.66 mmol) was added dropwise under an ice bath. After the addition was complete, the reaction mixture was stirred at room temperature for 2 hours. Under an ice bath, the reaction was quenched with water (200 mL) and extracted with ethyl acetate (100 mL x 3). The organic phase was washed with brine (100 mL x 3), dried over anhydrous magnesium sulfate, filtered, and concentrated. The crude product was purified by silica gel chromatography (petroleum ether:ethyl acetate=5:1) to obtain 4,5-dibromo-2-((2-(trimethylsilyl)ethoxy)methyl)pyridazin-3(2*H*)-one (I-5-a, 18.6 g, yield: 61.46%, colorless oil). ESI[M+H]$^+$ =355.1, 357.1

**Step 2: Preparation of 4-bromo-5-chloro-2-((2-(trimethylsilyl)ethoxy)methyl)pyridazin-3(2*H*)-one (I-5-b)**

To a solution of 4,5-dibromo-2-((2-(trimethylsilyl)ethoxy)methyl)pyridazin-3(2*H*)-one (I-5-a, 17.4 g, 45.30 mmol) in *N*-methylpyrrolidone (100 mL) was added lithium chloride (1.93 g, 45.30 mmol), and the resulting solution was stirred at 95°C for 4 hours. Then, 250 mL of water was added to the reaction, and the mixture was extracted with 3 x 250 mL of ethyl acetate. The organic layers were combined. The organic layer was washed with 3 x 250 mL of brine, dried over anhydrous sodium sulfate, and concentrated under reduced pressure to obtain crude 4-bromo-5-chloro-2-((2-(trimethylsilyl)ethoxy)methyl)pyridazin-3(2*H*)-one (I-5-b, 13 g), which was used directly in the next step. ESI[M+H]$^+$ =311.2, 313.2

**Step 3: Preparation of 5-chloro-4-(trifluoromethyl)-2-((2-(trimethylsilyl)ethoxy)methyl)pyridazin-3(2H)-one (I-5)**

To a solution of 4-bromo-5-chloro-2-((2-(trimethylsilyl)ethoxy)methyl)pyridazin-3(2*H*)-one (I-5-b, 13 g, 38.27 mmol) in N-methylpyrrolidone (80 mL) at room temperature was added cuprous iodide (1.47 g, 7.66 mmol), followed by the dropwise addition of methyl 2,2-difluoro-2-(fluorosulfonyl)acetate (22.17 g, 114.8 mmol). The resulting solution was stirred at 80°C for 2 hours. The reaction was quenched by adding 250 mL of water and extracted with 3 x 250 mL of ethyl acetate. The combined organic layers were washed with 3 x 250 mL of brine, dried over anhydrous sodium sulfate, and concentrated under reduced pressure. The crude product was purified by silica gel chromatography (ethyl acetate:petroleum ether = 1:99) to obtain compound 5-chloro-4-(trifluoromethyl)-2-((2-(trimethylsilyl)ethoxy)methyl)pyridazin-3(2*H*)-one (I-5, 9.4 g, yield 53.95%). ESI[2M+Na]$^+$= 679.1

**Preparation of Intermediate 1-6: 6-oxo-5-(trifluoromethyl)-1,6-dihydropyridazine-3-carbaldehyde (1-6)**

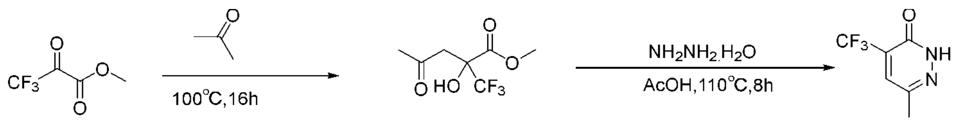


I-6-a

I-6-b

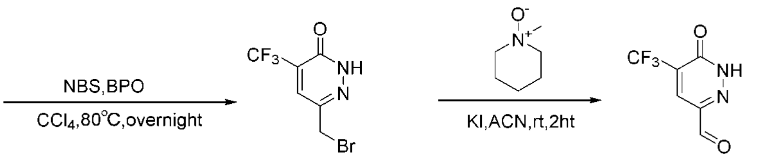


I-6-c

I-6

**Step 1: Preparation of methyl 2-hydroxy-4-oxo-2-(trifluoromethyl)pentanoate (I-6-a)**

Methyl 3,3,3-trifluoro-2-oxopropanoate (10 g, 64.08 mmol) was dissolved in acetone (5.2 mL) and stirred in a sealed tube at 100°C for 16 hours. The reaction mixture was concentrated under reduced pressure to obtain the target product methyl 2-hydroxy-4-oxo-2-(trifluoromethyl)pentanoate (I-6-a, 12.84 g, yield 93.65%). ESI$[M+H]^+$=215.05

**Step 2: Preparation of 6-methyl-4-(trifluoromethyl)pyridazin-3(2*H*)-one (I-6-b)**

Methyl 2-hydroxy-4-oxo-2-(trifluoromethyl)pentanoate (I-6-a, 12.13 g, 56.68 mmol) was dissolved in acetic acid (35 mL). Hydrazine hydrate (5.67 g, 113.26 mmol) was added, and the mixture was reacted at 110°C overnight. Saturated brine was added, and the mixture was extracted with ethyl acetate. The product was purified by column chromatography (petroleum ether:ethyl acetate = 88%:12%) to obtain the target product 6-methyl-4-(trifluoromethyl)pyridazin-3(2*H*)-one (I-6-b, 6.55 g, yield 64.88%). ESI$[M+H]^+$ =179.04

**Step 3: Preparation of 6-(bromomethyl)-4-(trifluoromethyl)pyridazin-3(2*H*)-one (I-6-c)**

6-methyl-4-(trifluoromethyl)pyridazin-3(2*H*)-one (I-6-b, 6.55 g, 36.8 mmol) was dissolved in carbon tetrachloride solution (190 mL). *N*-bromosuccinimide (9.82 g, 55.17 mmol) and dibenzoyl peroxide (1.78 g, 7.35 mmol) were added sequentially, and the mixture was reacted at 80°C overnight. Saturated brine was added, and the mixture was extracted with ethyl acetate. The organic phase was concentrated under reduced pressure and purified by column chromatography (petroleum ether:ethyl acetate = 91:9) to obtain the target product 6-(bromomethyl)-4-(trifluoromethyl)pyridazin-3(2*H*)-one (I-6-c, 4.7 g, yield 49.91%). ESI$[M+H]^+$ =256.95

**Step 4: Preparation of 6-oxo-5-(trifluoromethyl)-1,6-dihydropyridazine-3-carbaldehyde (1-6)**

6-(bromomethyl)-4-(trifluoromethyl)pyridazin-3(2*H*)-one (I-6-c, 500 mg, 1.95 mmol) was dissolved in acetonitrile (5 mL). Under an ice bath, *N*-methylmorpholine-*N*-oxide (803 mg, 6.84 mmol) and potassium iodide (32.4 mg, 0.195 mmol) were added. After reacting at room temperature for 2 hours, the mixture was concentrated under reduced pressure and purified by column chromatography (petroleum ether:ethyl acetate = 80:20) to obtain the target product 6-oxo-5-(trifluoromethyl)-1,6-dihydropyridazine-3-carbaldehyde (I-6, 270 mg, yield 72%). (ESI) $[M+H]^+$=193.02 $^1$HNMR (600 MHz, DMSO-$d_6$) $\delta$ 14.45 (S, 1H), 9.74 (S, 1H), 8.04 (S, 1H).

**Preparation of Intermediate 1-7: 6-oxo-5-(trifluoromethyl)-1,6-dihydropyridine-3-carbaldehyde (1-7)**

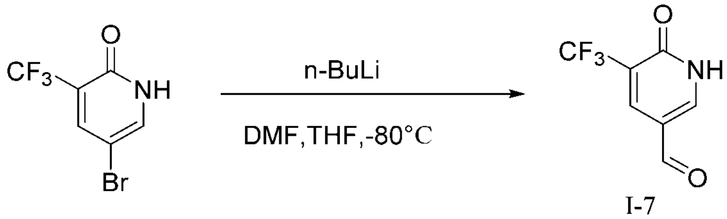


I-7

5-bromo-3-(trifluoromethyl)pyridin-2(1*H*)-one (2 g, 0.826 mmol) was dissolved in anhydrous tetrahydrofuran

solution (3 mL). After stirring at -80°C under a nitrogen atmosphere for 20 minutes, n-butyllithium (9.9 mL, 2.479 mmol) was slowly added dropwise, and the reaction was continued at -80°C for 3 hours. *N,N*-dimethylformamide (1.53 mL, 19.824 mmol) was added dropwise again, and the reaction was continued for 1 hour. The reaction was quenched by adding saturated aqueous ammonium chloride solution, extracted with ethyl acetate, and then extracted with dichloromethane. The organic phase was concentrated under reduced pressure.

The aqueous phase was filtered after distillation under reduced pressure and combined. The crude product was purified by column chromatography (petroleum ether-ethyl acetate: 0-70%) to obtain the target product 6-oxo-5-(trifluoromethyl)-1,6-dihydropyridine-3-carbaldehyde (I-7, 1.01 g, yield 63.96%). ESI[M+H]$^+$ =192.1

**Preparation of Intermediate 1-8: 1-(5-(trifluoromethyl)pyridin-2-yl)piperazine hydrochloride (I-8)**

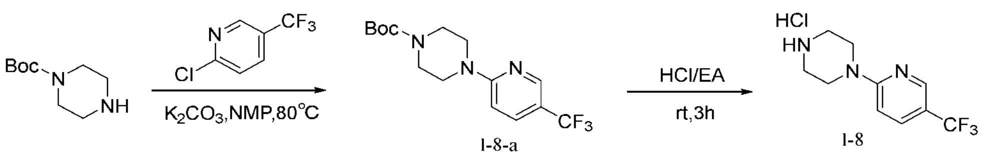

I-8-a
I-8

**Step 1: Preparation of tert-butyl 4-(5-(trifluoromethyl)pyridin-2-yl)piperazine-1-carboxylate (I-8-a)**

2-chloro-5-(trifluoromethyl)pyridine (500 mg, 2.68 mmol) was dissolved in *N*-methylpyrrolidone (13 mL), and tert-butyl piperazine-1-carboxylate (2.82 mg, 10 mmol) and potassium carbonate (557 mg, 4.02 mmol) were added. The mixture was reacted at 80°C for 3 hours. Water (150 mL) was added, and the solid was collected by stirring and filtration and dried under vacuum to obtain the target product tert-butyl 4-(5-(trifluoromethyl)pyridin-2-yl)piperazine-1-carboxylate (I-8-a, 520 mg, yield 58.46%).

**Step 2: Preparation of 1-(5-(trifluoromethyl)pyridin-2-yl)piperazine hydrochloride (I-8)**

tert-butyl 4-(5-(trifluoromethyl)pyridin-2-yl)piperazine-1-carboxylate (I-8-a, 520 mg, 1.57 mmol) was dissolved in 4M hydrogen chloride in ethyl acetate (6 mL) and reacted at room temperature for 2 hours. The reaction mixture was filtered, and the solid was washed with petroleum ether to obtain the target product 1-(5-(trifluoromethyl)pyridin-2-yl) piperazine hydrochloride (I-8, 400 mg, yield 95.22%). ESI [M+H]$^+$=232.0

**Preparation of Intermediate 1-9: 3-(5-(trifluoromethyl)pyrimidin-2-yl)-3,8-diazabicyclo[3.2.1]octane hydrochloride (1-9)**

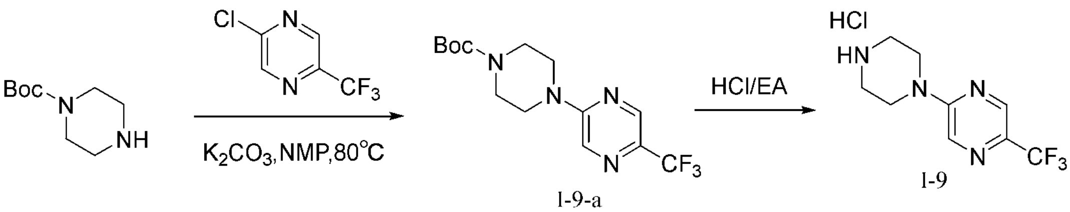

I-9-a
I-9

**Step 1: Preparation of tert-butyl 4-(5-(trifluoromethyl)pyrazin-2-yl)piperazine-1-carboxylate (I-9-a)**

tert-butyl piperazine-1-carboxylate (486 mg, 2.61 mmol) was dissolved in *N*-methylpyrrolidone (20 mL). Under an ice bath, 2-chloro-5-(trifluoromethyl)pyrazine (500 mg, 2.74 mmol) and potassium carbonate (757 mg, 5.22 mmol) were added. After reacting at 80°C for 4 hours, water (50 mL) was added to the reaction mixture under an ice bath. The mixture was filtered, the filter cake was washed with water, and the collected filter cake was concentrated under reduced pressure to obtain the target product tert-butyl 4-(5-(trifluoromethyl)pyrazin-2-yl)piperazine-1-carboxylate (I-9-a, 880 mg, yield 96.8%). ESI [M-56+H]$^+$=277.2

**Step 2: Preparation of 2-(piperazin-1-yl)-5-(trifluoromethyl)pyrazine hydrochloride (1-9)**

tert-butyl 4-(5-(trifluoromethyl)pyrazin-2-yl)piperazine-1-carboxylate (I-9-a, 880 mg, 2.61 mmol) was dissolved in ethyl acetate (10 mL). Under an ice bath, 4M hydrogen chloride in ethyl acetate solution (10 mL) was added. After reacting at room temperature for 2 hours, the mixture was concentrated under reduced pressure to obtain the target 2-(piperazin-1-yl)-5-(trifluoromethyl)pyrazine hydrochloride (I-9, 638 mg, yield 89.6%). ESI $[M+H]^+$=233.2

**Preparation of Intermediate 1-10: (*R*)-3-(trifluoromethyl)-7,7a,8,9,10,11-hexahydro-6*H*-pyrazino[1,2-d]pyrido [3,2-*b*][1,4]oxazepine hydrochloride (I-10)**

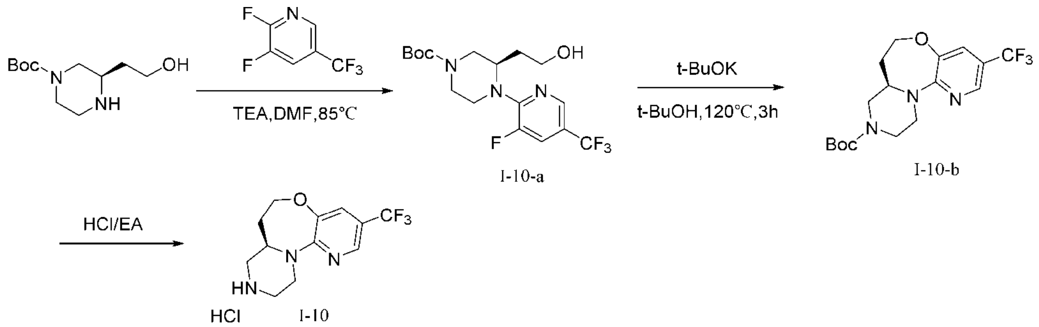


**Step 1: Preparation of tert-butyl (*R*)-4-(3-fluoro-5-(trifluoromethyl)pyridin-2-yl)-3-(2-hydroxyethyl)piperazine-1-carboxylate (I-10-a)**

(*R*)-tert-butyl 3-(2-hydroxyethyl)piperazine-1-carboxylate (100 mg, 0.434 mmol) was dissolved in *N,N*-dimethylformamide (3 mL), and then 2,3-difluoro-5-(trifluoromethyl)pyridine (100 mg, 0.564 mmol) and triethylamine (270 μL, 1.95 mmol) were added. The mixture was heated to 85°C and reacted overnight. After cooling, water was added to the reaction mixture, which was then extracted with ethyl acetate. The combined organic phases were washed with saturated brine, and the organic phase was dried over anhydrous sodium sulfate and filtered. The filtrate was concentrated under reduced pressure and purified by Flash column (ethyl acetate:petroleum ether = 0-27%) to obtain the target product tert-butyl (*R*)-4-(3-fluoro-5-(trifluoromethyl)pyridin-2-yl)-3-(2-hydroxyethyl)piperazine-1-carboxylate (I-10-a, 136 mg, yield 79.62%). ESI $[M-100+H]^+$=294.3, ESI $[M+H]^+$=394.3

**Step 2: Preparation of tert-butyl (R)-3-(trifluoromethyl)-6,7,7a,8,10,11-hexahydro-9H-pyrazino[1,2-d]pyrido[3,2-b][1,4]oxazepine-9-carboxylate (I-10-b)**

tert-butyl (*R*)-4-(3-fluoro-5-(trifluoromethyl)pyridin-2-yl)-3-(2-hydroxyethyl)piperazine-1-carboxylate (I-10-a, 136 mg, 0.345 mmol) was dissolved in tert-butanol (4 mL), and potassium tert-butoxide (117 mg, 1.04 mmol) was added. The mixture was reacted at 120°C for 3 hours. After cooling, the reaction mixture was distilled under reduced pressure. Ethyl acetate was added to dissolve the residue, which was then filtered. The filtrate was concentrated under reduced pressure and purified by Flash column (ethyl acetate:petroleum ether = 0-43%) to obtain the target product tert-butyl (*R*)-3-(trifluoromethyl)-6,7,7a,8,10,11-hexahydro-9*H*-pyrazino[1,2-*d*]pyrido[3,2-*b*][1,4]oxazepine-9-carboxylate (I-10-b, 102 mg, yield 79.02%). ESI $[M-100+H]^+$=274.3, ESI $[M+H]^+$=374.4

**Step 3: Preparation of (*R*)-3-(trifluoromethyl)-7,7a,8,9,10,11-hexahydro-6*H*-pyrazino[1,2-d]pyrido[3,2-*b*][1,4]oxazepine hydrochloride (I-10)**

To tert-butyl (*R*)-3-(trifluoromethyl)-6,7,7a,8,10,11-hexahydro-9*H*-pyrazino[1,2-*d*]pyrido[3,2-*b*][1,4]oxazepine-9-carboxylate (I-10-b, 102 mg, 0.273 mmol) at room temperature was added 4M hydrogen chloride in ethyl acetate solution (3 mL). The reaction mixture was stirred at room temperature for 1 hour, then ethyl acetate was added multiple times and the mixture was concentrated under reduced pressure to obtain crude (*R*)-3-(trifluoromethyl)-7,7a,8,9,10,11-hexahydro-6*H*-pyrazino[1,2-*d*]pyrido[3,2-*b*][1,4]oxazepine hydrochloride (I-10, 96 mg, yield 113.46%). ESI $[M+H]^+$=274.3

**Preparation of Intermediate I-11: (*S*)-3-(trifluoromethyl)-7,7*a*,8,9,10,11-hexahydro-6*H*-pyrazino[1,2-*d*]pyrido[3,2-*b*][1,4]oxazepine hydrochloride (I-11)**

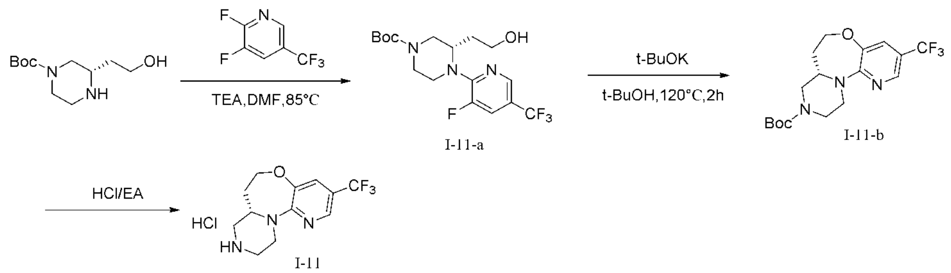


I-11-a I-11-b I-11

**Step 1: Preparation of tert-butyl (*S*)-4-(3-fluoro-5-(trifluoromethyl)pyridin-2-yl)-3-(2-hydroxyethyl)piperazine-1-carboxylate (I-11-a)**

(*S*)-tert-butyl 3-(2-hydroxyethyl)piperazine-1-carboxylate (200 mg, 0.868 mmol) and 2,3-difluoro-5-(trifluoromethyl)pyridine (183 mg, 0.999 mmol) were dissolved in *N*,*N*-dimethylformamide (6 mL). Triethylamine (540 μL, 3.91 mmol) was added, and the mixture was reacted in a sealed tube at 85°C overnight. The reaction was quenched with water (10 mL) and extracted with ethyl acetate. The organic phase was dried over anhydrous sodium sulfate and filtered. The filtrate was concentrated under reduced pressure and purified by normal phase column (petroleum ether:ethyl acetate = 3:1) to obtain the target product (*S*)-tert-butyl 4-(3-fluoro-5-(trifluoromethyl)pyridin-2-yl)-3-(2-hydroxyethyl)piperazine-1-carboxylate (I-11-a, 287 mg, yield 67.2%). ESI $[M+H]^+$=394.4, ESI $[M-56+H]^+$=338.2

**Step 2: Preparation of tert-butyl (*S*)-3-(trifluoromethyl)-6,7,7*a*,8,10,11-hexahydro-9*H*-pyrazino[1,2-*d*]pyrido[3,2-*b*][1,4]oxazepine-9-carboxylate (I-11-b)**

(*S*)-tert-butyl 4-(3-fluoro-5-(trifluoromethyl)pyridin-2-yl)-3-(2-hydroxyethyl)piperazine-1-carboxylate (I-11-a, 237 mg, 0.603 mmol) was dissolved in tert-butanol (8 mL). Potassium tert-butoxide (203 mg, 1.81 mmol) was added, and the mixture was reacted in a sealed tube at 120°C for 2 hours. After filtration, the filtrate was concentrated under reduced pressure to obtain the target product tert-butyl (*S*)-3-(trifluoromethyl)-6,7,7*a*,8,10,11-hexahydro-9*H*-pyrazino[1,2-*d*]pyrido[3,2-*b*][1,4]oxazepine-9-carboxylate (I-11-b, 260 mg, crude). ESI $[M-56+H]^+$=318.3

**Step 3: Preparation of (*S*)-3-(trifluoromethyl)-7,7*a*,8,9,10,11-hexahydro-6*H*-pyrazino[1,2-*d*]pyrido[3,2-*b*][1,4]oxazepine hydrochloride (I-11)**

(*S*)-tert-butyl 3-(trifluoromethyl)-6,7,7*a*,8,10,11-hexahydro-9*H*-pyrazino[1,2-*d*]pyrido[3,2-*b*][1,4]oxazepine-9-carboxylate (I-11-b, 260 mg, 0.697 mmol) was dissolved in ethyl acetate (2 mL), and 4M hydrogen chloride in ethyl acetate solution (4 mL) was added. After reacting at room temperature for 2 hours, the mixture was concentrated under reduced pressure to obtain the target product (*S*)-3-(trifluoromethyl)-7,7a,8,9,10,11-hexahydro-6H-pyrazino[1,2-d]pyrido[3,2-b][1,4]oxazepine hydrochloride (I-11, 280 mg, crude). ESI $[M+H]^+$=274.4

**Preparation of Intermediate 1-12: (R)-3-(trifluoromethyl)-7,7a,8,9,10,11-hexahydro-5*H*-pyrazino[2,1-*c*]pyrido[2,3-*e*][1,4]oxazepine hydrochloride (I-12)**

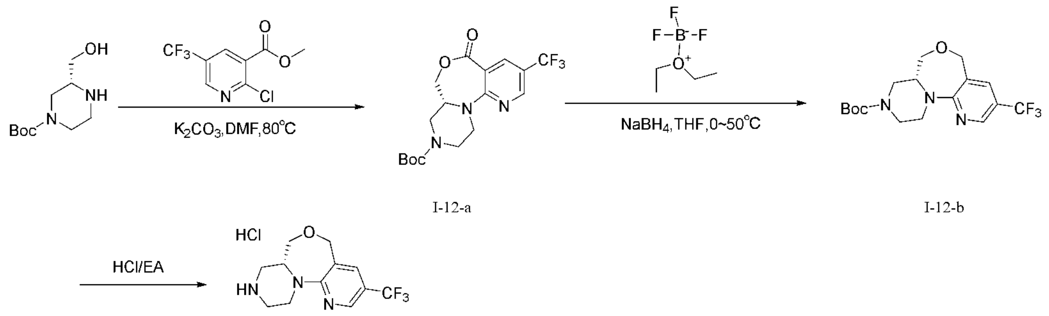


I-12-a

I-12-b

I-12

### Step 1: Preparation of tert-butyl (*R*)-5-oxo-3-(trifluoromethyl)-7*a*,8,10,11-tetrahydro-5*H*-pyrazino[2,1-*c*]pyrido[2,3-*e*][1,4]oxazepine-9(7*H*)-carboxylate (I-12-a)

tert-butyl (*R*)-3-(hydroxymethyl)piperazine-1-carboxylate (1.0 g, 4.62 mmol) was dissolved in *N,N*-dimethylformamide (15 mL), and methyl 2-chloro-5-(trifluoromethyl)nicotinate (1.11 g, 4.62 mmol) and potassium carbonate (1.28 g, 9.25 mmol) were added. The mixture was reacted at 80°C overnight. The reaction mixture was added dropwise to water (150 mL), and a solid precipitated. The mixture was filtered, and the filter cake was concentrated under reduced pressure to obtain the target product tert-butyl (*R*)-5-oxo-3-(trifluoromethyl)-7*a*,8,10,11-tetrahydro-5*H*-pyrazino[2,1-*c*]pyrido[2,3-*e*][1,4]oxazepine-9(7*H*)-carboxylate (I-12-a, 1.0 g, yield 55.9%). ESI [M-56+H]$^+$=332.4

### Step 2: Preparation of tert-butyl (*R*)-3-(trifluoromethyl)-7*a*,8,10,11-tetrahydro-5*H*-pyrazino[2,1-*c*]pyrido[2,3-e][1,4]oxazepine-9(7*H*)-carboxylate (I-12-b)

To a solution of tert-butyl (*R*)-5-oxo-3-(trifluoromethyl)-7*a*,8,10,11-tetrahydro-5*H*-pyrazino[2,1-*c*]pyrido[2,3-*e*][1,4]oxazepine-9(7*H*)-carboxylate (I-12-a, 800 mg, 2.07 mmol) in tetrahydrofuran (15 mL) under an ice bath were added sodium borohydride (210.9 mg, 5.58 mmol) and boron trifluoride etherate solution (293 mg, 2.07 mmol). The mixture was reacted at 50°C for 4 hours under a nitrogen atmosphere. The reaction mixture was concentrated under reduced pressure and purified by normal phase column chromatography (petroleum ether:ethyl acetate = 3:1) to obtain the target product tert-butyl (*R*)-3-(trifluoromethyl)-7*a*,8,10,11-tetrahydro-5*H*-pyrazino[2,1-c]pyrido[2,3-e][1,4]oxazepine-9(7H)-carboxylate (I-12-b, 110 mg, yield 11.4%). ESI [M+H]$^+$=374.4, ESI [M-56+H]$^+$=318.4

### Step 3: Preparation of (R)-3-(trifluoromethyl)-7,7a,8,9,10,11-hexahydro-5H-pyrazino[2,1-c]pyrido[2,3-e][1,4]oxazepine hydrochloride (I-12)

tert-butyl (*R*)-3-(trifluoromethyl)-7*a*,8,10,11-tetrahydro-5*H*-pyrazino[2,1-*c*]pyrido[2,3-*e*][1,4]oxazepine-9(7*H*)-carboxylate (I-12-b, 110 mg, 0.295 mmol) was dissolved in ethyl acetate (2 mL), and 4M hydrogen chloride in ethyl acetate solution (4 mL) was added. After reacting at room temperature for 2 hours, the mixture was concentrated under reduced pressure to obtain the target product (*R*)-3-(trifluoromethyl)-7,7a,8,9,10,11-hexahydro-5*H*-pyrazino[2,1-*c*]pyrido[2,3-*e*][1,4]oxazepine hydrochloride (I-12, 110 mg, crude). ESI [M+H]$^+$=274.4

### Preparation of Intermediate 1-13: (S)-3-(trifluoromethyl)-7,7a,8,9,10,11-hexahydro-6H-pyrazino[1,2-d]pyrido[3,2-b][1,4]oxazepine hydrochloride (I-13)

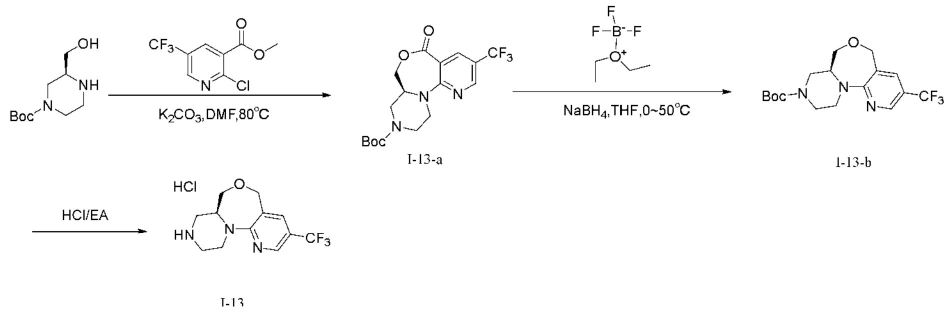

I-13-a

I-13-b

I-13

**Step 1: Synthesis of tert-butyl (*S*)-5-oxo-3-(trifluoromethyl)-7a,8,10,11-tetrahydro-5H-pyrazino[2,1-*c*]pyrido[2,3-*e*][1,4]oxazepine-9(7*H*)-carboxylate (I-13-a)**

tert-butyl (*S*)-3-(hydroxymethyl)piperazine-1-carboxylate (500 mg, 2.31 mmol) and methyl 2-chloro-5-(trifluoromethyl)nicotinate (554 mg, 2.31 mmol) were dissolved in *N*,*N*-dimethylformamide (10 mL), and then potassium carbonate (639 mg, 4.62 mmol) was added. The mixture was heated to 80°C and reacted overnight. After the reaction was complete, the reaction mixture was filtered to remove insoluble matter. The filtrate was concentrated under reduced pressure and purified by Flash column chromatography (ethyl acetate:petroleum ether = 0%~35%) to obtain the target product tert-butyl (*S*)-5-oxo-3-(trifluoromethyl)-7a,8,10,11-tetrahydro-5*H*-pyrazino[2,1-c]pyrido[2,3-*e*][1,4]oxazepine-9(7*H*)-carboxylate (I-13-a, 800 mg, yield 62%). ESI $[M+H]^+$=332.2

**Step 2: Preparation of tert-butyl (*S*)-3-(trifluoromethyl)-7*a*,8,10,11-tetrahydro-5*H*-pyrazino[2,1-*c*]pyrido[2,3-e][1,4]oxazepine-9(7*H*)-carboxylate (I-13-b)**

To a solution of tert-butyl (*S*)-5-oxo-3-(trifluoromethyl)-7a,8,10,11-tetrahydro-5*H*-pyrazino[2,1-c]pyrido[2,3-*e*][1,4]oxazepine-9(7*H*)-carboxylate (I-13-a, 730 mg, 1.88 mmol) in tetrahydrofuran (8 mL) under an ice bath were added sodium borohydride (187 mg, 4.71 mmol) and boron trifluoride etherate solution (267 mg, 1.88 mmol). The mixture was reacted at 50°C for 4 hours under a nitrogen atmosphere. The reaction mixture was concentrated under reduced pressure and purified by normal phase column chromatography (petroleum ether:ethyl acetate = 3:1) to obtain the target product tert-butyl (*S*)-3-(trifluoromethyl)-7*a*,8,10,11-tetrahydro-5*H*-pyrazino[2,1-*c*]pyrido[2,3-*e*][1,4]oxazepine-9(7*H*)-carboxylate (I-13-b, 100 mg, yield 14.2%). ESI $[M+H]^+$=374.4, ESI $[M-56+H]^+$=318.4

**Step 3: Preparation of (*S*)-3-(trifluoromethyl)-7,7*a*,8,9,10,11-hexahydro-5*H*-pyrazino[2,1-*c*]pyrido[2,3-*e*][1,4]oxazepine hydrochloride (I-13)**

tert-butyl (*S*)-3-(trifluoromethyl)-7*a*,8,10,11-tetrahydro-5*H*-pyrazino[2,1-*c*]pyrido[2,3-*e*][1,4]oxazepine-9(7*H*)-carboxylate (I-13-b, 100 mg, 0.241 mmol) was dissolved in ethyl acetate (2 mL), and 4M hydrogen chloride in ethyl acetate solution (4 mL) was added. After reacting at room temperature for 2 hours, the mixture was concentrated under reduced pressure to obtain the target product (*S*)-3-(trifluoromethyl)-7,7*a*,8,9,10,11-hexahydro-5*H*-pyrazino[2,1-c]pyrido[2,3-e][1,4]oxazepine hydrochloride (I-13, 110 mg, crude). ESI $[M+H]^+$=274.4

**Preparation of Intermediate I-14: 2-(piperazin-1-yl)-5-(trifluoromethyl)nicotinonitrile hydrochloride (I-14)**

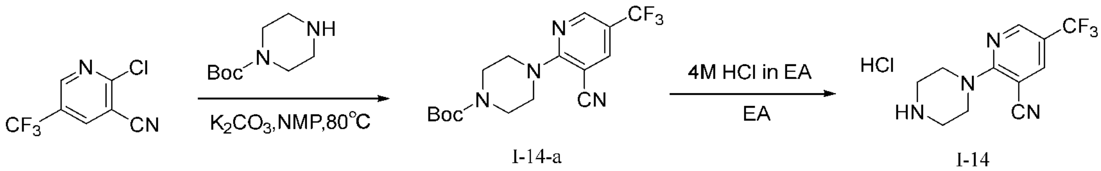

I-14-a

I-14

**Step 1: tert-butyl 4-(3-cyano-5-(trifluoromethyl)pyridin-2-yl)piperazine-1-carboxylate (I-14-a)**

2-chloro-5-(trifluoromethyl)nicotinonitrile (200 mg, 0.968 mmol) and tert-butyl piperazine-1-carboxylate (178.6 mg, 0.959 mmol) were dissolved in *N*-methylpyrrolidone (3 mL), and potassium carbonate (199 mg, 1.44 mmol) was added. The mixture was reacted at 80°C for 3 hours. Water (10 mL) was added dropwise to the reaction mixture, and a

white solid precipitated. The mixture was filtered, the filter cake was washed with water, and the collected filter cake was concentrated under reduced pressure to obtain the target product tert-butyl 4-(3-cyano-5-(trifluoromethyl)pyridin-2-yl) piperazine-1-carboxylate (I-14-a, 350 mg, yield 97.6%). ESI $[M-100+H]^+$=257.4, ESI $[M-56+H]^+$=301.5

**Step 2: Preparation of 2-(piperazin-1-yl)-5-(trifluoromethyl)nicotinonitrile hydrochloride (I-14)**

tert-butyl 4-(3-cyano-5-(trifluoromethyl)pyridin-2-yl)piperazine-1-carboxylate (I-14-a, 350 mg, 0.983 mmol) was dissolved in ethyl acetate (3 mL), and 4M hydrogen chloride in ethyl acetate solution (4 mL) was added. After reacting at room temperature for 4 hours, the mixture was concentrated under reduced pressure to obtain the target product 2-(piperazin-1-yl)-5-(trifluoromethyl)nicotinonitrile hydrochloride (I-14, 310 mg, crude). ESI $[M+H]^+$=257.2

**Preparation of Intermediate I-15: 1-(3-methoxy-5-(trifluoromethyl)pyridin-2-yl)piperazine hydrochloride (I-15)**

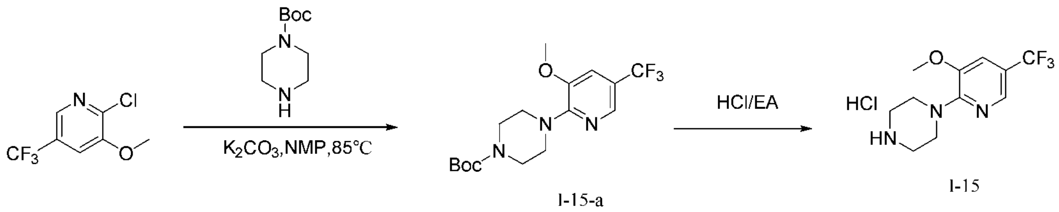


I-15-a

I-15

**Step 1: Preparation of tert-butyl 4-(3-methoxy-5-(trifluoromethyl)pyridin-2-yl)piperazine-1-carboxylate (I-15-a)**

tert-butyl piperazine-1-carboxylate (301 mg, 1.62 mmol) and 2-chloro-3-methoxy-5-(trifluoromethyl)pyridine (156 mg, 0.737 mmol) were dissolved in N-methylpyrrolidone (4 mL), and potassium carbonate (153 mg, 1.11 mmol) was added. The mixture was reacted at 85°C for 4 hours in a sealed tube. After cooling, the reaction mixture was quenched with water and extracted with ethyl acetate. The organic phase was dried over anhydrous sodium sulfate and filtered. The filtrate was concentrated under reduced pressure and purified by Flash chromatography (10g silica gel column, petroleum ether:ethyl acetate = 10:1) to obtain the target product tert-butyl 4-(3-methoxy-5-(trifluoromethyl)pyridin-2-yl)piperazine-1-carboxylate (I-15-a, 153 mg, yield 57.42%). ESI $[M+H]^+$=362.6

**Step 2: 1-(3-methoxy-5-(trifluoromethyl)pyridin-2-yl)piperazine hydrochloride (I-15)**

To tert-butyl 4-(3-methoxy-5-(trifluoromethyl)pyridin-2-yl)piperazine-1-carboxylate (I-15-a, 153 mg, 0.423 mmol) was added 4M hydrogen chloride in ethyl acetate solution (3 mL). After reacting at room temperature for 3 hours, the reaction mixture was diluted with ethyl acetate several times and then concentrated under reduced pressure to obtain the crude target product 1-(3-methoxy-5-(trifluoromethyl)pyridin-2-yl)piperazine hydrochloride (I-15, 114 mg, crude). ESI $[M+H]^+$=262.5

**Preparation of Intermediate I-16: 6-(1-bromoethyl)-4-(trifluoromethyl)pyridazin-3(2*H*)-one (I-16)**

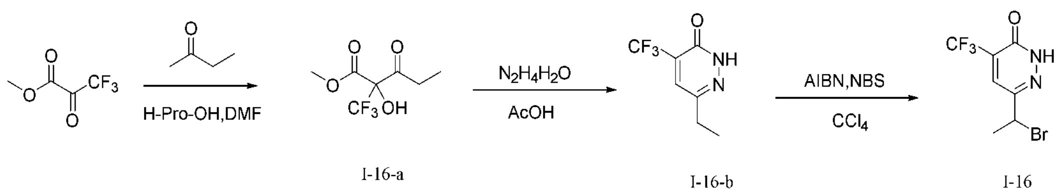


I-16-a

I-16-b

I-16

**Step 1: Preparation of methyl 2-hydroxy-3-oxo-2-(trifluoromethyl)pentanoate (I-16-a)**

Methyl 3,3,3-trifluoro-2-oxopropanoate (5 g, 32.0 mmol) and butan-2-one (11.5 mL, 129 mmol) were dissolved in *N*,*N*-dimethylformamide (10 mL), and L-proline (1.1 g, 12.8 mmol) was added. After reacting at room temperature for 48 hours, the reaction was quenched with water (20 mL) and extracted with ethyl acetate. The organic phase was dried over anhydrous sodium sulfate and filtered. The filtrate was concentrated under reduced pressure to obtain the target product

methyl 2-hydroxy-3-oxo-2-(trifluoromethyl)pentanoate (I-16-a, 7 g, yield 95%).

**Step 2: Preparation of 6-ethyl-4-(trifluoromethyl)pyridazin-3(2*H*)-one (I-16-b)**

Methyl 2-hydroxy-3-oxo-2-(trifluoromethyl)pentanoate (I-16-a, 7 g, 30.7 mmol) was dissolved in acetic acid (20 mL), and hydrazine hydrate (3 mL, 256 mmol) was added. The mixture was reacted at 110°C overnight. The reaction mixture was concentrated under reduced pressure and purified by normal phase column chromatography (petroleum ether:ethyl acetate = 3:1) to obtain the target product 6-ethyl-4-(trifluoromethyl)pyridazin-3(2H)-one (I-16-b, 2.2 g, yield 37.3%). ESI $[M+H]^+$=193.4

Step 3: **Preparation of 6-(1-bromoethyl)-4-(trifluoromethyl)pyridazin-3(2*H*)-one (I-16)**

6-ethyl-4-(trifluoromethyl)pyridazin-3(2H)-one (I-16-b, 1 g, 5.21 mmol) was dissolved in carbon tetrachloride (10 mL), and N-bromosuccinimide (927 mg, 5.21 mmol) and azobisisobutyronitrile (85.5 mg, 0.521 mmol) were added. The mixture was reacted at 80°C overnight. The reaction mixture was concentrated under reduced pressure and purified by normal phase column chromatography (petroleum ether:ethyl acetate = 3:1) to obtain the target product 6-(1-bromoethyl)-4-(trifluoromethyl)pyridazin-3(2H)-one (I-16, 1.1 g, yield 70.1%). ESI $[M+H]^+$=298.3, 300.3

**Preparation of Intermediate 1-17: 6-(2-bromopropan-2-yl)-4-(trifluoromethyl)pyridazin-3(2*H*)-one (1-17)**

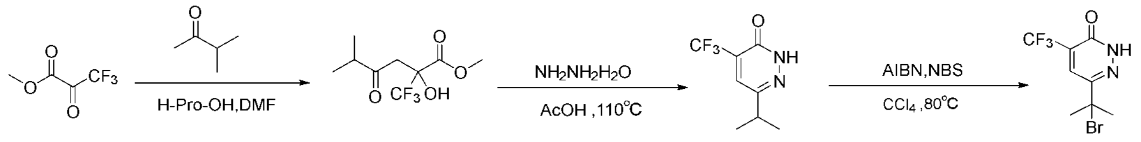


I-17-a I-17-b I-17

**Step 1: Preparation of methyl 2-hydroxy-5-methyl-4-oxo-2-(trifluoromethyl)hexanoate (I-17-a)**

Methyl 3,3,3-trifluoro-2-oxopropanoate (3 g, 0.019 mmol) and 3-methylbutan-2-one (6.6 g, 0.076 mmol) were dissolved in *N*,*N*-dimethylformamide (10 mL), then L-proline (0.66 g, 0.0058 mmol) was added, and the reaction was stirred at room temperature for 3 minutes, then reacted at 40°C overnight. After the reaction was complete, it was cooled to room temperature, the reaction was quenched with water, and then extracted with ethyl acetate. The organic phase was collected and washed repeatedly with saturated sodium chloride solution, dried over anhydrous sodium sulfate, filtered, and concentrated under reduced pressure to obtain the target product methyl 2-hydroxy-5-methyl-4-oxo-2-(trifluoromethyl)hexanoate (I-17-a, 3 g, crude).

**Step 2: Preparation of 6-isopropyl-4-(trifluoromethyl)pyridazin-3(2*H*)-one (I-17-b)**

Methyl 2-hydroxy-5-methyl-4-oxo-2-(trifluoromethyl)hexanoate (I-17-a, 3 g, 0.012 mmol) was dissolved in ethanol (10 mL), and hydrazine hydrate (1.2 mL, 0.024 mmol) was added with stirring. The tube was sealed and heated to 110°C to react overnight. After the reaction was complete, it was quenched with water and extracted with ethyl acetate. The organic phase was collected and washed repeatedly with saturated sodium chloride solution, dried over anhydrous sodium sulfate, filtered, and concentrated under reduced pressure, followed by purification by Flash column chromatography (petroleum ether:ethyl acetate = 3:1) to obtain the target product 6-isopropyl-4-(trifluoromethyl)pyridazin-3(2H)-one (I-17-b, 250 mg, yield 9.8%). ESI $[M+H]^+$=207.03

**Step 3: Preparation of 6-(2-bromopropan-2-yl)-4-(trifluoromethyl)pyridazin-3(2*H*)-one (I-17)**

6-isopropyl-4-(trifluoromethyl)pyridazin-3(2H)-one (I-17-b, 150 mg, 0.728 mmol) was dissolved in carbon tetrachloride (6 mL), and N-bromosuccinimide (140 mg, 0.80 mmol) and azobisisobutyronitrile (12 mg, 0.072 mmol) were added. The tube was sealed and heated to 80°C to react overnight. After the reaction was complete, it was cooled to room temperature. The filtrate was concentrated under reduced pressure and purified by Flash column chromatography (petroleum ether:ethyl acetate = 3:1) to obtain the target product 6-(2-bromopropan-2-yl)-4-(trifluoromethyl)pyridazin-3(2H)-one (I-17, 70 mg, yield 25.4%). ESI $[M+H]^+$=285.2, 287.2

**Preparation of Intermediate 1-18: 6-(bromomethyl)-5-(trifluoromethyl)pyridazin-3(2H)-one (I-18)**

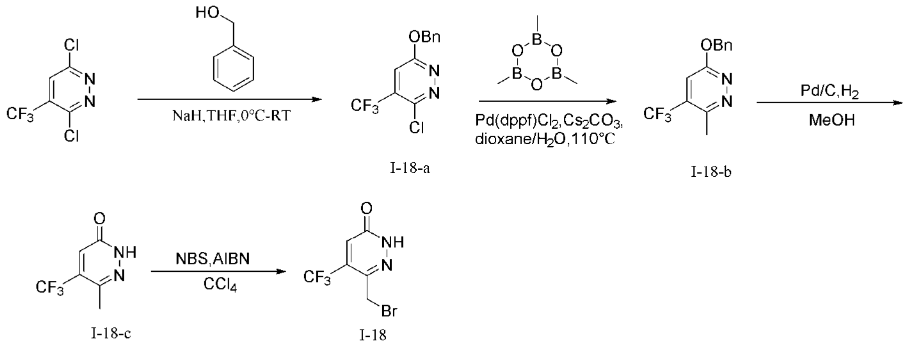


I-18-a I-18-b I-18-c I-18

**Step 1: Preparation of 6-(benzyloxy)-3-chloro-4-(trifluoromethyl)pyridazine (I-18-a)**

Benzyl alcohol (262 mg, 2.42 mmol) was dissolved in tetrahydrofuran (10 mL), placed in an ice bath, and 60% sodium hydride (138 mg, 3.46 mmol) was added under a nitrogen atmosphere and stirred for 15 minutes. Then, 3,6-dichloro-4-(trifluoromethyl)pyridazine (500 mg, 2.3 mmol) was added, and the mixture was warmed to room temperature and reacted for 4 hours. The reaction mixture was quenched with water and extracted with ethyl acetate. The organic phase was collected and washed repeatedly with saturated sodium chloride solution, dried over anhydrous sodium sulfate, filtered, and concentrated under reduced pressure, followed by purification by Flash column chromatography (ethyl acetate:petroleum ether = 0~10%) to obtain the target product 6-(benzyloxy)-3-chloro-4-(trifluoromethyl)pyridazine (I-18-a, 597 mg, yield 89.75%).

**Step 2: Preparation of 6-(benzyloxy)-3-methyl-4-(trifluoromethyl)pyridazine (I-18-b)**

Under a nitrogen atmosphere, 6-(benzyloxy)-3-chloro-4-(trifluoromethyl)pyridazine (I-18-a, 547 mg, 1.9 mmol), 2,4,6-trimethyl-1,3,5,2,4,6-trimethoxytriborane (360 mg, 2.84 mmol), cesium carbonate (1.85 g, 5.68 mmol), and [1,1'-bis(diphenylphosphino)ferrocene]palladium(II) dichloride (69 mg, 0.095 mmol) were dissolved in dioxane (21 mL) and water (3 mL), and then heated to 110°C and reacted for 5 hours. After the reaction was complete, it was quenched with water and extracted with ethyl acetate. The organic phase was collected and washed repeatedly with saturated sodium chloride solution, dried over anhydrous sodium sulfate, filtered, and concentrated under reduced pressure, followed by purification by Flash column chromatography (ethyl acetate:petroleum ether = 0~10%) to obtain the target product 6-(benzyloxy)-3-methyl-4-(trifluoromethyl)pyridazine (I-18-b, 300 mg, yield 59.06%). ESI $[M+H]^+$=269.0

**Step 3: Preparation of 6-methyl-5-(trifluoromethyl)pyridazin-3(2*H*)-one (I-18-c)**

6-(benzyloxy)-3-methyl-4-(trifluoromethyl)pyridazine (I-18-b, 300 mg, 1.12 mmol) was dissolved in methanol (5 mL). After purging with nitrogen, 5% palladium on carbon (20 mg) was added, and the atmosphere was replaced with hydrogen three times. The reaction mixture was reacted at room temperature under a hydrogen atmosphere for 2 hours. After the reaction was complete, it was filtered. The filtrate was concentrated under reduced pressure and purified by preparative thin-layer chromatography (petroleum ether:ethyl acetate = 1:1) to obtain the target product 6-methyl-5-(trifluoromethyl)pyridazin-3(2*H*)-one (I-18-c, 195 mg, yield 97.99%). ESI $[M+H]^+$=179.1

**Step 4: Preparation of 6-(bromomethyl)-5-(trifluoromethyl)pyridazin-3(2H)-one (I-18)**

6-methyl-5-(trifluoromethyl)pyridazin-3(2*H*)-one (I-18-c, 195 mg, 1.095 mmol) was dissolved in carbon tetrachloride (10 mL), and *N*-bromosuccinimide (877 mg, 4.928 mmol) and azobisisobutyronitrile (54 mg, 0.329 mmol) were added. The tube was sealed and heated to 85°C to react overnight. After the reaction was complete, it was cooled to room temperature. The filtrate was concentrated under reduced pressure and purified by Flash column chromatography (ethyl acetate:petroleum ether = 0-36%) to obtain the target product 6-(bromomethyl)-5-(trifluoromethyl)pyridazin-3(2H)-one (I-18, 59 mg, yield 21%). ESI $[M+H]^+$=256.8, 258.8

**Preparation of Intermediate I-19: 6-(bromomethyl)-5-cyclobutoxypyridazin-3(2*H*)-one (I-19)**

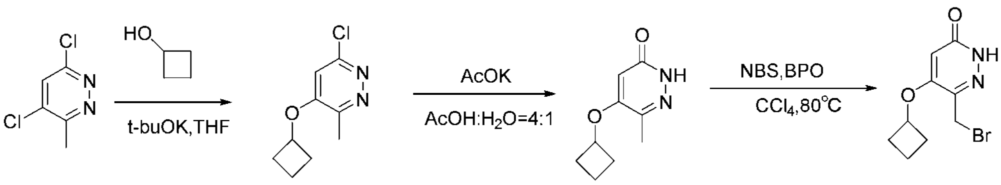


I-19-a I-19-b I-19

**Step 1: Preparation of 6-chloro-4-cyclobutoxy-3-methylpyridazine (I-19-a)**

4,6-dichloro-3-methylpyridazine (500 mg, 3.07 mmol) was dissolved in tetrahydrofuran (15 mL), and potassium tert-butoxide (517 mg, 4.61 mmol) and cyclobutanol (265 μL, 3.37 mmol) were added. After reacting at room temperature for 2 hours, the reaction was quenched with water and extracted with ethyl acetate. The organic phase was dried over anhydrous sodium sulfate and filtered. The filtrate was concentrated under reduced pressure, and the crude product was purified by normal phase column chromatography (petroleum ether:ethyl acetate = 3:1) to obtain the target product 6-chloro-4-cyclobutoxy-3-methylpyridazine (I-19-a, 340 mg, yield 55.9%). ESI $[M+H]^+$=199.1, 201.1

**Step 2: Preparation of 5-cyclobutoxy-6-methylpyridazin-3(2*H*)-one (I-19-b)**

6-chloro-4-cyclobutoxy-3-methylpyridazine (I-19-a, 340 mg, 1.51 mmol) was dissolved in acetic acid (8 mL), and water (2 mL) and potassium acetate (257.4 mg, 3.03 mmol) were added. The mixture was reacted at 100°C for 5 hours. The mixture was concentrated under reduced pressure, and the crude product was purified by normal phase column chromatography (petroleum ether:ethyl acetate = 3:1) to obtain the target product 5-cyclobutoxy-6-methylpyridazin-3(2*H*)-one (I-19-b, 257 mg, yield 83.2%). ESI $[M+H]^+$=361.1

**Step 3: Preparation of 6-(bromomethyl)-5-cyclobutoxypyridazin-3(2*H*)-one (1-19)**

5-cyclobutoxy-6-methylpyridazin-3(2H)-one (I-19-b, 120 mg, 0.666 mmol) was dissolved in carbon tetrachloride (2 mL), and *N*-bromosuccinimide (118.6 mg, 0.666 mmol) and dibenzoyl peroxide (16 mg, 0.067 mmol) were added. The mixture was reacted at 80°C overnight. The mixture was concentrated under reduced pressure, and the crude product was purified by normal phase column chromatography (petroleum ether:ethyl acetate = 1:1) to obtain the target product 6-(bromomethyl)-5-cyclobutoxypyridazin-3(2*H*)-one (I-19, 62 mg, yield 36.1%). ESI $[M+H]^+$=259.2, 261.2

**Preparation of Intermediate 1-20: 1-(3-ethoxy-5-(trifluoromethyl)pyridin-2-yl)piperazine hydrochloride (I-20)**

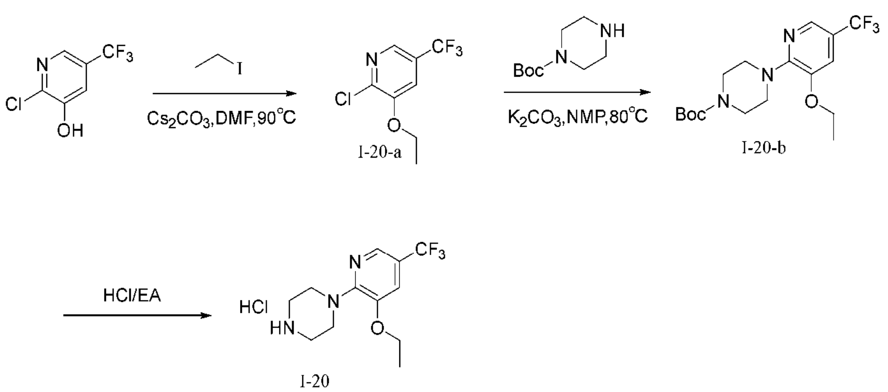


I-20-a I-20-b

I-20

**Step 1: Preparation of 2-chloro-3-ethoxy-5-(trifluoromethyl)pyridine (I-20-a)**

2-chloro-5-(trifluoromethyl)pyridin-3-ol (250 mg, 1.27 mmol) and iodoethane (203 μL, 2.53 mmol) were dissolved in *N*,*N*-dimethylformamide (15 mL), and cesium carbonate (619 mg, 1.89 mmol) was added. The mixture was reacted at 90°C for 1 hour in a sealed tube. After cooling, the reaction mixture was quenched with water and extracted with ethyl acetate. The organic phase was dried over anhydrous sodium sulfate and filtered. The filtrate was concentrated under reduced pressure and purified by Flash chromatography (10g silica gel column, petroleum ether:ethyl acetate = 6:1) to obtain the target product 2-chloro-3-ethoxy-5-(trifluoromethyl)pyridine (I-20-a, 220 mg, yield 77.04%). ESI $[M+H]^+$=225.9

**Step 2: Preparation of tert-butyl 4-(3-ethoxy-5-(trifluoromethyl)pyridin-2-yl)piperazine-1-carboxylate (I-20-b)**

tert-butyl piperazine-1-carboxylate (218 mg, 1.17 mmol) and 2-chloro-3-ethoxy-5-(trifluoromethyl)pyridine (I-20-a, 220 mg, 0.98 mmol) were dissolved in *N*-methylpyrrolidone (8 mL), and potassium carbonate (203 mg, 1.47 mmol) was added. The mixture was reacted at 80°C overnight in a sealed tube. After cooling, the reaction mixture was quenched with water and extracted with ethyl acetate. The organic phase was dried over anhydrous sodium sulfate and filtered. The filtrate was concentrated under reduced pressure and purified by Flash chromatography (10g silica gel column, petroleum ether:ethyl acetate = 6:1) to obtain the target product tert-butyl 4-(3-ethoxy-5-(trifluoromethyl)pyridin-2-yl)piperazine-1-carboxylate (I-20-b, 120 mg, yield 32.69%). ESI $[M+H]^+$=366.0

**Step 3: Preparation of 1-(3-ethoxy-5-(trifluoromethyl)pyridin-2-yl)piperazine hydrochloride (I-20)**

tert-butyl 4-(3-ethoxy-5-(trifluoromethyl)pyridin-2-yl)piperazine-1-carboxylate (I-20-b, 115 mg, 0.31 mmol) was dissolved in 4M hydrogen chloride in ethyl acetate (3 mL) and reacted at room temperature for 2 hours, then concentrated under reduced pressure to obtain the target product 1-(3-ethoxy-5-(trifluoromethyl)pyridin-2-yl)piperazine hydrochloride (I-20, 110 mg, yield 115.79%). ESI $[M+H]^+$=276.0

**Preparation of Intermediate I-21: 1-(3,5-bis(trifluoromethyl)pyridin-2-yl)piperazine hydrochloride (I-21)**

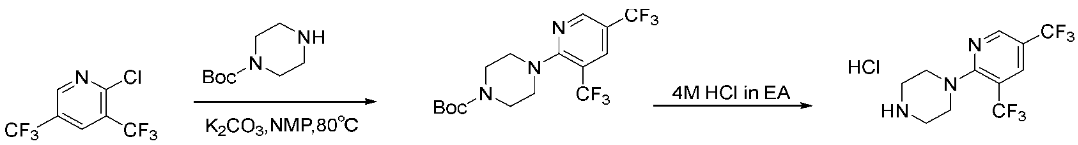

I-21-a I-21

**Step 1: Preparation of tert-butyl 4-(3,5-bis(trifluoromethyl)pyridin-2-yl)piperazine-1-carboxylate (I-21-a)**

2-chloro-3,5-bis(trifluoromethyl)pyridine (100 mg, 0.359 mmol) and tert-butyl piperazine-1-carboxylate (73.6 mg, 0.355 mmol) were dissolved in N-methylpyrrolidone (2 mL), and potassium carbonate (92.3 mg, 0.533 mmol) was added. The mixture was reacted at 80°C for 3 hours. Water (10 mL) was added dropwise to the reaction mixture, and a white solid precipitated. The mixture was filtered, the filter cake was washed with water, and the collected filter cake was concentrated under reduced pressure to obtain the target product tert-butyl 4-(3,5-bis(trifluoromethyl)pyridin-2-yl)piperazine-1-carboxylate (I-21-a, 155 mg, yield 96.9%).

**Step 2: Preparation of 1-(3,5-bis(trifluoromethyl)pyridin-2-yl)piperazine hydrochloride (I-21)**

tert-butyl 4-(3,5-bis(trifluoromethyl)pyridin-2-yl)piperazine-1-carboxylate (I-21-a, 155 mg, 0.388 mmol) was dissolved in ethyl acetate (3 mL), and 4M hydrogen chloride in ethyl acetate solution (4 mL) was added. After reacting at room temperature for 4 hours, the mixture was concentrated under reduced pressure to obtain the target product 1-(3,5-bis(trifluoromethyl)pyridin-2-yl)piperazine hydrochloride (I-21, 150 mg, crude product). ESI $[M+H]^+$=300.1

**Preparation of Intermediate 1-22: 1-(3-methyl-5-(trifluoromethyl)pyridin-2-yl)piperazine (I-22)**

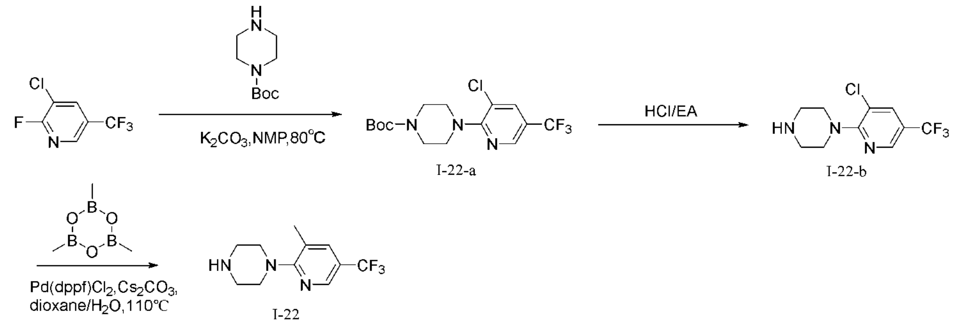


I-22-a

I-22-b

I-22

**Step 1: Preparation of tert-butyl 4-(3-chloro-5-(trifluoromethyl)pyridin-2-yl)piperazine-1-carboxylate (I-22-a)**

3-chloro-2-fluoro-5-(trifluoromethyl)pyridine (300 mg, 1.5 mmol) was dissolved in *N*-methylpyrrolidone (10 mL), and tert-butyl piperazine-1-carboxylate (336 mg, 1.8 mmol) and potassium carbonate (415.6 mg, 3.01 mmol) were added. The mixture was reacted in an oil bath at 80°C overnight. After the reaction was complete, the filtrate was concentrated under reduced pressure and purified by Flash column chromatography (petroleum ether:ethyl acetate = 4:1) to obtain the target product tert-butyl 4-(3-chloro-5-(trifluoromethyl)pyridin-2-yl)piperazine-1-carboxylate (I-22-a, 450 mg, yield 82%). ESI $[M+H]^+$=366.0, 368.0

**Step 2: Preparation of 1-(3-chloro-5-(trifluoromethyl)pyridin-2-yl)piperazine (I-22-b)**

tert-butyl 4-(3-chloro-5-(trifluoromethyl)pyridin-2-yl)piperazine-1-carboxylate (I-22-a, 450 mg) was dissolved in ethyl acetate solution (4 mL), and hydrogen chloride in ethyl acetate solution (4 mL) was added. The mixture was reacted at room temperature overnight. After the reaction was complete, the reaction mixture was concentrated under reduced pressure, then dissolved in dichloromethane and concentrated under reduced pressure again, repeating twice, to obtain the target product 1-(3-chloro-5-(trifluoromethyl)pyridin-2-yl)piperazine (I-22-b, 420 mg, crude product). ESI $[M+H]^+$=266.0, 268.0

**Step 3: Preparation of 1-(3-methyl-5-(trifluoromethyl)pyridin-2-yl)piperazine (I-22)**

1-(3-chloro-5-(trifluoromethyl)pyridin-2-yl)piperazine (I-22-b, 340 mg, 1.28 mmol), 2,4,6-trimethyl-1,3,5,2,4,6-trimethoxytriborane (193 mg, 1.54 mmol), cesium carbonate (836 mg, 2.57 mmol), and [1,1'-bis(diphenylphosphino) ferrocene]palladium(II) dichloride (16 mg, 0.022 mmol) were dissolved in dioxane (7 mL) and water (1 mL), and reacted in a sealed reaction flask under a nitrogen atmosphere in an oil bath at 110°C overnight. After the reaction was complete, the reaction mixture was poured into water and extracted three times with ethyl acetate. The organic phases were collected and washed repeatedly with saturated sodium chloride solution, dried over anhydrous sodium sulfate, filtered, and concentrated under reduced pressure to obtain the target product 1-(3-methyl-5-(trifluoromethyl)pyridin-2-yl)piperazine (I-22, 390 mg, crude product). ESI $[M+H]^+$=246.3

**Preparation of Intermediate 1-23: 1-(3-cyclopropyl-5-(trifluoromethyl)pyridin-2-yl)piperazine (I-23)**

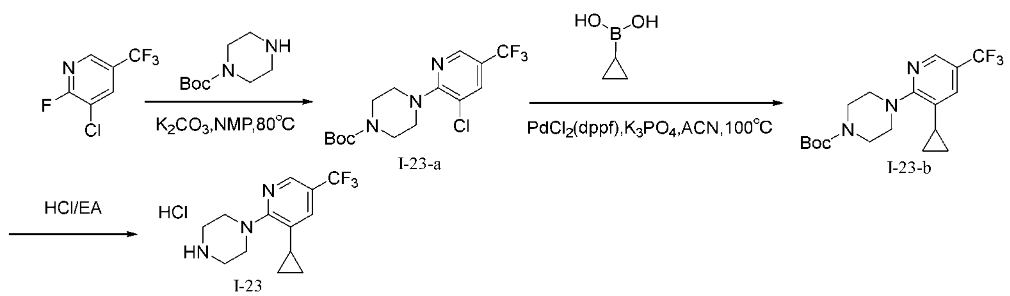


I-23-a

I-23-b

I-23

**Step 1: Preparation of tert-butyl 4-(3-chloro-5-(trifluoromethyl)pyridin-2-yl)piperazine-1-carboxylate (I-23-a)**

tert-butyl piperazine-1-carboxylate (448 mg, 2.41 mmol) and 3-chloro-2-fluoro-5-(trifluoromethyl)pyridine (400

mg, 2.01 mmol) were dissolved in *N*-methylpyrrolidone (10 mL), and potassium carbonate (416 mg, 3.01 mmol) was added. The mixture was reacted in a sealed tube at 80°C for 2 hours. After cooling, the reaction mixture was quenched with water and extracted with ethyl acetate. The organic phase was dried over anhydrous sodium sulfate and filtered. The filtrate was concentrated under reduced pressure and purified by Flash chromatography (25g silica gel column, petroleum ether:ethyl acetate = 8:1) to obtain the target product tert-butyl 4-(3-chloro-5-(trifluoromethyl)pyridin-2-yl)piperazine-1-carboxylate (I-23-a, 600 mg, yield 82.02%). ESI $[M+H]^+$=366.0

**Step 2: Preparation of tert-butyl 4-(3-cyclopropyl-5-(trifluoromethyl)pyridin-2-yl)piperazine-1-carboxylate (I-23-b)**

tert-butyl 4-(3-chloro-5-(trifluoromethyl)pyridin-2-yl)piperazine-1-carboxylate (I-23-a, 530 mg, 1.45 mmol) was dissolved in acetonitrile (15 mL), and cyclopropylboronic acid (166 mg, 1.93 mmol), [1,1'-bis(diphenylphosphino) ferrocene]palladium(II) dichloride (53 mg, 0.07 mmol), and potassium phosphate (923 mg, 4.35 mmol) were added. The mixture was reacted under microwave irradiation at 100°C for 80 minutes under a nitrogen atmosphere. After concentration under reduced pressure, the crude product was purified by Flash chromatography (25g silica gel column, petroleum ether:ethyl acetate = 5:1) to obtain the target product tert-butyl 4-(3-cyclopropyl-5-(trifluoromethyl)pyridin-2-yl) piperazine-1-carboxylate (I-23-b, 290 mg, yield 47.62%). ESI $[M+H]^+$ =372.2

**Step 3: Preparation of 1-(3-cyclopropyl-5-(trifluoromethyl)pyridin-2-yl)piperazine (I-23)**

tert-butyl 4-(3-cyclopropyl-5-(trifluoromethyl)pyridin-2-yl)piperazine-1-carboxylate (I-23-b, 290 mg, 0.78 mmol) was dissolved in 4M hydrogen chloride in ethyl acetate (4 mL) and reacted at room temperature for 2 hours, then concentrated under reduced pressure. Water (1 mL) was added, and the pH was adjusted to 8 with $NaHCO_3$. The mixture was extracted with (dichloromethane:methanol = 10:1), the organic phase was dried over anhydrous sodium sulfate and filtered, and the filtrate was concentrated under reduced pressure to obtain the target product 1-(3-cyclopropyl-5-(trifluoromethyl)pyridin-2-yl)piperazine hydrochloride (I-23, 310 mg, yield 129.17%). ESI $[M+H]^+$=272.6

**Preparation of Intermediate 1-24: 1-(4-methoxy-5-(trifluoromethyl)pyridin-2-yl)piperazine hydrochloride (I-24)**

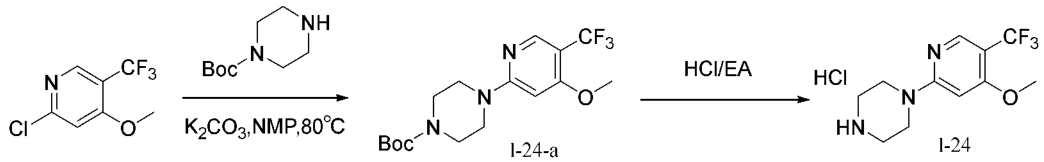


**Step 1: Preparation of tert-butyl 4-(4-methoxy-5-(trifluoromethyl)pyridin-2-yl)piperazine-1-carboxylate (I-24-a)**

tert-butyl piperazine-1-carboxylate (158 mg, 0.85 mmol) and 2-chloro-4-methoxy-5-(trifluoromethyl)pyridine (150 mg, 0.71 mmol) were dissolved in *N*-methylpyrrolidone (5 mL), and potassium carbonate (147 mg, 1.06 mmol) was added. The mixture was reacted in a sealed tube at 80°C overnight. After cooling, the reaction mixture was quenched with water and extracted with ethyl acetate. The organic phase was dried over anhydrous sodium sulfate and filtered. The filtrate was concentrated under reduced pressure and purified by Flash chromatography (10g silica gel column, petroleum ether:ethyl acetate = 8:1) to obtain the target product tert-butyl 4-(4-methoxy-5-(trifluoromethyl)pyridin-2-yl)piperazine-1-carboxylate (I-24-a, 140 mg, yield 54.64%). ESI $[M+H]^+$=362.0

**Step 2: Preparation of 1-(4-methoxy-5-(trifluoromethyl)pyridin-2-yl)piperazine hydrochloride (I-24)**

tert-butyl 4-(4-methoxy-5-(trifluoromethyl)pyridin-2-yl)piperazine-1-carboxylate (I-24-a, 140 mg, 0.39 mmol) was dissolved in 4M hydrogen chloride in ethyl acetate (2 mL) and reacted at room temperature for 2 hours, then concentrated under reduced pressure. Water (1 mL) was added, and the pH was adjusted to 8 with $NaHCO_3$. The mixture was extracted with (dichloromethane:methanol = 10:1), the organic phase was dried over anhydrous sodium sulfate and filtered, and the filtrate was concentrated under reduced pressure to obtain the target product 1-(4-methoxy-5-(trifluoromethyl)pyridin-2-yl)piperazine hydrochloride (I-24, 144 mg, yield 125.22%). ESI $[M+H]^+$=262.0

**Preparation of Intermediate 1-25: *N*-(2-(piperazin-1-yl)-5-(trifluoromethyl)pyridin-3-yl)acetamide (I-25)**

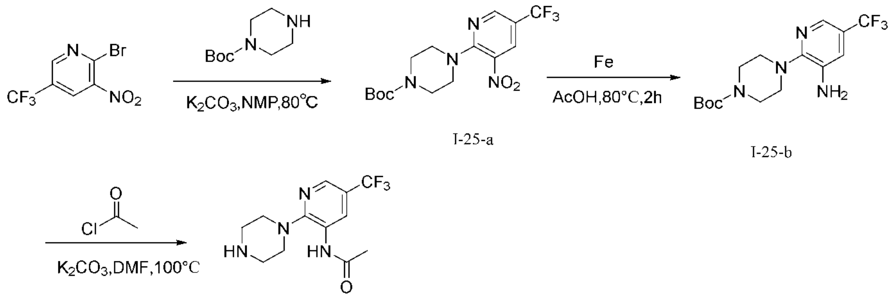


I-25-a I-25-b I-25

**Step 1: Preparation of tert-butyl 4-(3-nitro-5-(trifluoromethyl)pyridin-2-yl)piperazine-1-carboxylate (I-25-a)**

2-bromo-3-nitro-5-(trifluoromethyl)pyridine (100 mg, 0.369 mmol) and tert-butyl piperazine-1-carboxylate (76 mg, 0.406 mmol) were dissolved in *N*-methylpyrrolidone (3 mL), and potassium carbonate (153 mg, 1.107 mmol) was added. The mixture was reacted at 80°C for 1 hour. The reaction was quenched by adding water and extracted with ethyl acetate. The organic phases were combined, washed with brine, dried over anhydrous sodium sulfate, filtered, and the filtrate was concentrated under reduced pressure to obtain the target product tert-butyl 4-(3-nitro-5-(trifluoromethyl) pyridin-2-yl)piperazine-1-carboxylate (I-25-a, 120 mg, yield 86.41%). ESI $[M+H-100]^+$=277.0, ESI $[M+H-56]^+$=321.3

**Step 2: Preparation of tert-butyl 4-(3-amino-5-(trifluoromethyl)pyridin-2-yl)piperazine-1-carboxylate (I-25-b)**

tert-butyl 4-(3-nitro-5-(trifluoromethyl)pyridin-2-yl)piperazine-1-carboxylate (I-25-a, 120 mg, 0.319 mmol) and iron powder (356 mg, 6.377 mmol) were added to acetic acid (4 mL) and reacted at 80°C for 2 hours. After cooling, the reaction mixture was filtered, and the filtrate was concentrated under reduced pressure to obtain the target product tert-butyl 4-(3-amino-5-(trifluoromethyl)pyridin-2-yl)piperazine-1-carboxylate (I-25-b, 110 mg, yield 99.61%). ESI $[M+H]^+$=347.9

**Step 3: Preparation of N-(2-(piperazin-1-yl)-5-(trifluoromethyl)pyridin-3-yl)acetamide (I-25)**

tert-butyl 4-(3-amino-5-(trifluoromethyl)pyridin-2-yl)piperazine-1-carboxylate (I-25-b, 110 mg, 0.318 mmol) and potassium carbonate (132 mg, 0.953 mmol) were added to *N*,*N*-dimethylformamide (3 mL). Acetyl chloride (37 mg, 0.476 mmol) was added dropwise under a nitrogen atmosphere, and the mixture was heated to 100°C and reacted for 2 hours. After cooling, the reaction was quenched with water and extracted with ethyl acetate. The organic phases were combined, washed with brine, dried over anhydrous sodium sulfate, filtered, and the filtrate was concentrated under reduced pressure to obtain the target product *N*-(2-(piperazin-1-yl)-5-(trifluoromethyl)pyridin-3-yl)acetamide (I-25, 400 mg, purity 25%, yield 109.23%). ESI $[M+H]^+$=289.1

**Preparation of Intermediate I-26: 2-(piperazin-1-yl)-5-(trifluoromethyl)nicotinamide hydrochloride (I-26)**

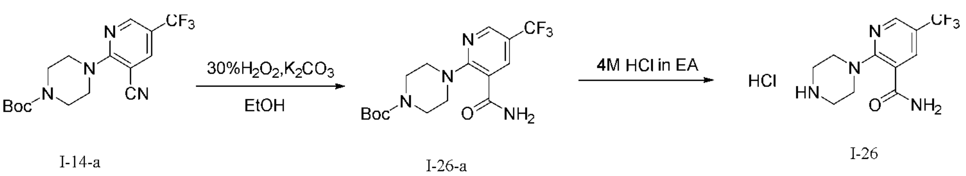


I-14-a I-26-a I-26

**Step 1: Preparation of tert-butyl 4-(3-carbamoyl-5-(trifluoromethyl)pyridin-2-yl)piperazine-1-carboxylate (I-26-a)**

tert-butyl 4-(3-cyano-5-(trifluoromethyl)pyridin-2-yl)piperazine-1-carboxylate (I-14-a, 550 mg, 1.54 mmol) was dissolved in ethanol (15 mL), and water (5 mL), potassium carbonate (640 mg, 4.63 mmol), and 30% hydrogen peroxide

(3.3 mL) were added. After reacting at room temperature for 5 hours, the solvent was removed by concentration under reduced pressure. Water (15 mL) was added, and the mixture was extracted with ethyl acetate. The organic phase was dried over anhydrous sodium sulfate and filtered. The filtrate was concentrated under reduced pressure, and the crude product was purified by normal phase column chromatography (petroleum ether:ethyl acetate = 3:1) to obtain the target product tert-butyl 4-(3-carbamoyl-5-(trifluoromethyl)pyridin-2-yl)piperazine-1-carboxylate (I-26-a, 256 mg, yield 44.3%). ESI $[M+H]^+$=375.2

**Step 2: Preparation of 2-(piperazin-1-yl)-5-(trifluoromethyl)nicotinamide hydrochloride (I-26)**

tert-butyl 4-(3-carbamoyl-5-(trifluoromethyl)pyridin-2-yl)piperazine-1-carboxylate (I-26-a, 256 mg, 0.684 mmol) was dissolved in ethyl acetate (4 mL), and 4M hydrogen chloride in ethyl acetate solution (4 mL) was added. After reacting at room temperature for 3 hours, the mixture was concentrated under reduced pressure to obtain the target product 2-(piperazin-1-yl)-5-(trifluoromethyl)nicotinamide hydrochloride (I-26, 194 mg, yield 91.5%). ESI $[M+H]^+$=275.1

**Preparation of Intermediate 1-27: 6-(2-chloropropan-2-yl)-4-(trifluoromethyl)pyridazin-3(2H)-one (I-27)**

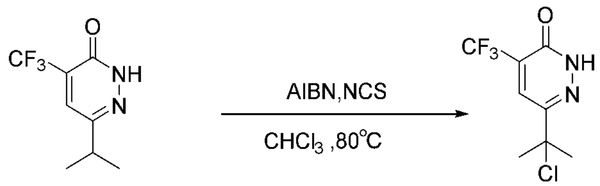


I-17-b I-27

6-isopropyl-4-(trifluoromethyl)pyridazin-3(2*H*)-one (I-17-b, 2.28 g, 11.06 mmol) was dissolved in chloroform (70 mL), and *N*-chlorosuccinimide (2.22 g, 16.59 mmol) and azobisisobutyronitrile (1.82 g, 11.06 mmol) were added. The tube was sealed and heated to 80°C to react overnight. After the reaction was complete, the mixture was cooled to room temperature, and the filtrate was concentrated under reduced pressure and purified by Flash column chromatography (petroleum ether:ethyl acetate = 3:1) to obtain the target product 6-(2-chloropropan-2-yl)-4-(trifluoromethyl)pyridazin-3(2H)-one (I-27, 2.9 g, yield 109.02%). ESI $[M+H]^+$=241.0, 243.0

**Preparation of Intermediate I-28: 7-(piperazin-1-yl)-4-(trifluoromethyl)-1*H*-pyrrolo[2,3-*c*]pyridine hydrochloride (I-28)**

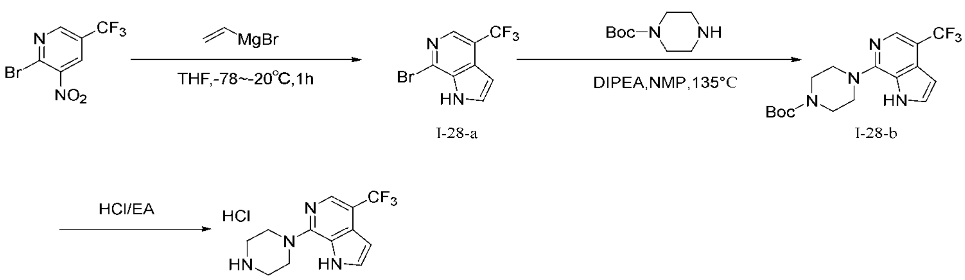


I-28-a I-28-b I-28

**Step 1: Preparation of 7-bromo-4-(trifluoromethyl)-1*H*-pyrrolo[2,3-*c*]pyridine (I-28-a)**

2-bromo-3-nitro-5-(trifluoromethyl)pyridine (500 mg, 1.78 mmol) was dissolved in tetrahydrofuran (15 mL). After purging with nitrogen, vinylmagnesium bromide (11 mL, 10.7 mmol) was added dropwise at -78°C, and the mixture was then stirred at -20°C for 1 hour. After the reaction was complete, it was warmed to room temperature, quenched with saturated ammonium chloride solution, and extracted with ethyl acetate. The organic phase was collected, washed with saturated sodium chloride solution, dried over anhydrous sodium sulfate, filtered, and concentrated under reduced pressure. The residue was purified by Flash column chromatography (petroleum ether:ethyl acetate = 4:1) to obtain the target product 7-bromo-4-(trifluoromethyl)-1H-pyrrolo[2,3-c]pyridine (I-28-a, 130 mg, yield 26.5%). ESI $[M+H]^+$=266.2

**Step 2: Preparation of tert-butyl 4-(4-(trifluoromethyl)-1*H*-pyrrolo[2,3-c]pyridin-7-yl)piperazine-1-carboxylate (I-28-b)**

7-bromo-4-(trifluoromethyl)-1*H*-pyrrolo[2,3-c]pyridine (I-28-a, 130 mg, 0.49 mmol) was dissolved in *N*-methylpyrrolidone (6 mL), and *N*,*N*-diisopropylethylamine (325 μL, 1.96 mmol) and tert-butyl piperazine-1-carboxylate (275 mg, 1.47 mmol) were added with stirring. The tube was sealed and heated to 135°C to react overnight. After the reaction was complete, it was cooled to room temperature, diluted with water, and extracted with ethyl acetate. The organic phases were collected and washed repeatedly with saturated sodium chloride solution, dried over anhydrous sodium sulfate, filtered, and concentrated under reduced pressure. The residue was purified by Flash column chromatography (petroleum ether:ethyl acetate = 1:1) to obtain the target product tert-butyl 4-(4-(trifluoromethyl)-1*H*-pyrrolo[2,3-*c*]pyridin-7-yl)piperazine-1-carboxylate (1-28-b, 180 mg, yield 98%). ESI $[M+H]^+$ =371.4

**Step 3: Preparation of 7-(piperazin-1-yl)-4-(trifluoromethyl)-1*H*-pyrrolo[2,3-*c*]pyridine hydrochloride (I-28)**

tert-butyl 4-(4-(trifluoromethyl)-1*H*-pyrrolo[2,3-*c*]pyridin-7-yl)piperazine-1-carboxylate (I-28-b, 80 mg, 0.22 mmol) was dissolved in ethyl acetate (2 mL), and 4M hydrogen chloride in ethyl acetate solution (2 mL) was added. The mixture was reacted at room temperature overnight. After the reaction was complete, the mixture was concentrated under reduced pressure, and dichloromethane solution was added and evaporated twice to obtain the target product 7-(piperazin-1-yl)-4-(trifluoromethyl)-1*H*-pyrrolo[2,3-*c*]pyridine hydrochloride (I-28, 70 mg, crude product). ESI $[M+H]^+$=271.4

**Preparation of Intermediate 1-29: 1-methyl-7-(piperazin-1-yl)-4-(trifluoromethyl)-1*H*-pyrrolo[2,3-c]pyridine hydrochloride (I-29)**

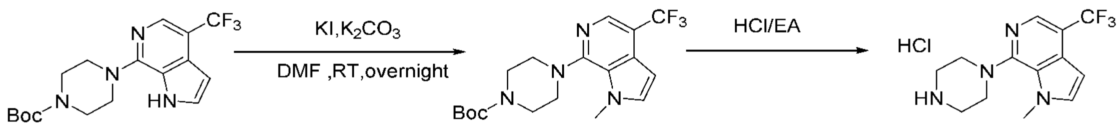


I-28-b I-29-a I-29

**Step 1: Preparation of tert-butyl 4-(1-methyl-4-(trifluoromethyl)-1*H*-pyrrolo[2,3-*c*]pyridin-7-yl)piperazine-1-carboxylate (I-29-a)**

tert-butyl 4-(4-(trifluoromethyl)-1*H*-pyrrolo[2,3-c]pyridin-7-yl)piperazine-1-carboxylate (I-28-b, 30 mg, 0.081 mmol) was dissolved in *N*,*N*-dimethylformamide (2 mL), and methyl iodide (4.3 μL, 0.081 mmol) and potassium carbonate (16.8 mg, 0.121 mmol) were added with stirring. The mixture was reacted at room temperature overnight. After the reaction was complete, it was diluted with water and extracted with ethyl acetate. The organic phases were collected and washed repeatedly with saturated sodium chloride solution, dried over anhydrous sodium sulfate, filtered, and concentrated under reduced pressure. The residue was purified by Flash column chromatography (petroleum ether:ethyl acetate = 4:1) to obtain the target product tert-butyl 4-(1-methyl-4-(trifluoromethyl)-1*H*-pyrrolo[2,3-c]pyridin-7-yl)piperazine-1-carboxylate (I-29-a, 30 mg, yield 96%). ESI $[M+H]^+$ =385.4

**Step 4: Preparation of 1-methyl-7-(piperazin-1-yl)-4-(trifluoromethyl)-1H-pyrrolo[2,3-c]pyridine hydrochloride (I-29)**

tert-butyl 4-(1-methyl-4-(trifluoromethyl)-1*H*-pyrrolo[2,3-*c*]pyridin-7-yl)piperazine-1-carboxylate (I-29-a, 30 mg, 0.078 mmol) was dissolved in ethyl acetate (1 mL), and 4M hydrogen chloride in ethyl acetate solution (1 mL) was added. The mixture was reacted at room temperature overnight. After the reaction was complete, the mixture was concentrated under reduced pressure, and dichloromethane solution was added and evaporated twice to obtain the target product 1-methyl-7-(piperazin-1-yl)-4-(trifluoromethyl)-1*H*-pyrrolo[2,3-c]pyridine hydrochloride (I-29, 30 mg, crude product). ESI $[M+H]^+$=285.4

**Preparation of Intermediate I-30: (2-(piperazin-1-yl)-5-(trifluoromethyl)pyridin-3-yl)(pyrrolidin-1-yl)methanone hydrochloride (I-30)**

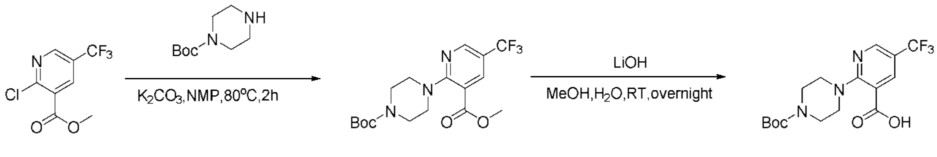


I-30-a

I-30-b

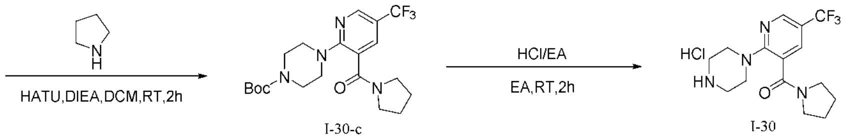


I-30-c

I-30

**Step 1: Preparation of tert-butyl 4-(3-(methoxycarbonyl)-5-(trifluoromethyl)pyridin-2-yl)piperazine-1-carboxylate (I-30-a)**

Methyl 2-chloro-5-(trifluoromethyl)nicotinate (500 mg, 2.087 mmol) and tert-butyl piperazine-1-carboxylate (408 mg, 2.194 mmol) were dissolved in N-methylpyrrolidone (15 mL), and potassium carbonate (432 mg, 3.13 mmol) was added. The mixture was reacted at 80°C for 2 hours. Water was added to the reaction mixture, and a solid precipitated. The mixture was filtered, and the filter cake was washed with water and dried at 70°C for 1.5 hours to obtain the target product tert-butyl 4-(3-(methoxycarbonyl)-5-(trifluoromethyl)pyridin-2-yl)piperazine-1-carboxylate (I-30-a, 764 mg, yield 93.81%). ESI $[M+H]^+$ =376.3

**Step 2: Preparation of 2-(4-(tert-butoxycarbonyl)piperazin-1-yl)-5-(trifluoromethyl)nicotinic acid (I-30-b)**

tert-butyl 4-(3-(methoxycarbonyl)-5-(trifluoromethyl)pyridin-2-yl)piperazine-1-carboxylate (I-30-a, 740 mg, 1.9 mmol) and lithium hydroxide (146 mg, 3.476 mmol) were dissolved in methanol (20 mL) and water (4 mL) and reacted at room temperature overnight. After monitoring showed the reaction was complete, acetic acid was added to adjust the pH to 7, and the mixture was extracted three times with ethyl acetate. The combined organic phases were dried over anhydrous sodium sulfate and filtered. The filtrate was concentrated under reduced pressure to obtain the target product 2-(4-(tert-butoxycarbonyl)piperazin-1-yl)-5-(trifluoromethyl)nicotinic acid (I-30-b, 820 mg, crude product). ESI $[M+H]^+$ =376.3

**Step 3: Preparation of tert-butyl 4-(3-(pyrrolidine-1-carbonyl)-5-(trifluoromethyl)pyridin-2-yl)piperazine-1-carboxylate (I-30-c)**

2-(4-(tert-butoxycarbonyl)piperazin-1-yl)-5-(trifluoromethyl)nicotinic acid (I-30-b, 200 mg, 0.533 mmol) was dissolved in dichloromethane (10 mL), and *O*-(7-azabenzotriazol-1-yl)-*N,N,N',N'*-tetramethyluronium hexafluorophosphate (200 mg, 0.526 mmol) was added and stirred for 10 minutes. Then, *N,N*-diisopropylethylamine (279 μL, 1.578 mmol) and pyrrolidine (44 μL, 0.53 mmol) were added, and the mixture was reacted at room temperature overnight. The reaction mixture was concentrated under reduced pressure and purified by Flash chromatography (10g silica gel column, petroleum ether:ethyl acetate = 1:1) to obtain the target product tert-butyl 4-(3-(pyrrolidine-1-carbonyl)-5-(trifluoromethyl)pyridin-2-yl)piperazine-1-carboxylate (I-30-c, 180 mg, yield 71.72%). ESI $[M+H]^+$=429.0

**Step 4: Preparation of (2-(piperazin-1-yl)-5-(trifluoromethyl)pyridin-3-yl)(pyrrolidin-1-yl)methanone hydrochloride (I-30)**

tert-butyl 4-(3-(pyrrolidine-1-carbonyl)-5-(trifluoromethyl)pyridin-2-yl)piperazine-1-carboxylate (I-30-c, 180 mg, 0.42 mmol) was dissolved in 4M hydrogen chloride in ethyl acetate (3 mL) and reacted at room temperature for 2 hours, then concentrated under reduced pressure to obtain the target product (2-(piperazin-1-yl)-5-(trifluoromethyl)pyridin-3-yl)(pyrrolidin-1-yl)methanone hydrochloride (I-30, 190 mg, crude product). ESI $[M+H]^+$=329.0

**Preparation of Intermediate 1-31: *N*-methyl-2-(piperazin-1-yl)-5-(trifluoromethyl)nicotinamide hydrochloride (I-31)**

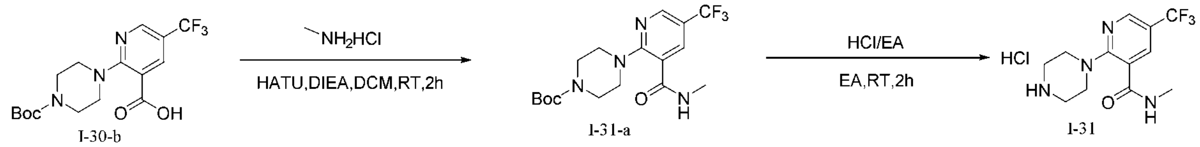

I-30-b I-31-a I-31

**Step 1: Preparation of tert-butyl 4-(3-(methylcarbamoyl)-5-(trifluoromethyl)pyridin-2-yl)piperazine-1-carboxylate (I-31-a)**

2-(4-(tert-butoxycarbonyl)piperazin-1-yl)-5-(trifluoromethyl)nicotinic acid (I-30-b, 200 mg, 0.533 mmol) was dissolved in dichloromethane (10 mL), and *O*-(7-azabenzotriazol-1-yl)-*N,N,N',N'*-tetramethyluronium hexafluorophosphate (200 mg, 0.526 mmol) was added and stirred for 10 minutes. Then, *N,N*-diisopropylethylamine (279 μL, 1.578 mmol) and methylamine hydrochloride (36 mg, 0.53 mmol) were added, and the mixture was reacted at room temperature for 3 hours. The reaction mixture was concentrated under reduced pressure and purified by Flash chromatography (10g silica gel column, petroleum ether:ethyl acetate = 1:1) to obtain the target product tert-butyl 4-(3-(methylcarbamoyl)-5-(trifluoromethyl)pyridin-2-yl)piperazine-1-carboxylate (I-31-a, 220 mg, yield 106.38%). ESI $[M+H]^+$=389.0

**Step 2: Preparation of *N*-methyl-2-(piperazin-1-yl)-5-(trifluoromethyl)nicotinamide hydrochloride (I-31)**

tert-butyl 4-(3-(methylcarbamoyl)-5-(trifluoromethyl)pyridin-2-yl)piperazine-1-carboxylate (I-31-a, 220 mg, 0.567 mmol) was dissolved in 4M hydrogen chloride in ethyl acetate (3 mL) and reacted at room temperature for 2 hours, then concentrated under reduced pressure to obtain the target product *N*-methyl-2-(piperazin-1-yl)-5-(trifluoromethyl)nicotinamide hydrochloride (I-31, 193 mg, yield 105.46%). ESI $[M+H]^+$=289.0

**Preparation of Intermediate I-32: 2-methyl-7-(piperazin-1-yl)-4-(trifluoromethyl)-1*H*-pyrrolo[2,3-c]pyridine hydrochloride (I-32)**

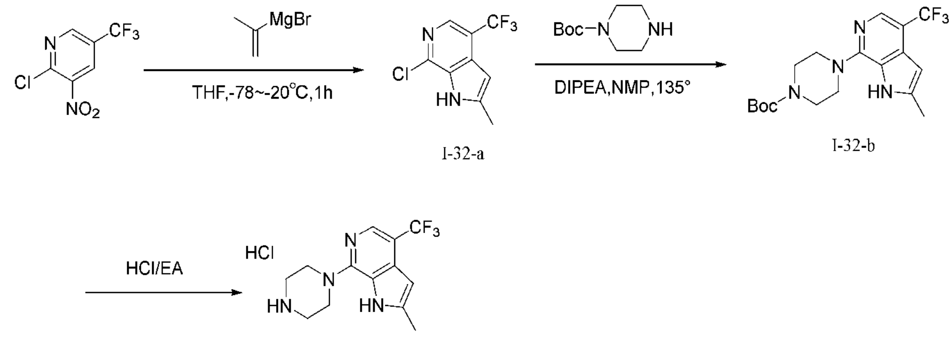

I-32-a I-32-b
I-32

**Step 1: Preparation of 7-chloro-2-methyl-4-(trifluoromethyl)-1*H*-pyrrolo[2,3-*c*]pyridine (I-32-a)**

2-chloro-3-nitro-5-(trifluoromethyl)pyridine (250 mg, 1.10 mmol) was dissolved in tetrahydrofuran (12 mL). After purging with nitrogen, isopropenylmagnesium bromide (6.62 mL, 6.62 mmol) was added dropwise at - 78°C, and the mixture was then stirred at -20°C for 1 hour. After the reaction was complete, it was warmed to room temperature, quenched with saturated ammonium chloride solution, and extracted with ethyl acetate. The organic phase was collected, washed with saturated sodium chloride solution, dried over anhydrous sodium sulfate, filtered, and concentrated under reduced pressure. The residue was purified by Flash column chromatography (petroleum ether:ethyl acetate = 4:1) to obtain the target product 7-chloro-2-methyl-4-(trifluoromethyl)-1*H*-pyrrolo[2,3-c]pyridine (I-32-a, 90 mg, yield 34.75%). ESI $[M+H]^+$=235.8

**Step 2: Preparation of tert-butyl 4-(2-methyl-4-(trifluoromethyl)-1*H*-pyrrolo[2,3-c]pyridin-7-yl)piperazine-1-carboxylate (I-32-b)**

7-chloro-2-methyl-4-(trifluoromethyl)-1*H*-pyrrolo[2,3-c]pyridine (I-32-a, 90 mg, 0.38 mmol) was dissolved in *N*-methylpyrrolidone (4 mL), and then *N*,*N*-diisopropylethylamine (254 μL, 1.53 mmol) and tert-butyl piperazine-1-carboxylate (214 mg, 1.15 mmol) were added with stirring. The tube was sealed and heated to 135°C to react overnight. After the reaction was complete, it was cooled to room temperature, diluted with water, and extracted with ethyl acetate. The organic phases were collected and washed repeatedly with saturated sodium chloride solution, dried over anhydrous sodium sulfate, filtered, and concentrated under reduced pressure. The residue was purified by Flash column chromatography (petroleum ether:ethyl acetate = 3:1) to obtain the target product tert-butyl 4-(2-methyl-4-(trifluoromethyl)-1*H*-pyrrolo[2,3-*c*]pyridin-7-yl)piperazine-1-carboxylate (I-32-b, 80 mg, yield 54.4%). ESI $[M+H]^+$=385.3

**Step 3: Preparation of 2-methyl-7-(piperazin-1-yl)-4-(trifluoromethyl)-1*H*-pyrrolo[2,3-*c*]pyridine hydrochloride (1-32)**

tert-butyl 4-(2-methyl-4-(trifluoromethyl)-1*H*-pyrrolo[2,3-c]pyridin-7-yl)piperazine-1-carboxylate (I-32-b, 80 mg, 0.21 mmol) was dissolved in ethyl acetate (1 mL), and 4M hydrogen chloride in ethyl acetate solution (3 mL) was added. The mixture was reacted at room temperature for 2 hours. After the reaction was complete, the mixture was concentrated under reduced pressure, and dichloromethane was added to dissolve the residue, which was then evaporated twice to obtain the crude target product 2-methyl-7-(piperazin-1-yl)-4-(trifluoromethyl)-1*H*-pyrrolo[2,3-c]pyridine hydrochloride (I-32, 100 mg, crude product). ESI $[M+H]^+$=285.3

**Preparation of Intermediate I-33: 3-(2-(piperazin-1-yl)-5-(trifluoromethyl)pyridin-3-yl)-1,2,4-oxadiazole trifluoroacetate (I-33)**

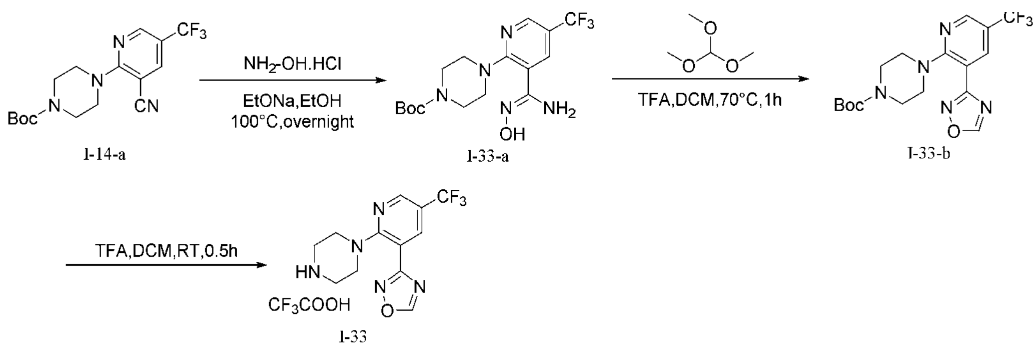

I-14-a I-33-a I-33-b I-33

**Step 1: Preparation of tert-butyl 4-(3-(N'-hydroxycarbamimidoyl)-5-(trifluoromethyl)pyridin-2-yl)piperazine-1-carboxylate (I-33-a)**

tert-butyl 4-(3-cyano-5-(trifluoromethyl)pyridin-2-yl)piperazine-1-carboxylate (I-14-a, 100 mg, 0.28 mmol) was dissolved in ethanol (5 mL), and hydroxylamine hydrochloride (29 mg, 0.42 mmol) and sodium ethoxide (43 mg, 0.63 mmol) were added. After reacting at 100°C overnight, the reaction mixture was concentrated under reduced pressure, and the crude product was purified by Flash chromatography (10g silica gel column, petroleum ether:ethyl acetate = 0-40%) to obtain the target product tert-butyl 4-(3-(*N'*-hydroxycarbamimidoyl)-5-(trifluoromethyl)pyridin-2-yl)piperazine-1-carboxylate (I-33-a, 50 mg, yield 45.9%). ESI $[M+H]^+$=390.1

**Step 2: Preparation of tert-butyl 4-(3-(1,2,4-oxadiazol-3-yl)-5-(trifluoromethyl)pyridin-2-yl)piperazine-1-carboxylate (I-33-b)**

tert-butyl 4-(3-(*N'*-hydroxycarbamimidoyl)-5-(trifluoromethyl)pyridin-2-yl)piperazine-1-carboxylate (I-33-a, 50 mg, 0.13 mmol) was dissolved in trimethyl orthoformate (5 mL), and trifluoroacetic acid (0.5 mL) was added. After reacting at 70°C for 1 hour, the reaction mixture was concentrated under reduced pressure to obtain the crude target product tert-butyl 4-(3-(1,2,4-oxadiazol-3-yl)-5-(trifluoromethyl)pyridin-2-yl)piperazine-1-carboxylate (I-33-b, 60 mg, crude product). ESI $[M+H]^+$=400.1

**Step 3: Preparation of 3-(2-(piperazin-1-yl)-5-(trifluoromethyl)pyridin-3-yl)-1,2,4-oxadiazole trifluoroacetate (I-33)**

tert-butyl 4-(3-(1,2,4-oxadiazol-3-yl)-5-(trifluoromethyl)pyridin-2-yl)piperazine-1-carboxylate (I-33-b, 60 mg, 0.15 mmol) was dissolved in dichloromethane (3 mL), and trifluoroacetic acid (0.6 mL) was added. After reacting at

room temperature for 0.5 hours, the mixture was concentrated under reduced pressure to obtain the crude target product 3-(2-(piperazin-1-yl)-5-(trifluoromethyl)pyridin-3-yl)-1,2,4-oxadiazole trifluoroacetate (I-33, 110 mg, crude product). ESI $[M+H]^+$=300.1

**Embodiment 1: Preparation of 3-(trifluoromethyl)-5-(((((1*S*,3*S*)-3-(3-(5-(trifluoromethyl)pyrimidin-2-yl)-3,8-diazabicyclo[3.2.1]octane-8-carbonyl)cyclopentyl)amino)methyl)pyridin-2(1*H*)-one (1)**

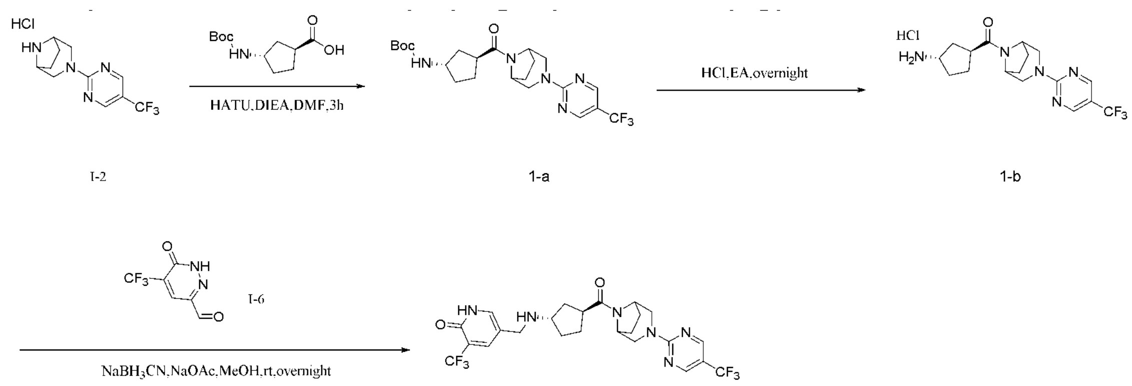


I-2 1-a 1-b I-6

**Step 1: Preparation of tert-butyl ((1*S*,3*S*)-3-(3-(5-(trifluoromethyl)pyrimidin-2-yl)-3,8-diazabicyclo[3.2.1]octane-8-carbonyl)cyclopentyl)carbamate (1-a)**

(1S,3S)-3-((tert-butoxycarbonyl)amino)cyclopentane-1-carboxylic acid (50 mg, 0.218 mmol), O-(7-azabenzotriazol-1-yl)-*N,N,N',N'*-tetramethyluronium hexafluorophosphate (99.6 mg, 0.262 mmol), and *N,N*-diisopropylethylamine (184 mg, 1.42 mmol) were dissolved in *N,N*-dimethylformamide (2 mL). Finally, 3-(5-(trifluoromethyl)pyrimidin-2-yl)-3,8-diazabicyclo[3.2.1]octane hydrochloride (I-2, 64.9 mg, 0.221 mmol) was added, and the reaction was stirred at room temperature for 3 hours. After monitoring showed the reaction was complete, it was quenched with water and extracted with ethyl acetate. The organic phase was collected and washed with saturated sodium chloride solution, dried over anhydrous sodium sulfate, filtered, and concentrated under reduced pressure. The residue was purified by preparative TLC silica gel plate (ethyl acetate:petroleum ether = 1:1) to obtain the target product tert-butyl ((1S,3S)-3-(3-(5-(trifluoromethyl)pyrimidin-2-yl)-3,8-diazabicyclo[3.2.1]octane-8-carbonyl)cyclopentyl)carbamate (1-a, 67.6 mg, yield 66.1%). ESI$[M+H]^+$ =470.2

**Step 2: Preparation of ((1*S*,3*S*)-3-aminocyclopentyl)(3-(5-(trifluoromethyl)pyrimidin-2-yl)-3,8-diazabicyclo [3.2.1]octan-8-yl)methanone hydrochloride (1-b)**

tert-butyl ((1*S*,3*S*)-3-(3-(5-(trifluoromethyl)pyrimidin-2-yl)-3,8-diazabicyclo[3.2.1]octane-8-carbonyl)cyclopentyl)carbamate (1-a, 67.6 mg, 0.144 mmol) was dissolved in 4 mol/L hydrogen chloride in ethyl acetate solution and reacted at room temperature overnight. After monitoring showed the reaction was complete, the mixture was concentrated under reduced pressure to obtain the target product ((1*S*,3*S*)-3-aminocyclopentyl)(3-(5-(trifluoromethyl)pyrimidin-2-yl)-3,8-diazabicyclo[3.2.1]octan-8-yl)methanone hydrochloride (1-b, 76.4 mg, yield 149%). ESI$[M+H]^+$ =370.2

**Step 3: Preparation of 3-(trifluoromethyl)-5-(((((1*S*,3*S*)-3-(3-(5-(trifluoromethyl)pyrimidin-2-yl)-3,8-diazabicyclo [3.2.1]octane-8-carbonyl)cyclopentyl)amino)methyl)pyridin-2(1*H*)-one (1)**

((1*S*,3*S*)-3-aminocyclopentyl)(3-(5-(trifluoromethyl)pyrimidin-2-yl)-3,8-diazabicyclo[3.2.1]octan-8-yl)methanone (1-b, 30 mg, 0.0739 mmol) and 6-oxo-5-(trifluoromethyl)-1,6-dihydropyridazine-3-carbaldehyde (I-6, 16.9 mg, 0.088 mmol) were dissolved in methanol (2 mL) under an ice bath. Sodium acetate (3 μL) was added dropwise, and the mixture was stirred under an ice bath for 20 minutes. Then, sodium cyanoborohydride (13.9 mg, 0.221 mmol) was added, and the reaction was stirred at room temperature overnight. After monitoring showed the reaction was complete, it was quenched with water and extracted with dichloromethane:methanol = 10:1. The organic phase was dried over anhydrous sodium sulfate, filtered, and concentrated under reduced pressure. The residue was purified by preparative HPLC [Pack Column 20ID*250mm*10μm; A:$H_2O$(0.1%FA), B:ACN, B%: 5%-50% over 22 min] to obtain 3-(trifluoromethyl)-5-(((((1*S*,3*S*)-3-(3-(5-(trifluoromethyl)pyrimidin-2-yl)-3,8-diazabicyclo[3.2.1]octane-8-carbonyl)cyclopentyl)amino)methyl)pyridin-2(1*H*)-one (1, 1.46 mg, yield 3.30%), ESI $[M+H]^+$=545.2 $^1$H NMR (400 MHz, DMSO-$d_6$) δ 8.72 (S, 2H), 8.29 (S, 1H), 7.94 (S, 1H), 7.58 (S, 1H), 4.64 (d, J = 6.4 Hz, 1H), 4.49 (t, J = 12.6 Hz, 2H), 3.59 (d, J = 6.4 Hz, 1H), 3.46 (S, 1H), 3.16 - 3.01 (m, 3H), 2.07 (S, 1H), 1.83 (ddd, J = 21.6, 15.6, 8.8 Hz, 5H), 1.69 - 1.56 (m, 2H), 1.54 (d, J = 8.0 Hz, 1H), 1.44 - 1.34 (m, 1H), 1.23 (S, 1H), 1.19 - 1.09 (m, 1H), 1.08 - 0.98 (m, 1H).

**Embodiment 2: Preparation of 4-(trifluoromethyl)-5-((((2*S*)-1-(((3*R*)-3-(3-(5-(trifluoromethyl)pyrimidin-2-yl)-3,8-diazabicyclo[3.2.1]octane-8-carbonyl)pyrrolidin-1-yl)propan-2-yl)amino)pyridazin-3(2*H*)-one (2)**

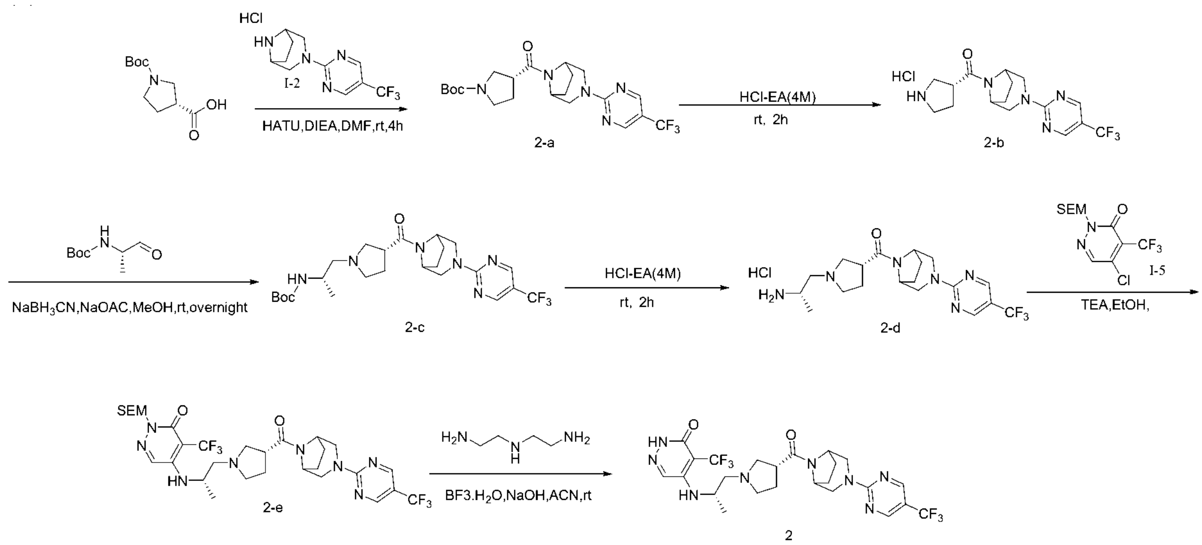


**Step 1: Preparation of tert-butyl (3*R*)-3-(3-(5-(trifluoromethyl)pyrimidin-2-yl)-3,8-diazabicyclo[3.2.1]octane-8-carbonyl)pyrrolidine-1-carboxylate (2-a)**

(*R*)-1-(tert-butoxycarbonyl)pyrrolidine-3-carboxylic acid (50 mg, 0.23 mmol) was dissolved in *N,N*-dimethylformamide (2 mL), and *O*-(7-azabenzotriazol-1-yl)-*N,N,N',N'*-tetramethyluronium hexafluorophosphate (88.5 mg, 0.23 mmol) and *N,N*-diisopropylethylamine (120 μL, 0.696 mmol) were added sequentially and reacted at 0°C for 20 minutes. Then, 3-(5-(trifluoromethyl)pyrimidin-2-yl)-3,8-diazabicyclo[3.2.1]octane hydrochloride (I-2, 69.6 mg, 0.238 mmol) was added, and stirring was continued for 4 hours. 10 volumes of water were added to the reaction mixture, and after stirring for 10 minutes, the mixture was filtered. The filter cake was washed with ethyl acetate solution, and the filtrate was concentrated under reduced pressure to obtain the target product tert-butyl (3*R*)-3-(3-(5-(trifluoromethyl)pyrimidin-2-yl)-3,8-diazabicyclo[3.2.1]octane-8-carbonyl)pyrrolidine-1-carboxylate (2-a, 97 mg, yield 91.60%). ESI$[M+H]^+$ =456.21

**Step 2: Preparation of ((*R*)-pyrrolidin-3-yl)(3-(5-(trifluoromethyl)pyrimidin-2-yl)-3,8-diazabicyclo[3.2.1]octan-8-yl)methanone hydrochloride (2-b)**

To tert-butyl (3R)-3-(3-(5-(trifluoromethyl)pyrimidin-2-yl)-3,8-diazabicyclo[3.2.1]octane-8-carbonyl)pyrrolidine-1-carboxylate (2-a, 97 mg, 0.213 mmol), 4M hydrogen chloride in ethyl acetate solution (1 mL) was added slowly, and the mixture was stirred at room temperature for 2 hours. The reaction mixture was concentrated under reduced pressure to obtain the crude target product ((R)-pyrrolidin-3-yl)(3-(5-(trifluoromethyl)pyrimidin-2-yl)-3,8-diazabicyclo [3.2.1]octan-8-yl)methanone hydrochloride (2-b, 131 mg). ESI$[M+H]^+$ =356.16

**Step 3: Preparation of tert-butyl ((2*S*)-1-((3*R*)-3-(3-(5-(trifluoromethyl)pyrimidin-2-yl)-3,8-diazabicyclo[3.2.1]octane-8-carbonyl)pyrrolidin-1-yl)propan-2-yl)carbamate (2-c)**

((*R*)-pyrrolidin-3-yl)(3-(5-(trifluoromethyl)pyrimidin-2-yl)-3,8-diazabicyclo[3.2.1]octan-8-yl)methanone hydrochloride (2-b, 131 mg, 0.37 mmol) was dissolved in methanol (7 mL). Then, tert-butyl (S)-(1-oxopropan-2-yl)carbamate (76.5 mg, 0.44 mmol) was added, followed by sodium acetate (62.84 mg, 1.10 mmol) at 0°C. The mixture was stirred at 0°C for 20 minutes, and then sodium cyanoborohydride (15.1 mg, 0.18 mmol) was added at 0°C. The reaction was stirred at

room temperature overnight. Saturated brine was added, and the mixture was extracted with ethyl acetate. The organic phase was concentrated under reduced pressure, and the residue was purified by column chromatography (dichloromethane:methanol = 10:1) to obtain the target product tert-butyl ((2*S*)-1-((3*R*)-3-(3-(5-(trifluoromethyl)pyrimidin-2-yl)-3,8-diazabicyclo[3.2.1]octane-8-carbonyl)pyrrolidin-1-yl)propan-2-yl)carbamate (2-c, 82 mg, yield 43.47%). ESI $[M+H]^+$=513.27

**Step 4: Preparation of ((*R*)-1-((*S*)-2-aminopropyl)pyrrolidin-3-yl)(3-(5-(trifluoromethyl)pyrimidin-2-yl). 3,8-diazabicyclo[3.2.1]octan-8-yl)methanone hydrochloride (2-d)**

To tert-butyl ((2S)-1-((3*R*)-3-(3-(5-(trifluoromethyl)pyrimidin-2-yl)-3,8-diazabicyclo[3.2.1]octane-8-carbonyl) pyrrolidin-1-yl)propan-2-yl)carbamate (2-c, 82 mg, 0.16 mmol), 4M hydrogen chloride in ethyl acetate solution (0.8 mL) was added slowly, and the mixture was stirred at room temperature for 2 hours. The reaction mixture was concentrated under reduced pressure to obtain the crude target product ((*R*)-1-((*S*)-2-aminopropyl)pyrrolidin-3-yl)(3-(5-(trifluoromethyl)pyrimidin-2-yl)-3,8-diazabicyclo[3.2.1]octan-8-yl)methanone hydrochloride (2-d, 80 mg). ESI$[M+H]^+$ =413.27

**Step 5: Preparation of 4-(trifluoromethyl)-5-((((2*S*)-1-(((3*R*)-3-(3-(5-(trifluoromethyl)pyrimidin-2-yl)-3,8-diazabicyclo[3.2.1]octane-8-carbonyl)pyrrolidin-1-yl)propan-2-yl)amino)-2-((2-(trimethylsilyl)ethoxy)methyl)pyridazin-3(2*H*)-one (2-e)**

((*R*)-1-((*S*)-2-aminopropyl)pyrrolidin-3-yl)(3-(5-(trifluoromethyl)pyrimidin-2-yl)-3,8-diazabicyclo[3.2.1]octan-8-yl)methanone hydrochloride (2-d, 80 mg, 0.194 mmol) was dissolved in ethanol (8 mL). 5-chloro-4-(trifluoromethyl)-2-((2-(trimethylsilyl)ethoxy)methyl)pyridazin-3(2*H*)-one (I-5, 95.6 mg, 0.291 mmol) and triethylamine (14.7 mg, 0.144 mmol) were added sequentially, and the mixture was stirred at 80°C for 2 hours. The reaction mixture was concentrated under reduced pressure, and the residue was purified by column chromatography (ethyl acetate) to obtain the target product 4-(trifluoromethyl)-5-((((2*S*)-1-(((3*R*)-3-(3-(5-(trifluoromethyl)pyrimidin-2-yl)-3,8-diazabicyclo[3.2.1] octane-8-carbonyl)pyrrolidin-1-yl)propan-2-yl)amino)-2-((2-(trimethylsilyl)ethoxy)methyl)pyridazin-3(2*H*)-one (2-e, 56 mg, yield 40.97%). ESI$[M+H]^+$ =705.31

**Step 6: Preparation of 4-(trifluoromethyl)-5-((((2*S*)-1-(((3*R*)-3-(3-(5-(trifluoromethyl)pyrimidin-2-yl)-3,8-diazabicyclo[3.2.1]octane-8-carbonyl)pyrrolidin-1-yl)propan-2-yl)amino)pyridazin-3(2*H*)-one (2)**

4-(trifluoromethyl)-5-((((2*S*)-1-(((3*R*)-3-(3-(5-(trifluoromethyl)pyrimidin-2-yl)-3,8-diazabicyclo[3.2.1]octane-8-carbonyl)pyrrolidin-1-yl)propan-2-yl)amino)-2-((2-(trimethylsilyl)ethoxy)methyl)pyridazin-3(2*H*)-one (2-e, 28 mg, 0.04 mmol) was dissolved in acetonitrile (3 mL). Boron trifluoride etherate (33.9 mg, 0.239 mmol) was added at -10°C, and the mixture was stirred at -10°C for 1 hour. Then, diethylenetriamine (14.4 mg, 0.14 mmol) and 1M sodium hydroxide solution (84 μL) were added sequentially, and the reaction mixture was stirred at room temperature overnight. The reaction mixture was concentrated under reduced pressure, and the residue was purified by preparative HPLC [Pack Column 20ID*250mm*10μm; A:$H_2O$(0.1%FA), B:ACN, B%: 10%-70% over 22 min] to obtain the target product 4-(trifluoromethyl)-5-((((2*S*)-1-(((3*R*)-3-(3-(5-(trifluoromethyl)pyrimidin-2-yl)-3,8-diazabicyclo[3.2.1]octane-8-carbonyl)pyrrolidin-1-yl)propan-2-yl)amino)pyridazin-3(2*H*)-one (2, 13 mg, yield 57.01%). ESI$[M+H]^+$=575.22 $^1H$ NMR (600 MHz, DMSO-$d_6$) δ 12.44 (d, J = 9.5 Hz, 1H), 8.71 (S, 2H), 8.18 (S, 1H), 7.91 (S, 1H), 4.64 (t, J = 6.6 Hz, 1H), 4.54 - 4.44 (m, 3H), 3.06 (dt, J = 27.1, 13.8 Hz, 3H), 2.93 (dt, J = 30.2, 8.7 Hz, 1H), 2.71 (dt, J = 13.5, 7.8 Hz, 2H), 2.62 (dt, J = 19.7, 10.0 Hz, 2H), 2.04 - 1.84 (m, 4H), 1.81 - 1.65 (m, 2H), 1.63 - 1.49 (m, 2H), 1.18 (d, J = 6.2 Hz, 3H).

**Embodiment 3: Preparation of 4-(trifluoromethyl)-6-(((((1*R*,3*R*)-3-(3-(5-(trifluoromethyl)pyrimidin-2-yl)-3,8-diazabicyclo[3.2.1]octane-8-carbonyl)cyclobutyl)amino)methyl)pyridazin-3(2*H*)-one (3)**

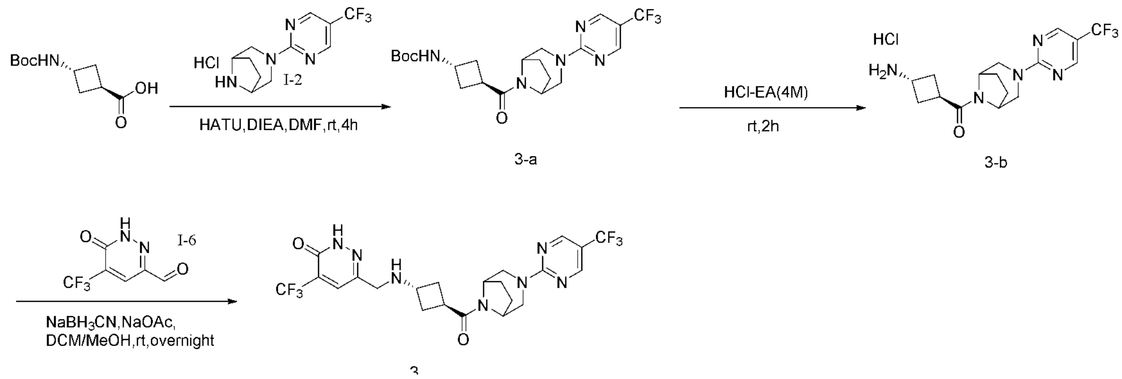


**Step 1: Preparation of tert-butyl ((1*R*,3*R*)-3-(3-(5-(trifluoromethyl)pyrimidin-2-yl)-3,8-diazabicyclo[3.2.1]octane-8-carbonyl)cyclobutyl)carbamate (3-a)**

(1*R*,3*R*)-3-((tert-butoxycarbonyl)amino)cyclobutane-1-carboxylic acid (60 mg, 0.279 mmol) was dissolved in dichloromethane (2 mL), and *O*-(7-azabenzotriazol-1-yl)-*N,N,N',N'*-tetramethyluronium hexafluorophosphate (106 mg, 0.279 mmol), *N,N*-diisopropylethylamine (108 mg, 0.837 mmol), and 3-(5-(trifluoromethyl)pyrimidin-2-yl)-3,8-diazabicyclo[3.2.1]octane hydrochloride (I-2, 55 mg, 0.223 mmol) were added. The mixture was reacted at room temperature overnight. The reaction mixture was filtered and concentrated under reduced pressure, and the residue was purified by preparative plate (petroleum ether:ethyl acetate = 1:1) to obtain the target product tert-butyl ((1*R*,3*R*)-3-(3-(5-(trifluoromethyl)pyrimidin-2-yl)-3,8-diazabicyclo[3.2.1]octane-8-carbonyl)cyclobutyl)carbamate (3-a, 81 mg, yield 63.8%). ESI $[M+H]^+$ =456.4 ESI$[M+H-56]^+$ =456.4 ESI$[2M+H]^+$ =911.6

**Step 2: Preparation of (((1*R*,3*R*)-3-aminocyclobutyl)(3-(5-(trifluoromethyl)pyrimidin-2-yl)-3,8-diazabicyclo [3.2.1]octan-8-yl)methanone hydrochloride (3-b)**

To tert-butyl ((1*R*,3*R*)-3-(3-(5-(trifluoromethyl)pyrimidin-2-yl)-3,8-diazabicyclo[3.2.1]octane-8-carbonyl)cyclobutyl)carbamate (3-a, 81 mg, 0.178 mmol), 4M hydrogen chloride in ethyl acetate solution (2 mL) was added slowly, and the mixture was stirred at room temperature for 1 hour. After adding 4 mL of ethyl acetate, the reaction mixture was filtered, and the filter cake was dried to obtain the crude target product (((1*R*,3*R*)-3-aminocyclobutyl)(3-(5-(trifluoromethyl) pyrimidin-2-yl)-3,8-diazabicyclo[3.2.1]octan-8-yl)methanone hydrochloride (3-b, 76 mg, yield 109.07%). ESI$[M+H]^+$ =356.3 ESI$[2M+H]^+$=711.5

**Step 3: Preparation of 4-(trifluoromethyl)-6-(((((1*R*,3*R*)-3-(3-(5-(trifluoromethyl)pyrimidin-2-yl)-3,8-diazabicyclo [3.2.1]octane-8-carbonyl)cyclobutyl)amino)methyl)pyridazin-3(2H)-one (3)**

(((1*R*,3*R*)-3-aminocyclobutyl)(3-(5-(trifluoromethyl)pyrimidin-2-yl)-3,8-diazabicyclo[3.2.1]octan-8-yl)methanone hydrochloride (3-b, 30 mg, 0.084 mmol) was dissolved in methanol (0.5 mL) and dichloromethane (0.5 mL). Then, 6-oxo-5-(trifluoromethyl)-1,6-dihydropyridazine-3-carbaldehyde (I-6, 24 mg, 0.127 mmol) was added, followed by sodium acetate (3.5 mg, 0.042 mmol) at 0°C. The mixture was stirred at 0°C for 20 minutes, and then sodium cyanoborohydride (16 mg, 0.252 mmol) was added at 0°C. The reaction was stirred at room temperature overnight. Saturated brine was added, and the mixture was extracted with ethyl acetate. The organic phase was concentrated under reduced pressure, and the residue was purified by preparative thin-layer chromatography silica gel plate (dichloromethane:methanol = 10:1) and then by preparative HPLC [Pack Column 20ID*250mm*10μm; A:$H_2O$(0.1%FA), B:ACN, B%: 10%-70% over 22 min] to obtain the target product 4-(trifluoromethyl)-6-(((((1*R*,3*R*)-3-(3-(5-(trifluoromethyl)pyrimidin-2-yl)-3,8-diazabicyclo[3.2.1] octane-8-carbonyl)cyclobutyl)amino)methyl)pyridazin-3(2H)-one (3, 10 mg, yield 22.29%). ESI$[M+H]^+$=532.4 $^1$HNMR (400 MHz, DMSO-$d_6$) δ 8.71 (S, 2H), 8.38 (S, 1H), 7.94 (S, 1H), 4.64 (d, J = 6.5 Hz, 1H), 4.46 (d, J = 12.9 Hz, 1H), 4.24 (d, J = 6.0 Hz, 1H), 4.10 (S, 2H), 3.57 (S, 2H), 3.28 - 3.18 (m, 2H), 3.05 (dd, J = 12.5, 5.1 Hz, 2H), 2.36 (S, 1H), 2.19 (S, 1H), 2.03 - 1.80 (m, 3H), 1.74 (d, J = 15.6 Hz, 1H), 1.63 - 1.44 (m, 2H).

**Embodiment 4: Preparation of 3-(trifluoromethyl)-5-(((((1*R*,3*R*)-3-(3-(5-(trifluoromethyl)pyrimidin-2-yl)-3,8-diazabicyclo[3.2.1]octane-8-carbonyl)cyclobutyl)amino)methyl)pyridin-2(1*H*)-one (4)**

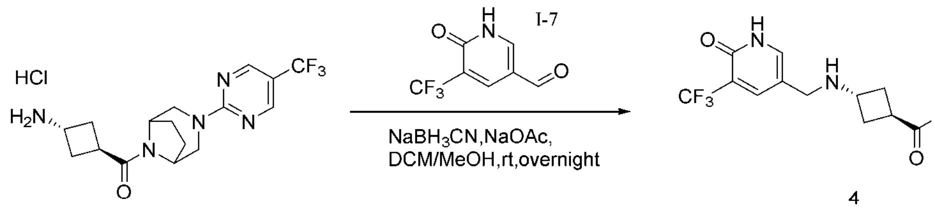


3-a

4

(((1*R*,3*R*)-3-aminocyclobutyl)(3-(5-(trifluoromethyl)pyrimidin-2-yl)-3,8-diazabicyclo[3.2.1]octan-8-yl)methanone hydrochloride (3-b, 50 mg, 0.141 mmol) was dissolved in methanol (1 mL) and dichloromethane (1 mL). Then, 6-oxo-5-(trifluoromethyl)-1,6-dihydropyridine-3-carbaldehyde (I-7, 40 mg, 0.211 mmol) was added, followed by sodium acetate (5.7 mg, 0.071 mmol) at 0°C. The mixture was stirred at 0°C for 20 minutes, and then sodium cyanoborohydride (16 mg, 0.252 mmol) was added at 0°C. The reaction was stirred at room temperature overnight. Saturated brine was added, and the mixture was extracted with ethyl acetate. The organic phase was concentrated under reduced pressure, and the residue was purified by preparative thin-layer chromatography silica gel plate (dichloromethane:methanol = 10:1) and then by preparative HPLC [Pack Column 20ID*250mm*10μm; A:$H_2O$(0.1%FA), B:ACN, B%: 10%-70% over 22 min] to obtain the target product 3-(trifluoromethyl)-5-(((((1R,3R)-3-(3-(5-(trifluoromethyl)pyrimidin-2-yl)-3,8-diazabicyclo[3.2.1]octane-8-carbonyl)cyclobutyl)amino)methyl)pyridin-2(1H)-one (4, 1.23 mg, yield 1.65%). ESI$[M+H]^+$=531.30 $^1$HNMR (400 MHz, DMSO-$d_6$) δ 8.71 (S, 2H), 8.34 (S, 1H), 7.92 (S, 1H), 7.55 (S, 1H), 4.64 (d, J = 6.4 Hz, 1H), 4.46 (d, J = 12.9 Hz, 2H), 4.24 (d, J = 6.0 Hz, 1H), 3.25 - 3.16 (m, 3H), 3.05 (dd, J = 12.4, 6.4 Hz, 2H), 2.38 (dd, J = 7.1, 3.7 Hz, 1H), 2.21 (dd, J = 7.0, 3.7 Hz, 1H), 1.94 (dd, J = 26.4, 15.0 Hz, 4H), 1.74 (d, J = 10.5 Hz, 2H), 1.57 (dd, J = 28.0, 11.8 Hz, 3H).

**Embodiment 5: Preparation of 4-(trifluoromethyl)-6-((3-(4-(5-(trifluoromethyl)pyrimidin-2-yl)piperazine-1-carbonyl)azetidin-1-yl)methyl)pyridazin-3(2*H*)-one (5)**

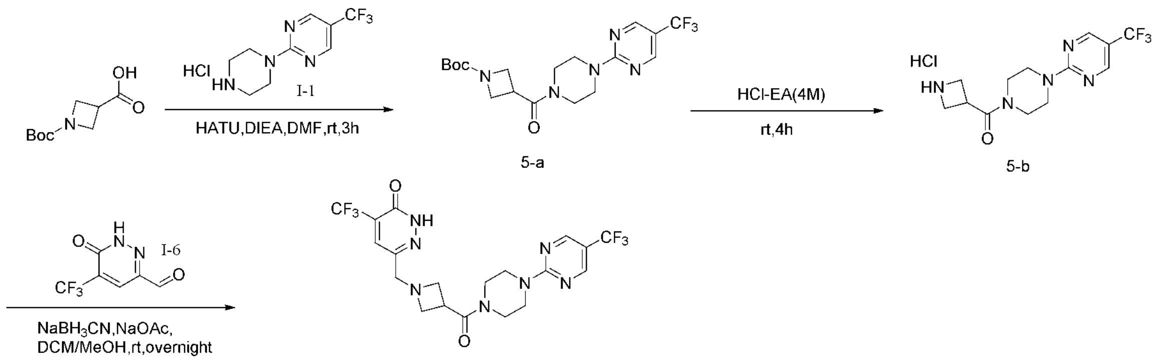


5

**Step 1: Preparation of tert-butyl 3-(4-(5-(trifluoromethyl)pyrimidin-2-yl)piperazine-1-carbonyl)azetidine-1-carboxylate (5-a)**

1-(tert-butoxycarbonyl)azetidine-3-carboxylic acid (1 g, 4.97 mmol) was dissolved in dichloromethane (50 mL), and *O*-(7-azabenzotriazol-1-yl)-*N,N,N',N'*-tetramethyluronium hexafluorophosphate (1.89 g, 4.97 mmol), *N,N*-diisopropylethylamine (2.6 mL, 14.91 mmol), and 2-(piperazin-1-yl)-5-(trifluoromethyl)pyrimidine hydrochloride (I-1, 1.6 g, 5.96 mmol) were added. The mixture was reacted at room temperature for 3 hours. The reaction mixture was filtered and concentrated under reduced pressure, then purified by Flash chromatography (petroleum ether:ethyl acetate = 1:1) to obtain the target product tert-butyl 3-(4-(5-(trifluoromethyl)pyrimidin-2-yl)piperazine-1-carbonyl)azetidine-1-carboxylate (5-a, 2.041 g, yield 98.86%). ESI$[M+H-56]^+$ =360.0 ESI$[2M+H]^+$=831.3

**Step 2: Preparation of azetidin-3-yl(4-(5-(trifluoromethyl)pyrimidin-2-yl)piperazin-1-yl)methanone hydrochloride (5-b)**

tert-butyl 3-(4-(5-(trifluoromethyl)pyrimidin-2-yl)piperazine-1-carbonyl)azetidine-1-carboxylate (5-a, 2.041 g, 4.91 mmol) was dissolved in ethyl acetate (20 mL), and 4M hydrogen chloride in ethyl acetate solution (40 mL) was added dropwise slowly. The mixture was stirred at room temperature for 4 hours. The reaction mixture was directly distilled

under reduced pressure to obtain the crude target product azetidin-3-yl(4-(5-(trifluoromethyl)pyrimidin-2-yl)piperazin-1-yl)methanone hydrochloride (5-b, 2 g, yield 99.52%). ESI[M+H]$^+$=316.1, ESI[2M+H]$^+$=631.4

**Step 3: Preparation of 4-(trifluoromethyl)-6-(((3-(4-(5-(trifluoromethyl)pyrimidin-2-yl)piperazine-1-carbonyl) azetidin-1-yl)methyl)pyridazin-3(2*H*)-one (5)**

azetidin-3-yl(4-(5-(trifluoromethyl)pyrimidin-2-yl)piperazin-1-yl)methanone hydrochloride (5-b, 50 mg, 0.159 mmol) was dissolved in methanol (1 mL) and dichloromethane (1 mL). Then, 6-oxo-5-(trifluoromethyl)-1,6-dihydropyridazine-3-carbaldehyde (I-6, 37 mg, 0.19 mmol) was added, followed by sodium acetate (6.5 mg, 0.08 mmol) at 0°C. The mixture was stirred at 0°C for 20 minutes, and then sodium cyanoborohydride (30 mg, 0.477 mmol) was added at 0°C. The reaction was stirred at room temperature overnight. Saturated brine was added, and the mixture was extracted with ethyl acetate. The organic phase was concentrated under reduced pressure and purified by preparative thin-layer chromatography silica gel plate (dichloromethane:methanol = 10:1), and then by preparative HPLC [Pack Column 20ID*250mm*10μm; A:$H_2O$(0.1%FA), B:ACN, B%: 10%-70% over 22 min] to obtain the target product 4-(trifluoromethyl)-6-(((3-(4-(5-(trifluoromethyl)pyrimidin-2-yl)piperazine-1-carbonyl)azetidin-1-yl)methyl)pyridazin-3(2H)-one (5, 1.27 mg, yield 1.63%). ESI[M+H]$^+$=492.30 $^1$HNMR (400 MHz, DMSO-$d_6$) δ 8.72 (S, 2H), 7.79 (S, 1H), 3.81 (d, J = 4.8 Hz, 4H), 3.54 (d, J = 7.2 Hz, 3H), 3.50 (S, 2H), 3.47 (d, J = 8.0 Hz, 2H), 3.28 - 3.23 (m, 4H).

**Embodiment 6: Preparation of 4-(trifluoromethyl)-5-((((2*S*)-1-((3*S*)-3-(3-(5-(trifluoromethyl)pyrimidin-2-yl)-3,8-diazabicyclo[3.2.1]octane-8-carbonyl)pyrrolidin-1-yl)propan-2-yl)amino)pyridazin-3(2*H*)-one (6)**

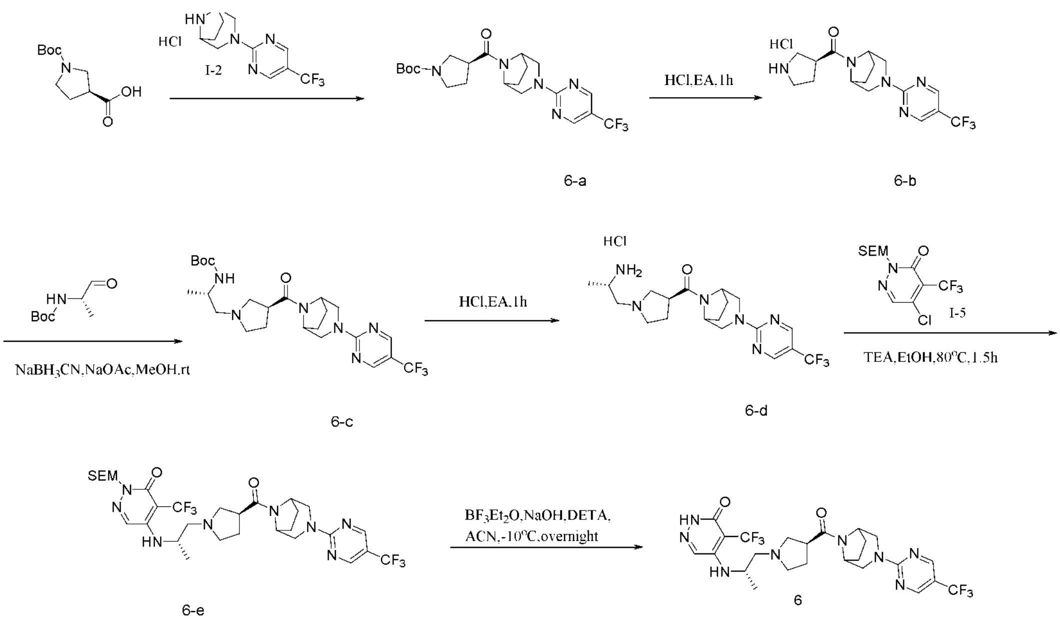

6-a
6-b
6-c
6-d
6-e
6

**Step 1: Preparation of tert-butyl (3*S*)-3-(3-(5-(trifluoromethyl)pyrimidin-2-yl)-3,8-diazabicyclo[3.2.1]octane-8-carbonyl)pyrrolidine-1-carboxylate (6-a)**

(*S*)-1-(tert-butoxycarbonyl)pyrrolidine-3-carboxylic acid (0.1 g, 0.465 mmol) was dissolved in *N,N*-dimethylformamide (4 mL). Under an ice bath, *O*-(7-azabenzotriazol-1-yl)-*N,N,N',N'*-tetramethyluronium hexafluorophosphate (0.177 g, 0.465 mmol) and *N,N*-diisopropylethylamine (243 μL, 1.395 mmol) were added. After stirring under an ice bath for 30 minutes, 3-(5-(trifluoromethyl)pyrimidin-2-yl)-3,8-diazabicyclo[3.2.1]octane hydrochloride (I-2, 0.15 g, 0.581 mmol) was added. After reacting at room temperature for 3 hours, the reaction mixture was added dropwise to water (40 mL) with stirring. The mixture was filtered, and the filter cake was concentrated under reduced pressure to obtain the target product tert-butyl (3*S*)-3-(3-(5-(trifluoromethyl)pyrimidin-2-yl)-3,8-diazabicyclo[3.2.1]octane-8-carbonyl)pyrrolidine-1-carboxylate (6-a, 0.1 g, yield 47.26%). (ESI) [M+H]$^+$=456.6

**Step 2: Preparation of ((*S*)-pyrrolidin-3-yl)(3-(5-(trifluoromethyl)pyrimidin-2-yl)-3,8-diazabicyclo[3.2.1]octan-8-yl)methanone hydrochloride (6-b)**

To tert-butyl (3S)-3-(3-(5-(trifluoromethyl)pyrimidin-2-yl)-3,8-diazabicyclo[3.2.1]octane-8-carbonyl)pyrrolidine-1-carboxylate (6-a, 0.1 g, 0.219 mmol) was added 4M hydrogen chloride in ethyl acetate solution (2 mL). After reacting at room temperature for 3 hours, the mixture was concentrated under reduced pressure to obtain the crude target product ((*S*)-pyrrolidin-3-yl)(3-(5-(trifluoromethyl)pyrimidin-2-yl)-3,8-diazabicyclo[3.2.1]octan-8-yl)methanone hydrochloride (6-b, 0.1 g). (ESI) $[M+H]^+$=356.5

**Step 3: Preparation of tert-butyl ((2*S*)-1-((3*S*)-3-(3-(5-(trifluoromethyl)pyrimidin-2-yl)-3,8-diazabicyclo[3.2.1]octane-8-carbonyl)pyrrolidin-1-yl)propan-2-yl)carbamate (6-c)**

((*S*)-pyrrolidin-3-yl)(3-(5-(trifluoromethyl)pyrimidin-2-yl)-3,8-diazabicyclo[3.2.1]octan-8-yl)methanone hydrochloride (6-b, 50 mg, 0.14 mmol) and tert-butyl (S)-(1-oxopropan-2-yl)carbamate (29.5 mg, 0.17 mmol) were dissolved in methanol (3 mL). Then, sodium acetate (6 mg, 0.07 mmol) was added under an ice bath and stirred for 20 minutes. Finally, sodium cyanoborohydride (26.5 mg, 0.42 mmol) was added slowly, and the reaction was stirred at room temperature for 3 hours. After monitoring showed the reaction was complete, it was quenched with water and extracted with dichloromethane. The organic phase was collected and washed with saturated sodium chloride solution, dried over anhydrous sodium sulfate, filtered, and concentrated under reduced pressure. The residue was purified by preparative TLC silica gel plate (dichloromethane/methanol = 10:1) to obtain tert-butyl ((2*S*)-1-((3*S*)-3-(3-(5-(trifluoromethyl)pyrimidin-2-yl)-3,8-diazabicyclo[3.2.1]octane-8-carbonyl)pyrrolidin-1-yl)propan-2-yl)carbamate (6-c, 39 mg, yield 54%), ESI $[M+H]^+$=513.5.

**Step 4: Preparation of ((*S*)-1-((*S*)-2-aminopropyl)pyrrolidin-3-yl)(3-(5-(trifluoromethyl)pyrimidin-2-yl). 3,8-diazabicyclo[3.2.1]octan-8-yl)methanone hydrochloride (6-d)**

tert-butyl ((2*S*)-1-((3*S*)-3-(3-(5-(trifluoromethyl)pyrimidin-2-yl)-3,8-diazabicyclo[3.2.1]octane-8-carbonyl)pyrrolidin-1-yl)propan-2-yl)carbamate (6-c, 39 mg, 0.076 mmol) was dissolved in 4 mol/L hydrogen chloride in ethyl acetate solution and reacted at room temperature for 1 hour. After monitoring showed the reaction was complete, the mixture was concentrated under reduced pressure to obtain the target product ((S)-1-((*S*)-2-aminopropyl)pyrrolidin-3-yl)(3-(5-(trifluoromethyl)pyrimidin-2-yl)-3,8-diazabicyclo[3.2.1]octan-8-yl)methanone hydrochloride (6-d, 45 mg, yield 143%). ESI $[M+H]^+$=413.7

**Step 5: Preparation of 4-(trifluoromethyl)-5-((((2*S*)-1-((3*S*)-3-(3-(5-(trifluoromethyl)pyrimidin-2-yl)-3,8-diazabicyclo[3.2.1]octane-8-carbonyl)pyrrolidin-1-yl)propan-2-yl)amino)-2-((2-(trimethylsilyl)ethoxy)methyl)pyridazin-3(2*H*)-one (6-e)**

((*S*)-1-((*S*)-2-aminopropyl)pyrrolidin-3-yl)(3-(5-(trifluoromethyl)pyrimidin-2-yl)-3,8-diazabicyclo[3.2.1]octan-8-yl)methanone (6-d, 45 mg, 0.109 mmol), 5-chloro-4-(trifluoromethyl)-2-((2-(trimethylsilyl)ethoxy)methyl)pyridazin-3(2*H*)-one (I-5, 49.4 mg, 0.15 mmol), and triethylamine (30.4 mg, 0.30 mmol) were dissolved in ethanol (2 mL) and heated to 80°C to react for 1.5 hours. After monitoring showed the reaction was complete, the mixture was concentrated under reduced pressure and purified by preparative TLC silica gel plate (pure ethyl acetate) to obtain the target product 4-(trifluoromethyl)-5-((((2S)-1-((3S)-3-(3-(5-(trifluoromethyl)pyrimidin-2-yl)-3,8-diazabicyclo[3.2.1]octane-8-carbonyl)pyrrolidin-1-yl)propan-2-yl)amino)-2-((2-(trimethylsilyl)ethoxy)methyl)pyridazin-3(2*H*)-one (6-e, 32 mg, yield 41.6%). ESI$[M+H]^+$ =705.6

**Step 6: Preparation of 4-(trifluoromethyl)-5-((((2*S*)-1-((3*S*)-3-(3-(5-(trifluoromethyl)pyrimidin-2-yl)-3,8-diazabicyclo[3.2.1]octane-8-carbonyl)pyrrolidin-1-yl)propan-2-yl)amino)pyridazin-3(2*H*)-one (6)**

4-(trifluoromethyl)-5-((((2*S*)-1-((3*S*)-3-(3-(5-(trifluoromethyl)pyrimidin-2-yl)-3,8-diazabicyclo[3.2.1]octane-8-carbonyl)pyrrolidin-1-yl)propan-2-yl)amino)-2-((2-(trimethylsilyl)ethoxy)methyl)pyridazin-3(2*H*)-one (6-e, 32 mg, 0.045 mmol) was dissolved in acetonitrile (3 mL). Boron trifluoride etherate (38.5 mg, 0.27 mmol) was added dropwise slowly at -10°C. After monitoring showed complete reaction to the intermediate, 1 mol/L aqueous sodium hydroxide solution (9 mL) and diethylenetriamine (16.4 mg, 0.159 mmol) were added dropwise slowly in sequence. The reaction was stirred from -10°C to room temperature overnight. After monitoring showed the reaction was complete, it was quenched with water and extracted with ethyl acetate. The organic phase was collected and washed with saturated sodium chloride solution, dried over anhydrous sodium sulfate, filtered, and concentrated under reduced pressure. The residue was purified by preparative HPLC [Pack Column 20ID*250mm*10μm; A:$H_2O$(0.1%FA), B:ACN, B%: 5%-50% over 22 min] to obtain

the target product 4-(trifluoromethyl)-5-((((2*S*)-1-((3*S*)-3-(3-(5-(trifluoromethyl)pyrimidin-2-yl)-3,8-diazabicyclo[3.2.1]octane-8-carbonyl)pyrrolidin-1-yl)propan-2-yl)amino)pyridazin-3(2*H)*-one (6, 8.2 mg, yield 31.4%). ESI[M+H]$^+$=575.4 $^1$H NMR (400 MHz, DMSO-$d_6$) δ 12.44 (S, 1H), 8.72 (S, 2H), 8.17 (S, 1H), 7.92 (S, 1H), 4.47 (d, J = 13.0 Hz, 2H), 3.40 (S, 2H), 3.16-2.98 (m, 4H), 2.98-2.84 (m, 2H), 2.65-2.55 (m, 2H), 2.43 (d, J = 8.3 Hz, 2H), 1.93 (dd, J = 23.9, 14.7 Hz, 4H), 1.57 (dd, J = 22.2, 10.5 Hz, 2H), 1.18 (d, J = 6.2 Hz, 3H).

**Embodiment 7: Preparation of 3-(trifluoromethyl)-5-((((4-(3-(5-(trifluoromethyl)pyrimidin-2-yl)-3,8-diazabicyclo [3.2.1]octane-8-carbonyl)phenyl)amino)methyl)pyridin-2(1*H*)-one (7)**

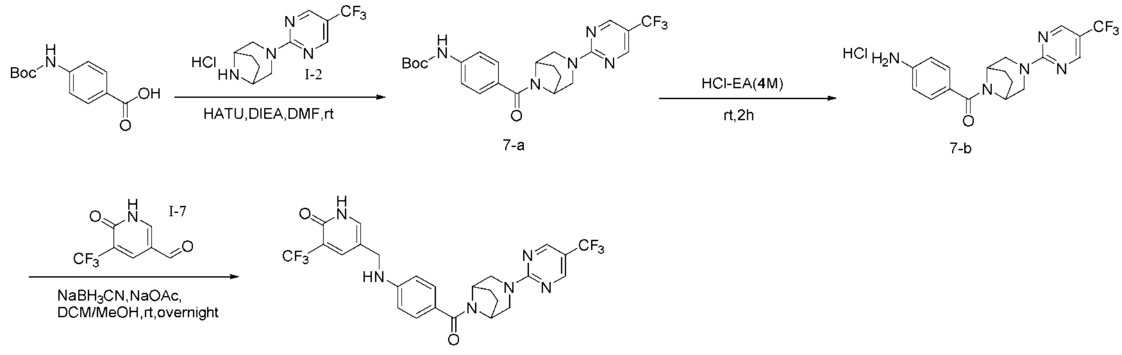


7

**Step 1: Preparation of tert-butyl (4-(3-(5-(trifluoromethyl)pyrimidin-2-yl)-3,8-diazabicyclo[3.2.1]octane-8-carbonyl)phenyl)carbamate (7-a)**

4-((tert-butoxycarbonyl)amino)benzoic acid (100 mg, 0.421 mmol) was dissolved in dichloromethane (5 mL), and *O*-(7-azabenzotriazol-1-yl)-*N,N,N',N'*-tetramethyluronium hexafluorophosphate (160 mg, 0.421 mmol), *N,N*-diisopropylethylamine (220 μL, 1.263 mmol), and 3-(5-(trifluoromethyl)pyrimidin-2-yl)-3,8-diazabicyclo[3.2.1]octane hydrochloride (I-2, 130 mg, 0.505 mmol) were added. The mixture was reacted at room temperature overnight. The reaction mixture was filtered and concentrated under reduced pressure, then purified by Flash chromatography (petroleum ether:ethyl acetate = 1:1) to obtain the target product tert-butyl (4-(3-(5-(trifluoromethyl)pyrimidin-2-yl)-3,8-diazabicyclo [3.2.1]octane-8-carbonyl)phenyl)carbamate (7-a, 176 mg, yield 87.45%). ESI[M+H]$^+$=478.2 ESI[2M+H]$^+$=955.5

**Step 2: Preparation of (4-aminophenyl)(3-(5-(trifluoromethyl)pyrimidin-2-yl)-3,8-diazabicyclo[3.2.1]octan-8-yl) methanone hydrochloride (7-b)**

To tert-butyl (4-(3-(5-(trifluoromethyl)pyrimidin-2-yl)-3,8-diazabicyclo[3.2.1]octane-8-carbonyl)phenyl)carbamate (7-a, 176 mg, 0.369 mmol) was added dropwise slowly 4M hydrogen chloride in ethyl acetate solution (3 mL), and the mixture was stirred at room temperature for 2 hours. After adding 10 mL of ethyl acetate to the reaction mixture, it was filtered. The filter cake was directly distilled under reduced pressure to obtain the crude target product (4-aminophenyl)(3-(5-(trifluoromethyl)pyrimidin-2-yl)-3,8-diazabicyclo[3.2.1]octan-8-yl)methanone hydrochloride (7-b, 164 mg). ESI[M+H]$^+$=378.2 ESI[2M+H]$^+$=755.5

**Step 3: Preparation of 3-(trifluoromethyl)-5-((((4-(3-(5-(trifluoromethyl)pyrimidin-2-yl)-3,8-diazabicyclo[3.2.1] octane-8-carbonyl)phenyl)amino)methyl)pyridin-2(1*H*)-one (7)**

(4-aminophenyl)(3-(5-(trifluoromethyl)pyrimidin-2-yl)-3,8-diazabicyclo[3.2.1]octan-8-yl)methanone hydrochloride (7-b, 74 mg, 0.196 mmol) was dissolved in methanol (1 mL) and dichloromethane (1 mL). Then, 6-oxo-5-(trifluoromethyl)-1,6-dihydropyridine-3-carbaldehyde (I-7, 49 mg, 0.255 mmol) was added, followed by sodium acetate (8 mg, 0.098 mmol) at 0°C. The mixture was stirred at 0°C for 20 minutes, and then sodium cyanoborohydride (25 mg, 0.588 mmol) was added at 0°C. The reaction was stirred at room temperature overnight. Saturated brine was added, and the mixture was extracted with ethyl acetate. The organic phase was concentrated under reduced pressure and purified by preparative thin-layer chromatography silica gel plate (dichloromethane:methanol = 10:1), and then by preparative thin-layer chromatography silica gel plate (pure ethyl acetate) to obtain the target product 3-(trifluoromethyl)-5-((((4-(3-(5-(trifluoromethyl)pyrimidin-2-yl)-3,8-diazabicyclo[3.2.1]octane-8-carbonyl)phenyl)amino)methyl)pyridin-2(1*H*)-one (7, 4.04

mg, yield 3.73%). ESI[M+H]$^+$=553.40 $^1$HNMR (400 MHz, DMSO-$d_6$) δ 8.71 (S, 2H), 7.97 (S, 1H), 7.68 (S, 1H), 7.35 (d, J = 8.4 Hz, 2H), 6.66 (dd, J = 17.7, 7.3 Hz, 3H), 4.49 (d, J = 12.6 Hz, 3H), 4.11 (d, J = 5.6 Hz, 2H), 3.23 (d, J = 12.3 Hz, 3H), 1.80 (d, J = 4.0 Hz, 2H), 1.56 (d, J = 7.4 Hz, 2H).

**Embodiment 8: Preparation of 5-(((((1*R*,3*R*)-3-((*R*)-2-methyl-4-(5-(trifluoromethyl)pyrimidin-2-yl)piperazine-1-carbonyl)cyclobutyl)amino)methyl)-3-(trifluoromethyl)pyridin-2(1*H*)-one (8)**

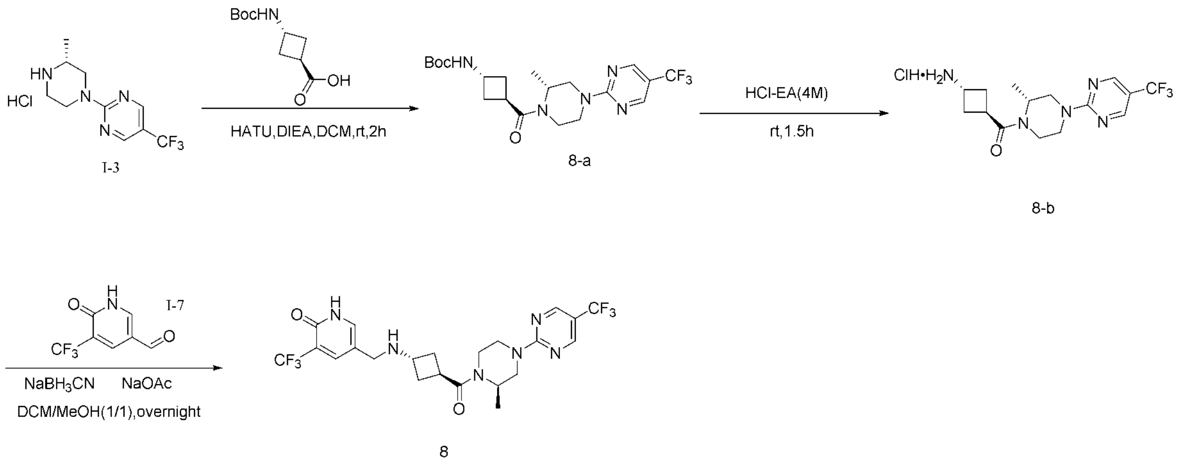


**Step 1: Preparation of tert-butyl ((1*R*,3*R*)-3-((*R*)-2-methyl-4-(5-(trifluoromethyl)pyrimidin-2-yl)piperazine-1-carbonyl)cyclobutyl)carbamate (8-a)**

(1*R*,3*R*)-3-((tert-butoxycarbonyl)amino)cyclobutane-1-carboxylic acid (80 mg, 0.372 mmol) was dissolved in dichloromethane (4 mL), and *O*-(7-azabenzotriazol-1-yl)-*N,N,N',N'*-tetramethyluronium hexafluorophosphate (141 mg, 0.372 mmol), *N,N*-diisopropylethylamine (198 μL, 1.116 mmol), and (R)-2-(3-methylpiperazin-1-yl)-5-(trifluoromethyl) pyrimidine hydrochloride (I-3, 100 mg, 0.409 mmol) were added. The mixture was reacted at room temperature for 2 hours. The reaction mixture was filtered and concentrated under reduced pressure, then purified by Flash chromatography (petroleum ether:ethyl acetate = 1:1) to obtain the target product tert-butyl ((1*R*,3*R*)-3-((*R*)-2-methyl-4-(5-(trifluoromethyl) pyrimidin-2-yl)piperazine-1-carbonyl)cyclobutyl)carbamate (8-a, 138 mg, yield 83.73%). ESI[M+H]$^+$ =444.7 ESI [M+H-56]$^+$ = 388.6

**Step 2: Preparation of ((1*R*,3*R*)-3-aminocyclobutyl)((*R*)-2-methyl-4-(5-(trifluoromethyl)pyrimidin-2-yl)piperazin-1-yl)methanone hydrochloride (8-b)**

To tert-butyl ((1*R*,3*R*)-3-((*R*)-2-methyl-4-(5-(trifluoromethyl)pyrimidin-2-yl)piperazine-1-carbonyl)cyclobutyl) carbamate (8-a, 138 mg, 0.311 mmol) was added dropwise slowly 4M hydrogen chloride in ethyl acetate solution (3 mL), and the mixture was stirred at room temperature for 1.5 hours. After adding 4 mL of ethyl acetate to the reaction mixture, it was filtered. The filter cake was dried to obtain the crude target product ((1*R*,3*R*)-3-aminocyclobutyl)((*R*)-2-methyl-4-(5-(trifluoromethyl)pyrimidin-2-yl)piperazin-1-yl)methanone hydrochloride (8-b, 157 mg, yield 132.84%). ESI [M+H]$^+$ = 344.6

**Step 3: Preparation of 5-(((((1*R*,3*R*)-3-((*R*)-2-methyl-4-(5-(trifluoromethyl)pyrimidin-2-yl)piperazine-1-carbonyl) cyclobutyl)amino)methyl)-3-(trifluoromethyl)pyridin-2(1*H*)-one (8)**

((1*R*,3*R*)-3-aminocyclobutyl)((*R*)-2-methyl-4-(5-(trifluoromethyl)pyrimidin-2-yl)piperazin-1-yl)methanone hydrochloride (8-b, 100 mg, 0.263 mmol) was dissolved in methanol (1 mL) and dichloromethane (1 mL). Then, 6-oxo-5-(trifluoromethyl)-1,6-dihydropyridine-3-carbaldehyde (I-7, 60 mg, 0.316 mmol) was added, followed by sodium acetate (11 mg, 0.132 mmol) at 0°C. The mixture was stirred at 0°C for 20 minutes, and then sodium cyanoborohydride (50 mg, 0.79 mmol) was added at 0°C. The reaction was stirred at room temperature overnight. Saturated brine was added, and the mixture was extracted with ethyl acetate. The organic phase was concentrated under reduced pressure and purified by preparative thin-layer chromatography silica gel plate (dichloromethane:methanol = 8:1), and then by preparative HPLC [Pack Column 20ID*250mm*10μm; A:$H_2O$(0.1%FA), B:ACN, B%: 10%-70% over 22 min] to obtain

the target product *5-(((((1R,3R)-3-((R)-2*-methyl-4-(5-(trifluoromethyl)pyrimidin-2-yl)piperazine-1-carbonyl)cyclobutyl) amino)methyl)-3-(trifluoromethyl)pyridin-2(1*H*)-one (8, 2.63 mg, yield 1.93%). ESI$[M+H]^+$=519.20

**Embodiment 9: Preparation of 3-(trifluoromethyl)-5-(((3*S*)-3-(3-(5-(trifluoromethyl)pyrimidin-2-yl)-3,8-diazabicyclo[3.2.1]octane-8-carbonyl)pyrrolidin-1-yl)methyl)pyridin-2(1*H*)-one (9)**

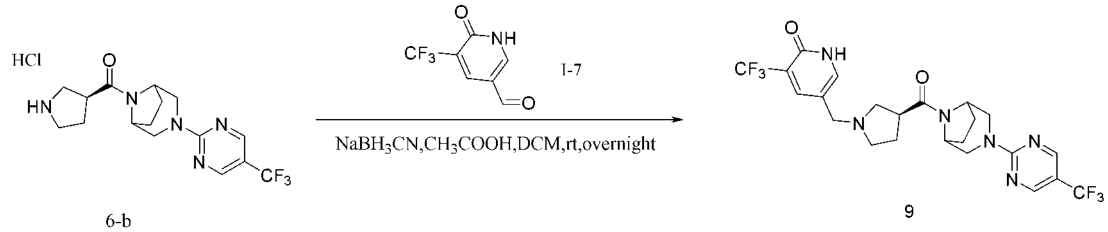


6-b

9

((*S*)-pyrrolidin-3-yl)(3-(5-(trifluoromethyl)pyrimidin-2-yl)-3,8-diazabicyclo[3.2.1]octan-8-yl)methanone hydrochloride (6-b, 30 mg, 0.0845 mmol) and 6-oxo-5-(trifluoromethyl)-1,6-dihydropyridine-3-carbaldehyde (I-7, 24.2 mg, 0.127 mmol) were dissolved in dichloromethane (1.5 mL). Acetic acid (2.5 mg, 0.0417 mmol) was added at 0°C. After stirring for 20 minutes, sodium cyanoborohydride (15.9 mg, 0.253 mmol) was added, and the reaction was stirred at room temperature overnight. After monitoring showed the reaction was complete, a large amount of water was added, and the mixture was extracted with dichloromethane:methanol = 10:1. The organic phase was dried over anhydrous sodium sulfate, filtered, and concentrated under reduced pressure. The residue was purified by preparative HPLC [Pack Column 20ID*250mm*10μm; A:$H_2O$(0.1%FA), B:ACN, B%: 5%-50% over 22 min] to obtain 3-(trifluoromethyl)-5-(((3*S*)-3-(3-(5-(trifluoromethyl)pyrimidin-2-yl)-3,8-diazabicyclo[3.2.1]octane-8-carbonyl)pyrrolidin-1-yl) methyl)pyridin-2(1*H*)-one (9, 1.24 mg, yield 3.05%). ESI $[M+H]^+$=531.2 $^1H$ NMR (400 MHz, DMSO-$d_6$) δ 12.29 (s, 1H), 8.72 (s, 2H), 7.92 (d, J = 6.5 Hz, 1H), 7.84 (s, 1H), 7.70 (d, J = 5.5 Hz, 1H), 7.59 (s, 1H), 6.30 (t, J = 6.7 Hz, 1H), 4.64 (d, J = 5.8 Hz, 1H), 4.55 - 4.42 (m, 2H), 3.06 (dd, J = 27.3, 14.6 Hz, 2H), 2.85 - 2.75 (m, 1H), 2.67 - 2.59 (m, 1H), 2.59 - 2.52 (m, 1H), 2.45 (d, J = 8.2 Hz, 1H), 2.42 - 2.31 (m, 1H), 2.00 (s, 1H), 1.94 - 1.78 (m, 2H), 1.74 (s, 1H), 1.56 (dd, J = 20.8, 11.0 Hz, 2H).

**Embodiment 10: Preparation of 3-(trifluoromethyl)-5-(((3*R*)-3-(3-(5-(trifluoromethyl)pyrimidin-2-yl)-3,8-diazabicyclo[3.2.1]octane-8-carbonyl)pyrrolidin-1-yl)methyl)pyridin-2(1*H*)-one (10)**

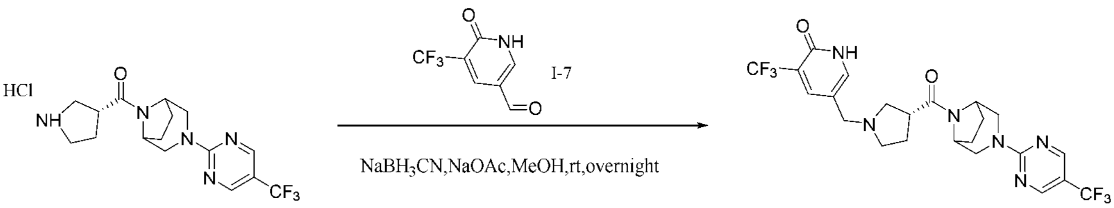


2-b

10

((*R*)-pyrrolidin-3-yl)(3-(5-(trifluoromethyl)pyrimidin-2-yl)-3,8-diazabicyclo[3.2.1]octan-8-yl)methanone hydrochloride (2-b, 30 mg, 0.0845 mmol) and 6-oxo-5-(trifluoromethyl)-1,6-dihydropyridine-3-carbaldehyde (I-7, 24.2 mg, 0.127 mmol) were dissolved in methanol (1.5 mL) under an ice bath. Acetic acid (2.5 mg) was added dropwise. After stirring under an ice bath for 20 minutes, sodium cyanoborohydride (15.9 mg, 0.253 mmol) was added, and the reaction was stirred at room temperature overnight. After monitoring showed the reaction was complete, it was quenched with water and extracted with dichloromethane:methanol = 10:1. The organic phase was dried over anhydrous sodium sulfate, filtered, and concentrated under reduced pressure. The residue was purified by preparative HPLC [Pack Column 20ID*250mm*10μm; A:$H_2O$(0.1%FA), B:ACN, B%: 5%-50% over 22 min] to obtain 3-(trifluoromethyl)-5-(((3*R*)-3-(3-(5-(trifluoromethyl)pyrimidin-2-yl)-3,8-diazabicyclo[3.2.1]octane-8-carbonyl)pyrrolidin-1-yl) methyl)pyridin-2(1*H*)-one (10, 1.01 mg, yield 2.25%). ESI $[M+H]^+$=531.2 $^1H$ NMR (400 MHz, DMSO-$d_6$) δ 12.22 (s, 1H), 8.72 (s, 2H), 7.84 (s, 1H), 7.59 (s, 1H), 4.64 (d, J = 6.2 Hz, 1H), 4.54 - 4.44 (m, 1H), 4.02 (dd, J = 14.2, 7.1 Hz, 1H), 3.60 (s, 4H), 3.10 - 3.00 (m, 1H), 2.83 (dd, J = 20.1, 11.4 Hz, 1H), 2.64 (d, J = 21.0 Hz, 1H), 2.41 - 2.32 (m, 1H), 1.99 (s, 1H), 1.76 (s, 4H), 1.58 (dd, J = 14.5, 7.5 Hz, 1H), 1.16 (dd, J = 14.3, 7.1 Hz, 1H), 0.88 - 0.80 (m, 1H).

**Embodiment 11: Preparation of 4-(trifluoromethyl)-6-(((3*S*)-3-(3-(5-(trifluoromethyl)pyrimidin-2-yl)-3,8-diazabicyclo[3.2.1]octane-8-carbonyl)pyrrolidin-1-yl)methyl)pyridazin-3(2*H*)-one (11)**

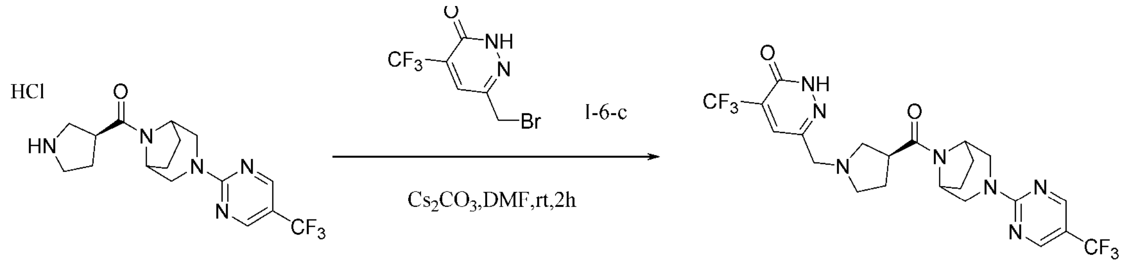


6-b

11

((*S*)-pyrrolidin-3-yl)(3-(5-(trifluoromethyl)pyrimidin-2-yl)-3,8-diazabicyclo[3.2.1]octan-8-yl)methanone (6-b, 50 mg, 0.141 mmol), 6-(bromomethyl)-4-(trifluoromethyl)pyridazin-3(2H)-one (I-6-c, 43.3 mg, 0.169 mmol), and cesium carbonate (137.6 mg, 0.422 mmol) were dissolved in *N*,*N*-dimethylformamide (2 mL), and the reaction was stirred at room temperature for 2 hours. After monitoring showed the reaction was complete, a large amount of water was added, and the mixture was extracted with dichloromethane:methanol = 10:1. The organic phase was dried over anhydrous sodium sulfate, filtered, and concentrated under reduced pressure. The residue was purified by preparative HPLC [Pack Column 20ID*250mm*10μm; A:$H_2O$(0.1%FA), B:ACN, B%: 5%-50% over 22 min] to obtain 4-(trifluoromethyl)-6-(((3S)-3-(3-(5-(trifluoromethyl)pyrimidin-2-yl)-3,8-diazabicyclo[3.2.1]octane-8-carbonyl)pyrrolidin-1-yl)methyl)pyridazin-3(2*H*)-one (11, 1.61 mg, yield 2.15%), ESI $[M+H]^+$=532.1 $^1H$ NMR (400 MHz, DMSO-$d_6$) δ 8.73 (S, 2H), 8.21 (S, 1H), 7.99 (S, 1H), 4.66 (S, 1H), 4.49 (d, J = 13.7 Hz, 2H), 3.10 (dd, J = 28.3, 12.7 Hz, 3H), 2.93 (td, J = 14.0, 7.1 Hz, 2H), 1.91 (S, 3H), 1.76 (S, 2H), 1.60 (dd, J = 23.9, 11.4 Hz, 3H), 1.38 (S, 1H), 1.25 (dd, J = 10.7, 4.5 Hz, 1H), 1.16 (t, J = 7.3 Hz, 1H).

**Embodiment 12: Preparation of 4-(trifluoromethyl)-6-(((((1*R*,3*R*)-3-(4-(5-(trifluoromethyl)pyrimidin-2-yl)piperazine-1-carbonyl)cyclobutyl)amino)methyl)pyridazin-3(2*H*)-one (12)**

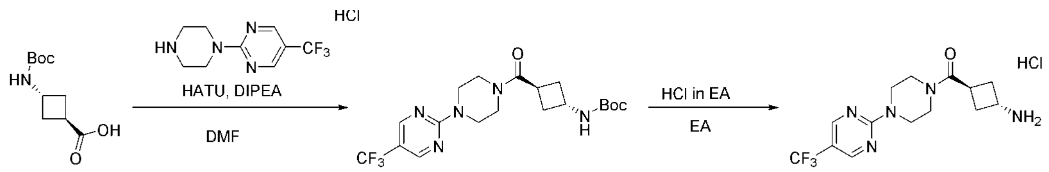


12-a

12-b

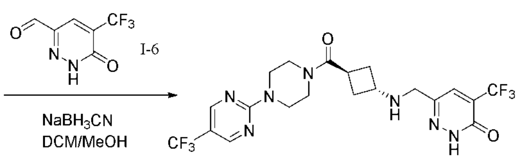


12

**Step 1: Preparation of tert-butyl ((1*R*,3*R*)-3-(4-(5-(trifluoromethyl)pyrimidin-2-yl)piperazine-1-carbonyl)cyclobutyl)carbamate (12-a)**

(1*R*,3*R*)-3-((tert-butoxycarbonyl)amino)cyclobutane-1-carboxylic acid (1 g, 4.65 mmol) was dissolved in N,N-dimethylformamide (10 mL). Under an ice bath, *O*-(7-azabenzotriazol-1-yl)-*N,N,N',N'*-tetramethyluronium hexafluorophosphate (1.77 g, 4.65 mmol) and N,N-diisopropylethylamine (2.44 mL, 13.95 mmol) were added. After stirring under an ice bath for 30 minutes, 2-(piperazin-1-yl)-5-(trifluoromethyl)pyrimidine hydrochloride (1.26 g, 4.65 mmol) was added. After reacting at room temperature for 3 hours, the reaction mixture was added dropwise to water (100 mL) with stirring. The mixture was filtered, and the filter cake was concentrated under reduced pressure to obtain the target product tert-butyl ((1*R*,3*R*)-3-(4-(5-(trifluoromethyl)pyrimidin-2-yl)piperazine-1-carbonyl)cyclobutyl)carbamate (12-a, 1.7 g, yield 85.4%). (ESI) [M+H]$^+$=430.2.

**Step 2: Preparation of ((1*R*,3*R*)-3-aminocyclobutyl)(4-(5-(trifluoromethyl)pyrimidin-2-yl)piperazin-1-yl)methanone hydrochloride (12-b)**

tert-butyl ((1*R*,3*R*)-3-(4-(5-(trifluoromethyl)pyrimidin-2-yl)piperazine-1-carbonyl)cyclobutyl)carbamate (12-a, 1.7 g, 3.96 mmol) was dissolved in ethyl acetate (10 mL), and 4M hydrogen chloride in ethyl acetate solution (20 mL) was added under an ice bath. After reacting at room temperature for 3 hours, the mixture was concentrated under reduced pressure to obtain the target product ((1*R*,3*R*)-3-aminocyclobutyl)(4-(5-(trifluoromethyl)pyrimidin-2-yl)piperazin-1-yl) methanone hydrochloride (12-b, 1.7 g, crude product). (ESI) [M+H]$^+$=330.2.

**Step 3: Preparation of 4-(trifluoromethyl)-6-(((((1*R*,3*R*)-3-(4-(5-(trifluoromethyl)pyrimidin-2-yl)piperazine-1-carbonyl)cyclobutyl)amino)methyl)pyridazin-3(2*H*)-one (12)**

((1*R*,3*R*)-3-aminocyclobutyl)(4-(5-(trifluoromethyl)pyrimidin-2-yl)piperazin-1-yl)methanone hydrochloride (12-b, 500 mg, 1.37 mmol) and 6-oxo-5-(trifluoromethyl)-1,6-dihydropyridazine-3-carbaldehyde (I-6, 368 mg, 1.91 mmol) were dissolved in methanol (5 mL) and dichloromethane (10 mL). Acetic acid (78 μL) was added under an ice bath. After stirring under an ice bath for 30 minutes, sodium cyanoborohydride (257.8 mg, 4.10 mmol) was added. After reacting at room temperature for 3 hours, the reaction mixture was concentrated under reduced pressure. After purification by column chromatography, the crude product was slurried with ethyl acetate to obtain the target product 4-(trifluoromethyl)-6-(((((1*R*,3*R*)-3-(4-(5-(trifluoromethyl)pyrimidin-2-yl)piperazine-1-carbonyl)cyclobutyl)amino)methyl)pyridazin-3(2*H*)-one (12, 150.25 mg, yield 21.7%). ESI [M+H]$^+$=506.2. $^1$H NMR (400 MHz, $CDCl_3$) δ 8.51 (s, 2H), 7.71 (s, 1H), 3.90 (s, 4H), 3.72 (s, 4H), 3.56 - 3.39 (m, 3H), 3.30 (s, 2H), 2.60 (s, 2H), 2.05 (d, J = 10.5 Hz, 2H).

**Embodiment 13: Preparation of 3-(trifluoromethyl)-5-(((((1*R*,3*R*)-3-(4-(5-(trifluoromethyl)pyrimidin-2-yl)piperazine-1-carbonyl)cyclobutyl)amino)methyl)pyridin-2(1*H*)-one (13)**

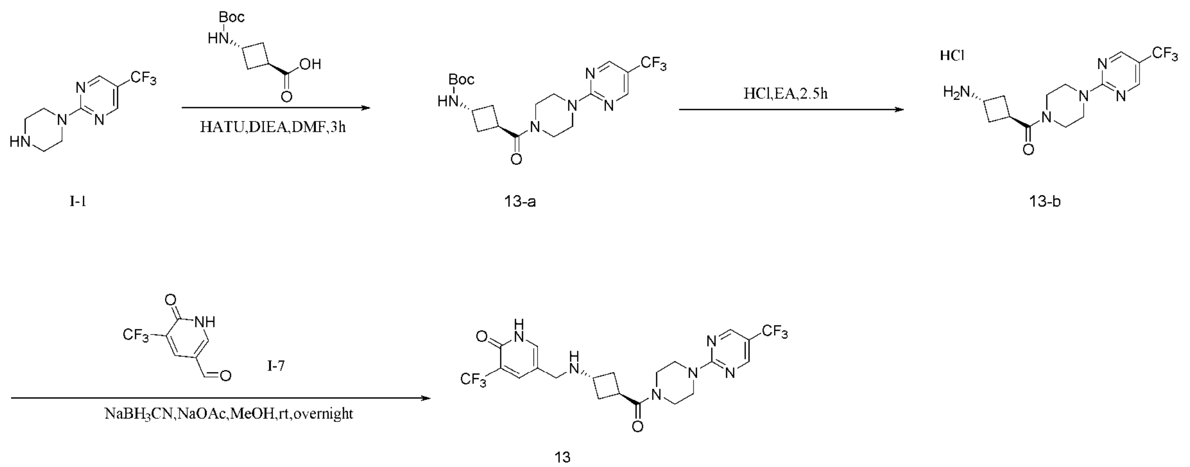


I-1 13-a 13-b

13

**Step 1: Preparation of tert-butyl ((1*R*,3*R*)-3-(4-(5-(trifluoromethyl)pyrimidin-2-yl)piperazine-1-carbonyl)cyclobutyl)carbamate (13-a)**

(1*R*,3*R*)-3-((tert-butoxycarbonyl)amino)cyclobutane-1-carboxylic acid (50 mg, 0.232 mmol), *O*-(7-azabenzotriazol-1-yl)-*N,N,N',N'*-tetramethyluronium hexafluorophosphate (88.5 mg, 0.232 mmol), and diisopropylethylamine (89.89 mg, 0.696 mmol) were dissolved in *N,N*-dimethylformamide (2 mL), and finally 2-(piperazin-1-yl)-5-(trifluoromethyl)pyrimidine (I-1, 63 mg, 0.234 mmol) was added. The reaction was stirred at room temperature for 3 hours. After monitoring showed the reaction was complete, ice water was added and stirred thoroughly. The mixture was filtered, and the filter cake was collected to obtain the target product tert-butyl ((1*R*,3*R*)-3-(4-(5-(trifluoromethyl)pyrimidin-2-yl)piperazine-1-carbonyl)cyclobutyl)carbamate (13-a, 85.6 mg, yield 85.9%). ESI[M+H]$^+$ =430.2

**Step 2: Preparation of ((1*R*,3*R*)-3-aminocyclobutyl)(4-(5-(trifluoromethyl)pyrimidin-2-yl)piperazin-1-yl)methanone hydrochloride (13-b)**

tert-butyl ((1*R*,3*R*)-3-(4-(5-(trifluoromethyl)pyrimidin-2-yl)piperazine-1-carbonyl)cyclobutyl)carbamate (13-a, 85.6 mg, 0.199 mmol) was dissolved in 4 mol/L hydrogen chloride in ethyl acetate solution and reacted at room temperature overnight. After monitoring showed the reaction was complete, the mixture was concentrated under reduced pressure to obtain the target product ((1*R*,3*R*)-3-aminocyclobutyl)(4-(5-(trifluoromethyl)pyrimidin-2-yl)piperazin-1-yl) methanone hydrochloride (13-b, 94.6 mg, yield 144%). ESI$[M+H]^+$ =330.1

**Step 3: Preparation of 3-(trifluoromethyl)-5-(((((1*R*,3*R*)-3-(4-(5-(trifluoromethyl)pyrimidin-2-yl)piperazine-1-carbonyl)cyclobutyl)amino)methyl)pyridin-2(1*H*)-one (13)**

((1*R*,3*R*)-3-aminocyclobutyl)(4-(5-(trifluoromethyl)pyrimidin-2-yl)piperazin-1-yl)methanone (13-b, 30 mg, 0.0739 mmol) and 6-oxo-5-(trifluoromethyl)-1,6-dihydropyridine-3-carbaldehyde (I-7, 18.8 mg, 0.098 mmol) were dissolved in methanol (2 mL) under an ice bath, and sodium acetate (4 μL) was added dropwise. After stirring under an ice bath for 20 minutes, sodium cyanoborohydride (15.5 mg, 0.246 mmol) was added, and the reaction was stirred at room temperature overnight. After monitoring showed the reaction was complete, it was quenched with water and extracted with dichloromethane:methanol = 10:1. The organic phase was dried over anhydrous sodium sulfate, filtered, and concentrated under reduced pressure. The residue was purified by preparative HPLC [Pack Column 20ID*250mm*10μm; A:$H_2O$(0.1%FA), B:ACN, B%: 5%-50% over 22 min] to obtain 3-(trifluoromethyl)-5-(((((1*R*,3*R*)-3-(4-(5-(trifluoromethyl) pyrimidin-2-yl)piperazine-1-carbonyl)cyclobutyl)amino)methyl)pyridin-2(1*H*)-one (13, 1.59 mg, yield 3.46%), ESI $[M+H]^+$=505.1 $^1H$ NMR (400 MHz, DMSO-$d_6$) δ 8.72 (S, 2H), 8.27 (S, 1H), 7.92 (S, 1H), 7.55 (S, 1H), 3.82 (d, J = 4.3 Hz, 2H), 3.55 (S, 2H), 3.39 (S, 4H), 3.27 (S, 3H), 3.15 (dd, J = 13.8, 6.9 Hz, 2H), 2.31 (dd, J = 10.3, 5.7 Hz, 2H), 1.94 (dd, J = 18.1, 9.7 Hz, 2H).

**Embodiment 14: Preparation of 3-(trifluoromethyl)-5-(((3-(4-(5-(trifluoromethyl)pyrimidin-2-yl)piperazine-1-carbonyl)azetidin-1-yl)methyl)pyridin-2(1*H*)-one (14)**

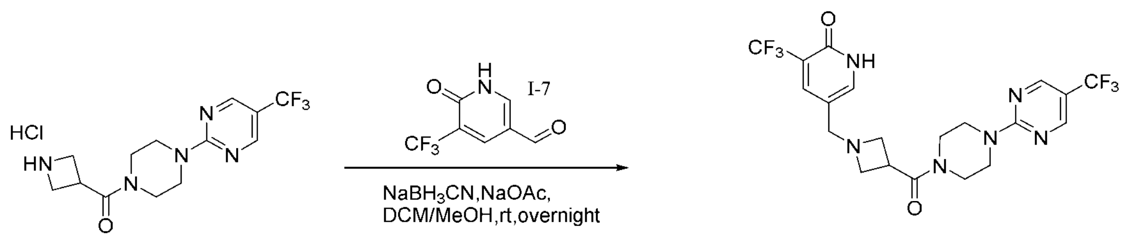


5-b 14

azetidin-3-yl(4-(5-(trifluoromethyl)pyrimidin-2-yl)piperazin-1-yl)methanone hydrochloride (5-b, 100 mg, 0.317 mmol) was dissolved in methanol (1 mL) and dichloromethane (1 mL), and 6-oxo-5-(trifluoromethyl)-1,6-dihydropyridine-3-carbaldehyde (I-7, 73 mg, 0.381 mmol) was added. Sodium acetate (13 mg, 0.159 mmol) was added at 0°C and the mixture was stirred at 0°C for 20 minutes, then sodium cyanoborohydride (60 mg, 0.951 mmol) was added at 0°C. The reaction was stirred at room temperature overnight. Saturated brine was added, and the mixture was extracted with ethyl acetate. The organic phase was concentrated under reduced pressure and purified by preparative thin-layer chromatography silica gel plate (dichloromethane:methanol = 10:1) to obtain the target product 3-(trifluoromethyl)-5-(((3-(4-(5-(trifluoromethyl)pyrimidin-2-yl)piperazine-1-carbonyl)azetidin-1-yl)methyl)pyridin-2(1H)-one (14, 5.37 mg, yield 3.45%). ESI$[M+H]^+$=491.30 $^1$NMR (400 MHz, $CD_3OD$) δ 8.58 (S, 2H), 7.96 (S, 1H), 7.59 (S, 1H), 4.56 (S, 2H), 3.96-3.88 (m, 4H), 3.69-3.64 (m, 2H), 3.57 (t, J = 7.6 Hz, 2H), 3.47 (d, J = 5.4 Hz, 1H), 3.45 (S, 2H), 3.37 (t, J = 7.2 Hz, 2H).

**Embodiment 15: Preparation of 3-(trifluoromethyl)-5-(((((1*R*,4*R*)-4-(4-(5-(trifluoromethyl)pyrimidin-2-yl)piperazine-1-carbonyl)cyclohexyl)amino)methyl)pyridin-2(1*H*)-one (15)**

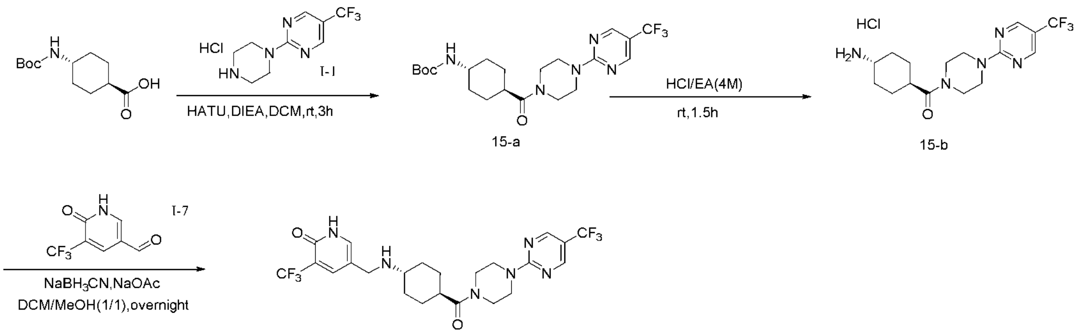


15-a

15-b

15

**Step 1: Preparation of tert-butyl ((1*R*,4*R*)-4-(4-(5-(trifluoromethyl)pyrimidin-2-yl)piperazine-1-carbonyl)cyclohexyl)carbamate (15-a)**

(1*R*,4*R*)-4-((tert-butoxycarbonyl)amino)cyclohexane-1-carboxylic acid (100 mg, 0.411 mmol) was dissolved in dichloromethane (5 mL), *O*-(7-azabenzotriazol-1-yl)-*N,N,N',N'*-tetramethyluronium hexafluorophosphate (156 mg, 0.411 mmol) and *N,N*-diisopropylethylamine (216 μL, 1.233 mmol) were added, and the mixture was reacted for 20 minutes, then 2-(piperazin-1-yl)-5-(trifluoromethyl)pyrimidine hydrochloride (I-1, 133 mg, 0.493 mmol) was added, and the reaction was stirred at room temperature for 3 hours. The reaction mixture was filtered and concentrated under reduced pressure, and the residue was purified by Flash chromatography (petroleum ether:ethyl acetate = 1:1) to obtain the target product tert-butyl ((1*R*,4*R*)-4-(4-(5-(trifluoromethyl)pyrimidin-2-yl)piperazine-1-carbonyl)cyclohexyl)carbamate (15-a, 202 mg, yield 107.45%). ESI $[M+H]^+$=458.7, ESI $[M+H-56]^+$=402.6

**Step 2: Preparation of ((1*R*,4*R*)-4-aminocyclohexyl)(4-(5-(trifluoromethyl)pyrimidin-2-yl)piperazin-1-yl)methanone hydrochloride (15-b)**

To tert-butyl ((1*R*,4*R*)-4-(4-(5-(trifluoromethyl)pyrimidin-2-yl)piperazine-1-carbonyl)cyclohexyl)carbamate (15-a, 202 mg, 0.442 mmol) was added dropwise slowly 4M hydrogen chloride in ethyl acetate solution (6 mL), and the mixture was stirred at room temperature for 1.5 hours. The reaction mixture was diluted with 4 mL of ethyl acetate and concentrated under reduced pressure to obtain the crude target product ((1*R*,4*R*)-4-aminocyclohexyl)(4-(5-(trifluoromethyl)pyrimidin-2-yl)piperazin-1-yl)methanone hydrochloride (15-b, 244 mg, crude product). ESI $[M+H]^+$=358.7

**Step 3: Preparation of 3-(trifluoromethyl)-5-(((((1*R*,4*R*)-4-(4-(5-(trifluoromethyl)pyrimidin-2-yl)piperazine-1-carbonyl)cyclohexyl)amino)methyl)pyridin-2(1*H*)-one (15)**

((1*R*,4*R*)-4-aminocyclohexyl)(4-(5-(trifluoromethyl)pyrimidin-2-yl)piperazin-1-yl)methanone hydrochloride (15-b, 100 mg, 0.255 mmol) was dissolved in methanol (1 mL) and dichloromethane (1 mL), and 6-oxo-5-(trifluoromethyl)-1,6-dihydropyridine-3-carbaldehyde (I-7, 58 mg, 0.305 mmol) was added. Sodium acetate (10 mg, 0.127 mmol) was added at 0°C and the mixture was stirred at 0°C for 20 minutes, then sodium cyanoborohydride (48 mg, 0.764 mmol) was added at 0°C. The reaction was stirred at room temperature overnight. 0.1 mL of water was added, and the mixture was filtered. The organic phase was concentrated under reduced pressure and purified twice by preparative thin-layer chromatography silica gel plate (dichloromethane:methanol = 8:1) to obtain the target product 3-(trifluoromethyl)-5-(((((1*R*,4*R*)-4-(4-(5-(trifluoromethyl)pyrimidin-2-yl)piperazine-1-carbonyl)cyclohexyl)amino)methyl)pyridin-2(1H)-one (15, 2.43 mg, yield 1.79%). ESI $[M+H]^+$=533.2, ESI $[2M+H]^+$=1065.3 $^1$HNMR (400 MHz, $CDCl_3$) δ 8.51 (s, 1H), 8.10 (s,1H), 7.92 (s, 1H), 7.56 (s, 1H), 3.92 (d, J = 20.8 Hz,2H), 3.78 - 3.67 (m, 2H), 3.63 - 3.55 (m, 1H), 2.60 (dd, J = 63.0, 17.2 Hz, 3H), 2.10 (d, J = 5.2 Hz, 2H), 1.86 (d, J = 12.7 Hz, 2H), 1.73 - 1.67 (m, 2H), 1.42 (d, J = 6.6 Hz, 3H), 1.30 (d, J = 8.7 Hz, 4H).

**Embodiment 16: Preparation of 3-(trifluoromethyl)-5-(((((1*S*,3*S*)-3-(4-(5-(trifluoromethyl)pyrimidin-2-yl)piperazine-1-carbonyl)cyclohexyl)amino)methyl)pyridin-2(1*H*)-one (16)**

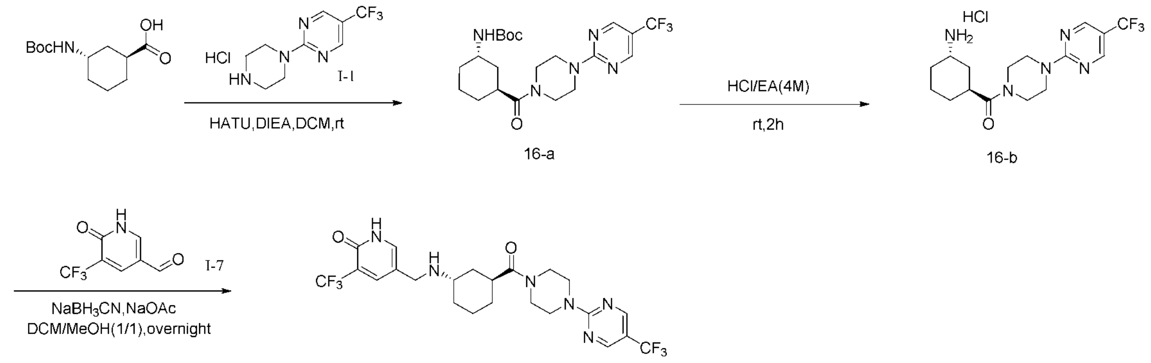


16-a 16-b 16

**Step 1: Preparation of tert-butyl ((1*S*,3*S*)-3-(4-(5-(trifluoromethyl)pyrimidin-2-yl)piperazine-1-carbonyl)cyclohexyl)carbamate (16-a)**

(1*S*,3*S*)-3-((tert-butoxycarbonyl)amino)cyclohexane-1-carboxylic acid (100 mg, 0.411 mmol) was dissolved in dichloromethane (5 mL), *O*-(7-azabenzotriazol-1-yl)-*N,N,N',N'*-tetramethyluronium hexafluorophosphate (156 mg, 0.411 mmol) and *N,N*-diisopropylethylamine (216 μL, 1.233 mmol) were added, and the mixture was reacted for 20 minutes, then 2-(piperazin-1-yl)-5-(trifluoromethyl)pyrimidine hydrochloride (I-1, 133 mg, 0.493 mmol) was added, and the reaction was stirred at room temperature overnight. The reaction mixture was filtered and concentrated under reduced pressure, and the residue was purified by Flash chromatography (petroleum ether:ethyl acetate = 1:1) to obtain the target product tert-butyl ((1S,3S)-3-(4-(5-(trifluoromethyl)pyrimidin-2-yl)piperazine-1-carbonyl)cyclohexyl)carbamate (16-a, 208 mg, yield 110%). ESI $[M+H]^+$=458.1, ESI $[M+H-56]^+$=401.9

**Step 2: Preparation of ((1*S*,3*S*)-3-aminocyclohexyl)(4-(5-(trifluoromethyl)pyrimidin-2-yl)piperazin-1-yl)methanone hydrochloride (16-b)**

To tert-butyl ((1S,3S)-3-(4-(5-(trifluoromethyl)pyrimidin-2-yl)piperazine-1-carbonyl)cyclohexyl)carbamate (16-a, 208 mg, 0.455 mmol) was added dropwise slowly hydrogen chloride in ethyl acetate solution (4M) (3 mL), and the mixture was stirred at room temperature for 2 hours. The reaction mixture was diluted with 4 mL of ethyl acetate and concentrated under reduced pressure to obtain the crude target product ((1S,3S)-3-aminocyclohexyl)(4-(5-(trifluoromethyl)pyrimidin-2-yl)piperazin-1-yl)methanone hydrochloride (16-b, 240 mg, crude product). ESI $[M+H]^+$=358.2, ESI $[2M+H]^+$=715.2

**Step 3: Preparation of 3-(trifluoromethyl)-5-(((((1*S*,3*S*)-3-(4-(5-(trifluoromethyl)pyrimidin-2-yl)piperazine-1-carbonyl)cyclohexyl)amino)methyl)pyridin-2(1*H*)-one (16)**

((1*S*,3*S*)-3-aminocyclohexyl)(4-(5-(trifluoromethyl)pyrimidin-2-yl)piperazin-1-yl)methanone hydrochloride (16-b, 100 mg, 0.254 mmol) was dissolved in methanol (1 mL) and dichloromethane (1 mL), and 6-oxo-5-(trifluoromethyl)-1,6-dihydropyridine-3-carbaldehyde (I-7, 58 mg, 0.305 mmol) was added. Sodium acetate (10 mg, 0.127 mmol) was added at 0°C and the mixture was stirred at 0°C for 20 minutes, then sodium cyanoborohydride (48 mg, 0.764 mmol) was added at 0°C. The reaction was stirred at room temperature overnight. 0.1 mL of water was added, and the mixture was filtered. The organic phase was concentrated under reduced pressure and purified twice by preparative thin-layer chromatography silica gel plate (dichloromethane:methanol = 8:1) to obtain the target product 3-(trifluoromethyl)-5-(((((1*S*,3*S*)-3-(4-(5-(trifluoromethyl)pyrimidin-2-yl)piperazine-1-carbonyl)cyclohexyl)amino)methyl)pyridin-2(1*H*)-one (16, 2.67 mg, yield 1.97%). ESI $[M+H]^+$=533.1, ESI $[2M+H]^+$=1065.3 $^1$HNMR (400 MHz,DMSO-$d_6$) δ 8.73 (s, 2H), 8.29 (s, 1H), 7.99 (s, 1H), 7.64 (s, 1H), 3.83 (d, J = 25.5 Hz, 4H), 3.56 (s, 6H), 3.11 - 3.02 (m, 2H), 2.91 (s, 1H), 1.61 (dd, J = 19.6, 13.6 Hz, 4H), 1.50 - 1.28 (m, 4H).

**Embodiment 17: Preparation of (*S*)-3-(trifluoromethyl)-5-(((3-(4-(5-(trifluoromethyl)pyrimidin-2-yl)piperazine-1-carbonyl)piperidin-1-yl)methyl)pyridin-2(1*H*)-one (17)**

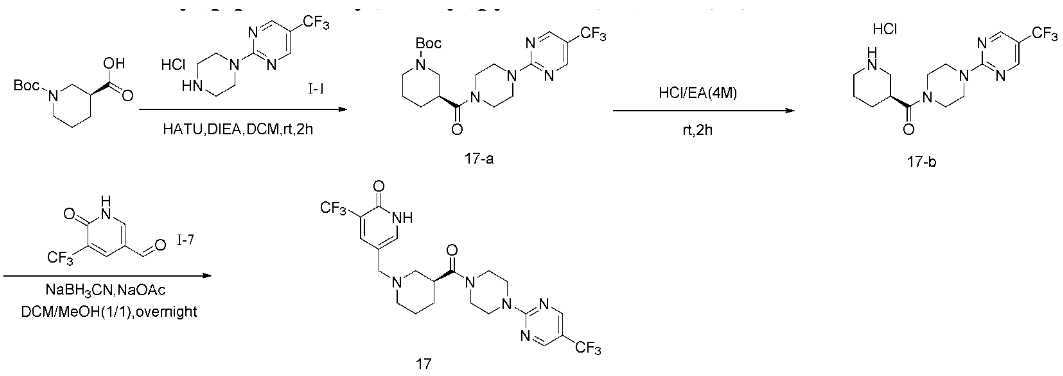


**Step 1: Preparation of tert-butyl (*S*)-3-(4-(5-(trifluoromethyl)pyrimidin-2-yl)piperazine-1-carbonyl)piperidine-1-carboxylate (17-a)**

(*S*)-1-(tert-butoxycarbonyl)piperidine-3-carboxylic acid (100 mg, 0.436 mmol) was dissolved in dichloromethane (5 mL), *O*-(7-azabenzotriazol-1-yl)-*N,N,N',N'*-tetramethyluronium hexafluorophosphate (166 mg, 0.436 mmol) and *N,N*-diisopropylethylamine (229 $\mu$L, 1.308 mmol) were added, and the mixture was reacted for 20 minutes, then 2-(piperazin-1-yl)-5-(trifluoromethyl)pyrimidine hydrochloride (I-1, 141 mg, 0.523 mmol) was added, and the reaction was stirred at room temperature for 2 hours. The reaction mixture was filtered and concentrated under reduced pressure, and the residue was purified by Flash chromatography (ethyl acetate:petroleum ether = 0-50%) to obtain the target product tert-butyl (S)-3-(4-(5-(trifluoromethyl)pyrimidin-2-yl)piperazine-1-carbonyl)piperidine-1-carboxylate (17-a, 187.8 mg, yield 96.95%). ESI $[M+H]^+$=444.2, ESI $[M+H-56]^+$=388.1, ESI $[M+H-100]^+$=344.2

**Step 2: Preparation of (*S*)-piperidin-3-yl(4-(5-(trifluoromethyl)pyrimidin-2-yl)piperazin-1-yl)methanone hydrochloride (17-b)**

To tert-butyl (*S*)-3-(4-(5-(trifluoromethyl)pyrimidin-2-yl)piperazine-1-carbonyl)piperidine-1-carboxylate (17-a, 187.8 mg, 0.423 mmol) was added dropwise slowly hydrogen chloride in ethyl acetate solution (4M) (3 mL), and the mixture was stirred at room temperature for 2 hours. The reaction mixture was diluted with 4 mL of ethyl acetate and concentrated under reduced pressure to obtain the crude target product (*S*)-piperidin-3-yl(4-(5-(trifluoromethyl)pyrimidin-2-yl)piperazin-1-yl)methanone hydrochloride (17-b, 194 mg, crude product). ESI $[M+H]^+$=344.2

**Step 3: Preparation of (*S*)-3-(trifluoromethyl)-5-(((3-(4-(5-(trifluoromethyl)pyrimidin-2-yl)piperazine-1-carbonyl)piperidin-1-yl)methyl)pyridin-2(1H)-one (17)**

(*S*)-piperidin-3-yl(4-(5-(trifluoromethyl)pyrimidin-2-yl)piperazin-1-yl)methanone hydrochloride (17-b, 100 mg, 0.263 mmol) was dissolved in methanol (1 mL) and dichloromethane (1 mL), and 6-oxo-5-(trifluoromethyl)-1,6-dihydropyridine-3-carbaldehyde (I-7, 60 mg, 0.316 mmol) was added. Sodium acetate (10 mg, 0.132 mmol) was added at 0°C and the mixture was stirred at 0°C for 20 minutes, then sodium cyanoborohydride (50 mg, 0.79 mmol) was added at 0°C. The reaction was stirred at room temperature overnight. 0.1 mL of water was added, and the mixture was filtered. The organic phase was concentrated under reduced pressure and purified by preparative thin-layer chromatography silica gel plate (dichloromethane:methanol = 8:1), and then purified by preparative HPLC [Pack Column 20ID*250mm*10$\mu$m; A:$H_2O$(0.1%FA), B:ACN, B%: 10%-70% over 22 min] to obtain the target product (*S*)-3-(trifluoromethyl)-5-(((3-(4-(5-(trifluoromethyl)pyrimidin-2-yl)piperazine-1-carbonyl)piperidin-1-yl)methyl)pyridin-2(1H)-one (17, 5.68 mg, yield 4.16%). ESI $[M+H]^+$=519.1, ESI $[2M+H]^+$=1037.2 $^1$HNMR (400 MHz, DMSO-$d_6$) $\delta$ 8.72 (s, 2H), 7.83 (s, 1H), 7.57 (s, 1H), 3.80 (d, J = 10.8 Hz, 4H), 3.55 (d, J = 20.2 Hz, 4H), 3.27 (s, 2H), 2.78 (dd, J = 20.9, 11.2 Hz, 3H), 2.01 (t, J = 10.7 Hz, 1H), 1.87 (t, J = 10.6 Hz, 1H), 1.73 (d, J = 12.1 Hz, 1H), 1.64 (d, J = 12.6 Hz, 1H), 1.57 - 1.46 (m, 1H), 1.27 (d, J = 15.3 Hz, 1H).

**Embodiment 18: Synthesis of 3-(trifluoromethyl)-5-(((4-(4-(5-(trifluoromethyl)pyrimidin-2-yl)piperazine-1-carbonyl)piperidin-1-yl)methyl)pyridin-2(1H)-one (18)**

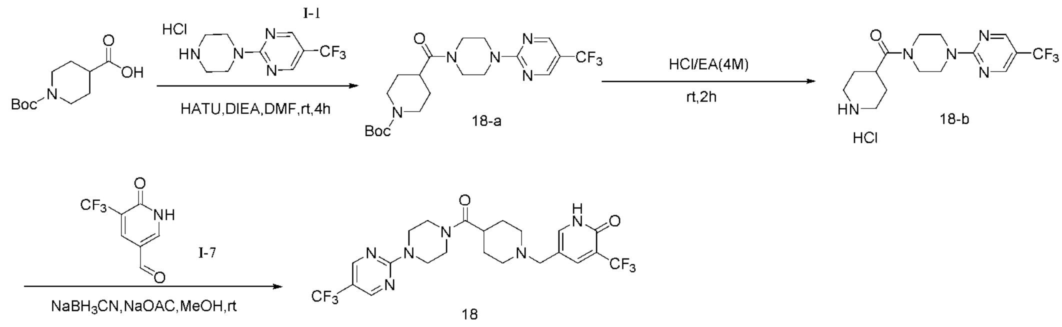


**Step 1: Preparation of tert-butyl 4-(4-(5-(trifluoromethyl)pyrimidin-2-yl)piperazine-1-carbonyl)piperidine-1-carboxylate (18-a)**

1-(tert-butoxycarbonyl)piperidine-4-carboxylic acid (100 mg, 0.44 mmol) was dissolved in *N,N*-dimethylformamide (3.5 mL), and *O*-(7-azabenzotriazol-1-yl)-*N,N,N',N'*-tetramethyluronium hexafluorophosphate (166 mg, 0.44 mmol) and *N,N*-diisopropylethylamine (228 μL) were added sequentially, and the mixture was stirred at 0°C for 20 minutes. Then, 2-(piperazin-1-yl)-5-(trifluoromethyl)pyrimidine (69.6 mg, 0.24 mmol) was added, and the reaction mixture was stirred at room temperature for 4 hours. 10 volumes of water were added to the reaction mixture, and after stirring for 15 minutes under an ice bath, the mixture was filtered. The filter cake was washed with a mixed solution of petroleum ether:ethyl acetate = 1:1, and the filtrate was concentrated under reduced pressure to obtain the target product tert-butyl 4-(4-(5-(trifluoromethyl)pyrimidin-2-yl)piperazine-1-carbonyl)piperidine-1-carboxylate (18-a, 128 mg, yield 66.25%). ESI $[M+H]^+$=444.2

**Step 2: Preparation of piperidin-4-yl(4-(5-(trifluoromethyl)pyrimidin-2-yl)piperazin-1-yl)methanone (18-b)**

tert-butyl 4-(4-(5-(trifluoromethyl)pyrimidin-2-yl)piperazine-1-carbonyl)piperidine-1-carboxylate (18-a, 128 mg, 0.29 mmol) was dissolved in 4M hydrogen chloride in ethyl acetate solution (1.3 mL). The reaction was stirred at room temperature for 2 hours. The reaction mixture was concentrated under reduced pressure to obtain the crude target product piperidin-4-yl(4-(5-(trifluoromethyl)pyrimidin-2-yl)piperazin-1-yl)methanone (18-b, 54 mg, crude product). ESI $[M+H]^+$=344.16

**Step 3: Preparation of 3-(trifluoromethyl)-5-(((4-(4-(5-(trifluoromethyl)pyrimidin-2-yl)piperazine-1-carbonyl)piperidin-1-yl)methyl)pyridin-2(1H)-one (18)**

piperidin-4-yl(4-(5-(trifluoromethyl)pyrimidin-2-yl)piperazin-1-yl)methanone (18-b, 54 mg, 0.16 mmol) was dissolved in methanol (1.5 mL). 6-oxo-5-(trifluoromethyl)-1,6-dihydropyridine-3-carbaldehyde (I-7, 36 mg, 0.19 mmol) was added and stirred for 10 minutes, then sodium acetate (11 mg, 0.13 mmol) was added at 0°C, and after stirring for 20 minutes, sodium cyanoborohydride (49 mg, 0.78 mmol) was added. The reaction was stirred at room temperature overnight. A small amount of water was added to the reaction mixture, which was then extracted with ethyl acetate. The filtrate was concentrated under reduced pressure and purified by column chromatography (dichloromethane:methanol = 10:1), and finally purified by preparative HPLC [Pack Column 20ID*250mm*10μm; A:$H_2O$(0.1%FA), B:ACN, B%: 10%-70% over 22 min] to obtain the target product 3-(trifluoromethyl)-5-(((4-(4-(5-(trifluoromethyl)pyrimidin-2-yl)piperazine-1-carbonyl)piperidin-1-yl)methyl)pyridin-2(1H)-one (18, 8 mg, yield 9.81%). ESI $[M+H]^+$=519.19 $^1$H NMR (400 MHz, DMSO-$d_6$) δ 11.72 (s, 1H), 8.73 (s, 2H), 7.95 (s, 1H), 7.71 (s, 1H), 4.42 (s, 2H), 3.91 - 3.77 (m, 8H), 2.45 - 2.30 (m, 4H), 2.05 - 1.93 (m, 5H).

**Embodiment 19: Preparation of 3-(trifluoromethyl)-5-(((((1*S*,3*S*)-3-(4-(5-(trifluoromethyl)pyrimidin-2-yl)piperazine-1-carbonyl)cyclobutyl)amino)methyl)pyridin-2(1H)-one (19)**

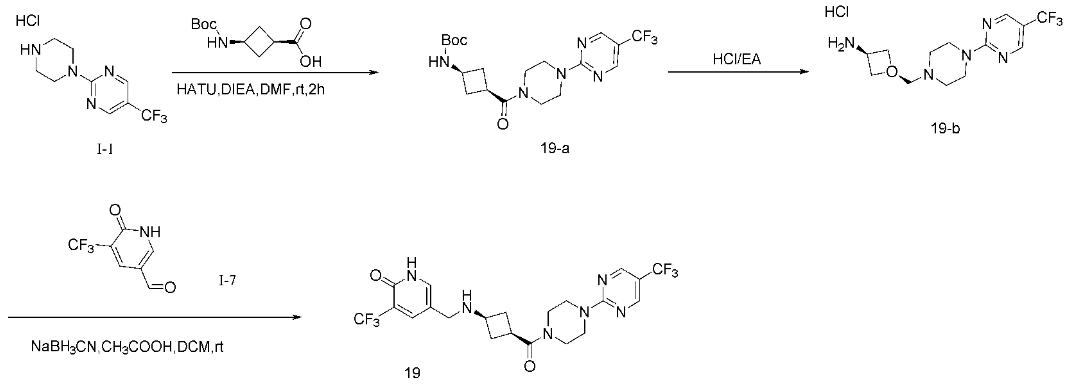


**Step 1: Preparation of tert-butyl ((1*S*,3*S*)-3-(4-(5-(trifluoromethyl)pyrimidin-2-yl)piperazine-1-carbonyl)cyclobutyl)carbamate (19-a)**

(1*S*,3*S*)-3-((tert-butoxycarbonyl)amino)cyclobutane-1-carboxylic acid (100 mg, 0.304 mmol), *O*-(7-azabenzotriazol-1-yl)-*N,N,N',N'*-tetramethyluronium hexafluorophosphate (181 mg, 0.476 mmol), and *N,N*-diisopropylethylamine (179.3 mg, 1.39 mmol) were dissolved in *N,N*-dimethylformamide (3 mL), and finally 2-(piperazin-1-yl)-5-(trifluoromethyl) pyrimidine hydrochloride (I-1, 127 mg, 0.472 mmol) was added. The reaction was stirred at room temperature overnight. After monitoring showed the reaction was complete, the reaction mixture was added dropwise to ice water with stirring. The precipitated solid was collected by filtration, and the filter cake was concentrated under reduced pressure to obtain the target product tert-butyl ((1S,3S)-3-(4-(5-(trifluoromethyl)pyrimidin-2-yl)piperazine-1-carbonyl)cyclobutyl)carbamate (19-a, 190 mg, yield 95.4%). ESI $[M+H]^+$=430.2

**Step 2: Preparation of ((1*S*,3*S*)-3-aminocyclobutyl)(4-(5-(trifluoromethyl)pyrimidin-2-yl)piperazin-1-yl)methanone hydrochloride (19-b)**

tert-butyl ((1*S*,3*S*)-3-(4-(5-(trifluoromethyl)pyrimidin-2-yl)piperazine-1-carbonyl)cyclobutyl)carbamate (19-a, 190 mg, 0.443 mmol) was dissolved in 4M hydrogen chloride in ethyl acetate solution (3 mL) and reacted at room temperature overnight. After monitoring showed the reaction was complete, the reaction mixture was concentrated under reduced pressure to obtain the target product ((1*S*,3*S*)-3-aminocyclobutyl)(4-(5-(trifluoromethyl)pyrimidin-2-yl)piperazin-1-yl)methanone hydrochloride (19-b, 215 mg, yield 147.2%). ESI $[M+H]^+$=330.2

**Step 3: Preparation of 3-(trifluoromethyl)-5-(((((1*S*,3*S*)-3-(4-(5-(trifluoromethyl)pyrimidin-2-yl)piperazine-1-carbonyl)cyclobutyl)amino)methyl)pyridin-2(1H)-one (19)**

((1*S*,3*S*)-3-aminocyclobutyl)(4-(5-(trifluoromethyl)pyrimidin-2-yl)piperazin-1-yl)methanone hydrochloride (19-b, 100 mg, 0.304 mmol) and 6-oxo-5-(trifluoromethyl)-1,6-dihydropyridine-3-carbaldehyde (I-7, 87 mg, 0.455 mmol) were dissolved in dichloromethane (3 mL). Acetic acid (9.1 mg, 0.151 mmol) was added at 0°C. After stirring for 20 minutes, sodium cyanoborohydride (57.3 mg, 0.912 mmol) was added, and the reaction was stirred at room temperature overnight. After monitoring showed the reaction was complete, a large amount of water was added, and the mixture was extracted with dichloromethane:methanol = 10:1. The organic phase was dried over anhydrous sodium sulfate, filtered, and concentrated under reduced pressure. The residue was purified by preparative HPLC [Pack Column 20ID*250mm* 10μm; A:$H_2O$(0.1%FA), B:ACN, B%: 5%-50% over 22 min] to obtain 3-(trifluoromethyl)-5-(((((1S,3S)-3-(4-(5-(trifluoromethyl) pyrimidin-2-yl)piperazine-1-carbonyl)cyclobutyl)amino)methyl)pyridin-2(1H)-one (19, 1.87 mg, yield 1.22%). ESI $[M+H]^+$=505.2 $^1$H NMR (400 MHz, DMSO-$d_6$)δ 9.16 (s, 2H), 8.70 (s, 1H), 8.35 (s, 1H), 7.98 (s, 1H), 4.22 (d, J = 4.3 Hz, 1H), 4.03 (s, 3H), 3.95 (d, J = 4.4 Hz, 1H), 3.50 - 3.44 (m, 1H), 3.40 - 3.29 (m, 2H), 2.93 (s, 4H), 2.67 (s, 1H), 2.33 (s, 1H), 2.20 - 2.16 (m, 3H).

**Embodiment 20: Preparation of 4-(trifluoromethyl)-6-(((((1*S*,3*S*)-3-(4-(5-(trifluoromethyl)pyrimidin-2-yl)piperazine-1-carbonyl)cyclobutyl)amino)methyl)pyridazin-3(2*H*)-one (20)**

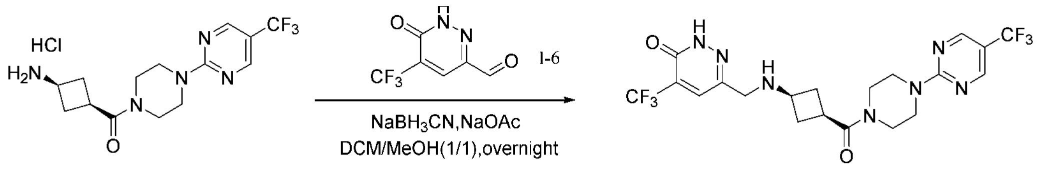


19-b

20

((1*S*,3*S*)-3-aminocyclobutyl)(4-(5-(trifluoromethyl)pyrimidin-2-yl)piperazin-1-yl)methanone hydrochloride (19-b, 190 mg, 0.521 mmol) and 6-oxo-5-(trifluoromethyl)-1,6-dihydropyridazine-3-carbaldehyde (I-6, 120 mg, 0.625 mmol) were dissolved in dichloromethane (2 mL) and methanol (2 mL). Sodium acetate (21 mg, 0.26 mmol) was added at 0°C, and the mixture was stirred for 30 minutes. Then, sodium cyanoborohydride (97 mg, 1.56 mmol) was added, and the reaction was stirred at room temperature overnight. The reaction was quenched by adding a small amount of water, filtered, and concentrated under reduced pressure. The residue was purified twice by preparative thin-layer chromatography silica gel plate (dichloromethane:methanol = 10:1) to obtain 4-(trifluoromethyl)-6-(((((1*S*,3*S*)-3-(4-(5-(trifluoromethyl)pyrimidin-2-yl)piperazine-1-carbonyl)cyclobutyl)amino)methyl)pyridazin-3(2*H*)-one (20, 43.25 mg, yield 16.43%). ESI [M+H]$^+$=506.3, ESI [2M+H]$^+$=1011.5 $^1$HNMR (400 MHz, DMSO-d$_6$) δ 8.72 (s, 2H), 7.95 (s, 1H), 3.79 (d, J = 4.1 Hz, 4H), 3.56 (s, 2H), 3.54 -3.50 (m, 2H), 3.44 (d, J = 4.3 Hz, 3H), 3.04 (dt, J = 15.7, 8.0 Hz, 1H), 2.93 (dt, J = 17.7, 9.0 Hz, 1H), 2.25 (dd, J = 15.6, 7.8 Hz, 2H), 1.82 (dd, J = 19.4, 9.9 Hz, 2H).

**Embodiment 21: Preparation of 3-(trifluoromethyl)-5-(((6-(4-(5-(trifluoromethyl)pyrimidin-2-yl)piperazine-1-carbonyl)-2-azaspiro[3.3]heptan-2-yl)methyl)pyridin-2(1*H*)-one (21)**

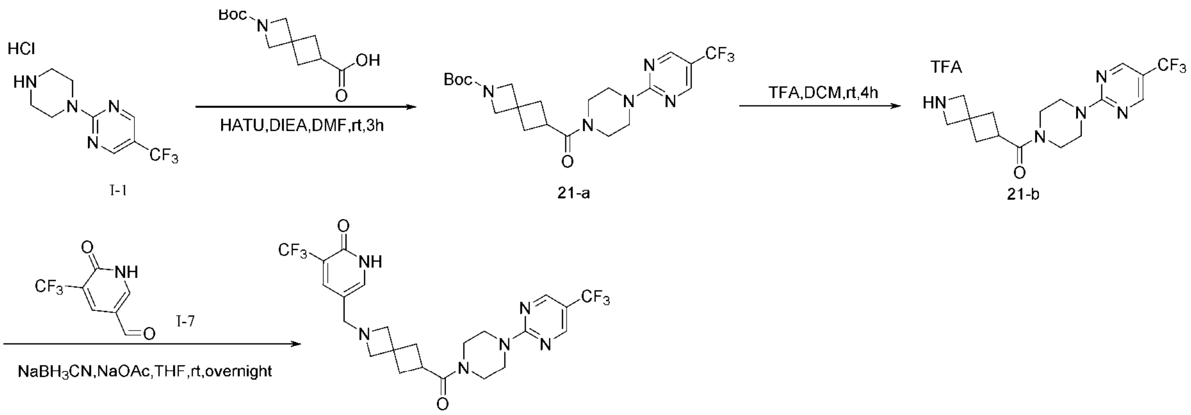


I-1

21-a

21-b

21

**Step 1: Preparation of tert-butyl 6-(4-(5-(trifluoromethyl)pyrimidin-2-yl)piperazine-1-carbonyl)-2-azaspiro[3.3]heptane-2-carboxylate (21-a)**

2-(tert-butoxycarbonyl)-2-azaspiro[3.3]heptane-6-carboxylic acid (100 mg, 0.413 mmol), *O*-(7-azabenzotriazol-1-yl)-*N,N,N',N'*-tetramethyluronium hexafluorophosphate (161 mg, 0.424 mmol), and *N,N*-diisopropylethylamine (216 μL, 1.24 mmol) were dissolved in N,N-dimethylformamide (3 mL). Finally, 2-(piperazin-1-yl)-5-(trifluoromethyl)pyrimidine hydrochloride (I-1, 113.6 mg, 0.423 mmol) was added, and the reaction was stirred at room temperature for 3 hours. After monitoring showed the reaction was complete, the reaction mixture was added dropwise to ice water with stirring. The precipitated solid was collected by filtration, and the filter cake was concentrated under reduced pressure to obtain the target product tert-butyl 6-(4-(5-(trifluoromethyl)pyrimidin-2-yl)piperazine-1-carbonyl)-2-azaspiro[3.3]heptane-2-carboxylate (21-a, 176 mg, yield 93.6%). ESI [M+H]$^+$=456.4

**Step 2: Preparation of (2-azaspiro[3.3]heptan-6-yl)(4-(5-(trifluoromethyl)pyrimidin-2-yl)piperazin-1-yl)methanone trifluoroacetate (21-b)**

tert-butyl 6-(4-(5-(trifluoromethyl)pyrimidin-2-yl)piperazine-1-carbonyl)-2-azaspiro[3.3]heptane-2-carboxylate (21-a, 176 mg, 0.387 mmol) was dissolved in dichloromethane (3 mL), and trifluoroacetic acid (0.3 mL) was added. The mixture was reacted at room temperature for 4 hours. After monitoring showed the reaction was complete, the mixture

was concentrated under reduced pressure to obtain the target product (2-azaspiro[3.3]heptan-6-yl)(4-(5-(trifluoromethyl) pyrimidin-2-yl)piperazin-1-yl)methanone trifluoroacetate (21-b, 220 mg). ESI $[M+H]^+$=356.4

**Step 3: Preparation of 3-(trifluoromethyl)-5-(((6-(4-(5-(trifluoromethyl)pyrimidin-2-yl)piperazine-1-carbonyl)-2-azaspiro[3.3]heptan-2-yl)methyl)pyridin-2(1*H*)-one (21)**

(2-azaspiro[3.3]heptan-6-yl)(4-(5-(trifluoromethyl)pyrimidin-2-yl)piperazin-1-yl)methanone trifluoroacetate (21-b, 30 mg, 0.085 mmol) and 6-oxo-5-(trifluoromethyl)-1,6-dihydropyridine-3-carbaldehyde (I-7, 24.2 mg, 0.127 mmol) were dissolved in tetrahydrofuran (1 mL). Sodium acetate (3.5 mg, 0.043 mmol) was added at 0°C, and the mixture was stirred for 20 minutes. Then, sodium cyanoborohydride (15.9 mg, 0.261 mmol) was added, and the reaction was stirred at room temperature overnight. After monitoring showed the reaction was complete, a large amount of water was added, and the mixture was extracted with dichloromethane:methanol = 10:1. The organic phase was dried over anhydrous sodium sulfate, filtered, and concentrated under reduced pressure. The residue was purified by preparative HPLC [Pack Column 20ID*250mm* 10μm; A:$H_2O$(0.1%FA), B:ACN, B%: 5%-50% over 22 min] to obtain 3-(trifluoromethyl)-5-(((6-(4-(5-(trifluoromethyl)pyrimidin-2-yl)piperazine-1-carbonyl)-2-azaspiro[3.3]heptan-2-yl)methyl)pyridin-2(1H)-one (21, 0.73 mg, yield 1.63%). ESI $[M+H]^+$=531.4 $^1H$ NMR (400 MHz, DMSO-$d_6$) δ 8.72 (s, 2H), 8.37 (s, 1H), 7.78 (s, 1H), 7.53 (s, 1H), 3.79 (s, 2H), 3.26 (s, 6H), 3.15 (s, 3H), 2.98 (s, 3H), 2.67 (s, 2H), 2.33 (s, 1H), 2.25 (d, J = 8.3 Hz, 2H).

**Embodiment 22: Preparation of (*R*)-3-(trifluoromethyl)-5-(((3-(4-(5-(trifluoromethyl)pyrimidin-2-yl)piperazine-1-carbonyl)piperidin-1-yl)methyl)pyridin-2(1*H*)-one (22)**

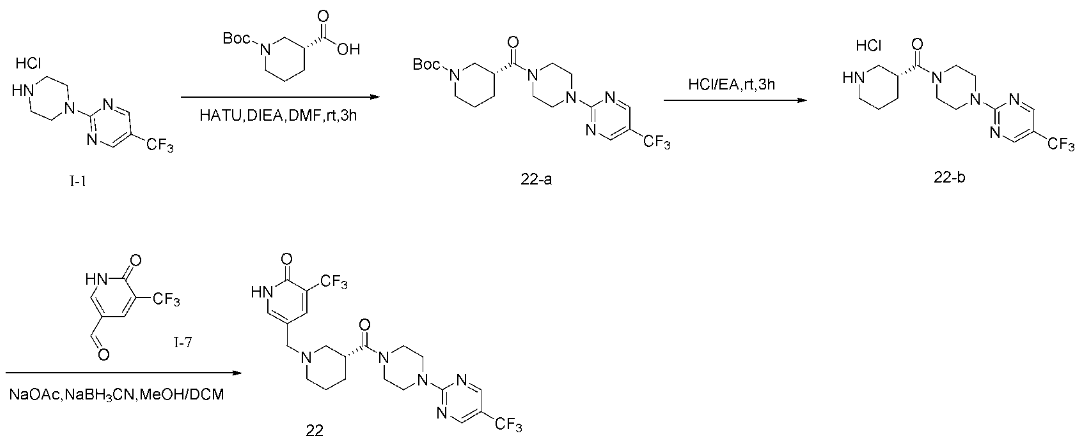

I-1 22-a 22-b 22

**Step 1: Preparation of tert-butyl (*R*)-3-(4-(5-(trifluoromethyl)pyrimidin-2-yl)piperazine-1-carbonyl)piperidine-1-carboxylate (22-a)**

(*R*)-1-(tert-butoxycarbonyl)piperidine-3-carboxylic acid (100 mg, 0.436 mmol), *O*-(7-azabenzotriazol-1-yl)-*N,N,N',N'*-tetramethyluronium hexafluorophosphate (169.1 mg, 0.445 mmol), and N,N-diisopropylethylamine (168.7 mg, 1.31 mmol) were dissolved in *N,N*-dimethylformamide (3 mL). Finally, 2-(piperazin-1-yl)-5-(trifluoromethyl) pyrimidine hydrochloride (119.5 mg, 0.445 mmol) was added, and the reaction was stirred at room temperature for 3 hours. After monitoring showed the reaction was complete, the reaction mixture was added to ice water. The precipitated solid was collected by filtration, and the filter cake was concentrated under reduced pressure to obtain the target product tert-butyl (*R*)-3-(4-(5-(trifluoromethyl)pyrimidin-2-yl)piperazine-1-carbonyl)piperidine-1-carboxylate (22-a, 184 mg, yield 95.3%). ESI $[M+H]^+$=444.19

**Step 2: Preparation of (*R*)-piperidin-3-yl(4-(5-(trifluoromethyl)pyrimidin-2-yl)piperazin-1-yl)methanone hydrochloride (22-b)**

tert-butyl (*R*)-3-(4-(5-(trifluoromethyl)pyrimidin-2-yl)piperazine-1-carbonyl)piperidine-1-carboxylate (22-a, 184 mg, 0.415 mmol) was dissolved in 4M hydrogen chloride in ethyl acetate solution (2 mL) and reacted at room temperature overnight. After monitoring showed the reaction was complete, the mixture was concentrated under reduced pressure to obtain the crude target product (*R*)-piperidin-3-yl(4-(5-(trifluoromethyl)pyrimidin-2-yl)piperazin-1-yl)methanone hydrochloride (22-b, 213.2 mg, yield 150%). ESI $[M+H]^+$=343.2

**Step 3: Preparation of (*R*)-3-(trifluoromethyl)-5-(((3-(4-(5-(trifluoromethyl)pyrimidin-2-yl)piperazine-1-carbonyl)piperidin-1-yl)methyl)pyridin-2(1*H*)-one (22)**

(*R*)-piperidin-3-yl(4-(5-(trifluoromethyl)pyrimidin-2-yl)piperazin-1-yl)methanone hydrochloride (22-b, 100 mg, 0.263 mmol) and 6-oxo-5-(trifluoromethyl)-1,6-dihydropyridine-3-carbaldehyde (I-7, 60 mg, 0.316 mmol) were dissolved in dichloromethane (2 mL) and methanol (2 mL). Sodium acetate (10 mg, 0.132 mmol) was added at 0°C, and the mixture was stirred for 30 minutes. Then, sodium cyanoborohydride (48 mg, 0.789 mmol) was added, and the reaction was stirred at room temperature overnight. The reaction was quenched by adding a small amount of water, filtered, and concentrated under reduced pressure. The residue was purified twice by preparative thin-layer chromatography silica gel plate (dichloromethane:methanol = 10:1) to obtain (*R*)-3-(trifluoromethyl)-5-(((3-(4-(5-(trifluoromethyl)pyrimidin-2-yl)piperazine-1-carbonyl)piperidin-1-yl)methyl)pyridin-2(1*H*)-one (22, 4.32 mg, yield 3.16%). ESI $[M+H]^+$=519.4 $^1H$ NMR (400 MHz, DMSO-$d_6$)$\delta$ 8.72 (s, 2H), 7.84 (s, 1H), 7.57 (s, 1H), 3.93 - 3.70 (m, 4H), 3.54 (d, J = 20.4 Hz, 4H), 3.27 (s, 2H), 2.78 (dd, J = 21.0, 11.2 Hz, 3H), 2.02 (t, J = 10.8 Hz, 1H), 1.92 - 1.82 (m, 1H), 1.61 (ddd, J = 37.3, 31.0, 12.2 Hz, 4H).

**Embodiment 23: Preparation of 5-(((((1*S*,3*R*)-3-((*S*)-3-methyl-4-(5-(trifluoromethyl)pyrimidin-2-yl)piperazine-1-carbonyl)cyclobutyl)amino)methyl)-3-(trifluoromethyl)pyridin-2(1*H*)-one (23)**

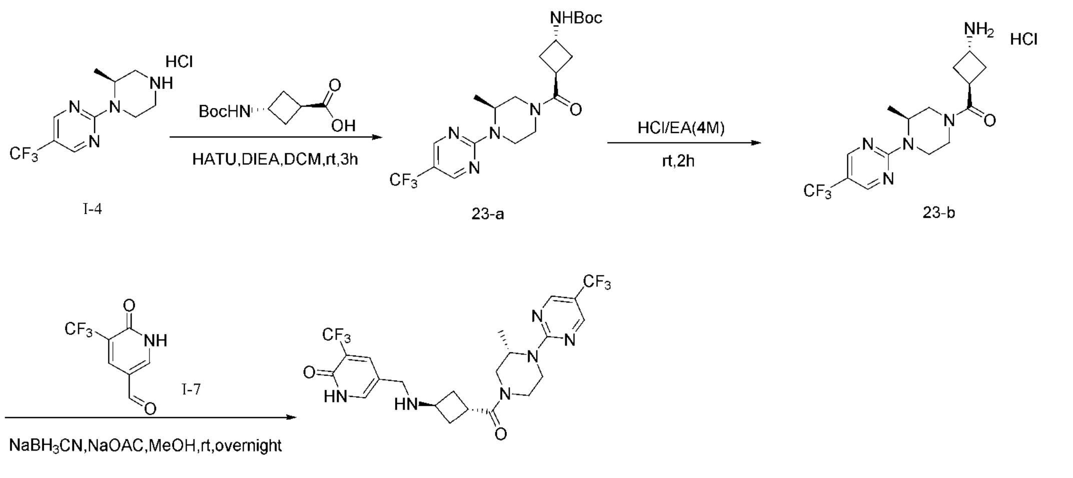

I-4 23-a 23-b

23

**Step 1: Preparation of tert-butyl ((1*S*,3*R*)-3-((*S*)-3-methyl-4-(5-(trifluoromethyl)pyrimidin-2-yl)piperazine-1-carbonyl)cyclobutyl)carbamate (23-a)**

(1*R*,3*R*)-3-((tert-butoxycarbonyl)amino)cyclobutane-1-carboxylic acid (80 mg, 0.372 mmol), *O*-(7-azabenzotriazol-1-yl)-*N,N,N',N'*-tetramethyluronium hexafluorophosphate (141 mg, 0.372 mmol), and *N,N*-diisopropylethylamine (198 μL, 1.116 mmol) were dissolved in dichloromethane (3 mL) and reacted for 30 minutes. Finally, (S)-2-(2-methylpiperazin-1-yl)-5-(trifluoromethyl)pyrimidine hydrochloride (I-4, 97 mg, 0.391 mmol) was added, and the reaction was stirred at room temperature for 3 hours. After monitoring showed the reaction was complete, the reaction mixture was concentrated under reduced pressure and purified by Flash chromatography (petroleum ether:ethyl acetate = 0-50%) to obtain the target product tert-butyl ((1*S*,3*R*)-3-((*S*)-3-methyl-4-(5-(trifluoromethyl)pyrimidin-2-yl)piperazine-1-carbonyl)cyclobutyl)carbamate (23-a, 156 mg, yield 94.65%). ESI $[M+H]^+$=444.3, ESI $[2M+H]^+$=887.6

**Step 2: Preparation of ((1*R*,3*S*)-3-aminocyclobutyl)((*S*)-3-methyl-4-(5-(trifluoromethyl)pyrimidin-2-yl)piperazin-1-yl)methanone hydrochloride (23-b)**

tert-butyl ((1*S*,3*R*)-3-((*S*)-3-methyl-4-(5-(trifluoromethyl)pyrimidin-2-yl)piperazine-1-carbonyl)cyclobutyl)carbamate (23-a, 156 mg, 0.352 mmol) was dissolved in 4 mol/L hydrogen chloride in ethyl acetate solution (3 mL) and reacted at room temperature for 2 hours. After monitoring showed the reaction was complete, the mixture was concentrated under reduced pressure to obtain the crude target product ((1*R*,3*S*)-3-aminocyclobutyl)((S)-3-methyl-4-(5-(trifluoromethyl)pyrimidin-2-yl)piperazin-1-yl)methanone hydrochloride (23-b, 156 mg). ESI $[M+H]^+$=344.3, ESI $[2M+H]^+$=687.5

**Step 3: Preparation of 5-(((((1*S*,3*R*)-3-((S)-3-methyl-4-(5-(trifluoromethyl)pyrimidin-2-yl)piperazine-1-carbonyl) cyclobutyl)amino)methyl)-3-(trifluoromethyl)pyridin-2(1*H*)-one (23)**

((1*R*,3*S*)-3-aminocyclobutyl)((S)-3-methyl-4-(5-(trifluoromethyl)pyrimidin-2-yl)piperazin-1-yl)methanone hydrochloride (23-b, 156 mg, 0.411 mmol) and 6-oxo-5-(trifluoromethyl)-1,6-dihydropyridine-3-carbaldehyde (I-7, 70 mg, 0.411 mmol) were dissolved in dichloromethane (1 mL) and methanol (2 mL). Sodium acetate (17 mg, 0.206 mmol) was added at 0°C, and the mixture was stirred for 30 minutes. Then, sodium cyanoborohydride (77 mg, 1.233 mmol) was added, and the reaction was stirred at room temperature overnight. The reaction was quenched by adding a small amount of water, filtered, and concentrated under reduced pressure. The residue was purified twice by Flash column chromatography (methanol:dichloromethane = 0-10%) to obtain 5-(((((1*S*,3*R*)-3-((*S*)-3-methyl-4-(5-(trifluoromethyl)pyrimidin-2-yl) piperazine-1-carbonyl)cyclobutyl)amino)methyl)-3-(trifluoromethyl)pyridin-2(1*H*)-one (23, 11.73 mg, yield 5.51%). ESI $[M+H]^+$=519.3, ESI $[2M+H]^+$=1037.5 $^1H$ NMR (400 MHz, DMSO-$d_6$)δ 8.73 (s, 2H), 8.24 (s, 1H), 7.96 (s, 1H), 7.1H), 4.46 (d, J = 12.5 Hz, 1H), 4.27 (t, J = 14.1 Hz, 1H), 3.40 - 3.10 (m,2.85 - 2.76 (m, 1H), 2.40 - 2.22 (m, 2H), 2.06 (dd, J = 15.6, 10.0 Hz, 2H), 1.09 (dd, J = 21.8, 6.7 Hz, 3H).

**Embodiment 24: Preparation of (R)-3-(trifluoromethyl)-5-(((3-(4-(5-(trifluoromethyl)pyrimidin-2-yl)piperazine-1-carbonyl)pyrrolidin-1-yl)methyl)pyridin-2(1*H*)-one (24)**

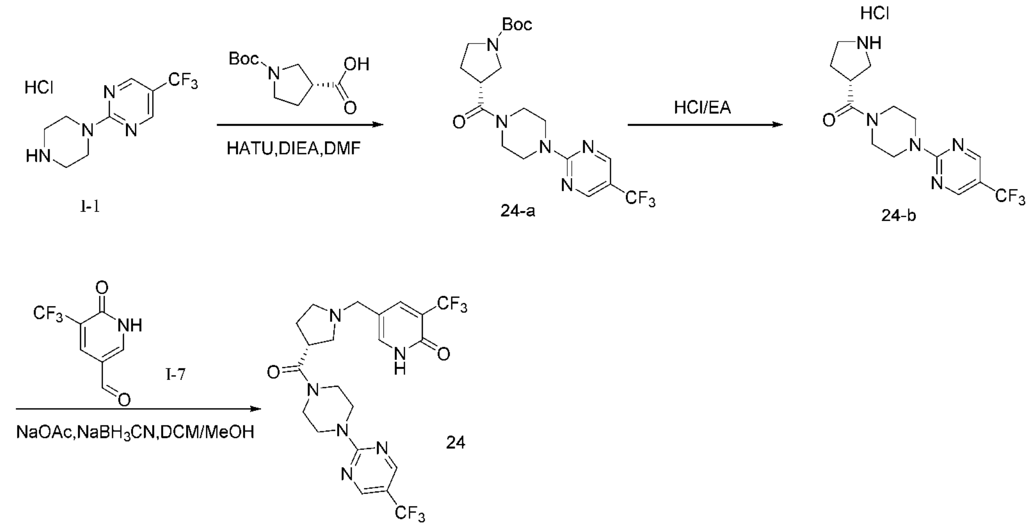


I-1, 24-a, 24-b, I-7, 24

**Step 1: Preparation of tert-butyl (*R*)-3-(4-(5-(trifluoromethyl)pyrimidin-2-yl)piperazine-1-carbonyl)pyrrolidine-1-carboxylate (24-a)**

(*R*)-1-(tert-butoxycarbonyl)pyrrolidine-3-carboxylic acid (100 mg, 0.465 mmol) was dissolved in *N,N*-dimethylformamide (6 mL). Under an ice bath, *O*-(7-azabenzotriazol-1-yl)-*N,N,N',N'*-tetramethyluronium hexafluorophosphate (176.7 mg, 0.465 mmol) and *N,N*-diisopropylethylamine (244 μL, 1.39 mmol) were added. After stirring under an ice bath for 30 minutes, 2-(piperazin-1-yl)-5-(trifluoromethyl)pyrimidine hydrochloride (124 mg, 0.465 mmol) was added. After reacting at room temperature for 3 hours, the reaction mixture was added dropwise to water (12 mL) with stirring. The mixture was filtered, and the filter cake was concentrated under reduced pressure to obtain the target product tert-butyl (*R*)-3-(4-(5-(trifluoromethyl)pyrimidin-2-yl)piperazine-1-carbonyl)pyrrolidine-1-carboxylate (24-a, 130 mg, yield 65.2%). ESI $[M-56+H]^+$=374.4

**Step 2: Preparation of (*R*)-pyrrolidin-3-yl(4-(5-(trifluoromethyl)pyrimidin-2-yl)piperazin-1-yl)methanone hydrochloride (24-b)**

tert-butyl (*R*)-3-(4-(5-(trifluoromethyl)pyrimidin-2-yl)piperazine-1-carbonyl)pyrrolidine-1-carboxylate (24-a, 130 mg, 0.303 mmol) was dissolved in 4M hydrogen chloride in ethyl acetate solution (3 mL) and reacted at room temperature for 2 hours. After monitoring showed the reaction was complete, ethyl acetate was added and the mixture was concentrated under reduced pressure multiple times to obtain the crude target product (R)-pyrrolidin-3-yl(4-(5-(trifluoromethyl)pyrimidin-2-yl)piperazin-1-yl)methanone hydrochloride (24-b, 137 mg, crude product). ESI $[M+H]^+$=330.2, ESI

$[2M+H]^+$=659.5

**Step 3: Preparation of (*R*)-3-(trifluoromethyl)-5-(((3-(4-(5-(trifluoromethyl)pyrimidin-2-yl)piperazine-1-carbonyl) pyrrolidin-1-yl)methyl)pyridin-2(1*H*)-one (24)**

(*R*)-pyrrolidin-3-yl(4-(5-(trifluoromethyl)pyrimidin-2-yl)piperazin-1-yl)methanone hydrochloride (24-b, 137 mg, 0.375 mmol) and 6-oxo-5-(trifluoromethyl)-1,6-dihydropyridine-3-carbaldehyde (I-7, 72 mg, 0.375 mmol) were dissolved in dichloromethane (1 mL) and methanol (2 mL). Sodium acetate (15 mg, 0.187 mmol) was added at 0°C, and the mixture was stirred for 30 minutes. Then, sodium cyanoborohydride (71 mg, 1.125 mmol) was added, and the reaction was stirred at room temperature overnight. After adding 0.3 mL of water, the mixture was concentrated under reduced pressure and then filtered. The filtrate was purified by preparative thin-layer chromatography silica gel plate (dichloromethane:methanol = 10:1) and then by preparative HPLC [Pack Column 20ID*250mm*10μm; A:$H_2O$(0.1%FA), B:ACN, B%: 5%-50% over 22 min] to obtain (*R*)-3-(trifluoromethyl)-5-(((3-(4-(5-(trifluoromethyl)pyrimidin-2-yl)piperazine-1-carbonyl)pyrrolidin-1-yl) methyl)pyridin-2(1H)-one (24, 2.17 mg, yield 1.15%). ESI $[M+H]^+$=505.3 $^1H$ NMR (600 MHz, DMSO-$d_6$) δ 8.72 (s, 2H), 7.84 (s, 1H), 7.58 (s, 1H), 3.82 (dd, J = 13.1, 4.4 Hz, 4H),3.61 - 3.53 (m, 4H), 3.28 (dd, J = 15.9, 8.0 Hz, 2H), 2.77 (t, J = 8.7 Hz, 1H), 2.60 (dd, J = 13.2, 7.1 Hz, 1H), 2.56 - 2.52 (m, 1H), 2.38 (dd, J = 15.4, 7.9 Hz, 1H), 1.99 (dd, J = 20.5, 9.2 Hz, 2H), 1.91 (dd, J =11.8, 6.0 Hz, 1H).

**Embodiment 25: Preparation of 3-(trifluoromethyl)-5-((((3-(4-(5-(trifluoromethyl)pyrimidin-2-yl)piperazine-1-carbonyl)bicyclo[1.1.1]pentan-1-yl)amino)methyl)pyridin-2(1*H*)-one (25)**

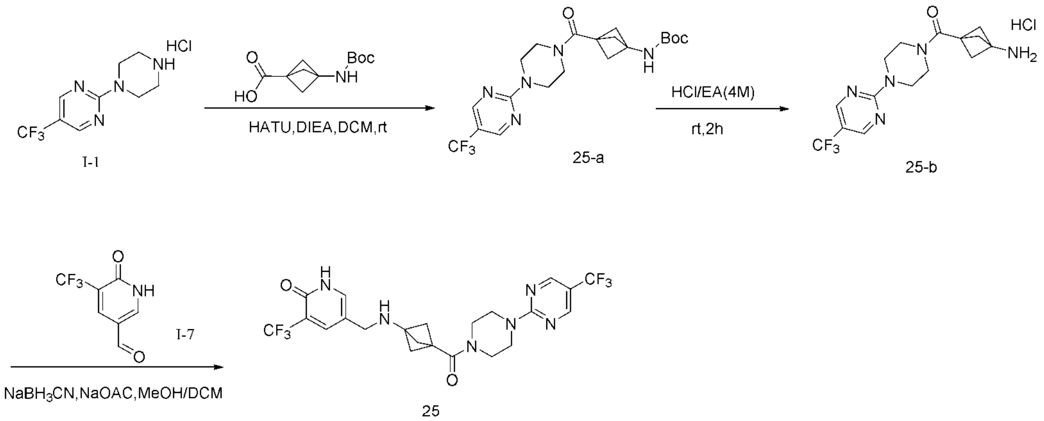

I-1
25-a
25-b
I-7
25

**Step 1: Preparation of tert-butyl (3-(4-(5-(trifluoromethyl)pyrimidin-2-yl)piperazine-1-carbonyl)bicyclo[1.1.1] pentan-1-yl)carbamate (25-a)**

3-((tert-butoxycarbonyl)amino)bicyclo[1.1.1]pentane-1-carboxylic acid (100 mg, 0.440 mmol), *O*-(7-azabenzotriazol-1-yl)-*N,N,N',N'*-tetramethyluronium hexafluorophosphate (167 mg, 0.440 mmol), and *N,N*-diisopropylethylamine (230 μL, 1.32 mmol) were dissolved in dichloromethane (4 mL) and reacted for 30 minutes. Finally, 2-(piperazin-1-yl)-5-(trifluoromethyl)pyrimidine hydrochloride (I-1, 142 mg, 0.528 mmol) was added, and the reaction was stirred at room temperature overnight. After monitoring showed the reaction was complete, the reaction mixture was concentrated under reduced pressure and purified by Flash chromatography (ethyl acetate:petroleum ether = 0-50%) to obtain the target product tert-butyl (3-(4-(5-(trifluoromethyl)pyrimidin-2-yl)piperazine-1-carbonyl)bicyclo[1.1.1]pentan-1-yl)carbamate (25-a, 190 mg, yield 97.81%). ESI $[M+H]^+$=442.1

**Step 2: Preparation of (3-aminobicyclo[1.1.1]pentan-1-yl)(4-(5-(trifluoromethyl)pyrimidin-2-yl)piperazin-1-yl) methanone hydrochloride (25-b)**

tert-butyl (3-(4-(5-(trifluoromethyl)pyrimidin-2-yl)piperazine-1-carbonyl)bicyclo[1.1.1]pentan-1-yl)carbamate (25-a, 190 mg, 0.43 mmol) was dissolved in 4M hydrogen chloride in ethyl acetate solution (3 mL) and reacted at room temperature for 2 hours. After monitoring showed the reaction was complete, ethyl acetate was added and the mixture was concentrated under reduced pressure multiple times to obtain the crude target product (3-aminobicyclo[1.1.1]pentan-1-yl) (4-(5-(trifluoromethyl)pyrimidin-2-yl)piperazin-1-yl)methanone hydrochloride (25-b, 190 mg, crude product). ESI

$[M+H]^+$=342.2

**Step 3: Preparation of 3-(trifluoromethyl)-5-((((3-(4-(5-(trifluoromethyl)pyrimidin-2-yl)piperazine-1-carbonyl)bicyclo[1.1.1]pentan-1-yl)amino)methyl)pyridin-2(1*H*)-one (25)**

(3-aminobicyclo[1.1.1]pentan-1-yl)(4-(5-(trifluoromethyl)pyrimidin-2-yl)piperazin-1-yl)methanone hydrochloride (25-b, 90 mg, 0.241 mmol) and 6-oxo-5-(trifluoromethyl)-1,6-dihydropyridine-3-carbaldehyde (I-7, 46 mg, 0.241 mmol) were dissolved in dichloromethane (1 mL) and methanol (2 mL). Sodium acetate (10 mg, 0.121 mmol) was added at 0°C, and the mixture was stirred for 30 minutes. Then, sodium cyanoborohydride (45 mg, 0.723 mmol) was added, and the reaction was stirred at room temperature overnight. The reaction mixture was concentrated under reduced pressure and purified by preparative thin-layer chromatography silica gel plate (dichloromethane:methanol = 10:1), and then purified by preparative HPLC [Pack Column 20ID*250mm*10μm; A:$H_2O$(0.1%FA), B:ACN, B%: 5%-70% over 22 min] and lyophilized to obtain 3-(trifluoromethyl)-5-((((3-(4-(5-(trifluoromethyl)pyrimidin-2-yl)piperazine-1-carbonyl)bicyclo[1.1.1]pentan-1-yl)amino)methyl)pyridin-2(1*H*)-one (25, 0.67 mg, yield 0.54%). ESI $[M+H]^+$=517.3, ESI $[2M+H]^+$=1033.5 $^1H$ NMR (600 MHz, DMSO-$d_6$) δ 8.72 (s, 2H), 7.92 (s, 1H), 7.59 (s, 1H), 3.81 (d, J = 20.4 Hz, 4H), 3.63 (s, 2H), 3.52 (s, 3H), 3.49 (s, 2H), 1.99 (s, 6H).

**Embodiment 26: Preparation of (*S*)-5-(((3-(3-methyl-4-(5-(trifluoromethyl)pyrimidin-2-yl)piperazine-1-carbonyl)azetidin-1-yl)methyl)-3-(trifluoromethyl)pyridin-2(1*H*)-one (26)**

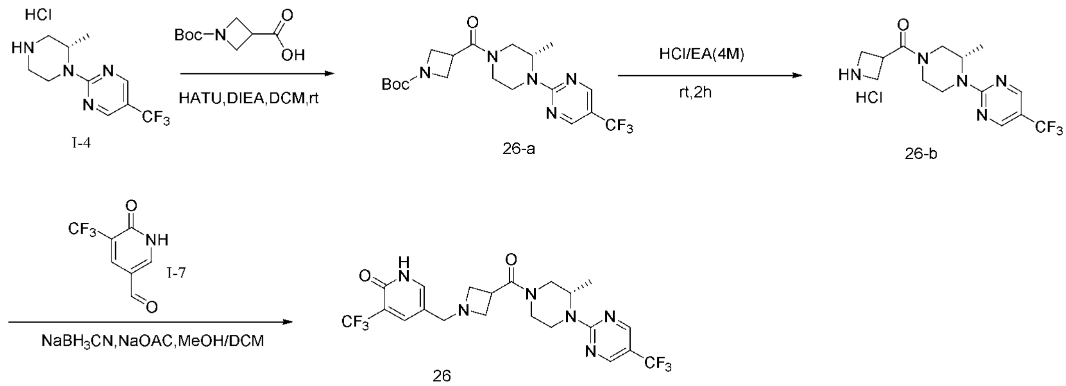


**Step 1: Preparation of tert-butyl (*S*)-3-(3-methyl-4-(5-(trifluoromethyl)pyrimidin-2-yl)piperazine-1-carbonyl)azetidine-1-carboxylate (26-a)**

1-(tert-butoxycarbonyl)azetidine-3-carboxylic acid (100 mg, 0.497 mmol), *O*-(7-azabenzotriazol-1-yl)-*N,N,N',N'*-tetramethyluronium hexafluorophosphate (189 mg, 0.497 mmol), and *N,N*-diisopropylethylamine (260 μL, 1.491 mmol) were dissolved in dichloromethane (4 mL) and reacted for 20 minutes. Finally, (S)-2-(2-methylpiperazin-1-yl)-5-(trifluoromethyl)pyrimidine hydrochloride (I-4, 135 mg, 0.547 mmol) was added, and the reaction was stirred at room temperature for 3 hours. After monitoring showed the reaction was complete, the reaction mixture was concentrated under reduced pressure and purified by Flash chromatography (ethyl acetate: petroleum ether = 0-50%) to obtain the target product tert-butyl (*S*)-3-(3-methyl-4-(5-(trifluoromethyl)pyrimidin-2-yl)piperazine-1-carbonyl)azetidine-1-carboxylate (26-a, 185 mg, yield 86.85%). ESI $[M+H-56]^+$=374.2, ESI $[M+H-100]^+$=330.1

**Step 2: Preparation of (*S*)-azetidin-3-yl(3-methyl-4-(5-(trifluoromethyl)pyrimidin-2-yl)piperazin-1-yl)methanone hydrochloride (26-b)**

tert-butyl (*S*)-3-(3-methyl-4-(5-(trifluoromethyl)pyrimidin-2-yl)piperazine-1-carbonyl)azetidine-1-carboxylate (26-a, 185 mg, 0.431 mmol) was dissolved in 4 mol/L hydrogen chloride in ethyl acetate solution (3 mL) and reacted at room temperature for 2 hours. After monitoring showed the reaction was complete, dichloromethane was added and the mixture was concentrated under reduced pressure multiple times to obtain the crude target product (*S*)-azetidin-3-yl(3-methyl-4-(5-(trifluoromethyl)pyrimidin-2-yl)piperazin-1-yl)methanone hydrochloride (26-b, 171 mg, crude product). ESI $[M+H]^+$=330.2

**Step 3: Preparation of (*S*)-5-(((3-(3-methyl-4-(5-(trifluoromethyl)pyrimidin-2-yl)piperazine-1-carbonyl)azetidin-1-yl)methyl)-3-(trifluoromethyl)pyridin-2(1*H*)-one (26)**

(S)-azetidin-3-yl(3-methyl-4-(5-(trifluoromethyl)pyrimidin-2-yl)piperazin-1-yl)methanone hydrochloride (26-b, 171 mg, 0.479 mmol) and 6-oxo-5-(trifluoromethyl)-1,6-dihydropyridine-3-carbaldehyde (I-7, 89 mg, 0.479 mmol) were dissolved in dichloromethane (2 mL) and methanol (2 mL). Sodium acetate (19 mg, 0.24 mmol) was added at 0°C, and the mixture was stirred for 30 minutes. Then, sodium cyanoborohydride (87 mg, 1.437 mmol) was added, and the reaction was stirred at room temperature for 4 hours. The reaction mixture was concentrated under reduced pressure and purified twice by preparative thin-layer chromatography silica gel plate (dichloromethane:methanol = 10:1) to obtain (*S*)-5-(((3-(3-methyl-4-(5-(trifluoromethyl)pyrimidin-2-yl)piperazine-1-carbonyl)azetidin-1-yl)methyl)-3-(trifluoromethyl)pyridin-2(1H)-one (26, 4.74 mg, yield 2.0%). ESI $[M+H]^+$=505.3 $^1H$ NMR (600 MHz, DMSO-$d_6$) δ 8.72 (s, 2H), 7.81 (s, 1H), 7.57 (s, 1H), 4.86 (d, J = 53.8 Hz, 1H), 4.46 (dd, J = 18.2, 15.2 Hz, 1H), 4.25 (dd, J = 30.3, 13.1 Hz, 1H), 3.68 (d, J = 12.7 Hz, 1H), 3.60 - 3.53 (m, 1H), 3.51 - 3.42 (m, 3H), 3.31 (d, J = 3.0 Hz, 2H), 3.17 (dd, J = 16.5, 10.0 Hz, 2H), 3.14 - 3.07 (m, 1H), 2.95 - 2.76 (m, 1H), 1.08 (dd, J = 19.2, 6.7 Hz, 3H).

**Embodiment 27: Preparation of 3-(trifluoromethyl)-5-(((3-(4-(5-(trifluoromethyl)pyridin-2-yl)piperazine-1-carbonyl)azetidin-1-yl)methyl)pyridin-2(1*H*)-one (27)**

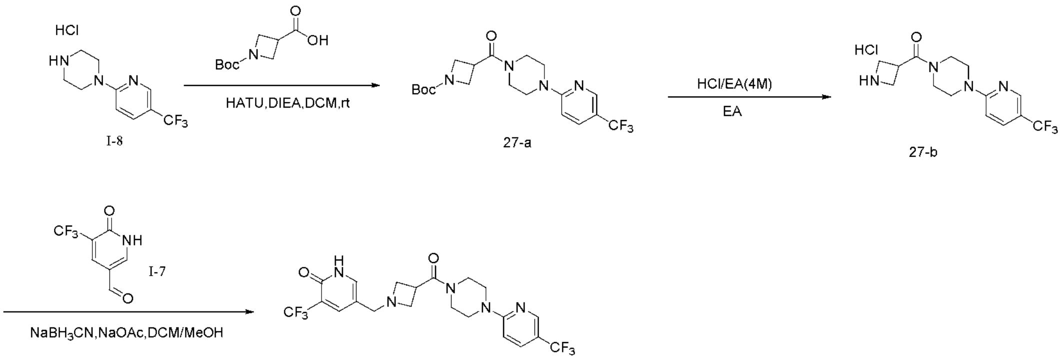

I-8
27-a
27-b
I-7
27

**Step 1: Preparation of tert-butyl 3-(4-(5-(trifluoromethyl)pyridin-2-yl)piperazine-1-carbonyl)azetidine-1-carboxylate (27-a)**

1-(tert-butoxycarbonyl)azetidine-3-carboxylic acid (100 mg, 0.497 mmol), *O*-(7-azabenzotriazol-1-yl)-*N,N,N',N'*-tetramethyluronium hexafluorophosphate (189 mg, 0.497 mmol), and *N,N*-diisopropylethylamine (260 μL, 1.491 mmol) were dissolved in dichloromethane (4 mL) and reacted for 20 minutes. Finally, 1-(5-(trifluoromethyl)pyridin-2-yl)piperazine hydrochloride (I-8, 146 mg, 0.646 mmol) was added, and the reaction was stirred at room temperature overnight. After monitoring showed the reaction was complete, the reaction mixture was concentrated under reduced pressure and purified by Flash column chromatography (petroleum ether:ethyl acetate = 0-50%) to obtain the target product tert-butyl 3-(4-(5-(trifluoromethyl)pyridin-2-yl)piperazine-1-carbonyl)azetidine-1-carboxylate (27-a, 161 mg, yield 78.17%). ESI $[M+H-56]^+$=359.2

**Step 2: Preparation of azetidin-3-yl(4-(5-(trifluoromethyl)pyridin-2-yl)piperazin-1-yl)methanone hydrochloride (27-b)**

tert-butyl 3-(4-(5-(trifluoromethyl)pyridin-2-yl)piperazine-1-carbonyl)azetidine-1-carboxylate (27-a, 161 mg, 0.388 mmol) was dissolved in ethyl acetate (2 mL), and 4M hydrogen chloride in ethyl acetate solution (4 mL) was added under an ice bath. After reacting at room temperature for 2 hours, the reaction mixture was concentrated under reduced pressure and purified by thin-layer chromatography (dichloromethane:methanol = 15:1) to obtain the target product azetidin-3-yl(4-(5-(trifluoromethyl)pyridin-2-yl)piperazin-1-yl)methanone hydrochloride (27-b, 60 mg, yield 49.2%). ESI $[M+H]^+$=315.2

**Step 3: Preparation of 3-(trifluoromethyl)-5-(((3-(4-(5-(trifluoromethyl)pyridin-2-yl)piperazine-1-carbonyl)azetidin-1-yl)methyl)pyridin-2(1H)-one (27)**

azetidin-3-yl(4-(5-(trifluoromethyl)pyridin-2-yl)piperazin-1-yl)methanone hydrochloride (27-b, 60 mg, 0.172 mmol) was dissolved in dichloromethane (4 mL) and methanol (2 mL). Under an ice bath, 6-oxo-5-(trifluoromethyl)-1,6-dihydropyridine-3-carbaldehyde (I-7, 42.7 mg, 0.223 mmol) and sodium acetate (7 mg, 0.0860 mmol) were added. After stirring under an ice bath for 20 minutes, sodium cyanoborohydride (32.4 mg, 0.516 mmol) was added, and the reaction was stirred at room temperature for 3 hours. The reaction mixture was concentrated under reduced pressure, and the crude product was purified by preparative thin-layer chromatography (dichloromethane:methanol = 10:1) to obtain the target product 3-(trifluoromethyl)-5-(((3-(4-(5-(trifluoromethyl)pyridin-2-yl)piperazine-1-carbonyl)azetidin-1-yl)methyl)pyridin-2(1H)-one (27, 3.97 mg, yield 4.72%). ESI $[M+H]^+$=490.2 $^1$H NMR (600MHz, DMSO-d6) δ 12.21 (s, 1H), 8.42 (s, 1H), 7.80 (s, 2H), 7.57 (s, 1H), 6.96 (d, J = 20.6 Hz, 1H), 5.73 (s, 2H), 5.33 (s, 1H), 3.61 (d, J = 5.5 Hz, 5H), 3.58 - 3.49 (m, 3H), 3.17 (s, 2H), 3.16 (s, 2H).

**Embodiment 28: Preparation of 3-(trifluoromethyl)-5-(((((1*R*,3*R*)-3-(4-(5-(trifluoromethyl)pyridin-2-yl)piperazine-1-carbonyl)cyclobutyl)amino)methyl)pyridin-2(1*H*)-one (28)**

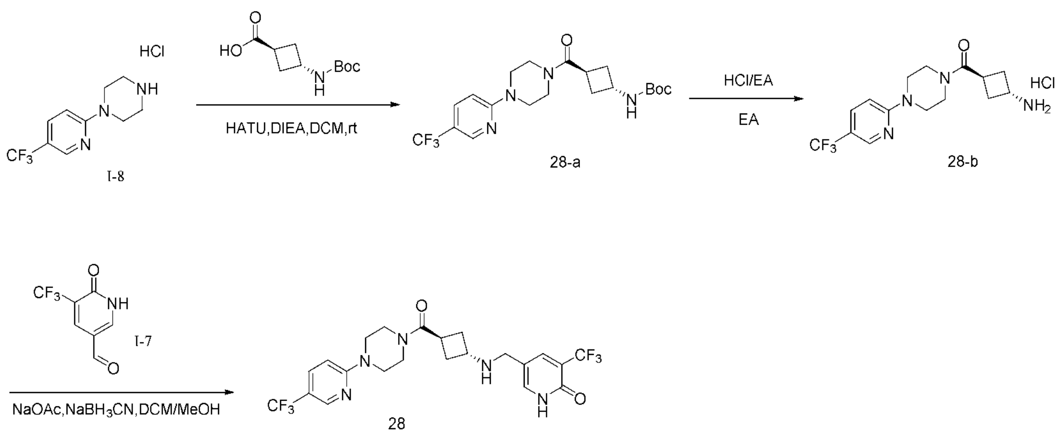


**Step 1: Preparation of tert-butyl ((1R,3R)-3-(4-(5-(trifluoromethyl)pyridin-2-yl)piperazine-1-carbonyl)cyclobutyl)carbamate (28-a)**

(1*R*,3*R*)-3-((tert-butoxycarbonyl)amino)cyclobutane-1-carboxylic acid (100 mg, 0.465 mmol), *O*-(7-azabenzotriazol-1-yl)-*N,N,N,N*-tetramethyluronium hexafluorophosphate (177 mg, 0.465 mmol), and diisopropylethylamine (245 μL, 1.395 mmol) were dissolved in dichloromethane (4 mL) and reacted for 20 minutes. Finally, 1-(5-(trifluoromethyl) pyridin-2-yl)piperazine hydrochloride (I-8, 137 mg, 0.511 mmol) was added, and the reaction was stirred at room temperature overnight. After monitoring showed the reaction was complete, the reaction mixture was concentrated under reduced pressure and purified by Flash chromatography (ethyl acetate:petroleum ether = 0-50%) to obtain the target product tert-butyl ((1*R*,3*R*)-3-(4-(5-(trifluoromethyl)pyridin-2-yl)piperazine-1-carbonyl)cyclobutyl)carbamate (28-a, 153 mg, yield 76.86%). ESI $[M+H]^+$=429.3

**Step 2: Preparation of ((1*R*,3*R*)-3-aminocyclobutyl)(4-(5-(trifluoromethyl)pyridin-2-yl)piperazin-1-yl)methanone hydrochloride (28-b)**

tert-butyl ((1*R*,3*R*)-3-(4-(5-(trifluoromethyl)pyridin-2-yl)piperazine-1-carbonyl)cyclobutyl)carbamate (28-a, 153 mg, 0.357 mmol) was dissolved in ethyl acetate (2 mL), and 4M hydrogen chloride in ethyl acetate solution (4 mL) was added under an ice bath. After reacting at room temperature for 2 hours, the reaction mixture was concentrated under reduced pressure to obtain the target product ((1*R*,3*R*)-3-aminocyclobutyl)(4-(5-(trifluoromethyl)pyridin-2-yl)piperazin-1-yl)methanone hydrochloride (28-b, 110 mg, yield 85.3%). ESI $[M+H]^+$=329.2

**Step 3: Preparation of 3-(trifluoromethyl)-5-(((((1*R*,3*R*)-3-(4-(5-(trifluoromethyl)pyridin-2-yl)piperazine-1-carbonyl)cyclobutyl)amino)methyl)pyridin-2(1*H*)-one (28)**

((1*R*,3*R*)-3-aminocyclobutyl)(4-(5-(trifluoromethyl)pyridin-2-yl)piperazin-1-yl)methanone hydrochloride (28-b, 110 mg, 0.303 mmol) was dissolved in dichloromethane (4 mL) and methanol (2 mL). Under an ice bath, 6-oxo-5-(trifluoromethyl)-1,6-dihydropyridine-3-carbaldehyde (I-7, 75.3 mg, 0.394 mmol) and sodium acetate (12.5 mg, 0.152 mmol) were added. After stirring under an ice bath for 20 minutes, sodium cyanoborohydride (57 mg, 0.909 mmol) was added, and the reaction was stirred at room temperature for 3 hours. The reaction mixture was concentrated under reduced pressure, and the crude product was purified by preparative thin-layer chromatography (dichloromethane:methanol = 10:1) to obtain the target product 3-(trifluoromethyl)-5-(((((1*R*,3*R*)-3-(4-(5-(trifluoromethyl)pyridin-2-yl)piperazine-1-carbonyl)cyclobutyl)amino)methyl)pyridin-2(1H)-one (28, 1.03 mg, yield 0.67%). ESI $[M+H]^+$=504.8 $^1H$ NMR (400 MHz, DMSO-$d_6$) δ 8.42 (s, 1H), 8.18 (s, 1H), 8.01 - 7.75 (m, 2H), 7.56 (s, 1H), 7.21 (s, 1H), 6.96 (d, J = 8.7 Hz, 1H), 6.68 (s, 1H), 5.76 (s, 2H), 5.32 (d, J = 3.2 Hz, 1H), 3.92 (ddd, J = 174.5, 96.7, 25.2 Hz, 6H), 3.14 (d, J = 16.4 Hz, 2H), 2.31 (d, J = 11.0 Hz, 2H), 1.97 (t, J = 13.0 Hz, 2H).

**Embodiment 29: Preparation of 3-(trifluoromethyl)-5-(((3-(4-(5-(trifluoromethyl)pyrazin-2-yl)piperazine-1-carbonyl)azetidin-1-yl)methyl)pyridin-2(1*H*)-one (29)**

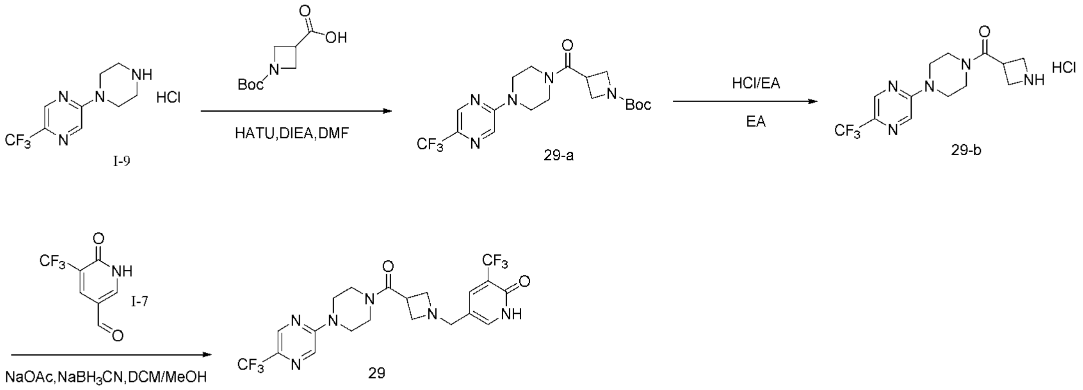


**Step 1: Preparation of tert-butyl 3-(4-(5-(trifluoromethyl)pyrazin-2-yl)piperazine-1-carbonyl)azetidine-1-carboxylate (29-a)**

1-(tert-butoxycarbonyl)azetidine-3-carboxylic acid (100 mg, 0.497 mmol) was dissolved in *N,N*-dimethylformamide (6 mL). Under an ice bath, *O*-(7-azabenzotriazol-1-yl)-*N,N,N',N'*-tetramethyluronium hexafluorophosphate (189 mg, 0.497 mmol) and *N,N*-diisopropylethylamine (261 μL, 1.49 mmol) were added. After stirring under an ice bath for 30 minutes, 2-(piperazin-1-yl)-5-(trifluoromethyl)pyrazine hydrochloride (I-9, 133.5 mg, 0.497 mmol) was added. After reacting at room temperature for 3 hours, the reaction mixture was added dropwise to water (12 mL) with stirring. The mixture was filtered, and the filter cake was concentrated under reduced pressure to obtain the target product tert-butyl 3-(4-(5-(trifluoromethyl)pyrazin-2-yl)piperazine-1-carbonyl)azetidine-1-carboxylate (29-a, 161 mg, yield 78.2%). ESI $[M-56+H]^+$=359.3

**Step 2: Preparation of azetidin-3-yl(4-(5-(trifluoromethyl)pyrazin-2-yl)piperazin-1-yl)methanone hydrochloride (29-b)**

tert-butyl 3-(4-(5-(trifluoromethyl)pyrazin-2-yl)piperazine-1-carbonyl)azetidine-1-carboxylate (29-a, 161 mg, 0.389 mmol) was dissolved in ethyl acetate (2 mL), and 4M hydrogen chloride in ethyl acetate solution (4 mL) was added under an ice bath. After reacting at room temperature for 3 hours, the reaction mixture was concentrated under reduced pressure, and the crude product was purified by thin-layer chromatography (dichloromethane:methanol = 20:1) to obtain the target product azetidin-3-yl(4-(5-(trifluoromethyl)pyrazin-2-yl)piperazin-1-yl)methanone hydrochloride (29-b, 80 mg, 65.3%). ESI $[M+H]^+$=316.3

**Step 3: Preparation of 3-(trifluoromethyl)-5-(((3-(4-(5-(trifluoromethyl)pyrazin-2-yl)piperazine-1-carbonyl)azetidin-1-yl)methyl)pyridin-2(1*H*)-one (29)**

azetidin-3-yl(4-(5-(trifluoromethyl)pyrazin-2-yl)piperazin-1-yl)methanone hydrochloride (29-b, 80 mg, 0.228 mmol) was dissolved in dichloromethane (4 mL) and methanol (2 mL). Under an ice bath, 6-oxo-5-(trifluoromethyl)-1,6-dihydropyridine-3-carbaldehyde (I-7, 57 mg, 0.296 mmol) and sodium acetate (10 mg, 0.114 mmol) were added. After stirring under an ice bath for 20 minutes, sodium cyanoborohydride (43 mg, 0.684 mmol) was added, and the reaction was stirred at room temperature for 3 hours. The reaction mixture was concentrated under reduced pressure, and the crude product was purified by preparative thin-layer chromatography (dichloromethane:methanol = 10:1) to obtain the target product 3-(trifluoromethyl)-5-(((3-(4-(5-(trifluoromethyl)pyrazin-2-yl)piperazine-1-carbonyl)azetidin-1-yl)methyl)pyridin-2(1*H*)-one (29, 5.65 mg, yield 5.06%). ESI $[M+H]^+$=491.8 $^1$H NMR (400 MHz, DMSO-$d_6$) δ 8.50 (s, 1H), 8.42 (s, 1H), 8.25 (s, 1H), 7.80 (s, 1H), 7.58 (s, 1H), 5.76 (s, 1H), 3.89 (d, J = 12.4 Hz, 2H), 3.70 (s, 4H), 3.58 (d, J = 1.9 Hz, 4H), 3.43 - 3.29 (m, 4H).

**Embodiment 30: Preparation of 6-((((*R*)-3-((*S*)-3-methyl-4-(5-(trifluoromethyl)pyrimidin-2-yl)piperazine-1-carbonyl)pyrrolidin-1-yl)methyl)-4-(trifluoromethyl)pyridazin-3(2*H*)-one (30)**

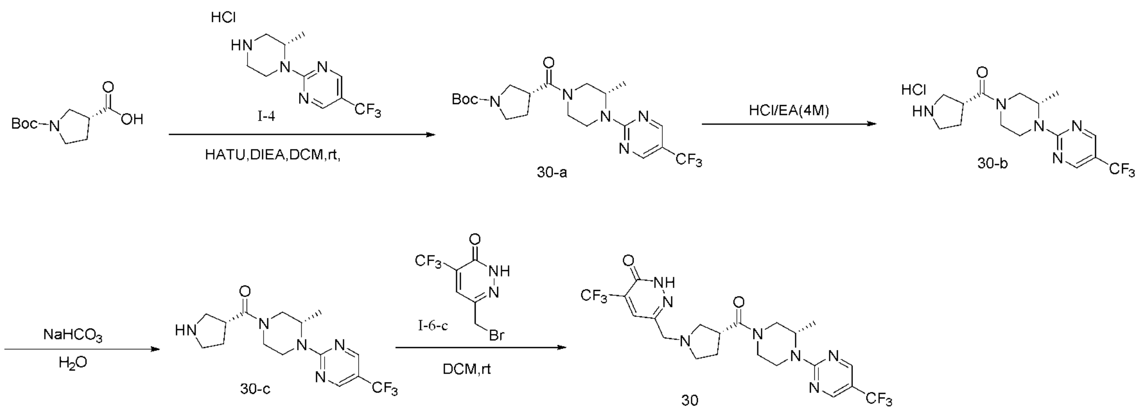


**Step 1: Preparation of tert-butyl (*R*)-3-((*S*)-3-methyl-4-(5-(trifluoromethyl)pyrimidin-2-yl)piperazine-1-carbonyl)pyrrolidine-1-carboxylate (30-a)**

(*R*)-1-(tert-butoxycarbonyl)pyrrolidine-3-carboxylic acid (100 mg, 0.465 mmol) was dissolved in dichloromethane (4 mL). Under an ice bath, *O*-(7-azabenzotriazol-1-yl)-*N,N,N,N*-tetramethyluronium hexafluorophosphate (177 mg, 0.465 mmol) and *N,N*-diisopropylethylamine (244 μL, 1.395 mmol) were added. After stirring under an ice bath for 30 minutes, (*S*)-2-(2-methylpiperazin-1-yl)-5-(trifluoromethyl)pyrimidine hydrochloride (I-4, 144 mg, 0.512 mmol) was added, and the mixture was reacted at room temperature for 1.5 hours. The reaction mixture was filtered, and the filtrate was concentrated under reduced pressure and purified by Flash chromatography (petroleum ether:ethyl acetate = 0-50%) to obtain the target product tert-butyl (*R*)-3-((*S*)-3-methyl-4-(5-(trifluoromethyl)pyrimidin-2-yl)piperazine-1-carbonyl)pyrrolidine-1-carboxylate (30-a, 190 mg, yield 92.22%). ESI $[M+H]^+$=444.8, ESI $[M+H-100]^+$=344.6

**Step 2: Preparation of ((*S*)-3-methyl-4-(5-(trifluoromethyl)pyrimidin-2-yl)piperazin-1-yl)((*R*)-pyrrolidin-3-yl)methanone hydrochloride (30-b)**

tert-butyl (*R*)-3-((*S*)-3-methyl-4-(5-(trifluoromethyl)pyrimidin-2-yl)piperazine-1-carbonyl)pyrrolidine-1-carboxylate (30-a, 0.19 g, 0.428 mmol) was dissolved in 4M hydrogen chloride in ethyl acetate solution (4 mL). After reacting at room temperature for 4 hours, the mixture was concentrated under reduced pressure to obtain the target product ((*S*)-3-methyl-4-(5-(trifluoromethyl)pyrimidin-2-yl)piperazin-1-yl)((*R*)-pyrrolidin-3-yl)methanone hydrochloride (30-b, 120 mg, crude product). ESI $[M+H]^+$= 344.8

**Step 3: Preparation of ((S)-3-methyl-4-(5-(trifluoromethyl)pyrimidin-2-yl)piperazin-1-yl)((*R*)-pyrrolidin-3-yl)methanone (30-c)**

((*S*)-3-methyl-4-(5-(trifluoromethyl)pyrimidin-2-yl)piperazin-1-yl)((*R*)-pyrrolidin-3-yl)methanone hydrochloride

(30-b, 120 mg, 0.315 mmol) was dissolved in water (1 mL), and sodium bicarbonate (29 mg, 0.347 mmol) was added. The mixture was extracted with dichloromethane, the organic phase was dried over anhydrous magnesium sulfate and filtered, and the filtrate was concentrated under reduced pressure to obtain the target product ((*S*)-3-methyl-4-(5-(trifluoromethyl)pyrimidin-2-yl)piperazin-1-yl)((*R*)-pyrrolidin-3-yl)methanone (30-c, 73 mg, yield 67.6%).

**Step 4: Preparation of 6-((((*R*)-3-((*S*)-3-methyl-4-(5-(trifluoromethyl)pyrimidin-2-yl)piperazine-1-carbonyl)pyrrolidin-1-yl)methyl)-4-(trifluoromethyl)pyridazin-3(2*H*)-one (30)**

((*S*)-3-methyl-4-(5-(trifluoromethyl)pyrimidin-2-yl)piperazin-1-yl)((*R*)-pyrrolidin-3-yl)methanone (30-c, 73 mg, 0.213 mmol) was dissolved in dichloromethane (2 mL), and 6-(bromomethyl)-4-(trifluoromethyl)pyridazin-3(2H)-one (I-6-c, 27 mg, 0.106 mmol) was added. After reacting at room temperature for 3 hours, the reaction mixture was concentrated under reduced pressure and purified by thin-layer chromatography (dichloromethane:methanol = 10:1) to obtain the target product 6-((((*R*)-3-((*S*)-3-methyl-4-(5-(trifluoromethyl)pyrimidin-2-yl)piperazine-1-carbonyl)pyrrolidin-1-yl)methyl)-4-(trifluoromethyl)pyridazin-3(2*H*)-one (30, 6.16 mg, yield 5.6%). ESI $[M+H]^+$=520.5 $^1H$ NMR (400 MHz, DMSO- $d_6$) δ 13.56 (s, 1H), 8.73 (s, 2H), 7.84 (s, 1H), 4.97 - 4.78 (m, 1H), 4.47 (d, J = 11.0 Hz, 1H), 4.28 (t, J = 11.7 Hz, 1H), 4.11 - 3.95 (m, 1H), 3.88 (d, J = 13.5 Hz, 1H), 3.63 - 3.49 (m, 2H), 3.27 - 3.12 (m, 2H), 2.86 (dd, J = 37.0, 15.7 Hz, 2H), 2.73 - 2.53 (m, 2H), 2.20 - 1.91 (m, 2H), 1.90 - 1.72 (m, 1H), 1.09 (dd, J = 25.0, 6.6 Hz, 3H).

**Embodiment 31: Preparation of (*R*)-4-(trifluoromethyl)-6-(((3-(4-(5-(trifluoromethyl)pyrimidin-2-yl)piperazine-1-carbonyl)pyrrolidin-1-yl)methyl)pyridazin-3(2*H*)-one (31)**

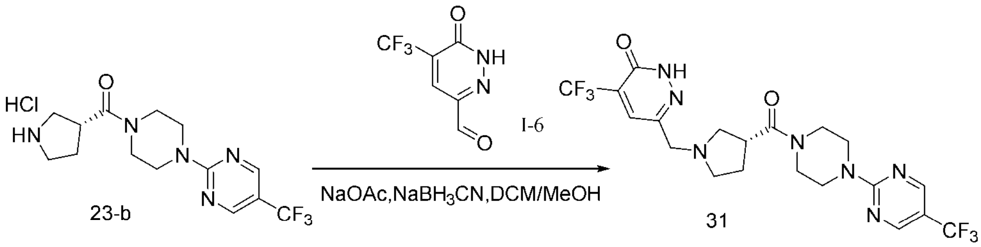

23-b
31

(*R*)-pyrrolidin-3-yl(4-(5-(trifluoromethyl)pyrimidin-2-yl)piperazin-1-yl)methanone hydrochloride (24-b, 120 mg, 0.365 mmol) was dissolved in dichloromethane/methanol (4 mL/2 mL). Under an ice bath, 6-oxo-5-(trifluoromethyl)-1,6-dihydropyridazine-3-carbaldehyde (I-6, 98 mg, 0.510 mmol) and sodium acetate (15 mg, 0.182 mmol) were added. After stirring under an ice bath for 20 minutes, sodium cyanoborohydride (69 mg, 1.09 mmol) was added. After reacting at room temperature for 3 hours, the reaction mixture was concentrated under reduced pressure, and the crude product was purified by preparative thin-layer chromatography (dichloromethane:methanol = 10:1) to obtain the target product (*R*)-4-(trifluoromethyl)-6-(((3-(4-(5-(trifluoromethyl)pyrimidin-2-yl)piperazine-1-carbonyl)pyrrolidin-1-yl)methyl)pyridazin-3(2*H*)-one (31, 2.73 mg, yield 1.48%). ESI $[M+H]^+$=506.8 $^1H$ NMR (400 MHz, DMSO- $d_6$) δ 13.56 (s, 1H), 8.73 (s, 2H), 7.84 (s, 1H), 3.82 (d, J = 5.1 Hz, 4H), 3.55 (s, 4H), 3.30 (d, J = 7.7 Hz, 2H), 2.82 (t, J = 8.6 Hz, 1H), 2.69 - 2.57 (m, 2H), 2.05 - 1.86 (m, 4H).

**Embodiment 32: Preparation of 4-(trifluoromethyl)-6-(((3-(4-(5-(trifluoromethyl)pyridin-2-yl)piperazine-1-carbonyl)azetidin-1-yl)methyl)pyridazin-3(2*H*)-one (32)**

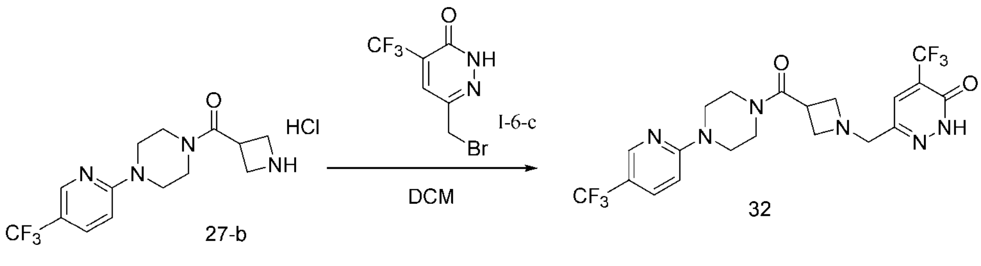

27-b
32

azetidin-3-yl(4-(5-(trifluoromethyl)pyridin-2-yl)piperazin-1-yl)methanone hydrochloride (27-b, 120 mg, 0.382

mmol) was dissolved in dichloromethane (2 mL), and 6-(bromomethyl)-4-(trifluoromethyl)pyridazin-3(2H)-one (I-6-c, 48 mg, 0.191 mmol) was added. After reacting at room temperature for 3 hours, the reaction mixture was concentrated under reduced pressure and purified by thin-layer chromatography (dichloromethane:methanol = 10:1) to obtain the target product 4-(trifluoromethyl)-6-(((3-(4-(5-(trifluoromethyl)pyridin-2-yl)piperazine-1-carbonyl)azetidin-1-yl)methyl)pyridazin-3(2*H*)-one (32, 6.07 mg, yield 3.24%). ESI $[M+H]^+$=491.5 $^1H$ NMR (400 MHz, DMSO-$d_6$) δ 13.56 (s, 1H), 8.42 (s, 1H), 8.15 (s, 1H), 7.80 (s, 1H), 6.96 (d, J = 9.1 Hz, 1H), 3.61 (s, 4H), 3.55 (d, J = 6.0 Hz, 4H), 3.50 (s, 2H), 3.46 (d, J = 7.6 Hz, 2H), 3.27 (d, J = 6.8 Hz, 2H), 3.16 (s, 1H).

**Embodiment 33: Preparation of ((1*R*,3*R*)-3-(((6-methoxy-5-(trifluoromethyl)pyridazin-3-yl)methyl)(methyl)amino)cyclobutyl)(4-(5-(trifluoromethyl)pyrimidin-2-yl)piperazin-1-yl)methanone (33)**

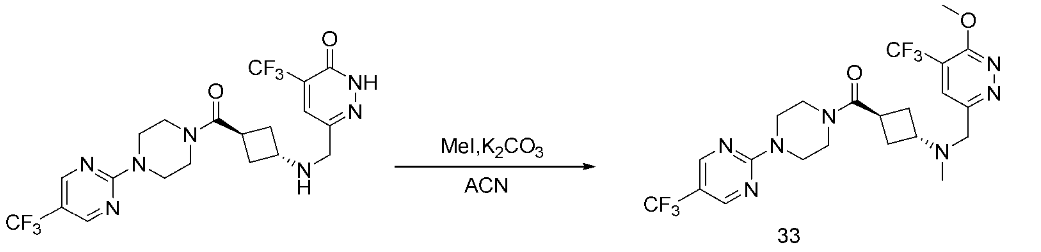

33

12

4-(trifluoromethyl)-6-(((((1*R*,3*R*)-3-(4-(5-(trifluoromethyl)pyrimidin-2-yl)piperazine-1-carbonyl)cyclobutyl)amino)methyl)pyridazin-3(2H)-one (12, 30 mg, 0.0595 mmol) was dissolved in acetonitrile (1 mL), and potassium carbonate (29 mg, 0.0893 mmol) and iodomethane (8 μL, 0.119 mmol) were added. After reacting at room temperature for 3 hours, the reaction mixture was concentrated under reduced pressure and purified by thin-layer chromatography (ethyl acetate) to obtain the target product ((1*R*,3*R*)-3-(((6-methoxy-5-(trifluoromethyl)pyridazin-3-yl)methyl)(methyl)amino)cyclobutyl)(4-(5-(trifluoromethyl)pyrimidin-2-yl)piperazin-1-yl)methanone (33, 5.33 mg, yield 16.8%). ESI $[M+H]^+$=534.7 $^1H$ NMR (400 MHz, DMSO- $d_6$) δ 8.73 (s, 2H), 7.84 (s, 1H), 3.83 (s, 4H), 3.69 (s, 3H), 3.57 (s, 2H), 3.42 (s, 2H), 3.31 (s, 2H), 3.22 (s, 1H), 2.93 (dd, J = 17.8, 10.7 Hz, 1H), 2.34 - 2.11 (m, 4H), 1.98 (s, 3H).

**Embodiment 34: Preparation of 6-((methyl(((1*R*,3*R*)-3-(4-(5-(trifluoromethyl)pyrimidin-2-yl)piperazine-1-carbonyl)cyclobutyl)amino)methyl)-4-(trifluoromethyl)pyridazin-3(2*H*)-one (34)**

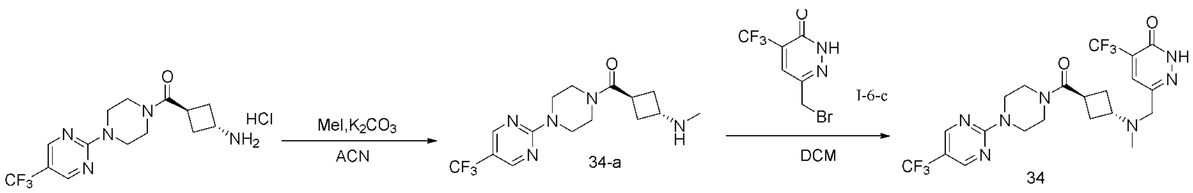

34-a
34

**Step 1: Preparation of ((1*R*,3*R*)-3-(methylamino)cyclobutyl)(4-(5-(trifluoromethyl)pyrimidin-2-yl)piperazin-1-yl)methanone (34-a)**

((1*R*,3*R*)-3-aminocyclobutyl)(4-(5-(trifluoromethyl)pyrimidin-2-yl)piperazin-1-yl)methanone hydrochloride (100 mg, 0.274 mmol) was dissolved in acetonitrile (2 mL), and cesium carbonate (133.7 mg, 0.410 mmol) and potassium iodide (6 μL, 0.274 mmol) were added. After reacting at room temperature for 20 minutes, the mixture was filtered, and the filtrate was concentrated under reduced pressure to obtain the target product ((1*R*,3*R*)-3-(methylamino)cyclobutyl)(4-(5-(trifluoromethyl)pyrimidin-2-yl)piperazin-1-yl)methanone (34-a, 100 mg, crude product). ESI $[M+H]^+$=344.5

**Step 2: Preparation of 6-((methyl(((1*R*,3*R*)-3-(4-(5-(trifluoromethyl)pyrimidin-2-yl)piperazine-1-carbonyl)cyclobutyl)amino)methyl)-4-(trifluoromethyl)pyridazin-3(2*H*)-one (34)**

((1*R*,3*R*)-3-(methylamino)cyclobutyl)(4-(5-(trifluoromethyl)pyrimidin-2-yl)piperazin-1-yl)methanone (34-a, 100

mg, 0.292 mmol) was dissolved in dichloromethane (2 mL), and 6-(bromomethyl)-4-(trifluoromethyl)pyridazin-3(2H)-one (I-6-c, 37 mg, 0.146 mmol) was added. After reacting at room temperature for 3 hours, the reaction mixture was concentrated under reduced pressure and purified by thin-layer chromatography (dichloromethane:methanol = 10:1), followed by preparative HPLC [Pack Column 20ID*250mm*10μm; A:$H_2O$(0.1%$NH_4HCO_3$), B:ACN, B%: 10%-100% over 25 min] to obtain the target product 6-((methyl(((1*R*,3*R*)-3-(4-(5-(trifluoromethyl)pyrimidin-2-yl)piperazine-1-carbonyl)cyclobutyl)amino)methyl)-4-(trifluoromethyl)pyridazin-3(2*H*)-one (34, 0.56 mg, yield 0.37%). ESI $[M+H]^+$=520.4 $^1$H NMR (400 MHz, DMSO-$d_6$) δ 13.56 (s, 1H), 8.74 (s, 2H), 7.82 (s, 1H), 3.83 (s, 4H), 3.57 (s, 2H), 3.42 (s, 2H), 3.22 (s, 2H), 2.94 (s, 2H), 2.22 (dd, J = 54.5, 18.5 Hz, 4H), 2.00 (d, J = 16.9 Hz, 3H).

**Embodiment 35: Preparation of 4-(trifluoromethyl)-6-(1-((((1*R*, 3*R*)-3-(4-(5-(trifluoromethyl)pyrimidin-2-yl)piperazine-1-carbonyl)cyclobutyl)amino)ethyl)pyridazin-3(2*H*)-one and 4-(trifluoromethyl)-6-(1-((((1*S*, 3*S*)-3-(4-(5-(trifluoromethyl)pyrimidin-2-yl)piperazine-1-carbonyl)cyclobutyl)amino)ethyl)pyridazin-3(2*H*)-one (35-P1, 35-P2)**

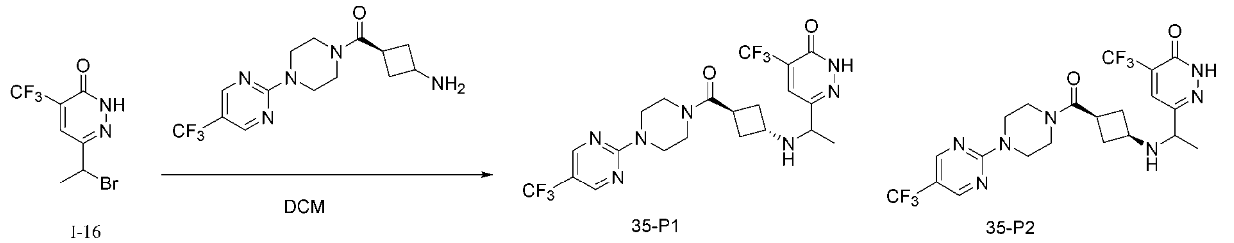


I-16 35-P1 35-P2

(3-aminocyclobutyl)(4-(5-(trifluoromethyl)pyrimidin-2-yl)piperazin-1-yl)methanone (120 mg, 0.363 mmol) was dissolved in dichloromethane (2 mL), and 6-(1-bromoethyl)-4-(trifluoromethyl)pyridazin-3(2*H*)-one (I-16, 54 mg, 0.182 mmol) was added. After reacting at room temperature for 2 hours, the reaction mixture was concentrated under reduced pressure and purified by preparative thin-layer chromatography (dichloromethane:methanol = 20:1), followed by chiral separation to obtain the target product 4-(trifluoromethyl)-6-(1-((((1*R*,3*R*)-3-(4-(5-(trifluoromethyl)pyrimidin-2-yl)piperazine-1-carbonyl)cyclobutyl)amino)ethyl)pyridazin-3(2*H*)-one (35-P1, 10.55 mg) and the target product 4-(trifluoromethyl)-6-(1-((((1*S*,3*S*)-3-(4-(5-(trifluoromethyl)pyrimidin-2-yl)piperazine-1-carbonyl)cyclobutyl)amino)ethyl)pyridazin-3(2*H*)-one (35-P2, 10.86 mg). ESI $[M+H]^+$=520.4 $^1$H NMR (400 MHz, DMSO-$d_6$) δ 13.49 (s, 1H), 8.73 (s, 2H), 7.98 (s, 1H), 3.80 (s, 4H), 3.74 - 3.40 (m, 4H), 3.19 (s, 1H), 3.03 (s, 1H), 2.67 (s, 1H), 2.38 - 1.87 (m, 4H), 1.89 - 1.72 (m, 1H), 1.23 (d, J = 5.1 Hz, 3H).

**Embodiment 36: Preparation of (*R*)- 4-(trifluoromethyl)-6-(1-(3-(4-(5-(trifluoromethyl)pyrimidin-2-yl)piperazine-1-carbonyl)azetidin-1-yl)ethyl)pyridazin-3(2*H*)-one and (*S*)- 4-(trifluoromethyl)-6-(1-(3-(4-(5-(trifluoromethyl)pyrimidin-2-yl)piperazine-1-carbonyl)azetidin-1-yl)ethyl)pyridazin-3(2*H*)-one (36-P1, 36-P2)**

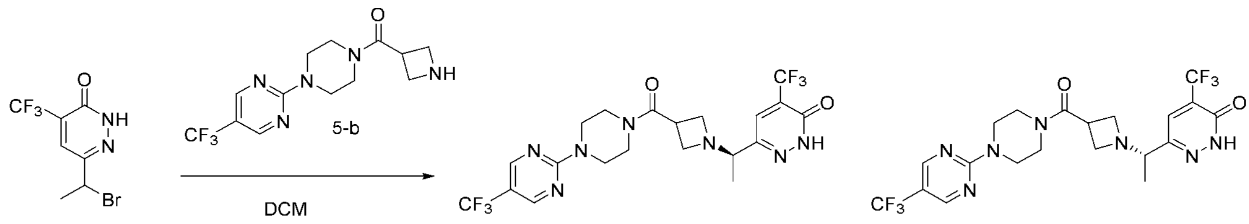


I-16 36-P1 36-P2

azetidin-3-yl(4-(5-(trifluoromethyl)pyrimidin-2-yl)piperazin-1-yl)methanone (5-b, 160 mg, 0.508 mmol) was dissolved in dichloromethane (10 mL), and 6-(1-bromoethyl)-4-(trifluoromethyl)pyridazin-3(2*H*)-one (I-16, 75.4 mg, 0.254 mmol) was added. After reacting at room temperature for 2 hours, the reaction mixture was concentrated under reduced pressure and purified by preparative thin-layer chromatography (dichloromethane:methanol = 20:1), followed by chiral separation to obtain the target product (*R*)-4-(trifluoromethyl)-6-(1-(3-(4-(5-(trifluoromethyl)pyrimidin-2-yl)piperazine-1-carbonyl)azetidin-1-yl)ethyl)pyridazin-3(2H)-one (36-P1, 7.99 mg) and the target product (S)-4-(trifluoromethyl)-6-(1-(3-(4-(5-(trifluoromethyl)pyrimidin-2-yl)piperazine-1-carbonyl)azetidin-1-yl)ethyl)pyridazin-3(2H)-one (36-P2, 7.71 mg). ESI $[M+H]^+$=506.5 $^1$H NMR (400 MHz, DMSO-$d_6$) δ 13.61 (s, 1H), 8.72 (s, 2H), 7.77 (s, 1H), 3.80 (s, 4H), 3.51 (d, J = 22.6 Hz, 4H), 3.29 (d, J = 6.2 Hz, 1H), 3.23 (s, 1H), 3.15 - 3.04 (m, 1H), 1.14 (d, J = 6.4 Hz, 3H).

**Embodiment 37: Preparation of (*R*)-4-(trifluoromethyl)-6-(((3-(3-(trifluoromethyl)-7,7*a*,8,9,10,11-hexahydro-6*H*-pyrazino[1,2-*d*]pyrido[3,2-*b*][1,4]oxazepine-9-carbonyl)azetidin-1-yl)methyl)pyridazin-3(2*H*)-one (37)**

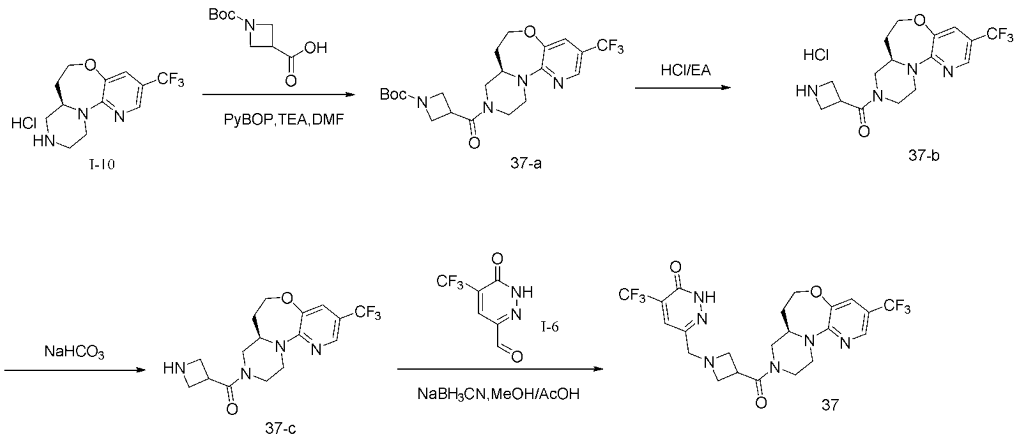


**Step 1: Preparation of tert-butyl (*R*)-3-(3-(trifluoromethyl)-7,7a,8,9,10,11-hexahydro-6*H*-pyrazino[1,2-*d*]pyrido [3,2-*b*][1,4]oxazepine-9-carbonyl)azetidine-1-carboxylate (37-a)**

1-(tert-butoxycarbonyl)azetidine-3-carboxylic acid (51 mg, 0.252 mmol), (*R*)-3-(trifluoromethyl)-7,7a,8,9,10,11-hexahydro-6*H*-pyrazino[1,2-*d*]pyrido[3,2-*b*][1,4]oxazepine hydrochloride (I-10, 60 mg, 0.194 mmol), and (benzotriazol-1-yloxy)tripyrrolidinophosphonium hexafluorophosphate (121 mg, 0.322 mmol) were dissolved in *N,N*-dimethylformamide (5 mL), and then triethylamine (81 μL, 0.423 mmol) was added. The reaction was stirred at room temperature for 2 hours. Water was added to the reaction mixture, which was then extracted with ethyl acetate. The combined organic phases were washed with saturated brine, and the organic phase was dried over anhydrous sodium sulfate and filtered. The filtrate was concentrated under reduced pressure and purified by Flash column chromatography (ethyl acetate: petroleum ether = 0-45%) to obtain the target product tert-butyl (*R*)-3-(3-(trifluoromethyl)-7,7*a*,8,9,10,11-hexahydro-6*H*-pyrazino[1,2-*d*] pyrido[3,2-b][1,4]oxazepine-9-carbonyl)azetidine-1-carboxylate (37-a, 75 mg, yield 84.81%). ESI $[M+H]^+$=457.3, ESI $[M+H-56]^+$=401.3

**Step 2: Preparation of (*R*)-azetidin-3-yl(3-(trifluoromethyl)-6,7,7a,8,10,11-hexahydro-9H-pyrazino[1,2-d]pyrido [3,2-b][1,4]oxazepin-9-yl)methanone hydrochloride (37-b)**

tert-butyl (*R*)-3-(3-(trifluoromethyl)-7,7*a*,8,9,10,11-hexahydro-6*H*-pyrazino[1,2-*d*]pyrido[3,2-*b*][1,4]oxazepine-9-carbonyl)azetidine-1-carboxylate (37-a, 75 mg, 0.164 mmol) was dissolved in 4M hydrogen chloride in ethyl acetate (2 mL) and reacted at room temperature for 1 hour. After monitoring showed the reaction was complete, the reaction mixture was diluted with dichloromethane multiple times and then concentrated under reduced pressure to obtain the target product (*R*)-azetidin-3-yl(3-(trifluoromethyl)-6,7,7*a*,8,10,11-hexahydro-9*H*-pyrazino[1,2-d]pyrido[3,2-b][1,4] oxazepin-9-yl)methanone hydrochloride (37-b, 74 mg, yield 114.66%). ESI $[M+H]^+$=357.4

**Step 3: Preparation of (*R*)-azetidin-3-yl(3-(trifluoromethyl)-6,7,7a,8,10,11-hexahydro-9H-pyrazino[1,2-d]pyrido [3,2-b][1,4]oxazepin-9-yl)methanone (37-c)**

(*R*)-azetidin-3-yl(3-(trifluoromethyl)-6,7,7,7a,8,10,11-hexahydro-9*H*-pyrazino[1,2-*d*]pyrido[3,2-*b*][1,4]oxazepin-9-yl)methanone hydrochloride (37-b, 74 mg, 0.188 mmol) was dissolved in water (2 mL), and sodium bicarbonate (19 mg, 0.226 mmol) was added. The mixture was reacted at room temperature for 5 minutes. After monitoring showed the pH was 8-9, the reaction mixture was concentrated under reduced pressure. The crude product was dissolved in a solution of dichloromethane:methanol = 10:1 by sonication and filtered. The filtrate was concentrated under reduced pressure to obtain the target product (R)-azetidin-3-yl(3-(trifluoromethyl)-6,7,7,7a,8,10,11-hexahydro-9*H*-pyrazino[1,2-*d*]pyrido[3,2-b][1,4]oxazepin-9-yl)methanone (37-c, 57 mg, yield 84.91%).

**Step 4: Preparation of (*R*)-4-(trifluoromethyl)-6-((3-(3-(trifluoromethyl)-7,7*a*,8,9,10,11-hexahydro-6*H*-pyrazino [1,2-*d*]pyrido[3,2-*b*][1,4]oxazepine-9-carbonyl)azetidin-1-yl)methyl)pyridazin-3(2H)-one (37)**

**(*R*)-azetidin-3-yl(3-(trifluoromethyl)-6,7,7,7*a*,8,10,11-hexahydro-9*H*-pyrazino[1,2-*d*]pyrido[3,2-*b*]**[1,4]oxazepin-9-yl)methanone (37-c, 57 mg, 0.16 mmol) and 6-oxo-5-(trifluoromethyl)-1,6-dihydropyridazine-3-carbaldehyde (I-6, 37 mg, 0.192 mmol) were dissolved in methanol (0.9 mL) and acetic acid (0.1 mL). The reaction was stirred at room temperature for 1.5 hours, then sodium cyanoborohydride (20 mg, 0.32 mmol) was added, and the reaction was stirred at room temperature overnight. After monitoring showed the reaction was complete, a large amount of water was added, and the mixture was extracted with dichloromethane:methanol = 10:1. The organic phase was dried over anhydrous sodium sulfate, filtered, and concentrated under reduced pressure. The residue was purified twice by preparative thin-layer chromatography silica gel plate (dichloromethane:methanol = 10:1) to obtain (R)-4-(trifluoromethyl)-6-((3-(3-(trifluoromethyl)-7,7*a*,8,9,10,11-hexahydro-6*H*-pyrazino[1,2-*d*]pyrido[3,2-*b*][1,4]oxazepine-9-carbonyl)azetidin-1-yl)methyl)pyridazin-3(2*H*)-one (37, 4.04 mg, yield 4.74%). ESI $[M+H]^+$=533.3 $^1$H NMR (600 MHz, DMSO-$d_6$)$\delta$ 13.55 (s, 1H), 8.14 (s, 1H), 7.79 (s, 1H), 7.34 (d, J = 7.3 Hz, 1H), 4.33 - 4.20 (m, 2H), 3.97 (s, 1H), 3.89 - 3.79 (m, 2H), 3.73 (d, J = 12.3 Hz, 1H), 3.67 - 3.58 (m, 2H), 3.53 (d, J = 6.7 Hz, 1H), 3.49 (s, 2H), 3.44 (d, J = 19.3 Hz, 2H), 3.29 - 3.18 (m, 2H), 2.09 (s, 1H), 1.94 - 1.83 (m, 1H).

**Embodiment 38: Preparation of 4-(trifluoromethyl)-6-(((((1*R*,3*R*)-3-(((R)-3-(trifluoromethyl)-7,7*a*,8,9,10,11-hexahydro-6*H*-pyrazino[1,2-*d*]pyrido[3,2-*b*][1,4]oxazepine-9-carbonyl)cyclobutyl)amino)methyl)pyridazin-3(2*H*)-one (38)**

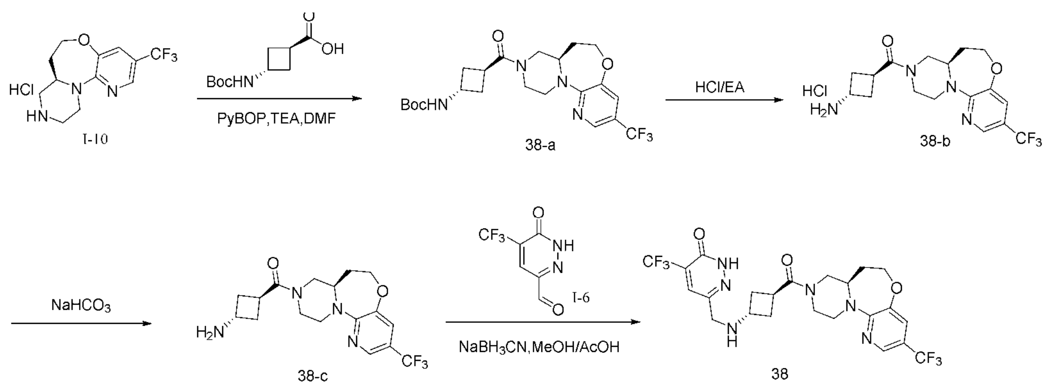


**Step 1: Preparation of tert-butyl ((1*R*,3*R*)-3-(((*R*)-3-(trifluoromethyl)-7,7*a*,8,9,10,11-hexahydro-6*H*-pyrazino[1,2-*d*]pyrido[3,2-*b*][1,4]oxazepine-9-carbonyl)cyclobutyl)carbamate (38-a)**

(1*R*,3*R*)-3-((tert-butoxycarbonyl)amino)cyclobutane-1-carboxylic acid (83 mg, 0.387 mmol), (*R*)-3-(trifluoromethyl)-7,7a,8,9,10,11-hexahydro-6*H*-pyrazino[1,2-*d*]pyrido[3,2-*b*][1,4]oxazepine hydrochloride (I-10, 100 mg, 0.323 mmol), and (benzotriazol-1-yloxy)tripyrrolidinophosphonium hexafluorophosphate (202 mg, 0.387 mmol) were dissolved in *N,N*-dimethylformamide (5 mL), and then triethylamine (135 μL, 0.969 mmol) was added. The reaction was stirred at room temperature overnight. Water was added to the reaction mixture, which was then extracted with ethyl acetate. The combined organic phases were washed with saturated brine, and the organic phase was dried over anhydrous sodium sulfate and filtered. The filtrate was concentrated under reduced pressure and purified by Flash column chromatography (ethyl acetate:petroleum ether = 0-57%) to obtain the target product tert-butyl ((1*R*,3*R*)-3-(((*R*)-3-(trifluoromethyl)-7,7*a*,8,9,10,11-hexahydro-6*H*-pyrazino[1,2-d]pyrido[3,2-b][1,4]oxazepine-9-carbonyl)cyclobutyl)carbamate (38-a, 120 mg, yield 79%). ESI $[M+H]^+$=471.4

**Step 2: Preparation of ((1*R*,3*R*)-3-aminocyclobutyl)((*R*)-3-(trifluoromethyl)-6,7,7*a*,8,10,11-hexahydro-9*H*-pyrazino[1,2-*d*]pyrido[3,2-*b*][1,4]oxazepin-9-yl)methanone hydrochloride (38-b)**

tert-butyl ((1*R*,3*R*)-3-(((*R*)-3-(trifluoromethyl)-7,7*a*,8,9,10,11-hexahydro-6*H*-pyrazino[1,2-*d*]pyrido[3,2-*b*][1,4] oxazepine-9-carbonyl)cyclobutyl)carbamate (38-a, 120 mg, 0.255 mmol) was dissolved in 4M hydrogen chloride in ethyl acetate (3 mL) and reacted at room temperature for 1.5 hours. After monitoring showed the reaction was complete, the reaction mixture was diluted with dichloromethane multiple times and then concentrated under reduced pressure to obtain the target product ((1*R*,3*R*)-3-aminocyclobutyl)((*R*)-3-(trifluoromethyl)-6,7,7a,8,10,11-hexahydro-9*H*-pyrazino[1,2-*d*]

pyrido[3,2-*b*][1,4]oxazepin-9-yl)methanone hydrochloride (38-b, 120 mg, yield 115.65%). ESI $[M+H]^+$=371.3

**Step 3: Preparation of ((1*R*,3*R*)-3-aminocyclobutyl)((*R*)-3-(trifluoromethyl)-6,7,7*a*,8,10,11-hexahydro-9*H*-pyrazino[1,2-*d*]pyrido[3,2-*b*][1,4]oxazepin-9-yl)methanone (38-c)**

((1*R*,3*R*)-3-aminocyclobutyl)((*R*)-3-(trifluoromethyl)-6,7,7*a*,8,10,11-hexahydro-9*H*-pyrazino[1,2-*d*]pyrido[3,2-*b*][1,4]oxazepin-9-yl)methanone hydrochloride (38-b, 120 mg, 0.295 mmol) was dissolved in water (2 mL), and sodium bicarbonate (30 mg, 0.355 mmol) was added. The mixture was reacted at room temperature for 5 minutes. After monitoring showed the pH was 8-9, the reaction mixture was concentrated under reduced pressure. The crude product was dissolved in a solution of dichloromethane:methanol = 10:1 by sonication and filtered. The filtrate was concentrated under reduced pressure to obtain the target product ((1R,3R)-3-aminocyclobutyl)((*R*)-3-(trifluoromethyl)-6,7,7*a*,8,10,11-hexahydro-9*H*-pyrazino[1,2-*d*]pyrido[3,2-*b*][1,4]oxazepin-9-yl)methanone (38-c, 99 mg, yield 90.62%).

**Step 4: Preparation of 4-(trifluoromethyl)-6-(((((1*R*,3*R*)-3-(((*R*)-3-(trifluoromethyl)-7,7*a*,8,9,10,11-hexahydro-6*H*-pyrazino[1,2-*d*]pyrido[3,2-*b*][1,4]oxazepine-9-carbonyl)cyclobutyl)amino)methyl)pyridazin-3(2*H*)-one (38)**

((1*R*,3*R*)-3-aminocyclobutyl)((*R*)-3-(trifluoromethyl)-6,7,7a,8,10,11-hexahydro-9*H*-pyrazino[1,2-*d*]pyrido[3,2-*b*][1,4]oxazepin-9-yl)methanone (38-c, 99 mg, 0.267 mmol) and 6-oxo-5-(trifluoromethyl)-1,6-dihydropyridazine-3-carbaldehyde (I-6, 62 mg, 0.321 mmol) were dissolved in methanol (1.8 mL) and acetic acid (0.2 mL). The reaction was stirred at room temperature for 1.5 hours, then sodium cyanoborohydride (34 mg, 0.535 mmol) was added, and the reaction was stirred at room temperature for 3 hours. After monitoring showed the reaction was complete, a large amount of water was added, and the mixture was extracted with dichloromethane:methanol = 10:1. The organic phase was dried over anhydrous sodium sulfate, filtered, and the filtrate was concentrated under reduced pressure and purified by Flash column chromatography (methanol:dichloromethane = 0-8%) to obtain 4-(trifluoromethyl)-6-(((((1*R*,3*R*)-3-(((*R*)-3-(trifluoromethyl)-7,7*a*,8,9,10,11-hexahydro-6*H*-pyrazino[1,2-*d*]pyrido[3,2-*b*][1,4]oxazepine-9-carbonyl)cyclobutyl)amino)methyl)pyridazin-3(2H)-one (38, 16.65 mg, yield 11.40%). ESI $[M+H]^+$=547.3 $^1H$ NMR (600 MHz, $CDCl_3$) δ 8.11 (d, J = 5.6 Hz, 1H), 7.72 (d, J = 3.6 Hz, 1H), 7.23 (d, J = 9.3 Hz, 1H), 4.32 (dd, J = 30.4, 7.3 Hz, 2H), 4.05 (dd, J = 13.6, 3.7 Hz, 1H), 4.01 - 3.86 (m, 2H), 3.77 (d, J = 6.8 Hz, 2H), 3.75 - 3.59 (m, 2H), 3.58 - 3.49 (m, 2H), 3.37 (dd, J = 13.5, 5.9 Hz, 1H), 3.31 (dd, J = 9.1, 4.5 Hz, 1H), 2.64 - 2.54 (m, 2H), 2.12 (dd, J = 16.3, 11.2 Hz, 3H), 2.05 - 1.92 (m, 1H).

**Embodiment 39: Preparation of 4-(trifluoromethyl)-6-(((((1*R*,3*R*)-3-(((R)-3-(trifluoromethyl)-7,7*a*,8,9,10,11-hexahydro-5*H*-pyrazino[2,1-*c*]pyrido[2,3-*e*][1,4]oxazepine-9-carbonyl)cyclobutyl)amino)methyl)pyridazin-3(2*H*)-one (39)**

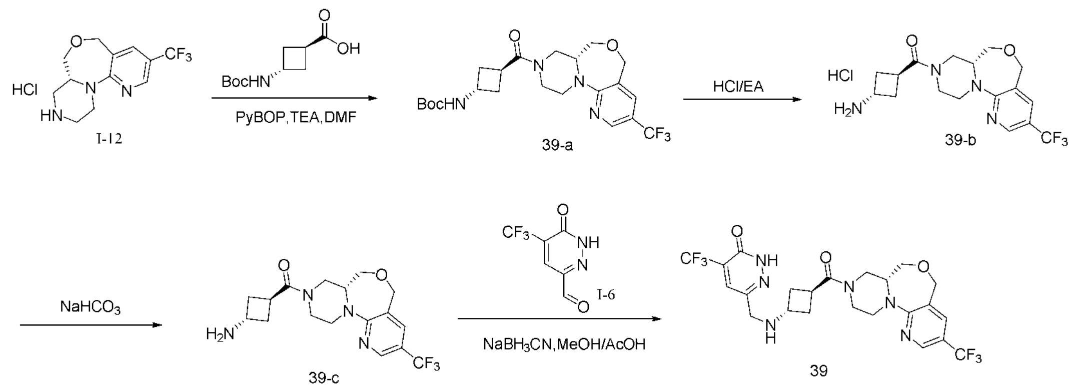


**Step 1: Preparation of tert-butyl ((1*R*,3*R*)-3-(((*R*)-3-(trifluoromethyl)-7,7*a*,8,9,10,11-hexahydro-5*H*-pyrazino[2,1-*c*]pyrido[2,3-*e*][1,4]oxazepine-9-carbonyl)cyclobutyl)carbamate (39-a)**

(1*R*,3*R*)-3-((tert-butoxycarbonyl)amino)cyclobutane-1-carboxylic acid (24 mg, 0.127 mmol), (*R*)-3-(trifluoromethyl)-7,7*a*,8,9,10,11-hexahydro-5*H*-pyrazino[2,1-*c*]pyrido[2,3-*e*][1,4]oxazepine hydrochloride (I-12, 29 mg, 0.106 mmol), and (benzotriazol-1-yloxy)tripyrrolidinophosphonium hexafluorophosphate (58 mg, 0.127 mmol) were dissolved in *N,N*-dimethylformamide (2 mL), and then triethylamine (38 μL, 0.318 mmol) was added. The reaction was stirred at room temperature overnight. Water was added to the reaction mixture, which was then extracted with ethyl acetate. The

combined organic phases were washed with saturated brine, and the organic phase was dried over anhydrous sodium sulfate and filtered. The filtrate was concentrated under reduced pressure and purified by Flash column chromatography (ethyl acetate:petroleum ether = 0-51%) to obtain the target product tert-butyl ((1*R*,3*R*)-3-(((*R*)-3-(trifluoromethyl)-7,7*a*,8,9,10,11-hexahydro-5*H*-pyrazino[2,1-*c*]pyrido[2,3-*e*][1,4]oxazepine-9-carbonyl)cyclobutyl)carbamate (39-a, 56 mg, yield 112.15%). ESI $[M+H]^+$=471.3, ESI $[M+H-56]^+$=415.2

**Step 2: Preparation of ((1*R*,3*R*)-3-aminocyclobutyl)((*R*)-3-(trifluoromethyl)-7*a*,8,10,11-tetrahydro-5*H*-pyrazino [2,1-*c*]pyrido[2,3-*e*][1,4]oxazepin-9(7*H*)-yl)methanone hydrochloride (39-b)**

tert-butyl ((1*R*,3*R*)-3-(((*R*)-3-(trifluoromethyl)-7,7*a*,8,9,10,11-hexahydro-5*H*-pyrazino[2,1-*c*]pyrido[2,3-*e*][1,4] oxazepine-9-carbonyl)cyclobutyl)carbamate (39-a, 56 mg, 0.119 mmol) was dissolved in 4M hydrogen chloride in ethyl acetate (2 mL) and reacted at room temperature for 3 hours. After monitoring showed the reaction was complete, the reaction mixture was diluted with dichloromethane multiple times and then concentrated under reduced pressure to obtain the target product ((1*R*,3*R*)-3-aminocyclobutyl)((R)-3-(trifluoromethyl)-7a,8,10,11-tetrahydro-5*H*-pyrazino[2,1-*c*]pyrido [2,3-*e*][1,4]oxazepin-9(7*H*)-yl)methanone hydrochloride (39-b, 58 mg, yield 119.78%). ESI $[M+H]^+$=371.2, ESI $[2M+H]^+$ =741.4

**Step 3: Preparation of ((1*R*,3*R*)-3-aminocyclobutyl)((*R*)-3-(trifluoromethyl)-7*a*,8,10,11-tetrahydro-5*H*-pyrazino [2,1-*c*]pyrido[2,3-e][1,4]oxazepin-9(7*H*)-yl)methanone (39-c)**

((1*R*,3*R*)-3-aminocyclobutyl)((*R*)-3-(trifluoromethyl)-7*a*,8,10,11-tetrahydro-5*H*-pyrazino[2,1-*c*]pyrido[2,3-*e*] [1,4]oxazepin-9(7*H*)-yl)methanone hydrochloride (39-b, 58 mg, 0.143 mmol) was dissolved in water (1.5 mL), and sodium bicarbonate (24 mg, 0.286 mmol) was added. The mixture was reacted at room temperature for 5 minutes. After monitoring showed the pH was 8-9, the reaction mixture was concentrated under reduced pressure. The crude product was dissolved in a solution of dichloromethane:methanol = 10:1 by sonication and filtered. The filtrate was concentrated under reduced pressure to obtain the target product ((1*R*,3*R*)-3-aminocyclobutyl)((*R*)-3-(trifluoromethyl)-7*a*,8,10,11-tetrahydro-5*H*-pyrazino[2,1-*c*]pyrido[2,3-e][1,4]oxazepin-9(7*H*)-yl)methanone (39-c, 52 mg, yield 98.31%).

**Step 4: Preparation of 4-(trifluoromethyl)-6-(((((1*R*,3*R*)-3-(((*R*)-3-(trifluoromethyl)-7,7*a*,8,9,10,11-hexahydro-5*H*-pyrazino[2,1-*c*]pyrido[2,3-*e*][1,4]oxazepine-9-carbonyl)cyclobutyl)amino)methyl)pyridazin-3(2*H*)-one (39)**

((1*R*,3*R*)-3-aminocyclobutyl)((*R*)-3-(trifluoromethyl)-7*a*,8,10,11-tetrahydro-5*H*-pyrazino[2,1-*c*]pyrido[2,3-e] [1,4]oxazepin-9(7*H*)-yl)methanone (39-c, 52 mg, 0.211 mmol) and 6-oxo-5-(trifluoromethyl)-1,6-dihydropyridazine-3-carbaldehyde (I-6, 40.5 mg, 0.14 mmol) were dissolved in methanol (0.9 mL) and acetic acid (0.1 mL). The reaction was stirred at room temperature for 1.5 hours, then sodium cyanoborohydride (18 mg, 0.281 mmol) was added, and the reaction was stirred at room temperature overnight. After monitoring showed the reaction was complete, a large amount of water was added, and the mixture was extracted with dichloromethane:methanol = 10:1. The organic phase was dried over anhydrous sodium sulfate, filtered, and the filtrate was concentrated under reduced pressure and purified by Flash column chromatography (methanol:dichloromethane = 0-8%), then by preparative thin-layer chromatography silica gel plate (dichloromethane:methanol = 10:1), and then by preparative HPLC [Pack Column 20ID*250mm*10μm; A:$H_2O$(0.1%FA), B:ACN, B%: 5%-80% over 22 min] to obtain 4-(trifluoromethyl)-6-(((((1*R*,3*R*)-3-(((*R*)-3-(trifluoromethyl)-7,7*a*,8,9,10,11-hexahydro-5*H*-pyrazino[2,1-*c*]pyrido[2,3-*e*][1,4]oxazepine-9-carbonyl)cyclobutyl)amino)methyl)pyridazin-3(2*H*)-one (39, 2.44 mg, yield 3.18%). ESI $[M+H]^+$=547.3, ESI $[2M+H]^+$=1093.5

**Embodiment 40: Preparation of (*S*)-4-(trifluoromethyl)-6-((3-(3-(trifluoromethyl)-7,7*a*,8,9,10,11-hexahydro-6*H*-pyrazino[1,2-*d*]pyrido[3,2-*b*][1,4]oxazepine-9-carbonyl)azetidin-1-yl)methyl)pyridazin-3(2*H*)-one (40)**

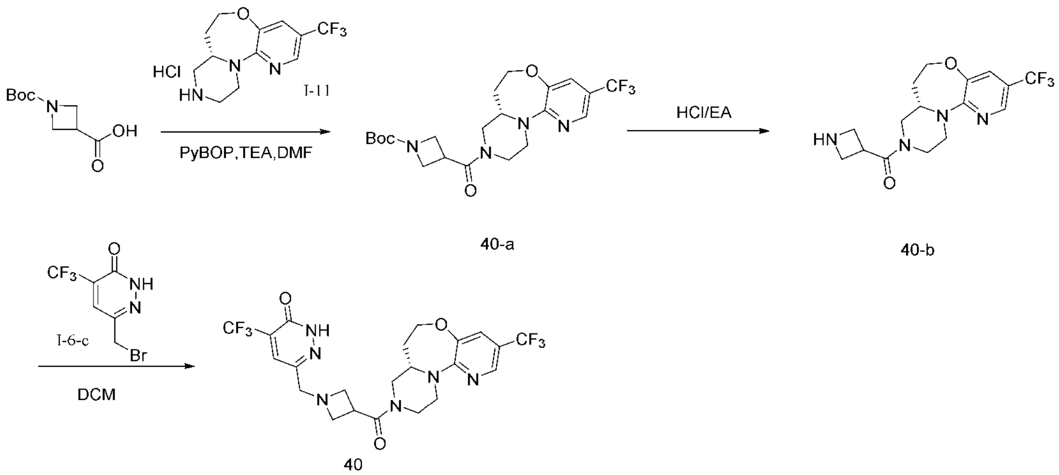


40-a

40-b

40

**Step 1: Preparation of tert-butyl (*S*)-3-(3-(trifluoromethyl)-7,7*a*,8,9,10,11-hexahydro-6*H*-pyrazino[1,2-*d*]pyrido[3,2-*b*][1,4]oxazepine-9-carbonyl)azetidine-1-carboxylate (40-a)**

1-(tert-butoxycarbonyl)azetidine-3-carboxylic acid (260 mg, 0.743 mmol) was dissolved in *N,N*-dimethylformamide (10 mL), and (benzotriazol-1-yloxy)tripyrrolidinophosphonium hexafluorophosphate (463.9 mg, 0.891 mmol), (*S*)-3-(trifluoromethyl)-7,7*a*,8,9,10,11-hexahydro-6*H*-pyrazino[1,2-*d*]pyrido[3,2-*b*][1,4]oxazepine hydrochloride (I-11, 230 mg, 0.473 mmol), and triethylamine (310 μL, 2.23 mmol) were added. After reacting at room temperature for 2 hours, the reaction mixture was quenched with water (15 mL) and extracted with ethyl acetate. The organic phase was dried over anhydrous sodium sulfate and filtered. The filtrate was concentrated under reduced pressure and purified by normal phase column chromatography (petroleum ether:ethyl acetate = 1:8) to obtain the target product tert-butyl (*S*)-3-(3-(trifluoromethyl)-7,7*a*,8,9,10,11-hexahydro-6*H*-pyrazino[1,2-d]pyrido[3,2-b][1,4]oxazepine-9-carbonyl)azetidine-1-carboxylate (40-a, 177 mg, yield 42.96%). ESI $[M-56+H]^+$=401.3

**Step 2: Preparation of (*S*)-azetidin-3-yl(3-(trifluoromethyl)-6,7,7,7*a*,8,10,11-hexahydro-9*H*-pyrazino[1,2-*d*]pyrido[3,2-*b*][1,4]oxazepin-9-yl)methanone (40-b)**

tert-butyl (*S*)-3-(3-(trifluoromethyl)-7,7*a*,8,9,10,11-hexahydro-6*H*-pyrazino[1,2-*d*]pyrido[3,2-*b*][1,4]oxazepine-9-carbonyl)azetidine-1-carboxylate (40-a, 177 mg, 0.381 mmol) was dissolved in ethyl acetate (2 mL), and 4M hydrogen chloride in ethyl acetate solution (3 mL) was added. After reacting at room temperature for 3 hours, the mixture was concentrated under reduced pressure. Water (1 mL) was added, the pH was adjusted to 8 with $NaHCO_3$, and the mixture was extracted with dichloromethane:methanol = 8:1. The organic phase was dried over anhydrous sodium sulfate and filtered. The filtrate was concentrated under reduced pressure and purified by normal phase column chromatography (dichloromethane:methanol = 8:1) to obtain the target product (*S*)-azetidin-3-yl(3-(trifluoromethyl)-6,7,7,7*a*,8,10,11-hexahydro-9*H*-pyrazino[1,2-*d*]pyrido[3,2-*b*][1,4]oxazepin-9-yl)methanone (40-b, 50 mg, yield 36.7%). ESI $[M+H]^+$=357.3.

**Step 3: Preparation of (*S*)-4-(trifluoromethyl)-6-((3-(3-(trifluoromethyl)-7,7*a*,8,9,10,11-hexahydro-6*H*-pyrazino[1,2-*d*]pyrido[3,2-*b*][1,4]oxazepine-9-carbonyl)azetidin-1-yl)methyl)pyridazin-3(2*H*)-one (40)**

(*S*)-azetidin-3-yl(3-(trifluoromethyl)-6,7,7,7a,8,10,11-hexahydro-9*H*-pyrazino[1,2-*d*]pyrido[3,2-*b*][1,4]oxazepin-9-yl)methanone (40-b, 50 mg, 0.195 mmol) was dissolved in dichloromethane (2 mL), and 6-(bromomethyl)-4-(trifluoromethyl)pyridazin-3(2*H*)-one (I-6-c, 17.5 mg, 0.098 mmol) was added. After reacting at room temperature for 3 hours, the mixture was purified by preparative thin-layer chromatography (dichloromethane:methanol = 10:1), and then purified by preparative HPLC [Pack Column 20ID*250mm* 10μm; A:$H_2O$(0.1%FA), B:ACN, B%: 10%-100% over 25 min] to obtain the target product (*S*)-4-(trifluoromethyl)-6-((3-(3-(trifluoromethyl)-7,7a,8,9,10,11-hexahydro-6*H*-pyrazino[1,2-*d*]pyrido[3,2-*b*][1,4]oxazepine-9-carbonyl)azetidin-1-yl)methyl)pyridazin-3(2*H*)-one (40, 1.51 mg, yield 3.17%). ESI $[M+H]^+$=533.4 $^1H$ NMR (400 MHz, DMSO-$d_6$) δ 13.59 (s, 1H), 8.15 (s, 1H), 7.80 (s, 1H), 7.35 (d, J = 14.3 Hz, 1H), 4.34 - 4.18 (m, 2H), 3.98 (dd, J = 17.1, 12.3 Hz, 2H), 3.81 (dd, J = 13.7, 3.6 Hz, 2H), 3.69 - 3.55 (m, 2H), 3.49 (d, J = 7.3 Hz, 4H), 3.25 (d, J = 7.0 Hz, 1H), 3.18 - 3.11 (m, 1H), 2.21 - 1.98 (m, 2H), 1.97 - 1.75 (m, 2H).

**Embodiment 41: Preparation of 4-(trifluoromethyl)-6-(((((1*S*,3*R*)-3-(((*S*)-3-(trifluoromethyl)-7,7*a*,8,9,10,11-hexahydro-6*H*-pyrazino[1,2-*d*]pyrido[3,2-*b*][1,4]oxazepine-9-carbonyl)cyclobutyl)amino)methyl)pyridazin-3(2*H*)-one (41)**

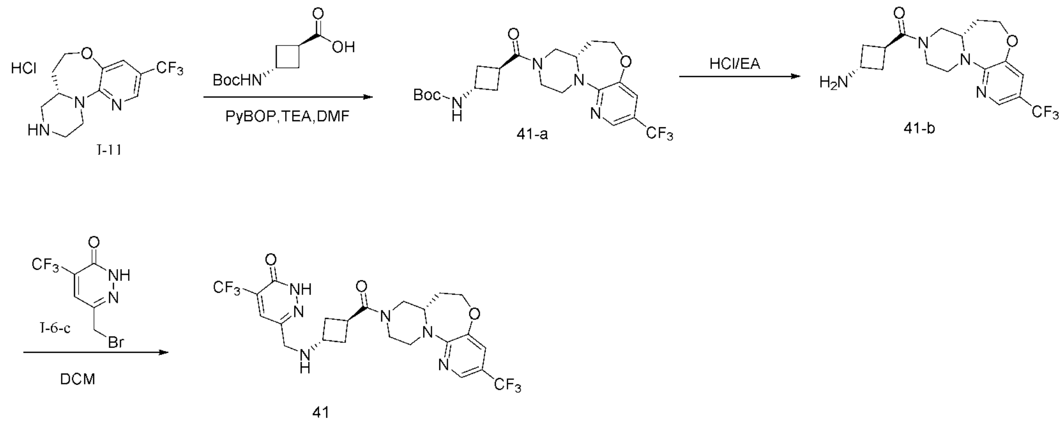


**Step 1: Preparation of tert-butyl ((1*S*,3*R*)-3-(((*S*)-3-(trifluoromethyl)-7,7*a*,8,9,10,11-hexahydro-6*H*-pyrazino[1,2-*d*]pyrido[3,2-*b*][1,4]oxazepine-9-carbonyl)cyclobutyl)carbamate (41-a)**

(1*R*,3*R*)-3-((tert-butoxycarbonyl)amino)cyclobutane-1-carboxylic acid (139 mg, 0.646 mmol) was dissolved in *N,N*-dimethylformamide (5 mL), and (benzotriazol-1-yloxy)tripyrrolidinophosphonium hexafluorophosphate (403.4 mg, 0.775 mmol), (*S*)-3-(trifluoromethyl)-7,7*a*,8,9,10,11-hexahydro-6*H*-pyrazino[1,2-*d*]pyrido[3,2-*b*][1,4]oxazepine hydrochloride (I-11, 200 mg, 0.646 mmol), and triethylamine (269 μL, 1.94 mmol) were added. After reacting at room temperature for 2 hours, the reaction mixture was quenched with water (15 mL) and extracted with ethyl acetate. The organic phase was dried over anhydrous sodium sulfate and filtered. The filtrate was concentrated under reduced pressure and purified by normal phase column chromatography (petroleum ether:ethyl acetate = 1:8) to obtain the target product tert-butyl ((1*S*,3*R*)-3-(((*S*)-3-(trifluoromethyl)-7,7*a*,8,9,10,11-hexahydro-6*H*-pyrazino[1,2-*d*]pyrido[3,2-*b*][1,4]oxazepine-9-carbonyl)cyclobutyl)carbamate (41-a, 201 mg, yield 66.2%). ESI $[M+H]^+$=471.3, ESI $[M-56+H]^+$=415.3

**Step 2: Preparation of (((1*R*,3*S*)-3-aminocyclobutyl)((*S*)-3-(trifluoromethyl)-6,7,7*a*,8,10,11-hexahydro-9*H*-pyrazino[1,2-*d*]pyrido[3,2-*b*][1,4]oxazepin-9-yl)methanone (41-b)**

tert-butyl ((1*S*,3*R*)-3-((*S*)-3-(trifluoromethyl)-7,7*a*,8,9,10,11-hexahydro-6*H*-pyrazino[1,2-*d*]pyrido[3,2-b][1,4]oxathiepine-9-carbonyl)cyclobutyl)carbamate (41-a, 201 mg, 0.447 mmol) was dissolved in ethyl acetate (2 mL), and 4M hydrogen chloride in ethyl acetate solution (2 mL) was added. After reacting at room temperature for 3 hours, the mixture was concentrated under reduced pressure. Water (1 mL) was added, the pH was adjusted to 8 with $NaHCO_3$, and the mixture was extracted with dichloromethane:methanol = 8:1. The organic phase was dried over anhydrous sodium sulfate and filtered, and the filtrate was concentrated under reduced pressure to obtain the target product (((1*R*,3*S*)-3-aminocyclobutyl)((*S*)-3-(trifluoromethyl)-6,7,7*a*,8,10,11-hexahydro-9*H*-pyrazino[1,2-*d*]pyrido[3,2-*b*][1,4]oxazepin-9-yl)methanone (41-b, 96 mg, yield 60.6%). ESI $[M+H]^+$=371.4

**Step 3: Preparation of 4-(trifluoromethyl)-6-(((((1*S*,3*R*)-3-((*S*)-3-(trifluoromethyl)-7,7*a*,8,9,10,11-hexahydro-6*H*-pyrazino[1,2-*d*]pyrido[3,2-*b*][1,4]oxazepine-9-carbonyl)cyclobutyl)amino)methyl)pyridazin-3(2H)-one (41)**

(((1*R*,3*S*)-3-aminocyclobutyl)((*S*)-3-(trifluoromethyl)-6,7,7*a*,8,10,11-hexahydro-9*H*-pyrazino[1,2-*d*]pyrido [3,2-*b*][1,4]oxazepin-9-yl)methanone (41-b, 96 mg, 0.259 mmol) was dissolved in dichloromethane (2 mL), and 6-(bromomethyl)-4-(trifluoromethyl)pyridazin-3(2*H*)-one (I-6-c, 33.2 mg, 0.130 mmol) was added. After reacting at room temperature for 3 hours, the mixture was purified by preparative thin-layer chromatography (dichloromethane:methanol = 10:1), and then purified by preparative HPLC [Pack Column 20ID*250mm*10μm; A:$H_2O$(0.1%FA), B:ACN, B%: 10%-100% over 25 min] to obtain the target product 4-(trifluoromethyl)-6-(((((1*S*,3*R*)-3-((*S*)-3-(trifluoromethyl)-7,7a,8,9,10,11-hexahydro-6*H*-pyrazino[1,2-*d*]pyrido[3,2-*b*][1,4]oxazepine-9-carbonyl)cyclobutyl)amino)methyl)pyridazin-3(2H)-one (41, 6.57 mg, yield 4.76%). ESI $[M+H]^+$=547.2 $^1$H NMR (600 MHz, DMSO-$d_6$) δ 13.40 (s, 1H), 8.14 (s, 1H), 7.91 (s, 1H), 7.32 (s, 1H), 4.26 (d, J = 30.7 Hz, 2H), 3.90 (dd, J = 97.5, 13.2 Hz, 2H), 3.65 (dd, J = 57.8, 22.5 Hz, 6H), 3.17 (t, J

= 45.3 Hz, 4H), 2.27 (s, 2H), 1.92 (d, J = 25.7 Hz, 2H).

**Embodiment 42: Preparation of (*R*)-4-(trifluoromethyl)-6-((3-(3-(trifluoromethyl)-7,7*a*,8,9,10,11-hexahydro-5*H*-pyrazino[2,1-*c*]pyrido[2,3-*e*][1,4]oxazepine-9-carbonyl)azetidin-1-yl)methyl)pyridazin-3(2*H*)-one (42)**

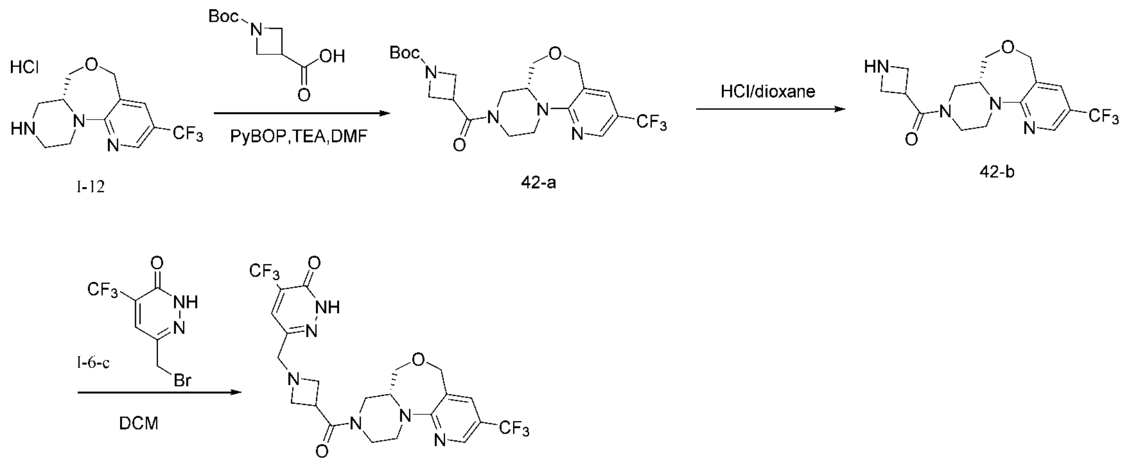


I-12 42-a 42-b 42

**Step 1: Preparation of tert-butyl (*R*)-3-(3-(trifluoromethyl)-7,7*a*,8,9,10,11-hexahydro-5*H*-pyrazino[2,1-*c*]pyrido[2,3-*e*][1,4]oxazepine-9-carbonyl)azetidine-1-carboxylate (42-a)**

1-(tert-butoxycarbonyl)azetidine-3-carboxylic acid (71.5 mg, 0.355 mmol) was dissolved in *N,N*-dimethylformamide (3 mL), and (benzotriazol-1-yloxy)tripyrrolidinophosphonium hexafluorophosphate (222 mg, 0.426 mmol), (*R*)-3-(trifluoromethyl)-7,7a,8,9,10,11-hexahydro-5*H*-pyrazino[2,1-c]pyrido[2,3-*e*][1,4]oxazepine hydrochloride (I-12, 110 mg, 0.355 mmol), and triethylamine (148 μL, 1.07 mmol) were added. After reacting at room temperature for 2 hours, the reaction mixture was quenched with water (10 mL) and extracted with ethyl acetate. The organic phase was dried over anhydrous sodium sulfate and filtered. The filtrate was concentrated under reduced pressure and purified by normal phase column chromatography (petroleum ether:ethyl acetate = 1:8) to obtain the target product tert-butyl (*R*)-3-(3-(trifluoromethyl)-7,7*a*,8,9,10,11-hexahydro-5*H*-pyrazino[2,1-c]pyrido[2,3-*e*][1,4]oxazepine-9-carbonyl)azetidine-1-carboxylate (42-a, 110 mg, yield 67.9%). ESI $[M-56+H]^+$=401.2

**Step 2: Preparation of (*R*)-azetidin-3-yl(3-(trifluoromethyl)-7a,8,10,11-tetrahydro-5*H*-pyrazino[2,1-*c*]pyrido[2,3-*e*][1,4]oxazepin-9(7*H*)-yl)methanone (42-b)**

To tert-butyl (*R*)-3-(3-(trifluoromethyl)-7,7*a*,8,9,10,11-hexahydro-5*H*-pyrazino[2,1-*c*]pyrido[2,3-*e*][1,4]oxazepine-9-carbonyl)azetidine-1-carboxylate (42-a, 110 mg, 0.241 mmol) was added 4M hydrogen chloride in dioxane solution (4 mL). After reacting at room temperature for 3 hours, the mixture was concentrated under reduced pressure. Water (1 mL) was added, the pH was adjusted to 8 with $NaHCO_3$, and the mixture was extracted with dichloromethane:methanol = 8:1. The organic phase was dried over anhydrous sodium sulfate and filtered, and the filtrate was concentrated under reduced pressure to obtain the target product (*R*)-azetidin-3-yl(3-(trifluoromethyl)-7a,8,10,11-tetrahydro-5*H*-pyrazino[2,1-*c*]pyrido[2,3-*e*][1,4]oxazepin-9(7*H*)-yl)methanone (42-b, 100 mg, crude). ESI $[M+H]^+$=357.3

**Step 3: Preparation of (*R*)-4-(trifluoromethyl)-6-((3-(3-(trifluoromethyl)-7,7*a*,8,9,10,11-hexahydro-5*H*-pyrazino[2,1-*c*]pyrido[2,3-*e*][1,4]oxazepine-9-carbonyl)azetidin-1-yl)methyl)pyridazin-3(2*H*)-one (42)**

(*R*)-azetidin-3-yl(3-(trifluoromethyl)-7*a*,8,10,11-tetrahydro-5*H*-pyrazino[2,1-*c*]pyrido[2,3-*e*][1,4]oxazepin-9(7*H*)-yl)methanone (42-b, 100 mg, 0.382 mmol) was dissolved in dichloromethane (2 mL), and 6-(bromomethyl)-4-(trifluoromethyl)pyridazin-3(2*H*)-one (I-6-c, 75.3 mg, 0.382 mmol) was added. After reacting at room temperature for 3 hours, the mixture was purified by normal phase column chromatography (dichloromethane:methanol = 10:1), and then purified by preparative HPLC [Pack Column 20ID*250mm* 10μm; A:$H_2O$(0.1%FA), B:ACN, B%: 10%-100% over 22 min] to obtain the target product (*R*)-4-(trifluoromethyl)-6-((3-(3-(trifluoromethyl)-7,7*a*,8,9,10,11-hexahydro-5*H*-

pyrazino[2,1-*c*]pyrido[2,3-*e*][1,4]oxazepine-9-carbonyl)azetidin-1-yl)methyl)pyridazin-3(2*H*)-one (42, 3.16 mg, yield 1.58%). ESI [M+H]$^+$=533.2 $^1$H NMR (400 MHz, DMSO-$d_6$) δ 13.99 (s, 1H), 8.43 (d, J = 13.2 Hz, 1H), 7.99 (s, 1H), 7.82 (d, J = 23.0 Hz, 1H), 4.88 (dd, J = 23.3, 12.2 Hz, 2H), 4.57 (s, 1H), 4.55 (d, J = 14.5 Hz, 1H), 4.45 (dd, J = 29.0, 9.0 Hz, 2H), 4.34 (dd, J = 32.1, 10.3 Hz, 2H), 4.24 (dd, J = 24.9, 16.6 Hz, 2H), 4.05 (dd, J = 25.9, 21.6 Hz, 2H), 3.97 - 3.74 (m, 4H), 3.38 - 3.21 (m, 2H).

**Embodiment 43: Preparation of 4-(trifluoromethyl)-6-(((((1*S*,3*R*)-3-((*S*)-3-(trifluoromethyl)-7,7*a*,8,9,10,11-hexahydro-5*H*-pyrazino[2,1-*c*]pyrido[2,3-*e*][1,4]oxazepine-9-carbonyl)cyclobutyl)amino)methyl)pyridazin-3(2*H*)-one (43)**

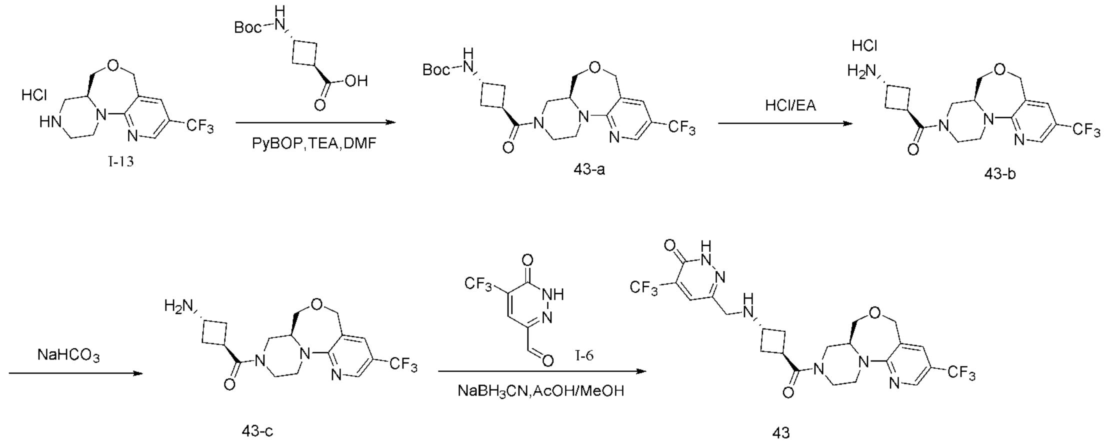


I-13 43-a 43-b 43-c I-6 43

**Step 1: Preparation of tert-butyl ((1*S*,3*R*)-3-((*S*)-3-(trifluoromethyl)-7,7*a*,8,9,10,11-hexahydro-5*H*-pyrazino[2,1-*c*]pyrido[2,3-e][1,4]oxathiepine-9-carbonyl)cyclobutyl)carbamate (43-a)**

(1*R*,3*R*)-3-((tert-butoxycarbonyl)amino)cyclobutane-1-carboxylic acid (46 mg, 0.213 mmol), (S)-3-(trifluoromethyl)-7,7*a*,8,9,10,11-hexahydro-5*H*-pyrazino[2,1-*c*]pyrido[2,3-*e*][1,4]oxazepine hydrochloride (I-13, 55 mg, 0.178 mmol), and (benzotriazol-1-yloxy)tripyrrolidinophosphonium hexafluorophosphate (111 mg, 0.213 mmol) were dissolved in *N,N*-dimethylformamide (4 mL), and then triethylamine (74 μL, 0.533 mmol) was added. The reaction was stirred at room temperature for 3 hours. Water was added to the reaction mixture, which was then extracted with ethyl acetate. The combined organic phases were washed with saturated brine, and the organic phase was dried over anhydrous sodium sulfate and filtered. The filtrate was concentrated under reduced pressure and purified by Flash silica gel column chromatography (ethyl acetate: petroleum ether=0~51%) to obtain the target product tert-butyl ((1*S*,3*R*)-3-((*S*)-3-(trifluoromethyl)-7,7*a*,8,9,10,11-hexahydro-5*H*-pyrazino[2,1-*c*]pyrido[2,3-*e*][1,4]oxathiepine-9-carbonyl)cyclobutyl)carbamate (43-a, 72 mg, yield 86.18%). ESI [M+H]$^+$=471.4

**Step 2: Preparation of ((1*R*,3*S*)-3-aminocyclobutyl)((*S*)-3-(trifluoromethyl)-7*a*,8,10,11-tetrahydro-5*H*-pyrazino[2,1-*c*]pyrido[2,3-*e*][1,4]oxazepin-9(7*H*)-yl)methanone hydrochloride (43-b)**

tert-butyl ((1*S*,3*R*)-3-((*S*)-3-(trifluoromethyl)-7,7*a*,8,9,10,11-hexahydro-5*H*-pyrazino[2,1-*c*]pyrido[2,3-*e*][1,4]oxathiepine-9-carbonyl)cyclobutyl)carbamate (43-a, 72 mg, 0.153 mmol) was dissolved in 4M hydrogen chloride in ethyl acetate (2 mL) and reacted at room temperature for 1 hour. After monitoring showed the reaction was complete, the reaction mixture was diluted with dichloromethane multiple times and then concentrated under reduced pressure to obtain the target product ((1*R*,3*S*)-3-aminocyclobutyl)((*S*)-3-(trifluoromethyl)-7*a*,8,10,11-tetrahydro-5*H*-pyrazino[2,1-c]pyrido[2,3-e][1,4]oxazepin-9(7*H*)-yl)methanone hydrochloride (43-b, 67 mg, yield 107.62%). ESI [M+H]$^+$=371.2

**Step 3: Preparation of ((1*R*,3*S*)-3-aminocyclobutyl)((*S*)-3-(trifluoromethyl)-7*a*,8,10,11-tetrahydro-5*H*-pyrazino[2,1-*c*]pyrido[2,3-*e*][1,4]oxazepin-9(7*H*)-yl)methanone (43-c)**

((1*R*,3*S*)-3-aminocyclobutyl)((*S*)-3-(trifluoromethyl)-7*a*,8,10,11-tetrahydro-5*H*-pyrazino[2,1-*c*]pyrido[2,3-*e*][1,4]oxazepin-9(7*H*)-yl)methanone hydrochloride (43-b, 67 mg, 0.165 mmol) was dissolved in water (2 mL), and then sodium bicarbonate (28 mg, 0.329 mmol) was added. The mixture was reacted at room temperature for 5 minutes. After

monitoring showed the pH was 8-9, the reaction mixture was concentrated under reduced pressure. The crude product was dissolved in a solution of dichloromethane:methanol = 10:1 by sonication and filtered. The filtrate was concentrated under reduced pressure to obtain the target product ((1*R*,3*S*)-3-aminocyclobutyl)((*S*)-3-(trifluoromethyl)-7*a*,8,10,11-tetrahydro-5*H*-pyrazino[2,1-*c*]pyrido[2,3-*e*][1,4]oxazepin-9(7*H*)-yl)methanone (43-c, 51 mg, yield 83.61%).

**Step 4: Preparation of 4-(trifluoromethyl)-6-(((((1*S*,3*R*)-3-((*S*)-3-(trifluoromethyl)-7,7*a*,8,9,10,11-hexahydro-5*H*-pyrazino[2,1-c]pyrido[2,3-*e*][1,4]oxazepine-9-carbonyl)cyclobutyl)amino)methyl)pyridazin-3(2*H*)-one (43)**

((1*R*,3*S*)-3-aminocyclobutyl)((*S*)-3-(trifluoromethyl)-7*a*,8,10,11-tetrahydro-5*H*-pyrazino[2,1-*c*]pyrido[2,3-*e*][1,4]oxazepin-9(7*H*)-yl)methanone (43-c, 51 mg, 0.137 mmol) and 6-oxo-5-(trifluoromethyl)-1,6-dihydropyridazine-3-carbaldehyde (I-6, 40 mg, 0.207 mmol) were dissolved in methanol (1.8 mL) and acetic acid (0.2 mL). The reaction was stirred at room temperature for 1.5 hours, then sodium cyanoborohydride (17 mg, 0.275 mmol) was added, and the reaction was stirred at room temperature overnight. After monitoring showed the reaction was complete, a large amount of water was added, and the mixture was extracted with dichloromethane:methanol = 10:1, dried over anhydrous sodium sulfate, and filtered. The filtrate was concentrated under reduced pressure and purified by preparative thin-layer chromatography silica gel plate (dichloromethane:methanol = 10:1), and then purified by preparative HPLC [Pack Column 30ID*250mm*10μm; A:$H_2O$(0.1%FA), B:ACN, B%: 5%-80% over 22 min] to obtain 4-(trifluoromethyl)-6-(((((1*S*,3*R*)-3-((*S*)-3-(trifluoromethyl)-7,7*a*,8,9,10,11-hexahydro-5*H*-pyrazino[2,1-*c*]pyrido[2,3-*e*][1,4]oxazepine-9-carbonyl)cyclobutyl)amino)methyl)pyridazin-3(2*H*)-one (43, 11.23 mg, yield 14.92%), ESI $[M+H]^+$=547.7 $^1$H NMR (400 MHz, DMSO-$d_6$) δ 8.40 (s, 1H), 7.94 (s, 1H), 7.79 (s, 1H), 4.88 (dd, J = 13.9, 10.1 Hz, 1H), 4.53 (d, J = 14.2 Hz, 1H), 4.00 (dt, J = 16.3, 9.9 Hz, 2H), 3.91 - 3.77 (m, 2H), 3.75 - 3.58 (m, 4H), 3.56 (s, 2H), 3.31 - 3.11 (m, 4H), 2.24 (d, J = 11.2 Hz, 2H), 1.90 (t, J = 12.2 Hz, 2H).

**Embodiment 44: Preparation of (*S*)-4-(trifluoromethyl)-6-((3-(3-(trifluoromethyl)-7,7*a*,8,9,10,11-hexahydro-5*H*-pyrazino[2,1-*c*]pyrido[2,3-*e*][1,4]oxazepine-9-carbonyl)azetidin-1-yl)methyl)pyridazin-3(2*H*)-one (44)**

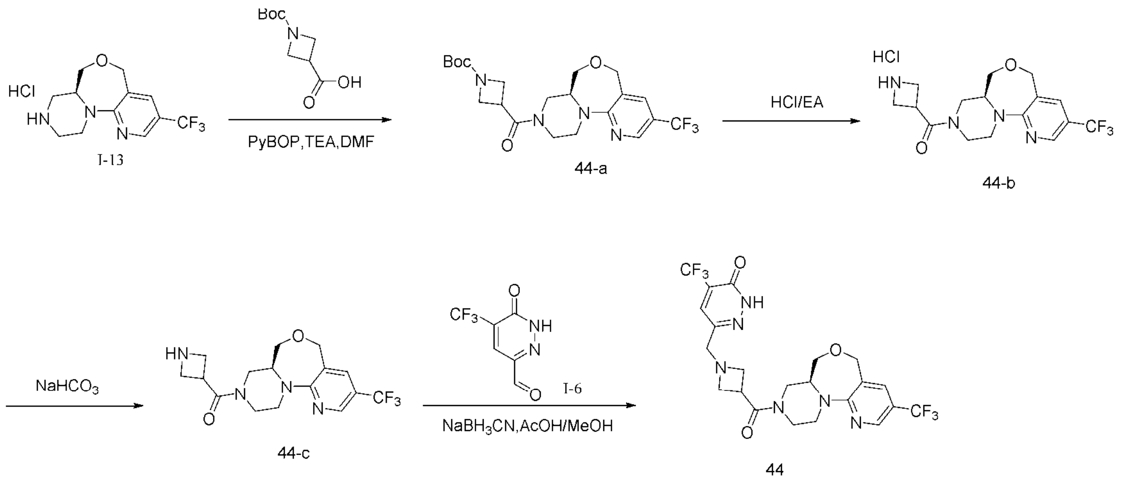


**Step 1: Preparation of tert-butyl (*S*)-3-(3-(trifluoromethyl)-7,7*a*,8,9,10,11-hexahydro-5*H*-pyrazino[2,1-*c*]pyrido[2,3-*e*][1,4]oxazepine-9-carbonyl)azetidine-1-carboxylate (44-a)**

1-(tert-butoxycarbonyl)azetidine-3-carboxylic acid (43 mg, 0.213 mmol), (*S*)-3-(trifluoromethyl)-7,7*a*,8,9,10,11-hexahydro-5*H*-pyrazino[2,1-*c*]pyrido[2,3-*e*][1,4]oxazepine hydrochloride (I-13, 55 mg, 0.178 mmol), and (benzotriazol-1-yloxy)tripyrrolidinophosphonium hexafluorophosphate (111 mg, 0.213 mmol) were dissolved in *N,N*-dimethylformamide (4 mL), and then triethylamine (74 μL, 0.533 mmol) was added. The reaction was stirred at room temperature for 3 hours. Water was added to the reaction mixture, which was then extracted with ethyl acetate. The combined organic phases were washed with saturated brine, and the organic phase was dried over anhydrous sodium sulfate and filtered. The filtrate was concentrated under reduced pressure and purified by Flash silica gel column chromatography (ethyl acetate:petroleum ether = 0~51%) to obtain the target product tert-butyl (*S*)-3-(3-(trifluoromethyl)-7,7*a*,8,9,10,11-hexahydro-5*H*-pyrazino[2,1-*c*]pyrido[2,3-*e*][1,4]oxazepine-9-carbonyl)azetidine-1-carboxylate (44-a, 65 mg, yield 80.19%). ESI $[M+H]^+$=457.2, ESI $[M+H-56]^+$=401.2

**Step 2: Preparation of (*S*)-azetidin-3-yl(3-(trifluoromethyl)-7*a*,8,10,11-tetrahydro-5*H*-pyrazino[2,1-*c*]pyrido[2,3-*e*][1,4]oxazepin-9(7*H*)-yl)methanone hydrochloride (44-b)**

tert-butyl (*S*)-3-(3-(trifluoromethyl)-7,7*a*,8,9,10,11-hexahydro-5*H*-pyrazino[2,1-*c*]pyrido[2,3-*e*][1,4]oxazepine-9-carbonyl)azetidine-1-carboxylate (44-a, 65 mg, 0.142 mmol) was dissolved in 4M hydrogen chloride in ethyl acetate (2 mL) and reacted at room temperature for 1 hour. After monitoring showed the reaction was complete, the reaction mixture was diluted with dichloromethane multiple times and then concentrated under reduced pressure to obtain the target product (*S*)-azetidin-3-yl(3-(trifluoromethyl)-7*a*,8,10,11-tetrahydro-5*H*-pyrazino[2,1-c]pyrido[2,3-*e*][1,4]oxazepin-9(7*H*)-yl)methanone hydrochloride (44-b, 60 mg, yield 107.27%). ESI $[M+H]^+$=357.2

**Step 3: Preparation of (*S*)-azetidin-3-yl(3-(trifluoromethyl)-7*a*,8,10,11-tetrahydro-5*H*-pyrazino[2,1-*c*]pyrido[2,3-*e*][1,4]oxazepin-9(7*H*)-yl)methanone (44-c)**

(*S*)-azetidin-3-yl(3-(trifluoromethyl)-7*a*,8,10,11-tetrahydro-5*H*-pyrazino[2,1-*c*]pyrido[2,3-*e*][1,4]oxazepin-9(7*H*)-yl)methanone hydrochloride (44-b, 60 mg, 0.152 mmol) was dissolved in water (2 mL), and then sodium bicarbonate (27 mg, 0.305 mmol) was added. The mixture was reacted at room temperature for 5 minutes. After monitoring showed the pH was 8-9, the reaction mixture was concentrated under reduced pressure. The crude product was dissolved in a solution of dichloromethane:methanol = 10:1 by sonication and filtered. The filtrate was concentrated under reduced pressure to obtain the target product (*S*)-azetidin-3-yl(3-(trifluoromethyl)-7*a*,8,10,11-tetrahydro-5*H*-pyrazino[2,1-*c*]pyrido [2,3-*e*][1,4]oxazepin-9(7*H*)-yl)methanone (44-c, 46 mg, yield 84.51%).

**Step 4: Preparation of (*S*)-4-(trifluoromethyl)-6-((3-(3-(trifluoromethyl)-7,7*a*,8,9,10,11-hexahydro-5*H*-pyrazino [2,1-*c*]pyrido[2,3-*e*][1,4]oxazepine-9-carbonyl)azetidin-1-yl)methyl)pyridazin-3(2*H*)-one (44)**

(*S*)-azetidin-3-yl(3-(trifluoromethyl)-7*a*,8,10,11-tetrahydro-5*H*-pyrazino[2,1-*c*]pyrido[2,3-*e*][1,4]oxazepin-9(7*H*)-yl)methanone (44-c, 46 mg, 0.129 mmol) and 6-oxo-5-(trifluoromethyl)-1,6-dihydropyridazine-3-carbaldehyde (I-6, 37 mg, 0.193 mmol) were dissolved in methanol (1.8 mL) and acetic acid (0.2 mL). The reaction was stirred at room temperature for 1.5 hours, then sodium cyanoborohydride (16 mg, 0.258 mmol) was added, and the reaction was stirred at room temperature overnight. After monitoring showed the reaction was complete, a large amount of water was added, and the mixture was extracted with dichloromethane:methanol = 10:1, dried over anhydrous sodium sulfate, and filtered. The filtrate was concentrated under reduced pressure and purified by preparative thin-layer chromatography silica gel plate (dichloromethane:methanol = 10:1), and then purified by preparative HPLC [Pack Column 30ID*250mm*10μm; A:$H_2O$(0.1%FA), B:ACN, B%: 5%-80% over 22 min] to obtain (*S*)-4-(trifluoromethyl)-6-((3-(3-(trifluoromethyl)-7,7*a*,8,9,10,11-hexahydro-5*H*-pyrazino[2,1-c]pyrido[2,3-e][1,4]oxazepine-9-carbonyl)azetidin-1-yl)methyl)pyridazin-3(2H)-one (44, 4.75 mg, yield 6.91%), ESI $[M+H]^+$=533.7 $^1$H NMR (400 MHz, DMSO-$d_6$) δ 8.41 (d, J = 6.2 Hz, 1H), 7.80 (s, 2H), 4.87 (t, J = 13.4 Hz, 1H), 4.53 (dd, J = 14.2, 4.9 Hz, 1H), 4.08 - 3.94 (m, 2H), 3.94 - 3.76 (m, 3H), 3.75 - 3.57 (m, 3H), 3.50 (s, 4H), 3.42 (dd, J = 13.7, 7.1 Hz, 1H), 3.31 - 3.17 (m, 3H).

**Embodiment 45: Preparation of 6-(((((1R,3R)-3-(4-(3-methoxy-5-(trifluoromethyl)pyridin-2-yl)piperazine-1-carbonyl)cyclobutyl)amino)methyl)-4-(trifluoromethyl)pyridazin-3(2*H*)-one (45)**

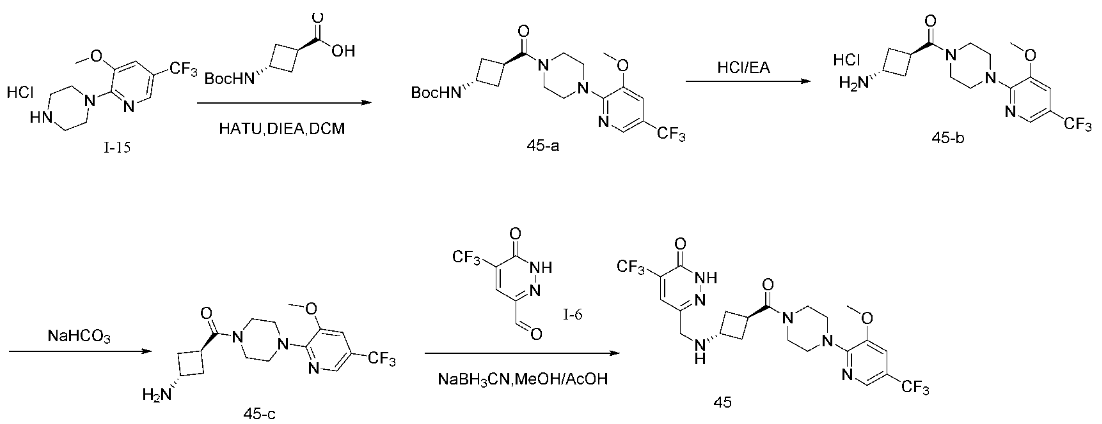

**Step 1: Preparation of tert-butyl (1*R*,3*R*)-3-(4-(3-methoxy-5-(trifluoromethyl)pyridin-2-yl)piperazine-1-carbonyl) cyclobutyl)carbamate (45-a)**

(1*R*,3*R*)-3-((tert-butoxycarbonyl)amino)cyclobutane-1-carboxylic acid (64 mg, 0.299 mmol) was dissolved in dichloromethane (5 mL), and *O*-(7-azabenzotriazol-1-yl)-*N,N,N',N'*-tetramethyluronium hexafluorophosphate (114 mg, 0.299 mmol) was added and stirred for 10 minutes. Then, *N,N*-diisopropylethylamine (157 μL, 0.897 mmol) and 1-(3-methoxy-5-(trifluoromethyl)pyridin-2-yl)piperazine hydrochloride (I-15, 89 mg, 0.299 mmol) were added. The reaction was stirred at room temperature for 2 hours. The reaction mixture was concentrated under reduced pressure and purified by Flash silica gel chromatography (ethyl acetate:petroleum ether = 0-49%) to obtain the target product tert-butyl ((1*R*,3*R*)-3-(4-(3-methoxy-5-(trifluoromethyl)pyridin-2-yl)piperazine-1-carbonyl)cyclobutyl)carbamate (45-a, 114 mg, yield 83.17%). ESI $[M+H]^+$=459.0

**Step 2: Preparation of ((1*R*,3*R*)-3-aminocyclobutyl)(4-(3-methoxy-5-(trifluoromethyl)pyridin-2-yl)piperazin-1-yl) methanone hydrochloride (45-b)**

tert-butyl ((1*R*,3*R*)-3-(4-(3-methoxy-5-(trifluoromethyl)pyridin-2-yl)piperazine-1-carbonyl)cyclobutyl)carbamate (45-a, 114 mg, 0.249 mmol) was dissolved in 4M hydrogen chloride in ethyl acetate (3 mL) and reacted at room temperature for 1 hour. After monitoring showed the reaction was complete, the reaction mixture was diluted with dichloromethane multiple times and then concentrated under reduced pressure to obtain the target product ((1*R*,3*R*)-3-aminocyclobutyl)(4-(3-methoxy-5-(trifluoromethyl)pyridin-2-yl)piperazin-1-yl)methanone hydrochloride (45-b, 118 mg, yield 120.20%).

**Step 3: Preparation of ((1*R*,3*R*)-3-aminocyclobutyl)(4-(3-methoxy-5-(trifluoromethyl)pyridin-2-yl)piperazin-1-yl) methanone (45-c)**

((1*R*,3*R*)-3-aminocyclobutyl)(4-(3-methoxy-5-(trifluoromethyl)pyridin-2-yl)piperazin-1-yl)methanone hydrochloride (45-b, 118 mg, 0.299 mmol) was dissolved in water (2 mL), and then sodium bicarbonate (51 mg, 0.598 mmol) was added. The mixture was reacted at room temperature for 5 minutes. After monitoring showed the pH was 8-9, the reaction mixture was concentrated under reduced pressure. The crude product was dissolved in a solution of dichloromethane:methanol = 10:1 by sonication and filtered. The filtrate was concentrated under reduced pressure to obtain the target product ((1*R*,3*R*)-3-aminocyclobutyl)(4-(3-methoxy-5-(trifluoromethyl)pyridin-2-yl)piperazin-1-yl)methanone (45-c, 106 mg, yield 98.97%).

**Step 4: Preparation of 6-(((((1R,3R)-3-(4-(3-methoxy-5-(trifluoromethyl)pyridin-2-yl)piperazine-1-carbonyl)cyclobutyl)amino)methyl)-4-(trifluoromethyl)pyridazin-3(2*H*)-one (45)**

((1*R*,3*R*)-3-aminocyclobutyl)(4-(3-methoxy-5-(trifluoromethyl)pyridin-2-yl)piperazin-1-yl)methanone (45-c, 106 mg, 0.296 mmol) and 6-oxo-5-(trifluoromethyl)-1,6-dihydropyridazine-3-carbaldehyde (I-6, 85 mg, 0.444 mmol) were dissolved in methanol (1.8 mL) and acetic acid (0.2 mL). The reaction was stirred at room temperature for 1.5 hours, then sodium cyanoborohydride (37 mg, 0.592 mmol) was added, and the reaction was stirred at room temperature overnight. After monitoring showed the reaction was complete, a large amount of water was added, and the mixture was extracted with dichloromethane:methanol = 10:1. The organic phase was dried over anhydrous sodium sulfate and filtered. The filtrate was concentrated under reduced pressure and purified by preparative HPLC [Pack Column 30ID*250mm*10μm; A:$H_2O$(0.1%FA), B:ACN, B%: 5%-80% over 22 min], and then purified twice by Flash chromatography (8g silica gel column, dichloromethane:methanol = 10:1) to obtain 6-(((((1*R*,3*R*)-3-(4-(3-methoxy-5-(trifluoromethyl)pyridin-2-yl)piperazine-1-carbonyl)cyclobutyl)amino)methyl)-4-(trifluoromethyl)pyridazin-3(2*H*)-one (45, 38.58 mg, yield 30.43%), ESI $[M+H]^+$=535.2 $^1$H NMR (400 MHz, DMSO-$d_6$) δ 8.11 (s, 1H), 7.94 (s, 1H), 7.44 (d, J = 1.6 Hz, 1H), 4.03 (dd, J = 14.2, 7.1 Hz, 1H), 3.88 (s, 3H), 3.56 (s, 4H), 3.45 (s, 4H), 3.40 (d, J = 5.1 Hz, 2H), 3.23 (ddd, J = 13.9, 9.4, 4.6 Hz, 1H), 3.14 (dt, J = 13.9, 6.9 Hz, 1H), 2.32 - 2.23 (m, 2H), 1.92 (dd, J = 18.5, 9.4 Hz, 2H).

**Embodiment 46: Preparation of 6-((3-(4-(3-methoxy-5-(trifluoromethyl)pyridin-2-yl)piperazine-1-carbonyl)azetidin-1-yl)methyl)-4-(trifluoromethyl)pyridazin-3(2*H*)-one (46)**

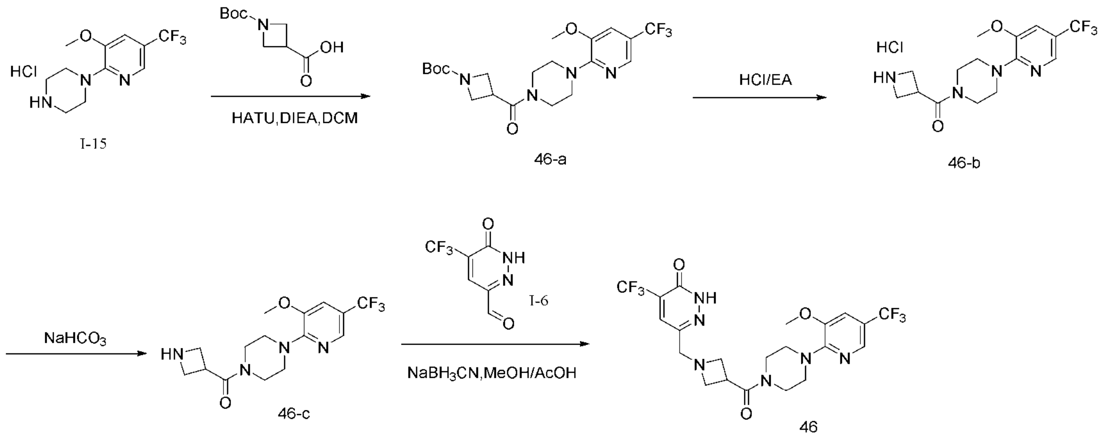


I-15 46-a 46-b I-6 46-c 46

**Step 1: Preparation of tert-butyl 3-(4-(3-methoxy-5-(trifluoromethyl)pyridin-2-yl)piperazine-1-carbonyl)azetidine-1-carboxylate (46-a)**

1-(tert-butoxycarbonyl)azetidine-3-carboxylic acid (60 mg, 0.299 mmol) was dissolved in dichloromethane (5 mL), and *O*-(7-azabenzotriazol-1-yl)-*N,N,N',N'*-tetramethyluronium hexafluorophosphate (114 mg, 0.299 mmol) was added and stirred for 10 minutes. Then, *N,N*-diisopropylethylamine (157 μL, 0.897 mmol) and 1-(3-methoxy-5-(trifluoromethyl)pyridin-2-yl)piperazine hydrochloride (I-15, 89 mg, 0.299 mmol) were added. The reaction was stirred at room temperature for 2 hours. The reaction mixture was concentrated under reduced pressure and purified by Flash silica gel chromatography (ethyl acetate:petroleum ether = 0-50%) to obtain the target product tert-butyl 3-(4-(3-methoxy-5-(trifluoromethyl)pyridin-2-yl)piperazine-1-carbonyl)azetidine-1-carboxylate (46-a, 119 mg, yield 89.56%). ESI $[M+H]^+$=445.0

**Step 2: Preparation of azetidin-3-yl(4-(3-methoxy-5-(trifluoromethyl)pyridin-2-yl)piperazin-1-yl)methanone hydrochloride (46-b)**

tert-butyl 3-(4-(3-methoxy-5-(trifluoromethyl)pyridin-2-yl)piperazine-1-carbonyl)azetidine-1-carboxylate (46-a, 119 mg, 0.268 mmol) was dissolved in 4M hydrogen chloride in ethyl acetate (3 mL) and reacted at room temperature for 1 hour. After monitoring showed the reaction was complete, the reaction mixture was diluted with dichloromethane multiple times and then concentrated under reduced pressure to obtain the target product azetidin-3-yl(4-(3-methoxy-5-(trifluoromethyl)pyridin-2-yl)piperazin-1-yl)methanone hydrochloride (46-b, 119 mg, yield 116.72%).

**Step 3: Preparation of azetidin-3-yl(4-(3-methoxy-5-(trifluoromethyl)pyridin-2-yl)piperazin-1-yl)methanone (46-c)**

azetidin-3-yl(4-(3-methoxy-5-(trifluoromethyl)pyridin-2-yl)piperazin-1-yl)methanone hydrochloride (46-b, 119 mg, 0.313 mmol) was dissolved in water (2 mL), and then sodium bicarbonate (53 mg, 0.626 mmol) was added. The mixture was reacted at room temperature for 5 minutes. After monitoring showed the pH was 8-9, the reaction mixture was concentrated under reduced pressure. The crude product was dissolved in a solution of dichloromethane:methanol = 10:1 by sonication and filtered. The filtrate was concentrated under reduced pressure to obtain the target product azetidin-3-yl(4-(3-methoxy-5-(trifluoromethyl)pyridin-2-yl)piperazin-1-yl)methanone (46-c, 103 mg, yield 95.72%).

**Step 4: Preparation of 6-((3-(4-(3-methoxy-5-(trifluoromethyl)pyridin-2-yl)piperazine-1-carbonyl)azetidin-1-yl)methyl)-4-(trifluoromethyl)pyridazin-3(2H)-one (46)**

azetidin-3-yl(4-(3-methoxy-5-(trifluoromethyl)pyridin-2-yl)piperazin-1-yl)methanone (46-c, 103 mg, 0.299 mmol) and 6-oxo-5-(trifluoromethyl)-1,6-dihydropyridazine-3-carbaldehyde (I-6, 86 mg, 0.449 mmol) were dissolved in methanol (1.8 mL) and acetic acid (0.2 mL). The reaction was stirred at room temperature for 1.5 hours, then sodium cyanoborohydride (38 mg, 0.598 mmol) was added, and the reaction was stirred at room temperature overnight. After monitoring showed the reaction was complete, a large amount of water was added, and the mixture was extracted with dichloromethane:methanol = 10:1. The organic phase was dried over anhydrous sodium sulfate and filtered. The filtrate was concentrated under reduced pressure and purified by preparative HPLC [Pack Column 30ID*250mm*10μm; A:$H_2O$(0.1%FA), B:ACN, B%: 5%-80% over 22 min], and then purified twice by Flash chromatography (8g silica gel column, dichloromethane:methanol = 10:1) to obtain 6-((3-(4-(3-methoxy-5-(trifluoromethyl)pyridin-2-yl)piperazine-1-carbonyl)azetidin-1-yl)methyl)-4-(trifluoromethyl)pyridazin-3(2*H*)-one (46, 9.83 mg, yield 6.31%), ESI $[M+H]^+$=521.3 $^1H$ NMR (400 MHz, DMSO-$d_6$) δ 13.54 (s, 1H), 8.11 (s, 1H), 7.79 (s, 1H), 7.44 (d, J = 1.7 Hz, 1H), 3.88 (s, 3H), 3.55 (d, J = 5.6 Hz, 2H), 3.50 (d, J = 9.1 Hz, 3H), 3.46 (d, J = 7.8 Hz, 5H), 3.37 (d, J = 3.2 Hz, 3H), 3.25 (t, J = 6.6 Hz, 2H).

**Embodiment 47: Preparation of 2-(4-((1*R*,3*R*)-3-(((6-oxo-5-(trifluoromethyl)-1,6-dihydropyridazin-3-yl)methyl) amino)cyclobutane-1-carbonyl)piperazin-1-yl)-5-(trifluoromethyl)nicotinonitrile (47)**

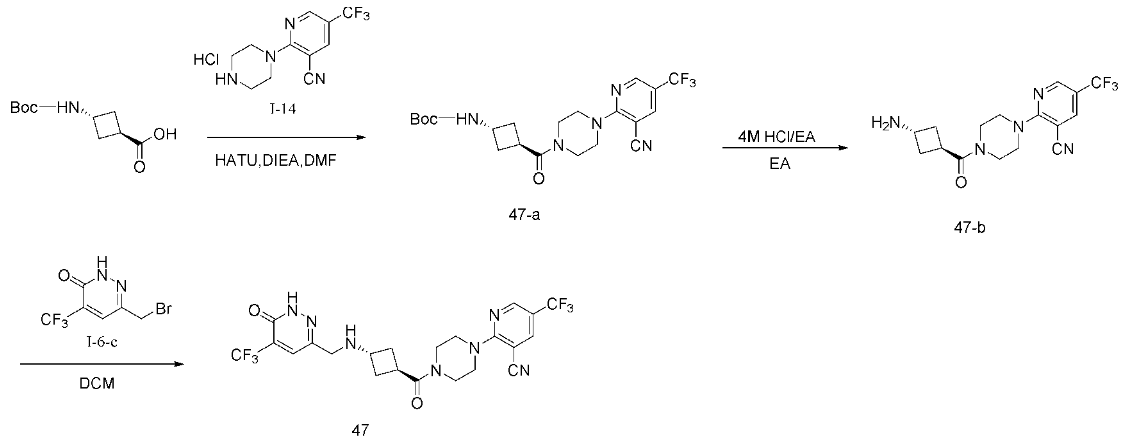


**Step 1: Preparation of tert-butyl ((1*R*,3*R*)-3-(4-(3-cyano-5-(trifluoromethyl)pyridin-2-yl)piperazine-1-carbonyl) cyclobutyl)carbamate (47-a)**

(1*R*,3*R*)-3-((tert-butoxycarbonyl)amino)cyclobutane-1-carboxylic acid (73.6 mg, 0.342 mmol) was dissolved in *N,N*-dimethylformamide (3 mL), and *O*-(7-azabenzotriazol-1-yl)-*N,N,N',N'*-tetramethyluronium hexafluorophosphate (130 mg, 0.342 mmol) and N,N-diisopropylethylamine (179 μL, 1.03 mmol) were added. After stirring under an ice bath for 20 minutes, 2-(piperazin-1-yl)-5-(trifluoromethyl)nicotinonitrile hydrochloride (I-14, 100 mg, 0.342 mmol) was added. After reacting at room temperature for 4 hours, water (20 mL) was added dropwise to the reaction mixture, and a white solid precipitated. The mixture was filtered, the filter cake was washed with water, and the collected filter cake was concentrated under reduced pressure to obtain the target product tert-butyl ((1*R*,3*R*)-3-(4-(3-cyano-5-(trifluoromethyl)pyridin-2-yl) piperazine-1-carbonyl)cyclobutyl)carbamate (47-a, 131 mg, yield 84.5%). ESI $[M-56+H]^+$=398.2

**Step 2: Preparation of 2-(4-((1*R*,3*R*)-3-aminocyclobutane-1-carbonyl)piperazin-1-yl)-5-(trifluoromethyl)nicotinonitrile (47-b)**

tert-butyl ((1*R*,3*R*)-3-(4-(3-cyano-5-(trifluoromethyl)pyridin-2-yl)piperazine-1-carbonyl)cyclobutyl)carbamate (47-a, 131 mg, 0.289 mmol) was dissolved in ethyl acetate (2 mL), and 4M hydrogen chloride in ethyl acetate solution (3 mL) was added. After reacting at room temperature for 3 hours, the mixture was concentrated under reduced pressure. Water (1 mL) was added, the pH was adjusted to 8 with $NaHCO_3$, and the mixture was extracted with dichloromethane:methanol = 8:1. The organic phase was dried over anhydrous sodium sulfate and filtered, and the filtrate was concentrated under reduced pressure to obtain the target product 2-(4-((1R,3R)-3-aminocyclobutane-1-carbonyl)piperazin-1-yl)-5-(trifluoromethyl)nicotinonitrile (47-b, 118 mg, crude product). ESI $[M+H]^+$=354.3

**Step 3: Preparation of 2-(4-((1*R*,3*R*)-3-(((6-oxo-5-(trifluoromethyl)-1,6-dihydropyridazin-3-yl)methyl)amino)cyclobutane-1-carbonyl)piperazin-1-yl)-5-(trifluoromethyl)nicotinonitrile (47)**

2-(4-((1*R*,3*R*)-3-aminocyclobutane-1-carbonyl)piperazin-1-yl)-5-(trifluoromethyl)nicotinonitrile (47-b, 118 mg, 0.303 mmol) was dissolved in dichloromethane (2 mL), and 6-(bromomethyl)-4-(trifluoromethyl)pyridazin-3(2*H*)-one (I-6-c, 17.5 mg, 0.098 mmol) was added. After reacting at room temperature for 3 hours, the mixture was purified by preparative thin-layer chromatography (dichloromethane:methanol = 10:1), and then purified by preparative HPLC [Pack Column 30ID*250mm*10μm; A:$H_2O$(0.1%FA), B:ACN, B%: 10%-100% over 25 min] to obtain the target product 2-(4-((1R,3R)-3-(((6-oxo-5-(trifluoromethyl)-1,6-dihydropyridazin-3-yl)methyl)amino)cyclobutane-1-carbonyl)piperazin-1-yl)-5-(trifluoromethyl)nicotinonitrile (47, 1.51 mg, yield 3.17%). ESI $[M+H]^+$=530.0 $^1H$ NMR (400 MHz,DMSO-$d_6$) δ 13.40 (s, 1H), 8.69 (s, 1H), 8.53 (s, 1H), 7.94 (s, 1H), 3.81 (t, J = 9.6 Hz, 4H), 3.64 - 3.59 (m, 2H), 3.57 (s, 2H), 3.49 (s, 2H),

3.28 - 3.21 (m, 1H), 3.14 (dd, J = 8.5, 5.4 Hz, 1H), 2.36 - 2.24 (m, 2H), 1.99 - 1.88 (m, 2H).

**Embodiment 48: Preparation of 4-(trifluoromethyl)-6-(2-((((1*R*,3*R*)-3-(4-(5-(trifluoromethyl)pyrimidin-2-yl)piperazine-1-carbonyl)cyclobutyl)amino)propan-2-yl)pyridazin-3(2*H*)-one (48)**

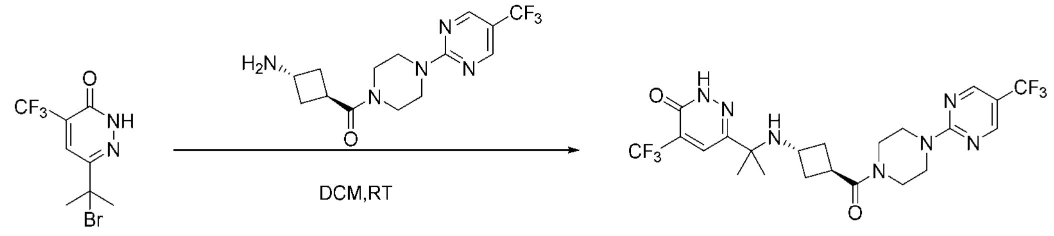


I-17

48

6-(2-bromopropan-2-yl)-4-(trifluoromethyl)pyridazin-3(2*H*)-one (I-17, 40 mg-50%, 0.14 mmol) and ((1*R*,3*R*)-3-aminocyclobutyl)(4-(5-(trifluoromethyl)pyrimidin-2-yl)piperazin-1-yl)methanone (23 mg, 0.14 mmol) were dissolved in dichloromethane (3 mL) and reacted at room temperature for 4 hours. After the reaction was complete, the filtrate was concentrated under reduced pressure and purified by preparative TLC plate (dichloromethane:methanol = 10:1) to obtain the target product 4-(trifluoromethyl)-6-(2-((((1*R*,3*R*)-3-(4-(5-(trifluoromethyl)pyrimidin-2-yl)piperazine-1-carbonyl)cyclobutyl)amino)propan-2-yl)pyridazin-3(2H)-one (48, 14.23 mg, yield 18.92%). ESI $[M+H]^+$=534.80 $^1H$ NMR (600 MHz, DMSO-$d_6$) δ 13.87 (s, 1H), 9.14 (s, 2H), 8.54 (s, 1H), 4.22 (d, J = 3.0 Hz, 4H), 3.98 - 3.93 (m, 2H), 3.43 (ddt, J = 20.7, 13.8, 8.2 Hz, 3H), 2.93 (dt, J = 3.4, 1.7 Hz, 2H), 2.63 - 2.58 (m, 2H), 2.37 - 2.30 (m, 2H), 1.70 (s, 6H).

**Embodiment 49: Preparation of 4-(trifluoromethyl)-6-(2-(3-(4-(5-(trifluoromethyl)pyrimidin-2-yl)piperazine-1-carbonyl)azetidin-1-yl)propan-2-yl)pyridazin-3(2*H*)-one (49)**

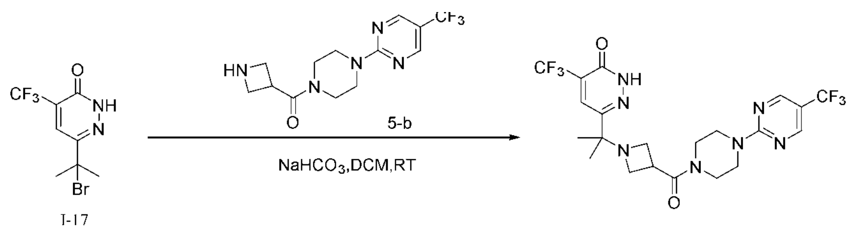


I-17

49

6-(2-bromopropan-2-yl)-4-(trifluoromethyl)pyridazin-3(2*H*)-one (I-17, 30 mg, purity 50%, 0.11 mmol) and azetidin-3-yl(4-(5-(trifluoromethyl)pyrimidin-2-yl)piperazin-1-yl)methanone (5-b, 19 mg, 0.11 mmol) were dissolved in dichloromethane (3 mL). After reacting at room temperature overnight with no obvious product, the pH was adjusted to weakly alkaline with aqueous sodium bicarbonate solution and the reaction was continued overnight. After the reaction was complete, the filtrate was concentrated under reduced pressure, purified once by preparative TLC plate (dichloromethane:methanol = 10:1), and then purified a second time by preparative HPLC [Pack Column 30ID*250mm*15um; A:$H_2O$(0.1%FA), B:CAN, B%: 10%-95% over 25 min] to obtain the target product 4-(trifluoromethyl)-6-(2-(3-(4-(5-(trifluoromethyl)pyrimidin-2-yl)piperazine-1-carbonyl)azetidin-1-yl)propan-2-yl)pyridazin-3(2*H*)-one (49, 5.11 mg, yield 9.4%). ESI $[M+H]^+$=520.1 $^1H$ NMR (400 MHz, DMSO-$d_6$) δ 13.51 (s, 1H), 8.72 (s, 2H), 7.95 (s, 1H), 3.80 (s, 5H), 3.50 (d, J = 31.5 Hz, 8H), 1.22 (s, 6H).

**Embodiment 50: Preparation of 2-(4-((1*R*,3*R*)-3-(((6-oxo-5-(trifluoromethyl)-1,6-dihydropyridazin-3-yl)methyl)amino)cyclobutane-1-carbonyl)piperazin-1-yl)-5-(trifluoromethyl)nicotinonitrile (50)**

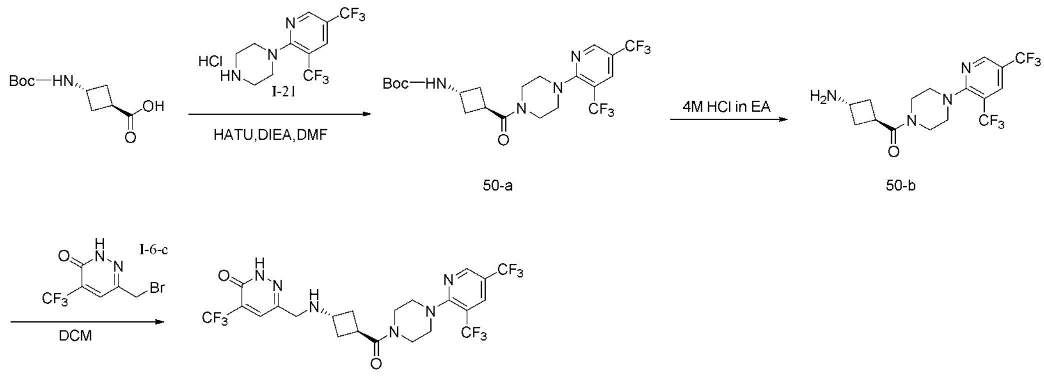


50-a

50-b

50

**Step 1: Preparation of tert-butyl (1*R*,3*R*)-3-(4-(3,5-bis(trifluoromethyl)pyridin-2-yl)piperazine-1-carbonyl)cyclobutyl)carbamate (50-a)**

(1*R*,3*R*)-3-((tert-butoxycarbonyl)amino)cyclobutane-1-carboxylic acid (96.2 mg, 0.447 mmol) was dissolved in *N,N*-dimethylformamide (5 mL), and *O*-(7-azabenzotriazol-1-yl)-*N,N,N',N'*-tetramethyluronium hexafluorophosphate (170 mg, 0.447 mmol) and *N,N*-diisopropylethylamine (235 μL, 1.34 mmol) were added. After stirring under an ice bath for 20 minutes, 1-(3,5-bis(trifluoromethyl)pyridin-2-yl)piperazine hydrochloride (I-21, 150 mg, 0.447 mmol) was added. After reacting at room temperature for 4 hours, water (20 mL) was added dropwise to the reaction mixture, and a white solid precipitated. The mixture was filtered, the filter cake was washed with water, and the collected filter cake was concentrated under reduced pressure to obtain the target product tert-butyl (1*R*,3*R*)-3-(4-(3,5-bis(trifluoromethyl)pyridin-2-yl)piperazine-1-carbonyl)cyclobutyl)carbamate (50-a, 80 mg, yield 36.2%). ESI $[M-56+H]^+$=441.2

**Step 2: Preparation of ((1*R*,3*R*)-3-aminocyclobutyl)(4-(3,5-bis(trifluoromethyl)pyridin-2-yl)piperazin-1-yl) methanone (50-b)**

tert-butyl (1*R*,3*R*)-3-(4-(3,5-bis(trifluoromethyl)pyridin-2-yl)piperazine-1-carbonyl)cyclobutyl)carbamate (50-a, 80 mg, 0.161 mmol) was dissolved in ethyl acetate (2 mL), and 4M hydrogen chloride in ethyl acetate solution (3 mL) was added. After reacting at room temperature for 3 hours, the mixture was concentrated under reduced pressure. Water (1 mL) was added, the pH was adjusted to 8 with $NaHCO_3$, and the mixture was extracted with dichloromethane:methanol = 8:1. The organic phase was dried over anhydrous sodium sulfate and filtered, and the filtrate was concentrated under reduced pressure to obtain the target product ((1*R*,3*R*)-3-aminocyclobutyl)(4-(3,5-bis(trifluoromethyl)pyridin-2-yl)piperazin-1-yl)methanone (50-b, 78 mg, crude product). ESI $[M+H]^+$=397.2

**Step 3: Preparation of 2-(4-((1*R*,3*R*)-3-(((6-oxo-5-(trifluoromethyl)-1,6-dihydropyridazin-3-yl)methyl)amino)cyclobutane-1-carbonyl)piperazin-1-yl)-5-(trifluoromethyl)nicotinonitrile (50)**

((1*R*,3*R*)-3-aminocyclobutyl)(4-(3,5-bis(trifluoromethyl)pyridin-2-yl)piperazin-1-yl)methanone (50-b, 78 mg, 0.188 mmol) was dissolved in dichloromethane (2 mL), and 6-(bromomethyl)-4-(trifluoromethyl)pyridazin-3(2*H*)-one (I-6-c, 54 mg, 0.188 mmol) was added. After reacting at room temperature for 3 hours, the mixture was purified by preparative thin-layer chromatography (dichloromethane:methanol = 10:1), and then purified by preparative HPLC [Pack Column 30ID*250mm*10μm; A:$H_2O$(0.1 %FA), B:ACN, B%: 10%-100% over 25 min] to obtain the target product 2-(4-((1*R*,3*R*)-3-(((6-oxo-5-(trifluoromethyl)-1,6-dihydropyridazin-3-yl)methyl)amino)cyclobutane-1-carbonyl)piperazin-1-yl)-5-(trifluoromethyl)nicotinonitrile (50, 7.26 mg, yield 6.75%). ESI $[M+H]^+$=573.0 $^1H$ NMR (400 MHz, DMSO-$d_6$) δ 13.99 (s, 1H), 8.84 - 8.76 (m, 1H), 8.37 (d, J = 1.4 Hz, 1H), 8.01 (s, 1H), 4.12 (s, 2H), 3.90 - 3.56 (m, 4H), 3.55 - 3.39 (m, 6H), 2.48 - 2.31 (m, 4H).

**Embodiment 51: Preparation of 5-cyclobutoxy-6-((3-(4-(5-(trifluoromethyl)pyrimidin-2-yl)piperazine-1-carbonyl)azetidin-1-yl)methyl)pyridazin-3(2*H*)-one (51)**

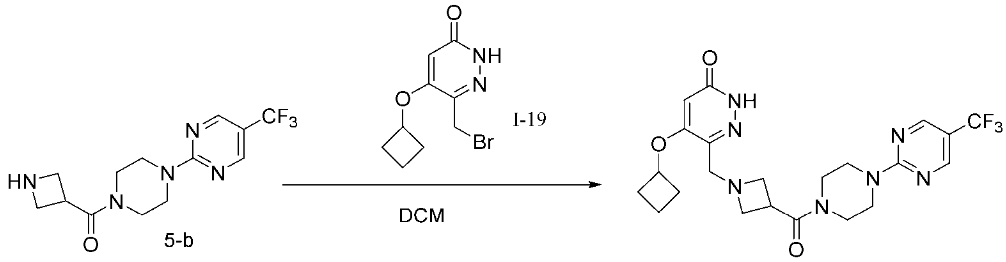


51

azetidin-3-yl(4-(5-(trifluoromethyl)pyrimidin-2-yl)piperazin-1-yl)methanone (5-b, 75.7 mg, 0.240 mmol) was dissolved in dichloromethane (2 mL), and 6-(bromomethyl)-5-cyclobutoxypyridazin-3(2*H*)-one (I-19, 31 mg, 0.120 mmol) was added. After reacting at room temperature for 1 hour, the mixture was purified by preparative thin-layer chromatography (dichloromethane:methanol = 10:1), and then purified by preparative HPLC [Pack Column 30ID*250mm*10μm; A:$H_2O$(0.1%FA), B:ACN, B%: 10%-100% over 30 min] to obtain the target product 5-cyclobutoxy-6-((3-(4-(5-(trifluoromethyl)pyrimidin-2-yl)piperazine-1-carbonyl)azetidin-1-yl)methyl)pyridazin-3(2H)-one (51, 1.11 mg, yield 6.75%). ESI $[M+H]^+$=494.2 $^1H$ NMR (600 MHz, DMSO-$d_6$) δ 12.48 (s, 1H), 8.72 (s, 2H), 5.94 (s, 1H), 4.78 - 4.68 (m, 2H), 3.79 (d, J = 17.0 Hz, 4H), 3.61 - 3.46 (m, 6H), 2.05 (dd, J = 28.2, 15.2 Hz, 4H), 1.83 - 1.75 (m, 2H), 1.64 (d, J = 22.7 Hz, 2H).

**Embodiment 52: Preparation of 5-cyclobutoxy-6-(((((1*R*,3*R*)-3-(4-(5-(trifluoromethyl)pyrimidin-2-yl)piperazine-1-carbonyl)cyclobutyl)amino)methyl)pyridazin-3(2*H*)-one (52)**

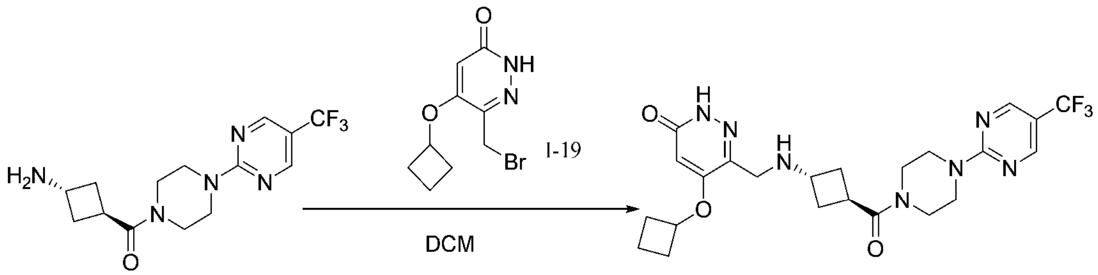


52

((1*R*,3*R*)-3-aminocyclobutyl)(4-(5-(trifluoromethyl)pyrimidin-2-yl)piperazin-1-yl)methanone (79.1 mg, 0.240 mmol) was dissolved in dichloromethane (2 mL), and 6-(bromomethyl)-5-cyclobutoxypyridazin-3(2*H*)-one (I-19, 31 mg, 0.120 mmol) was added. After reacting at room temperature for 1 hour, the mixture was purified by preparative thin-layer chromatography (dichloromethane:methanol = 10:1), and then purified by preparative HPLC [Pack Column 30ID*250mm*10μm; A:$H_2O$(0.1%FA), B:ACN, B%: 10%-100% over 30 min] to obtain the target product 5-cyclobutoxy-6-(((((1*R*,3*R*)-3-(4-(5-(trifluoromethyl)pyrimidin-2-yl)piperazine-1-carbonyl)cyclobutyl)amino)methyl)pyridazin-3(2H)-one (52, 2.29 mg, yield 3.76%). ESI $[M+H]^+$=508.2 $^1H$ NMR (600 MHz, DMSO-$d_6$) δ 12.91 (s, 1H), 8.72 (s, 2H), 6.09 (s, 1H), 4.80 (dd, J = 14.0, 7.0 Hz, 1H), 3.98 (s, 2H), 3.84 (s, 4H), 3.79 - 3.72 (m, 1H), 3.62 - 3.54 (m, 2H), 3.49 - 3.40 (m, 4H), 2.46 (dd, J = 14.9, 8.2 Hz, 5H), 2.12 (dd, J = 18.8, 9.0 Hz, 2H), 1.82 (dd, J = 20.5, 10.1 Hz, 1H), 1.65 (dd, J = 18.9, 9.6 Hz, 1H).

**Embodiment 53: Preparation of 6-(((((1*R*,3*R*)-3-(4-(3-methyl-5-(trifluoromethyl)pyridin-2-yl)piperazine-1-carbonyl)cyclobutyl)amino)methyl)-4-(trifluoromethyl)pyridazin-3(2*H*)-one (53)**

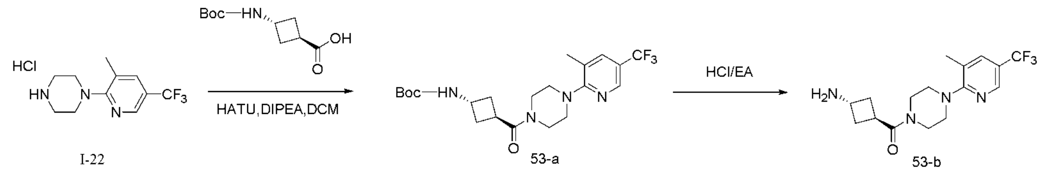


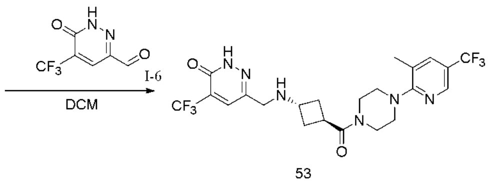


**Step 1: Preparation of tert-butyl (1*R*,3*R*)-3-(4-(3-methyl-5-(trifluoromethyl)pyridin-2-yl)piperazine-1-carbonyl) cyclobutyl)carbamate (53-a)**

(1*R*,3*R*)-3-((tert-butoxycarbonyl)amino)cyclobutane-1-carboxylic acid (245 mg, 1.138 mmol) and *O*-(7-azabenzotriazol-1-yl)-*N,N,N',N'*-tetramethyluronium hexafluorophosphate (433 mg, 1.138 mmol) were dissolved in dichloromethane (10 mL) and stirred for 10 minutes. Then, *N,N*-diisopropylethylamine (595 μL, 3.41 mmol) was added, and 1-(3-methyl-5-(trifluoromethyl)pyridin-2-yl)piperazine (I-22, 335 mg, 1.36 mmol) dissolved in dichloromethane (5 mL) was added to the reaction. The reaction was stirred at room temperature overnight. After the reaction was complete, the reaction mixture was concentrated under reduced pressure and purified by Flash column chromatography (petroleum ether:ethyl acetate = 1:1) to obtain the target product tert-butyl (1*R*,3*R*)-3-(4-(3-methyl-5-(trifluoromethyl)pyridin-2-yl) piperazine-1-carbonyl)cyclobutyl)carbamate (53-a, 330 mg, yield 54%). ESI $[M+H]^+$=443.4

**Step 2: Preparation of ((1*R*,3*R*)-3-aminocyclobutyl)(4-(3-methyl-5-(trifluoromethyl)pyridin-2-yl)piperazin-1-yl) methanone (53-b)**

tert-butyl (1*R*,3*R*)-3-(4-(3-methyl-5-(trifluoromethyl)pyridin-2-yl)piperazine-1-carbonyl)cyclobutyl)carbamate (53-a, 300 mg) was dissolved in ethyl acetate solution (4 mL), and hydrogen chloride in ethyl acetate solution (4 mL) was added. The mixture was reacted at room temperature overnight. After the reaction was complete, the reaction mixture was concentrated under reduced pressure, then dissolved in dichloromethane and concentrated under reduced pressure again, repeating twice. Aqueous sodium bicarbonate solution was slowly added until pH=7-9. After filtration, the solution was partially concentrated under reduced pressure to obtain the target product ((1*R*,3*R*)-3-aminocyclobutyl)(4-(3-methyl-5-(trifluoromethyl)pyridin-2-yl)piperazin-1-yl)methanone (53-b, 300 mg, crude product). ESI $[M+H]^+$=343.4

**Step 3: Preparation of 6-(((((1*R*,3*R*)-3-(4-(3-methyl-5-(trifluoromethyl)pyridin-2-yl)piperazine-1-carbonyl)cyclobutyl)amino)methyl)-4-(trifluoromethyl)pyridazin-3(2*H*)-one (53)**

((1*R*,3*R*)-3-aminocyclobutyl)(4-(3-methyl-5-(trifluoromethyl)pyridin-2-yl)piperazin-1-yl)methanone (53-b, 150 mg, 0.44 mmol) and 6-oxo-5-(trifluoromethyl)-1,6-dihydropyridazine-3-carbaldehyde (I-6, 143 mg, 0.48 mmol) were dissolved in dichloromethane (3 mL) and reacted at room temperature for 3 hours. After the reaction was complete, the filtrate was concentrated under reduced pressure and purified by preparative TLC silica gel plate (dichloromethane:methanol = 10:1), and then purified twice by preparative HPLC [Pack Column 30ID*250mm*15μm; A:$H_2O$(0.1% FA), B:ACN, B%: 10%-95% over 25 min] to obtain the target product 6-(((((1*R*,3*R*)-3-(4-(3-methyl-5-(trifluoromethyl) pyridin-2-yl)piperazine-1-carbonyl)cyclobutyl)amino)methyl)-4-(trifluoromethyl)pyridazin-3(2H)-one (53, 2.88 mg, yield 1.3%). ESI $[M+H]^+$=519.41 $^1$H NMR (600 MHz, DMSO-$d_6$) δ8.43 (s, 1H), 8.39 (s, 1H), 7.94 (s, 1H), 7.85 (s, 1H), 3.58 (d, J = 19.9 Hz, 4H), 3.41 (d, J = 19.8 Hz, 3H), 3.28 - 3.22 (m, 2H), 3.18 (d, J = 14.4 Hz, 4H), 2.31 (d, J = 6.9 Hz, 3H), 2.28 (s, 2H), 1.92 (dd, J = 17.0, 10.0 Hz, 2H).

**Embodiment 54: Preparation of 5-(trifluoromethyl)-6-((3-(4-(5-(trifluoromethyl)pyrimidin-2-yl)piperazine-1-carbonyl)azetidin-1-yl)methyl)pyridazin-3(2*H*)-one (54)**

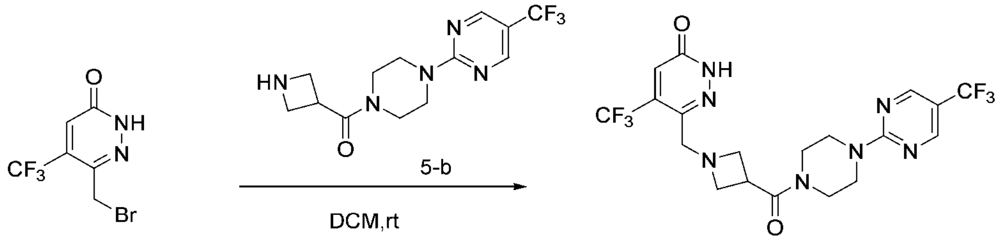


I-18

54

azetidin-3-yl(4-(5-(trifluoromethyl)pyrimidin-2-yl)piperazin-1-yl)methanone (5-b, 39 mg, 0.124 mmol) was dissolved in dichloromethane (2 mL), and 6-(bromomethyl)-5-(trifluoromethyl)pyridazin-3(2*H*)-one (I-18, 29 mg, 0.113 mmol) was added. The mixture was reacted at room temperature overnight. The reaction mixture was concentrated under reduced pressure and purified by preparative thin-layer chromatography (dichloromethane:methanol = 10:1), and then purified by preparative HPLC [Pack Column 30ID*250mm* 10μm; A:$H_2O$(0.1%FA), B:ACN, B%: 10%-60% over 30 min] to obtain the target product 5-(trifluoromethyl)-6-((3-(4-(5-(trifluoromethyl)pyrimidin-2-yl)piperazine-1-carbonyl)azetidin-1-yl)methyl)pyridazin-3(2*H*)-one (54, 0.8 mg, yield 1.44%). ESI $[M+H]^+$=492.5 $^1H$ NMR (600 MHz, DMSO-$d_6$)δ 8.72 (s, 2H), 7.33 (s, 1H), 3.80 (d, J = 4.2 Hz, 3H), 3.55 - 3.52 (m, 4H), 3.50 - 3.47 (m, 4H), 3.29 - 3.25 (m, 4H).

**Embodiment 55: Preparation of 5-(trifluoromethyl)-6-(((((1*R*,3*R*)-3-(4-(5-(trifluoromethyl)pyrimidin-2-yl)piperazine-1-carbonyl)cyclobutyl)amino)methyl)pyridazin-3(2*H*)-one (55)**

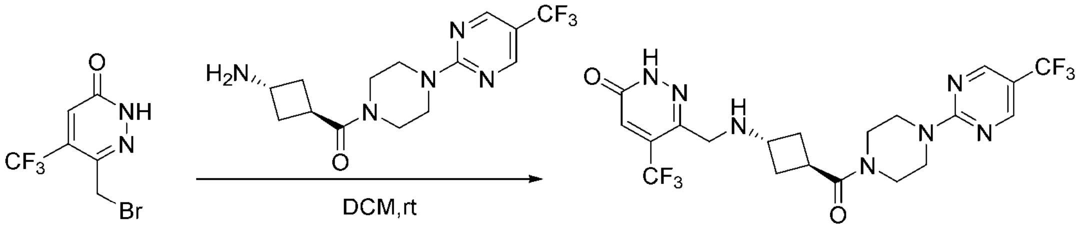


55

I-18

((1*R*,3*R*)-3-aminocyclobutyl)(4-(5-(trifluoromethyl)pyrimidin-2-yl)piperazin-1-yl)methanone (41 mg, 0.124 mmol) was dissolved in dichloromethane (2 mL), and 6-(bromomethyl)-5-(trifluoromethyl)pyridazin-3(2*H*)-one (I-18, 29 mg, 0.113 mmol) was added. The mixture was reacted at room temperature overnight. The reaction mixture was concentrated under reduced pressure and purified by preparative thin-layer chromatography (dichloromethane:methanol = 10:1), and then purified by preparative HPLC [Pack Column 30ID*250mm* 10μm; A:$H_2O$(0.1%FA), B:ACN, B%: 10%-60% over 30 min] to obtain the target product 5-(trifluoromethyl)-6-(((((1*R*,3*R*)-3-(4-(5-(trifluoromethyl)pyrimidin-2-yl)piperazine-1-carbonyl)cyclobutyl)amino)methyl)pyridazin-3(2H)-one (55, 1.34 mg, yield 2.35%). ESI $[M+H]^+$=506.5 $^1H$ NMR (600 MHz, DMSO-$d_6$)δ 8.72 (s, 2H), 7.33 (s, 1H), 3.81 (s, 4H), 3.63 (s, 2H), 3.55 (s, 2H), 3.47 - 3.33 (m, 3H), 3.28 - 3.18 (m, 2H), 2.30 (d, J = 4.3 Hz, 2H), 2.01 - 1.86 (m, 2H).

**Embodiment 56: Preparation of 6-(((((1*R*,3*R*)-3-(4-(3-cyclopropyl-5-(trifluoromethyl)pyridin-2-yl)piperazine-1-carbonyl)cyclobutyl)amino)methyl)-4-(trifluoromethyl)pyridazin-3(2*H*)-one (56)**

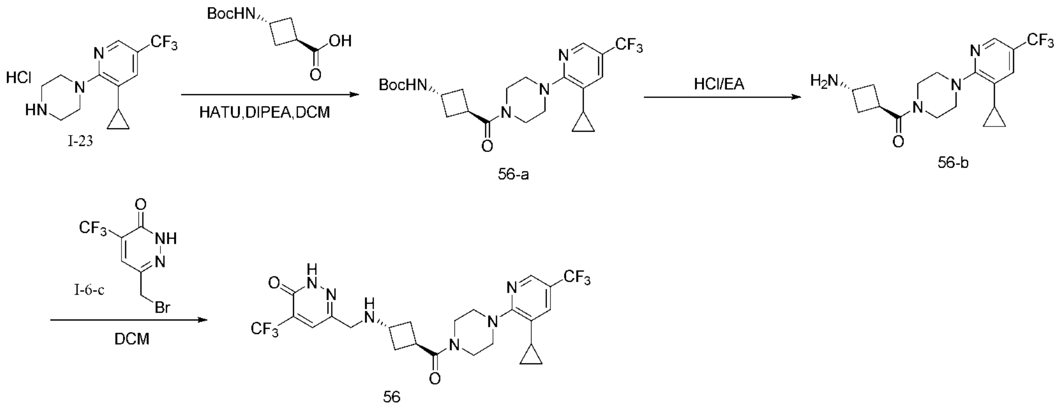


**Step 1: Preparation of tert-butyl ((1*R*,3*R*)-3-(4-(3-cyclopropyl-5-(trifluoromethyl)pyridin-2-yl)piperazine-1-carbonyl)cyclobutyl)carbamate (56-a)**

(1*R*,3*R*)-3-((tert-butoxycarbonyl)amino)cyclobutane-1-carboxylic acid (202 mg, 1.01 mmol) was dissolved in dichloromethane (14 mL), and *O*-(7-azabenzotriazol-1-yl)-*N,N,N',N'*-tetramethyluronium hexafluorophosphate (384 mg, 1.25 mmol) was added and stirred for 10 minutes. Then, *N,N*-diisopropylethylamine (528 μL, 3.03 mmol) and 1-(3-cyclopropyl-5-(trifluoromethyl)pyridin-2-yl)piperazine hydrochloride (I-23, 310 mg, 1.01 mmol) were added. The reaction was stirred at room temperature for 2 hours. The reaction mixture was concentrated under reduced pressure and purified by Flash chromatography (25g silica gel column, petroleum ether:ethyl acetate = 2:1) to obtain the target product tert-butyl ((1*R*,3*R*)-3-(4-(3-cyclopropyl-5-(trifluoromethyl)pyridin-2-yl)piperazine-1-carbonyl)cyclobutyl)carbamate (56-a, 185 mg, yield 79.02%). ESI $[M+H]^+$=469.8

**Step 2: Preparation of ((1*R*,3*R*)-3-aminocyclobutyl)(4-(3-cyclopropyl-5-(trifluoromethyl)pyridin-2-yl)piperazin-1-yl)methanone (56-b)**

tert-butyl ((1*R*,3*R*)-3-(4-(3-cyclopropyl-5-(trifluoromethyl)pyridin-2-yl)piperazine-1-carbonyl)cyclobutyl)carbamate (56-a, 185 mg, 0.39 mmol) was dissolved in 4M hydrogen chloride in ethyl acetate (3 mL) and reacted at room temperature for 2 hours, then concentrated under reduced pressure. Water (1 mL) was added, the pH was adjusted to 8 with $NaHCO_3$, and the mixture was extracted with (dichloromethane:methanol = 10:1). The organic phase was dried over anhydrous sodium sulfate and filtered, and the filtrate was concentrated under reduced pressure to obtain the target product ((1*R*,3*R*)-3-aminocyclobutyl)(4-(3-cyclopropyl-5-(trifluoromethyl)pyridin-2-yl)piperazin-1-yl)methanone (56-b, 135 mg, yield 92.75%). ESI $[M+H]^+$=369.4

**Step 3: Preparation of 6-(((((1R,3R)-3-(4-(3-cyclopropyl-5-(trifluoromethyl)pyridin-2-yl)piperazine-1-carbonyl)cyclobutyl)amino)methyl)-4-(trifluoromethyl)pyridazin-3(2*H*)-one (56)**

(1*R*,3*R*)-3-aminocyclobutyl)(4-(3-cyclopropyl-5-(trifluoromethyl)pyridin-2-yl)piperazin-1-yl)methanone (56-b, 80 mg, 0.22 mmol) was dissolved in dichloromethane (2 mL), and 6-(bromomethyl)-4-(trifluoromethyl)pyridazin-3(2H)-one (I-6-c, 67 mg, 0.26 mmol) was added. The reaction was stirred at room temperature for 1 hour and then purified by preparative thin-layer chromatography silica gel plate (dichloromethane:methanol = 10:1), and then purified by preparative HPLC [Pack Column 30ID*250mm* 10μm; A:$H_2O$(0.1%FA), B:ACN, B%: 5%-80% over 25 min] to obtain the target product 6-(((((1*R*,3*R*)-3-(4-(3-cyclopropyl-5-(trifluoromethyl)pyridin-2-yl)piperazine-1-carbonyl)cyclobutyl)amino)methyl)-4-(trifluoromethyl)pyridazin-3(2*H*)-one (56, 1.48 mg, yield 1.25%), ESI $[M+H]^+$=545.2 $^1H$ NMR (400 MHz, DMSO-$d_6$) δ 8.39 (s, 1H), 8.03 (s, 1H), 7.50 (s, 1H), 4.09 (s, 2H), 3.75 (s, 1H), 3.66 (s, 2H), 3.48 (s, 2H), 3.44 (s, 1H), 2.42 (d, J = 45.4 Hz, 4H), 2.02 - 1.96 (m, 4H), 1.07 (d, J = 7.6 Hz, 2H), 0.86 (d, J = 5.8 Hz, 3H).

**Embodiment 57: Preparation of 6-((3-(4-(4-methoxy-5-(trifluoromethyl)pyridin-2-yl)piperazine-1-carbonyl)azetidin-1-yl)methyl)-4-(trifluoromethyl)pyridazin-3(2*H*)-one (57)**

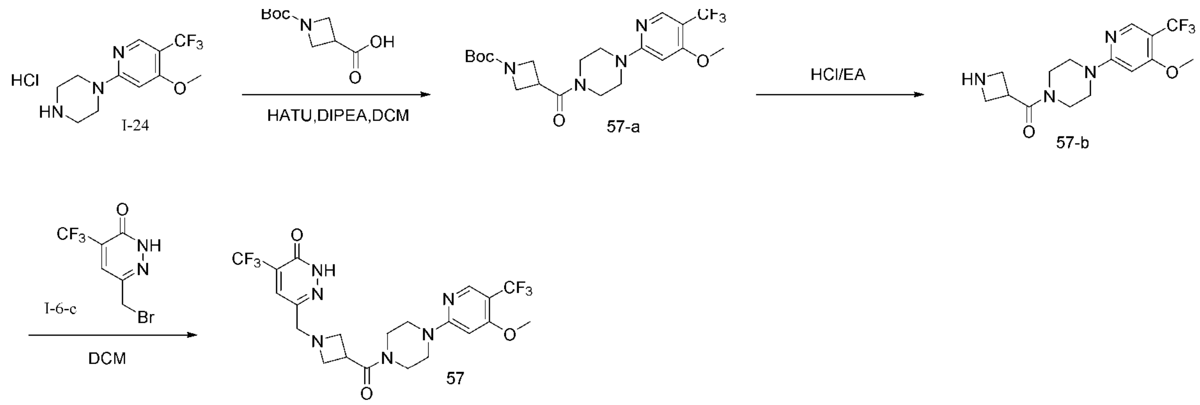


**Step 1: Preparation of tert-butyl 3-(4-(4-methoxy-5-(trifluoromethyl)pyridin-2-yl)piperazine-1-carbonyl)azetidine-1-carboxylate (57-a)**

1-(tert-butoxycarbonyl)azetidine-3-carboxylic acid (97 mg, 0.48 mmol) was dissolved in dichloromethane (8 mL), and *O*-(7-azabenzotriazol-1-yl)-*N,N,N',N'*-tetramethyluronium hexafluorophosphate (184 mg, 0.48 mmol) was added and stirred for 10 minutes. Then, N,N-diisopropylethylamine (254 μL, 1.45 mmol) and 1-(4-methoxy-5-(trifluoromethyl) pyridin-2-yl)piperazine hydrochloride (I-24, 310 mg, 1.01 mmol) were added, and the reaction was stirred at room temperature for 4 hours. The reaction mixture was concentrated under reduced pressure and purified by Flash chromatography (10g silica gel column, petroleum ether:ethyl acetate = 2:1) to obtain the target product tert-butyl 3-(4-(4-methoxy-5-(trifluoromethyl)pyridin-2-yl)piperazine-1-carbonyl)azetidine-1-carboxylate (57-a, 205 mg, yield 95.21%). ESI $[M+H]^+$=445.0

**Step 2: Preparation of azetidin-3-yl(4-(4-methoxy-5-(trifluoromethyl)pyridin-2-yl)piperazin-1-yl)methanone (57-b)**

tert-butyl 3-(4-(4-methoxy-5-(trifluoromethyl)pyridin-2-yl)piperazine-1-carbonyl)azetidine-1-carboxylate (57-a, 205 mg, 0.46 mmol) was dissolved in 4M hydrogen chloride in ethyl acetate (3 mL) and reacted at room temperature for 2 hours, then concentrated under reduced pressure. Water (1 mL) was added, the pH was adjusted to 8 with $NaHCO_3$, and the mixture was extracted with (dichloromethane:methanol = 10:1). The organic phase was dried over anhydrous sodium sulfate and filtered, and the filtrate was concentrated under reduced pressure to obtain the target product azetidin-3-yl(4-(4-methoxy-5-(trifluoromethyl)pyridin-2-yl)piperazin-1-yl)methanone (57-b, 150 mg, yield 94.34%). ESI $[M+H]^+$=345.2

**Step 3: Preparation of 6-((3-(4-(4-methoxy-5-(trifluoromethyl)pyridin-2-yl)piperazine-1-carbonyl)azetidin-1-yl) methyl)-4-(trifluoromethyl)pyridazin-3(2*H*)-one (57)**

azetidin-3-yl(4-(4-methoxy-5-(trifluoromethyl)pyridin-2-yl)piperazin-1-yl)methanone (57-b, 78 mg, 0.21 mmol) was dissolved in dichloromethane (2 mL), and 6-(bromomethyl)-4-(trifluoromethyl)pyridazin-3(2H)-one (I-6-c, 63 mg, 0.25 mmol) was added. The reaction was stirred at room temperature overnight and then purified by preparative thin-layer chromatography silica gel plate (dichloromethane:methanol = 10:1), and then purified by preparative HPLC [Pack Column 30ID*250mm*10μm; A:$H_2O$(0.1%FA), B:ACN, B%: 5%-80% over 25 min] to obtain 6-((3-(4-(4-methoxy-5-(trifluoromethyl)pyridin-2-yl)piperazine-1-carbonyl)azetidin-1-yl)methyl)-4-(trifluoromethyl)pyridazin-3(2*H*)-one (57, 2.76 mg, yield 2.59%), ESI $[M+H]^+$=521.0 $^1H$ NMR (400 MHz, DMSO-$d_6$) δ 13.96 (s, 1H), 8.18 (s, 1H), 7.99 (s, 1H), 6.46 (s, 1H), 4.51 - 4.15 (m, 6H), 3.99 (s, 1H), 3.92 (s, 3H), 3.89 - 3.79 (m, 1H), 3.71 (d, J = 47.4 Hz, 5H), 3.60 (s, 2H).

**Embodiment 58: Preparation of 6-(2-((((1*R*,3*R*)-3-(4-(3-ethoxy-5-(trifluoromethyl)pyridin-2-yl)piperazine-1-carbonyl)cyclobutyl)amino)propan-2-yl)-4-(trifluoromethyl)pyridazin-3(2*H*)-one (58)**

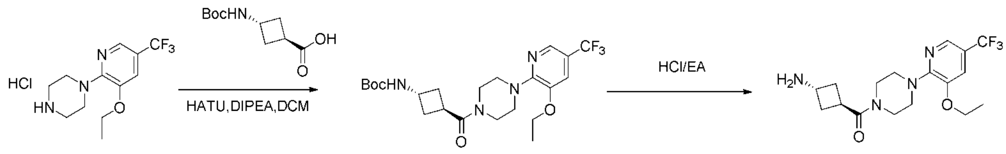


I-20 58-a 58-b

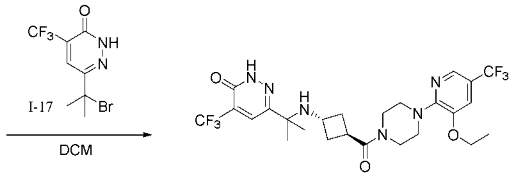


58

**Step 1: Preparation of tert-butyl ((1*R*,3*R*)-3-(4-(3-ethoxy-5-(trifluoromethyl)pyridin-2-yl)piperazine-1-carbonyl) cyclobutyl)carbamate (58-a)**

(1*R*,3*R*)-3-((tert-butoxycarbonyl)amino)cyclobutane-1-carboxylic acid (100 mg, 0.32 mmol) was dissolved in dichloromethane (6 mL), and *O*-(7-azabenzotriazol-1-yl)-*N,N,N',N'*-tetramethyluronium hexafluorophosphate (123 mg, 0.32 mmol) was added and stirred for 10 minutes. Then, *N,N*-diisopropylethylamine (169 $\mu$L, 0.97 mmol) and 1-(3-ethoxy-5-(trifluoromethyl)pyridin-2-yl)piperazine hydrochloride (I-20, 100 mg, 0.32 mmol) were added, and the reaction was stirred at room temperature for 2 hours. The reaction mixture was concentrated under reduced pressure and purified by Flash chromatography (10g silica gel column, petroleum ether:ethyl acetate = 1:1) to obtain the target product tert-butyl ((1*R*,3*R*)-3-(4-(3-ethoxy-5-(trifluoromethyl)pyridin-2-yl)piperazine-1-carbonyl)cyclobutyl)carbamate (58-a, 160 mg, yield 91.43%). ESI $[M+H]^+$=473.0

**Step 2: Preparation of (1*R*,3*R*)-3-aminocyclobutyl)(4-(3-ethoxy-5-(trifluoromethyl)pyridin-2-yl)piperazin-1-yl) methanone (58-b)**

tert-butyl ((1*R*,3*R*)-3-(4-(3-ethoxy-5-(trifluoromethyl)pyridin-2-yl)piperazine-1-carbonyl)cyclobutyl)carbamate (58-a, 160 mg, 0.34 mmol) was dissolved in 4M hydrogen chloride in ethyl acetate (3 mL) and reacted at room temperature for 2 hours, then concentrated under reduced pressure. Water (1 mL) was added, the pH was adjusted to 8 with $NaHCO_3$, and the mixture was extracted with (dichloromethane:methanol = 10:1). The organic phase was dried over anhydrous sodium sulfate and filtered, and the filtrate was concentrated under reduced pressure to obtain the target product (1*R*,3*R*)-3-aminocyclobutyl)(4-(3-ethoxy-5-(trifluoromethyl)pyridin-2-yl)piperazin-1-yl)methanone (58-b, 120 mg, yield 95.24%). ESI $[M+H]^+$=373.2

**Step 3: Preparation of 6-(2-((((1*R*,3*R*)-3-(4-(3-ethoxy-5-(trifluoromethyl)pyridin-2-yl)piperazine-1-carbonyl)cyclobutyl)amino)propan-2-yl)-4-(trifluoromethyl)pyridazin-3(2*H*)-one (58)**

(1*R*,3*R*)-3-aminocyclobutyl)(4-(3-ethoxy-5-(trifluoromethyl)pyridin-2-yl)piperazin-1-yl)methanone (58-b, 10 mg, 27.36 $\mu$mol) was dissolved in dichloromethane (3 mL), and 6-(2-bromopropan-2-yl)-4-(trifluoromethyl)pyridazin-3(2*H*)-one (I-17 , 100 mg, purity 6%, 21.05 umol) was added. After reacting at room temperature for 1.5 hours, the reaction mixture was distilled under reduced pressure, and the crude product was purified by preparative thin-layer chromatography (dichloromethane:methanol = 10:1), and then purified by preparative HPLC [Pack Column 30ID*250mm*10$\mu$m; A:$H_2O$(0.1%FA), B:ACN, B%: 10%-100% over 45 min] to obtain the target product 6-(2-((((1*R*,3*R*)-3-(4-(3-ethoxy-5-(trifluoromethyl)pyridin-2-yl)piperazine-1-carbonyl)cyclobutyl)amino)propan-2-yl)-4-(trifluoromethyl)pyridazin-3(2H)-one (58, 1.47 mg, yield 12.11%). ESI $[M+H]^+$=577.4 $^1$HNMR (600 MHz, DMSO-$d_6$) $\delta$ 8.29 (s, 1H), 8.11 (s, 1H), 7.40 (d, J = 1.5 Hz, 1H), 4.12 (q, J = 6.9 Hz, 2H), 3.55 - 3.52 (m, 2H), 3.48 - 3.43 (m, 6H), 3.06 (t, J = 9.5 Hz, 1H), 2.99 (dt, J = 15.9, 8.1 Hz, 2H), 2.20 - 2.14 (m, 2H), 1.89 (dd, J = 20.0, 9.6 Hz, 2H), 1.36 (t, J = 6.9 Hz, 3H), 1.27 (s, 6H).

**Embodiment 59: Preparation of 6-(2-((((1*R*,3*R*)-3-(4-(3-methoxy-5-(trifluoromethyl)pyridin-2-yl)piperazine-1-carbonyl)cyclobutyl)amino)propan-2-yl)-4-(trifluoromethyl)pyridazin-3(2*H*)-one (59)**

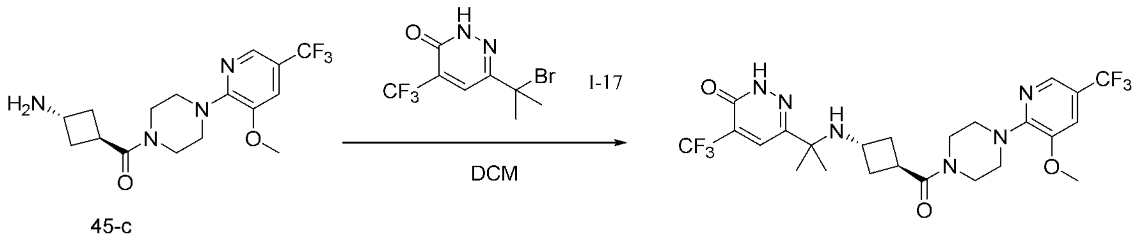


45-c 59

((1*R*,3*R*)-3-aminocyclobutyl)(4-(3-methoxy-5-(trifluoromethyl)pyridin-2-yl)piperazin-1-yl)methanone (45-c, 10 mg, 27.9 μmol) and 6-(2-bromopropan-2-yl)-4-(trifluoromethyl)pyridazin-3(2*H*)-one (I-17 , 30 mg, 0.103 mmol) were dissolved in dichloromethane (1 mL), and the reaction was stirred at room temperature for 1 hour. After monitoring showed the reaction was complete, the reaction mixture was concentrated under reduced pressure and purified by preparative HPLC [Pack Column 30ID*250mm*10μm; A:$H_2O$(0.1%TFA), B:ACN, B%: 10%-80% over 40 min] and lyophilized to obtain 6-(2-((((1*R*,3*R*)-3-(4-(3-methoxy-5-(trifluoromethyl)pyridin-2-yl)piperazine-1-carbonyl)cyclobutyl)amino)propan-2-yl)-4-(trifluoromethyl)pyridazin-3(2*H*)-one (59, 2.29 mg, yield 14.59%), ESI $[M+H]^+$=563.7 $^1$HNMR (600 MHz, DMSO-$d_6$) δ 8.12 (s, 2H), 7.45 (s, 1H), 3.88 (s, 3H), 3.79 (s, 2H), 3.57 (s, 3H), 3.46 (d, J = 2.8 Hz, 6H), 2.43 (dd, J = 27.5, 19.4 Hz, 4H), 1.59 (s, 6H).

**Embodiment 60: Preparation of 2-(4-((1*R*,3*R*)-3-((2-(6-oxo-5-(trifluoromethyl)-1,6-dihydropyridazin-3-yl)propan-2-yl)amino)cyclobutane-1-carbonyl)piperazin-1-yl)-5-(trifluoromethyl)nicotinamide (60)**

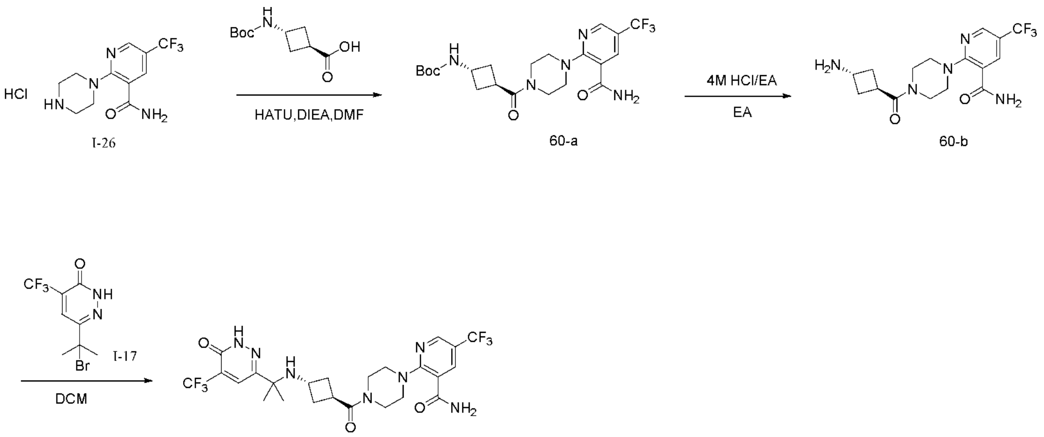


I-26 60-a 60-b 60

**Step 1: Preparation of tert-butyl ((1*R*,3*R*)-3-(4-(3-carbamoyl-5-(trifluoromethyl)pyridin-2-yl)piperazine-1-carbonyl)cyclobutyl)carbamate (60-a)**

(1R,3R)-3-((tert-butoxycarbonyl)amino)cyclobutane-1-carboxylic acid (69.4 mg, 0.323 mmol) was dissolved in N,*N*-dimethylformamide (5 mL), and *O*-(7-azabenzotriazol-1-yl)-*N,N,N',N'*-tetramethyluronium hexafluorophosphate (123 mg, 0.323 mmol) and N,N-diisopropylethylamine (170 μL, 0.968 mmol) were added. After stirring under an ice bath for 20 minutes, 2-(piperazin-1-yl)-5-(trifluoromethyl)nicotinamide hydrochloride (I-26, 100 mg, 0.323 mmol) was added. After reacting at room temperature for 4 hours, water (20 mL) was added dropwise to the reaction mixture, and a white solid precipitated. The mixture was filtered, the filter cake was washed with water, the collected filter cake was concentrated under reduced pressure to obtain the target product tert-butyl ((1R,3R)-3-(4-(3-carbamoyl-5-(trifluoromethyl)pyridin-2-yl)piperazine-1-carbonyl)cyclobutyl)carbamate (60-a, 120 mg, yield 78.8%). ESI $[M+H]^+$=472.2

**Step 2: Preparation of 2-(4-((1*R*,3*R*)-3-aminocyclobutane-1-carbonyl)piperazin-1-yl)-5-(trifluoromethyl)nicotinamide (60-b)**

((1*R*,3*R*)-3-(4-(3-carbamoyl-5-(trifluoromethyl)pyridin-2-yl)piperazine-1-carbonyl)cyclobutyl)carbamate (60-a, 120 mg, 0.255 mmol) was dissolved in ethyl acetate (2 mL), and 4M hydrogen chloride in ethyl acetate solution (3 mL) was added. After reacting at room temperature for 3 hours, the mixture was concentrated under reduced pressure.

Water (1 mL) was added, the pH was adjusted to 8 with $NaHCO_3$, and the mixture was extracted with dichloromethane:methanol = 8:1. The organic phase was dried over anhydrous sodium sulfate and filtered, and the filtrate was concentrated under reduced pressure to obtain the target product 2-(4-((1R,3R)-3-aminocyclobutane-1-carbonyl)piperazin-1-yl)-5-(trifluoromethyl)nicotinamide (60-b, 40 mg, yield 43%). ESI $[M+H]^+$=372.1

**Step 3: Preparation of 2-(4-((1*R*,3*R*)-3-((2-(6-oxo-5-(trifluoromethyl)-1,6-dihydropyridazin-3-yl)propan-2-yl)amino)cyclobutane-1-carbonyl)piperazin-1-yl)-5-(trifluoromethyl)nicotinamide (60)**

6-(2-bromopropan-2-yl)-4-(trifluoromethyl)pyridazin-3(2*H*)-one (I-17, 10 mg, 0.0352 mmol) was dissolved in dichloromethane (2 mL), and 2-(4-((1R,3R)-3-aminocyclobutane-1-carbonyl)piperazin-1-yl)-5-(trifluoromethyl)nicotinamide (60-b, 24 mg, 0.0634 mmol) was added. After reacting at room temperature for 30 minutes, the reaction was concentrated under reduced pressure, and the crude product was purified by normal phase column chromatography (dichloromethane:methanol = 10:1), and then purified by preparative HPLC [Pack Column 30ID*250mm*10μm; A:$H_2O$(0.1%$NH_3HCO_3$), B:ACN, B%: 10%-100% over 30 min] to obtain the target product 2-(4-((1R,3R)-3-((2-(6-oxo-5-(trifluoromethyl)-1,6-dihydropyridazin-3-yl)propan-2-yl)amino)cyclobutane-1-carbonyl)piperazin-1-yl)-5-(trifluoromethyl)nicotinamide (60, 0.7 mg, yield 3.4%). ESI $[M+H]^+$=576.2 $^1$H NMR (600 MHz, DMSO-$d_6$) δ 13.45 (s, 1H), 8.50 (s, 1H), 8.10 (s, 1H), 8.03 (s, 1H), 7.85 (s, 1H), 7.64 (s, 1H), 3.53 (dd, J = 13.0, 8.4 Hz, 2H), 3.48 - 3.43 (m, 2H), 3.43 - 3.36 (m, 4H), 3.08 - 3.03 (m, 1H), 2.99 (dd, J = 16.3, 8.2 Hz, 2H), 1.99 (dt, J = 12.4, 6.9 Hz, 4H), 1.23 (s, 6H).

**Embodiment 61: Preparation of *N*-(2-(4-((1*R*,3*R*)-3-((2-(6-oxo-5-(trifluoromethyl)-1,6-dihydropyridazin-3-yl)propan-2-yl)amino)cyclobutane-1-carbonyl)piperazin-1-yl)-5-(trifluoromethyl)pyridin-3-yl)acetamide (61)**

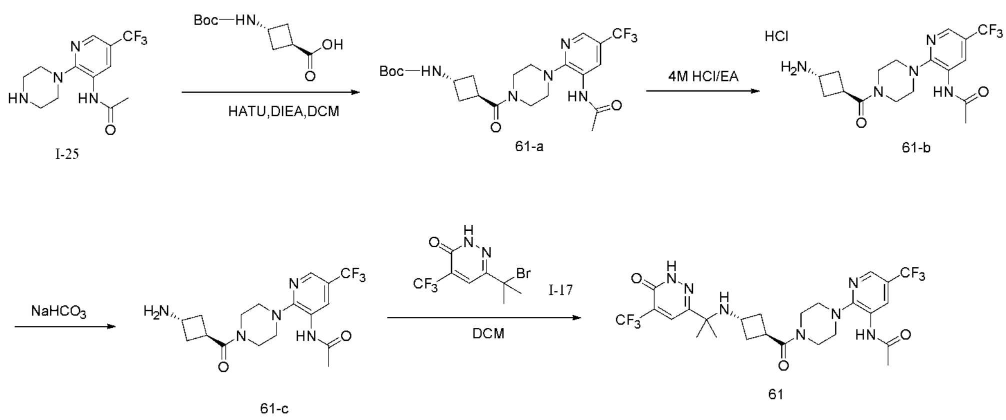


**Step 1: Preparation of tert-butyl (1*R*,3*R*)-3-(4-(3-acetamido-5-(trifluoromethyl)pyridin-2-yl)piperazine-1-carbonyl)cyclobutyl)carbamate (61-a)**

(1*R*,3*R*)-3-((tert-butoxycarbonyl)amino)cyclobutane-1-carboxylic acid (75 mg, 0.348 mmol) was dissolved in dichloromethane (5 mL), and *O*-(7-azabenzotriazol-1-yl)-*N,N,N',N'*-tetramethyluronium hexafluorophosphate (132 mg, 0.348 mmol) and *N,N*-diisopropylethylamine (243 μL, 1.045 mmol) were added. After stirring for 10 minutes, *N*-(2-(piperazin-1-yl)-5-(trifluoromethyl)pyridin-3-yl)acetamide (I-25, 400 mg, 0.348 mmol) was added, and the mixture was reacted at room temperature for 2 hours. The reaction mixture was concentrated under reduced pressure, and the crude product was purified by Flash column chromatography (10g silica gel column, methanol:dichloromethane = 0~7%) to obtain the target product tert-butyl (1*R*,3*R*)-3-(4-(3-acetamido-5-(trifluoromethyl)pyridin-2-yl)piperazine-1-carbonyl)cyclobutyl)carbamate (61-a, 110 mg, yield 65.09%). ESI $[M-56+H]^+$=485.4

**Step 2: Preparation of *N*-(2-(4-((1*R*,3*R*)-3-aminocyclobutane-1-carbonyl)piperazin-1-yl)-5-(trifluoromethyl)pyridin-3-yl)acetamide hydrochloride (61-b)**

tert-butyl (1*R*,3*R*)-3-(4-(3-acetamido-5-(trifluoromethyl)pyridin-2-yl)piperazine-1-carbonyl)cyclobutyl)carbamate (61-a, 110 mg, 0.227 mmol) was added to 4M hydrogen chloride in ethyl acetate solution (3 mL). After reacting at room temperature for 1 hour, the reaction mixture was concentrated under reduced pressure, and ethyl acetate was added for dilution and then concentrated under reduced pressure to obtain the target product *N*-(2-(4-((1*R*,3*R*)-3-aminocyclobutane-1-carbonyl)piperazin-1-yl)-5-(trifluoromethyl)pyridin-3-yl)acetamide hydrochloride (61-b, 100 mg,

yield 104.64%). ESI $[M+H]^+$=386.3

**Step 3: Preparation of *N*-(2-(4-((1*R*,3*R*)-3-aminocyclobutane-1-carbonyl)piperazin-1-yl)-5-(trifluoromethyl)pyridin-3-yl)acetamide (61-c)**

*N*-(2-(4-((1*R*,3*R*)-3-aminocyclobutane-1-carbonyl)piperazin-1-yl)-5-(trifluoromethyl)pyridin-3-yl)acetamide hydrochloride (61-b, 100 mg, 0.237 mmol) was dissolved in water (2 mL), and solid sodium bicarbonate (40 mg, 0.474 mmol) was added. The mixture was stirred at room temperature for 5 minutes. The pH of the reaction mixture was measured to be 8-10. The mixture was concentrated under reduced pressure, slurried with (dichloromethane:methanol = 10:1) and filtered. The filtrate was concentrated to obtain the target product *N*-(2-(4-((1*R*,3*R*)-3-aminocyclobutane-1-carbonyl) piperazin-1-yl)-5-(trifluoromethyl)pyridin-3-yl)acetamide (61-c, 45 mg, yield 49.26%).

**Step 4: Preparation of *N*-(2-(4-((1*R*,3*R*)-3-((2-(6-oxo-5-(trifluoromethyl)-1,6-dihydropyridazin-3-yl)propan-2-yl) amino)cyclobutane-1-carbonyl)piperazin-1-yl)-5-(trifluoromethyl)pyridin-3-yl)acetamide (61)**

*N*-(2-(4-((1*R*,3*R*)-3-aminocyclobutane-1-carbonyl)piperazin-1-yl)-5-(trifluoromethyl)pyridin-3-yl)acetamide (61-c, 10 mg, 26.25 μmol) was dissolved in dichloromethane (2 mL), and 6-(2-bromopropan-2-yl)-4-(trifluoromethyl) pyridazin-3(2*H*)-one (I-17, 100 mg, purity 6%, 21 μmol) was added. After reacting at room temperature for 3 hours, the reaction mixture was distilled under reduced pressure, and the crude product was purified by Flash column chromatography (4g silica gel column, methanol:dichloromethane = 0-10%), and then purified by preparative HPLC [Pack Column 30ID*250mm* 10μm; A:$H_2O$(0.1 %TFA), B:ACN, B%: 10%-100% over 45 min] to obtain the target product N-(2-(4-((1R,3R)-3-((2-(6-oxo-5-(trifluoromethyl)-1,6-dihydropyridazin-3-yl)propan-2-yl)amino)cyclobutane-1-carbonyl) piperazin-1-yl)-5-(trifluoromethyl)pyridin-3-yl)acetamide (61, 1.14 mg, yield 8.9%). ESI $[M+H]^+$=590.4 $^1$HNMR (600 MHz, DMSO-$d_6$) δ 8.37 (s, 1H), 8.21 (s, 1H), 8.13 (s, 1H), 3.81 (s, 1H), 3.63 (s, 3H), 3.44 (s, 3H), 3.24 (s, 4H), 2.18 (dd, J = 9.5, 5.8 Hz, 2H), 2.12 (s, 3H), 1.90 (d, J = 7.2 Hz, 2H), 1.60 (s, 6H).

**Embodiment 62: Preparation of 2-(4-((1*R*,3*R*)-3-(((6-oxo-5-(trifluoromethyl)-1,6-dihydropyridazin-3-yl)methyl) amino)cyclobutane-1-carbonyl)piperazin-1-yl)-5-(trifluoromethyl)nicotinamide (62)**

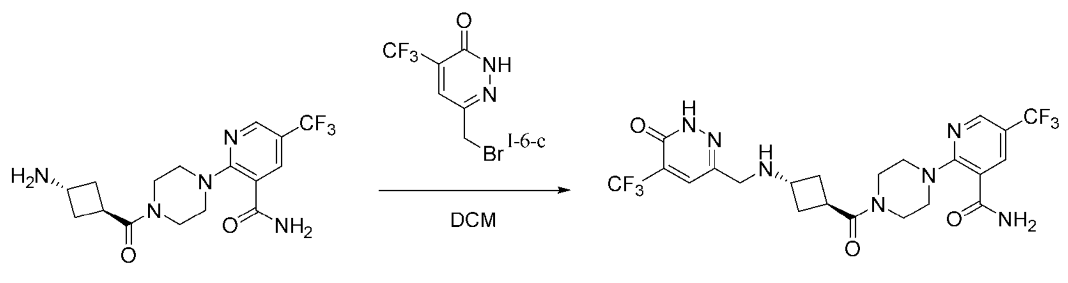


60-b 62

6-(bromomethyl)-4-(trifluoromethyl)pyridazin-3(2*H*)-one (I-6-c, 37.5 mg, 0.128 mmol) was dissolved in dichloromethane (5 mL), and 2-(4-((1*R*,3*R*)-3-aminocyclobutane-1-carbonyl)piperazin-1-yl)-5-(trifluoromethyl)nicotinamide (60-b, 86 mg, 0.231 mmol) was added. The mixture was reacted at room temperature for 1 hour. The reaction was concentrated under reduced pressure, and the crude product was purified by normal phase column chromatography (dichloromethane:methanol = 10:1), and then purified by preparative HPLC [Pack Column 30ID*250mm*10μm; A:$H_2O$(0.1%$NH_3HCO_3$), B:ACN, B%: 10%-100% over 50 min] to obtain the target product 2-(4-((1*R*,3*R*)-3-(((6-oxo-5-(trifluoromethyl)-1,6-dihydropyridazin-3-yl)methyl)amino)cyclobutane-1-carbonyl)piperazin-1-yl)-5-(trifluoromethyl)nicotinamide (62, 4.06 mg, yield 3.2%). ESI $[M+H]^+$=548.2 $^1$H NMR (600 MHz, DMSO-$d_6$) δ 8.50 (s, 1H), 8.04 (s, 1H), 7.87 (d, J = 20.8 Hz, 2H), 7.65 (s, 1H), 3.55 (s, 4H), 3.47 (s, 4H), 3.41 (d, J = 3.8 Hz, 2H), 3.23 (dd, J = 11.5, 7.0 Hz, 1H), 3.13 (t, J = 12.7 Hz, 1H), 2.26 (dd, J = 14.5, 7.5 Hz, 2H), 1.92 (dd, J = 17.0, 9.9 Hz, 2H).

**Embodiment 63: Preparation of *N*-(2-(4-((1*R*,3*R*)-3-(((6-oxo-5-(trifluoromethyl)-1,6-dihydropyridazin-3-yl) methyl)amino)cyclobutane-1-carbonyl)piperazin-1-yl)-5-(trifluoromethyl)pyridin-3-yl)acetamide (63)**

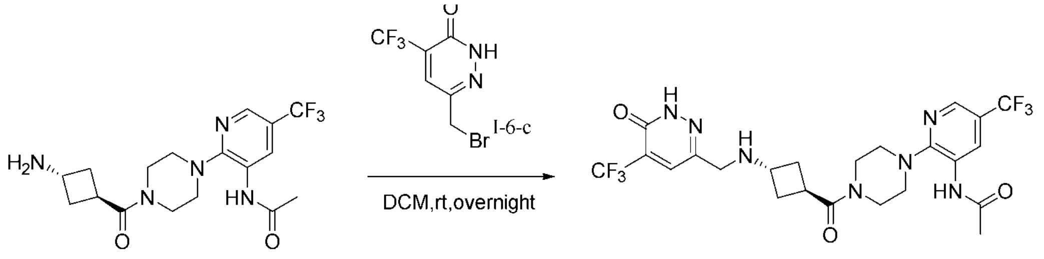


61-c 63

*N*-(2-(4-((1*R*,3*R*)-3-aminocyclobutane-1-carbonyl)piperazin-1-yl)-5-(trifluoromethyl)pyridin-3-yl)acetamide (61-c, 10 mg, 25.95 μmol) and 6-(bromomethyl)-4-(trifluoromethyl)pyridazin-3(2*H*)-one (I-6-c, 10 mg, 38.92 μmol) were dissolved in dichloromethane (2 mL), and the reaction was stirred at room temperature overnight. After monitoring showed the reaction was complete, the reaction mixture was concentrated under reduced pressure and purified by preparative thin-layer chromatography silica gel plate (dichloromethane:methanol = 10:1), and then purified by preparative HPLC [Pack Column 30ID*250mm*10μm; A:$H_2O$(0.1%FA), B:ACN, B%: 10%-80% over 40 min] and lyophilized to obtain N-(2-(4-((1R,3R)-3-(((6-oxo-5-(trifluoromethyl)-1,6-dihydropyridazin-3-yl)methyl)amino)cyclobutane-1-carbonyl)piperazin-1-yl)-5-(trifluoromethyl)pyridin-3-yl)acetamide (63, 1.01 mg, yield 6.93%). ESI $[M+H]^+$=562.4 $^1$HNMR (600 MHz, DMSO-$d_6$) δ 9.49 (s, 1H), 8.37 (s, 2H), 8.20 (s, 1H), 7.94 (s, 1H), 3.62 (s, 2H), 3.56 (s, 2H), 3.44 (s, 4H), 3.24 (s, 3H), 3.18 - 3.10 (m, 2H), 2.31 - 2.23 (m, 2H), 2.12 (s, 3H), 1.92 (dd, J = 17.4, 10.0 Hz, 2H).

**Embodiment 64: Preparation of 4-(trifluoromethyl)-6-(2-(((1R,3R)-3-(4-(4-(trifluoromethyl)-1*H*-pyrrolo[2,3-c]pyridin-7-yl)piperazine-1-carbonyl)cyclobutyl)amino)propan-2-yl)pyridazin-3(2*H*)-one (64)**

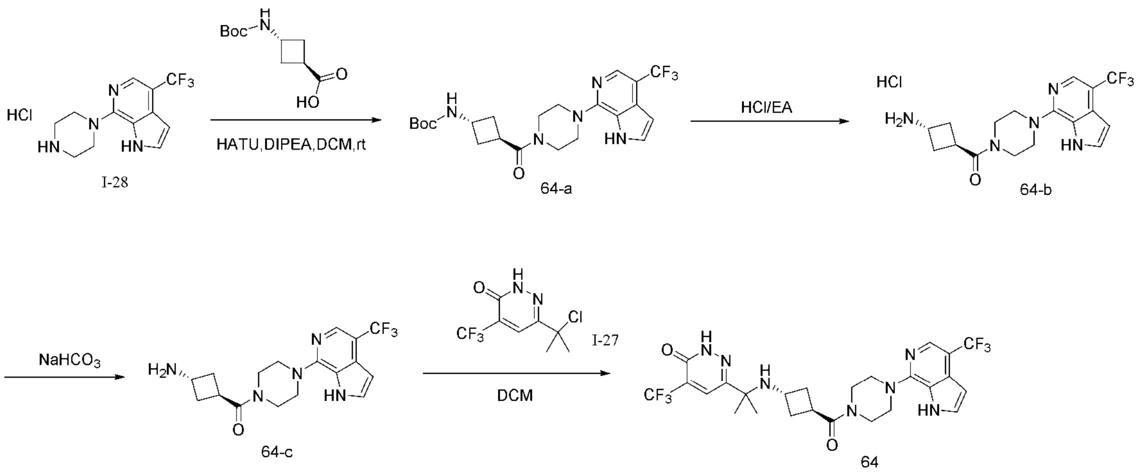


**Step 1: Preparation of tert-butyl ((1*R*,3*R*)-3-(4-(4-(trifluoromethyl)-1*H*-pyrrolo[2,3-c]pyridin-7-yl)piperazine-1-carbonyl)cyclobutyl)carbamate (64-a)**

(1*R*,3*R*)-3-((tert-butoxycarbonyl)amino)cyclobutane-1-carboxylic acid (42 mg, 0.195 mmol), *O*-(7-azabenzotriazol-1-yl)-*N,N,N',N'*-tetramethyluronium hexafluorophosphate (74.4 mg, 0.195 mmol), and *N,N*-diisopropylethylamine (102 μL, 0.59 mmol) were dissolved in dichloromethane (5 mL). The mixture was stirred for 0.5 hours, and then 7-(piperazin-1-yl)-4-(trifluoromethyl)-1*H*-pyrrolo[2,3-c]pyridine hydrochloride (I-28, 6 mg, 0.23 mmol) was added. The reaction was stirred at room temperature for 2 hours. After the reaction was complete, the reaction mixture was concentrated under reduced pressure, and the crude product was purified by Flash column chromatography (petroleum ether:ethyl acetate = 1:1) to obtain the target product tert-butyl ((1*R*,3*R*)-3-(4-(4-(trifluoromethyl)-1*H*-pyrrolo[2,3-*c*]pyridin-7-yl)piperazine-1-carbonyl)cyclobutyl)carbamate (64-a, 110 mg, yield 90%). ESI $[M+H]^+$=469.3

**Step 2: Preparation of ((1*R*,3*R*)-3-aminocyclobutyl)(4-(4-(trifluoromethyl)-1*H*-pyrrolo[2,3-*c*]pyridin-7-yl)piperazin-1-yl)methanone hydrochloride (64-b)**

tert-butyl ((1*R*,3*R*)-3-(4-(4-(trifluoromethyl)-1*H*-pyrrolo[2,3-*c*]pyridin-7-yl)piperazine-1-carbonyl)cyclobutyl)carbamate (64-a, 110 mg, 0.235 mmol) was dissolved in ethyl acetate (3 mL), and 4M hydrogen chloride in ethyl acetate solution (3 mL) was added. The mixture was reacted at room temperature overnight. After the reaction was complete, the mixture was concentrated under reduced pressure, dichloromethane solution was added and the mixture was evaporated twice to obtain the target product ((1*R*,3*R*)-3-aminocyclobutyl)(4-(4-(trifluoromethyl)-1*H*-pyrrolo[2,3-*c*]pyridin-7-yl)piperazin-1-yl)methanone hydrochloride (64-b, 110 mg, crude product). ESI $[M+H]^+$=369.3

**Step 3: Preparation of ((1*R*,3*R*)-3-aminocyclobutyl)(4-(4-(trifluoromethyl)-1*H*-pyrrolo[2,3-*c*]pyridin-7-yl)piperazin-1-yl)methanone (64-c)**

((1*R*,3*R*)-3-aminocyclobutyl)(4-(4-(trifluoromethyl)-1*H*-pyrrolo[2,3-*c*]pyridin-7-yl)piperazin-1-yl)methanone hydrochloride (64-b, 80 mg, 0.217 mmol) was dissolved in water (5 mL), and solid sodium bicarbonate (27 mg, 0.325 mmol) was added. The mixture was concentrated under reduced pressure to obtain the target product ((1*R*,3*R*)-3-aminocyclobutyl)(4-(4-(trifluoromethyl)-1*H*-pyrrolo[2,3-*c*]pyridin-7-yl)piperazin-1-yl)methanone (64-c, 80 mg, crude product). ESI $[M+H]^+$=369.3

**Step 4: Preparation of 4-(trifluoromethyl)-6-(2-(((1*R*,3*R*)-3-(4-(4-(trifluoromethyl)-1*H*-pyrrolo[2,3-*c*]pyridin-7-yl)piperazine-1-carbonyl)cyclobutyl)amino)propan-2-yl)pyridazin-3(2H)-one (64)**

((1*R*,3*R*)-3-aminocyclobutyl)(4-(4-(trifluoromethyl)-1*H*-pyrrolo[2,3-*c*]pyridin-7-yl)piperazin-1-yl)methanone (64-c, 80 mg, 0.217 mmol) was dissolved in dichloromethane (5 mL), and 6-(2-chloropropan-2-yl)-4-(trifluoromethyl)pyridazin-3(2H)-one (I-27, 100 mg, purity 50%, 0.217 mmol) was added. The mixture was reacted at room temperature overnight. The reaction was filtered and concentrated under reduced pressure. The crude product was purified by preparative HPLC [Pack Column 30ID*250mm* 10μm; A:$H_2O$(0.1%FA), B:ACN, B%: 10%-100% over 50 min] to obtain the target product 4-(trifluoromethyl)-6-(2-(((1*R*,3*R*)-3-(4-(4-(trifluoromethyl)-1*H*-pyrrolo[2,3-c]pyridin-7-yl)piperazine-1-carbonyl)cyclobutyl)amino)propan-2-yl)pyridazin-3(2H)-one (64, 2.12 mg, yield 1.71%). ESI $[M+H]^+$=572.4 $^1$H NMR (600 MHz, dmso) δ 11.86 (s, 1H), 8.20 (s, 1H), 8.12 (s, 1H), 8.04 (s, 1H), 7.64 (s, 1H), 6.51 (s, 1H), 3.64 (s, 2H), 3.46 (s, 6H), 3.04 (ddd, J = 23.5, 17.4, 8.7 Hz, 3H), 2.20 (t, J = 8.5 Hz, 2H), 1.91 (dd, J = 19.4, 9.7 Hz, 2H), 1.28 (s, 6H).

**Embodiment 65: Preparation of 6-(2-(((1R,3R)-3-(4-(1-methyl-4-(trifluoromethyl)-1H-pyrrolo[2,3-*c*]pyridin-7-yl)piperazine-1-carbonyl)cyclobutyl)amino)propan-2-yl)-4-(trifluoromethyl)pyridazin-3(2H)-one (65)**

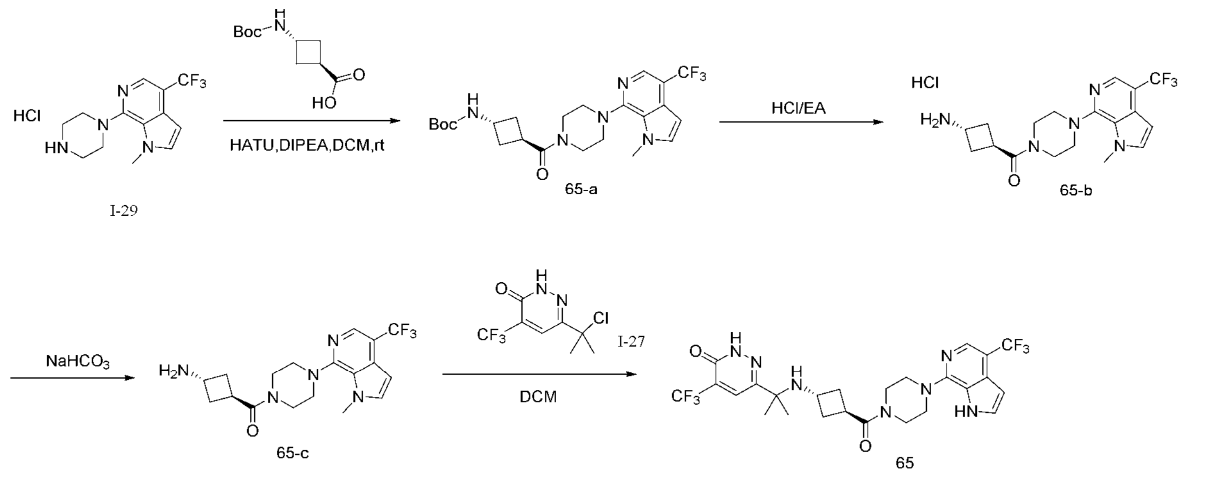


**Step 1: Preparation of tert-butyl ((1*R*,3*R*)-3-(4-(1-methyl-4-(trifluoromethyl)-1*H*-pyrrolo[2,3-*c*]pyridin-7-yl)piperazine-1-carbonyl)cyclobutyl)carbamate (65-a)**

(1*R*,3*R*)-3-((tert-butoxycarbonyl)amino)cyclobutane-1-carboxylic acid (20 mg, 0.092 mmol), *O*-(7-azabenzotriazol-1-yl)-*N,N,N',N'*-tetramethyluronium hexafluorophosphate (35 mg, 0.092 mmol), and *N,N*-diisopropylethylamine

(49 μL, 0.279 mmol) were dissolved in dichloromethane (2 mL). The mixture was stirred for 0.5 hours, and then 1-methyl-7-(piperazin-1-yl)-4-(trifluoromethyl)-1*H*-pyrrolo[2,3-c]pyridine hydrochloride (I-29, 30 mg, 0.111 mmol) was added. The reaction was stirred at room temperature for 2 hours. After the reaction was complete, the reaction mixture was concentrated under reduced pressure, and the crude product was purified by Flash column chromatography (petroleum ether:ethyl acetate = 1:1) to obtain the target product tert-butyl ((1*R*,3*R*)-3-(4-(1-methyl-4-(trifluoromethyl)-1*H*-pyrrolo[2,3-*c*]pyridin-7-yl)piperazine-1-carbonyl)cyclobutyl)carbamate (65-a, 28 mg, yield 56%). ESI $[M+H]^+$=482.4

**Step 2: Preparation of ((1*R*,3*R*)-3-aminocyclobutyl)(4-(1-methyl-4-(trifluoromethyl)-1*H*-pyrrolo[2,3-c]pyridin-7-yl)piperazin-1-yl)methanone hydrochloride (65-b)**

tert-butyl ((1*R*,3*R*)-3-(4-(1-methyl-4-(trifluoromethyl)-1*H*-pyrrolo[2,3-*c*]pyridin-7-yl)piperazine-1-carbonyl)cyclobutyl)carbamate (65-a, 28 mg, 0.058 mmol) was dissolved in ethyl acetate (1 mL), and 4M hydrogen chloride in ethyl acetate solution (1 mL) was added. The mixture was reacted at room temperature overnight. After the reaction was complete, the mixture was concentrated under reduced pressure, and dichloromethane was added to dissolve the residue, which was then evaporated twice to obtain the target product ((1*R*,3*R*)-3-aminocyclobutyl)(4-(1-methyl-4-(trifluoromethyl)-1*H*-pyrrolo[2,3-*c*]pyridin-7-yl)piperazin-1-yl)methanone hydrochloride (65-b, 32 mg, crude product). ESI $[M+H]^+$ =382.4

**Step 3: Preparation of ((1*R*,3*R*)-3-aminocyclobutyl)(4-(1-methyl-4-(trifluoromethyl)-1*H*-pyrrolo[2,3-*c*]pyridin-7-yl)piperazin-1-yl)methanone (65-c)**

((1*R*,3*R*)-3-aminocyclobutyl)(4-(1-methyl-4-(trifluoromethyl)-1*H*-pyrrolo[2,3-*c*]pyridin-7-yl)piperazin-1-yl) methanone hydrochloride (65-b, 32 mg, 0.084 mmol) was dissolved in water (3 mL), and solid sodium bicarbonate (14 mg, 0.167 mmol) was added. The mixture was concentrated under reduced pressure to obtain the target product ((1*R*,3*R*)-3-aminocyclobutyl)(4-(1-methyl-4-(trifluoromethyl)-1*H*-pyrrolo[2,3-*c*]pyridin-7-yl)piperazin-1-yl)methanone (65-c, 32 mg, crude product). ESI $[M+H]^+$=382.4

**Step 4: Preparation of 6-(2-(((1*R*,3*R*)-3-(4-(1-methyl-4-(trifluoromethyl)-1*H*-pyrrolo[2,3-*c*]pyridin-7-yl)piperazine-1-carbonyl)cyclobutyl)amino)propan-2-yl)-4-(trifluoromethyl)pyridazin-3(2H)-one (65)**

((1*R*,3*R*)-3-aminocyclobutyl)(4-(1-methyl-4-(trifluoromethyl)-1*H*-pyrrolo[2,3-*c*]pyridin-7-yl)piperazin-1-yl) methanone (65-c, 32 mg, 0.084 mmol) was dissolved in dichloromethane (2 mL), and 6-(2-chloropropan-2-yl)-4-(trifluoromethyl)pyridazin-3(2*H*)-one (I-27, 48 mg, purity 50%, 0.1 mmol) was added. The mixture was reacted at room temperature overnight. The reaction was filtered and concentrated under reduced pressure. The crude product was purified by preparative HPLC [Pack Column 30ID*250mm* 10μm; A:$H_2O$(0.1%FA), B:ACN, B%: 10%-100% over 50 min] to obtain the target product 6-(2-(((1*R*,3*R*)-3-(4-(1-methyl-4-(trifluoromethyl)-1*H*-pyrrolo[2,3-*c*]pyridin-7-yl)piperazine-1-carbonyl)cyclobutyl)amino)propan-2-yl)-4-(trifluoromethyl)pyridazin-3(2H)-one (65, 2.14 mg, yield 4.37%). ESI $[M+H]^+$=586.8 $^1H$ NMR (600 MHz, dmso) δ 13.45 (s, 1H), 8.11 (d, J = 5.9 Hz, 2H), 7.68 (d, J = 2.7 Hz, 1H), 6.53 (s, 1H), 4.09 (s, 3H), 3.67 (s, 3H), 3.48 (s, 2H), 3.11 (dd, J = 25.3, 14.0 Hz, 5H), 3.04 - 2.98 (m, 1H), 2.19 (t, J = 8.0 Hz, 2H), 1.92 (dd, J = 17.7, 8.8 Hz, 2H), 1.28 (s, 6H).

**Embodiment 66: Preparation of 6-(2-((1*R*,3*R*)-3-(4-(3-(3-(pyrrolidine-1-carbonyl)-5-(trifluoromethyl)pyridin-2-yl) piperazine-1-carbonyl)cyclobutyl)amino)propan-2-yl)-4-(trifluoromethyl)pyridazin-3(2*H*)-one (66)**

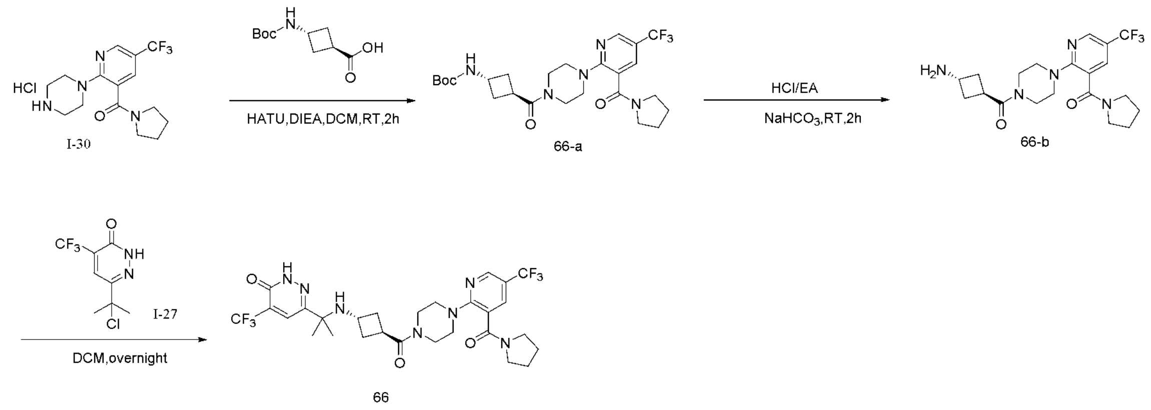


**Step 1: Preparation of tert-butyl ((1*R*,3*R*)-3-(4-(3-(pyrrolidine-1-carbonyl)-5-(trifluoromethyl)pyridin-2-yl)piperazine-1-carbonyl)cyclobutyl)carbamate (66-a)**

(1*R*,3*R*)-3-((tert-butoxycarbonyl)amino)cyclobutane-1-carboxylic acid (113 mg, 0.52 mmol) was dissolved in dichloromethane (8 mL), and *O*-(7-azabenzotriazol-1-yl)-*N,N,N',N'*-tetramethyluronium hexafluorophosphate (199 mg, 0.52 mmol) was added and stirred for 10 minutes. Then, *N,N*-diisopropylethylamine (274 μL, 1.56 mmol) and (2-(piperazin-1-yl)-5-(trifluoromethyl)pyridin-3-yl)(pyrrolidin-1-yl)methanone hydrochloride (I-30, 190 mg, 0.52 mmol) were added. The mixture was reacted at room temperature for 2 hours. The reaction mixture was concentrated under reduced pressure and purified by Flash column chromatography (10g silica gel column, dichloromethane:methanol = 20:1) to obtain the target product tert-butyl ((1*R*,3*R*)-3-(4-(3-(pyrrolidine-1-carbonyl)-5-(trifluoromethyl)pyridin-2-yl)piperazine-1-carbonyl) cyclobutyl)carbamate (66-a, 180 mg, yield 65.46%). ESI $[M+H]^+$=526.1

**Step 2: Preparation of (2-(4-((1*R*,3*R*)-3-aminocyclobutane-1-carbonyl)piperazin-1-yl)-5-(trifluoromethyl)pyridin-3-yl)(pyrrolidin-1-yl)methanone (66-b)**

tert-butyl ((1*R*,3*R*)-3-(4-(3-(pyrrolidine-1-carbonyl)-5-(trifluoromethyl)pyridin-2-yl)piperazine-1-carbonyl)cyclobutyl)carbamate (66-a, 180 mg, 0.34 mmol) was dissolved in 4M hydrogen chloride in ethyl acetate (3 mL) and reacted at room temperature for 2 hours, then concentrated under reduced pressure. Water (1 mL) was added, the pH was adjusted to 8 with $NaHCO_3$, and the mixture was extracted with (dichloromethane:methanol = 10:1). The organic phase was dried over anhydrous sodium sulfate and filtered, and the filtrate was concentrated under reduced pressure to obtain the target product (2-(4-((1*R*,3*R*)-3-aminocyclobutane-1-carbonyl)piperazin-1-yl)-5-(trifluoromethyl)pyridin-3-yl)(pyrrolidin-1-yl) methanone (66-b, 145 mg, yield 99.61%). ESI $[M+H]^+$=426.0

**Step 3: Preparation of 6-(2-(((1*R*,3*R*)-3-(4-(3-(3-(pyrrolidine-1-carbonyl)-5-(trifluoromethyl)pyridin-2-yl)piperazine-1-carbonyl)cyclobutyl)amino)propan-2-yl)-4-(trifluoromethyl)pyridazin-3(2H)-one (66)**

6-(2-chloropropan-2-yl)-4-(trifluoromethyl)pyridazin-3(2*H*)-one (I-27, 17 mg, 0.071 mmol) was dissolved in dichloromethane (2 mL), and (2-(4-((1*R*,3*R*)-3-aminocyclobutane-1-carbonyl)piperazin-1-yl)-5-(trifluoromethyl)pyridin-3-yl)(pyrrolidin-1-yl)methanone (66-b, 30 mg, 0.071 mmol) was added. After reacting at room temperature for 3 hours, the reaction mixture was concentrated under reduced pressure, and the crude product was purified by Flash column chromatography (dichloromethane:methanol = 20:1) to obtain the target product 6-(2-(((1*R*,3*R*)-3-(4-(3-(3-(pyrrolidine-1-carbonyl)-5-(trifluoromethyl)pyridin-2-yl)piperazine-1-carbonyl)cyclobutyl)amino)propan-2-yl)-4-(trifluoromethyl)pyridazin-3(2*H*)-one (66, 6.27 mg, yield 14.12%). ESI $[M+H]^+$=630.2 $^1H$ NMR (600 MHz, DMSO-$d_6$) δ 13.45 (s, 1H), 8.51 (s, 1H), 8.10 (s, 1H), 7.81 (s, 1H), 3.50 (d, J = 4.6 Hz, 2H), 3.45 - 3.42 (m, 6H), 3.35 (d, J = 3.5 Hz, 2H), 3.21 (s, 2H), 3.04 (t, J = 9.4 Hz, 1H), 3.01 - 2.96 (m, 1H), 2.16 (t, J = 8.3 Hz, 2H), 1.95 - 1.75 (m, 7H), 1.27 (s, 6H).

**Embodiment 67: Preparation of *N*-methyl-2-(4-((1*R*,3*R*)-3-((2-(6-oxo-5-(trifluoromethyl)-1,6-dihydropyridazin-3-yl)propan-2-yl)amino)cyclobutane-1-carbonyl)piperazin-1-yl)-5-(trifluoromethyl)nicotinamide (67)**

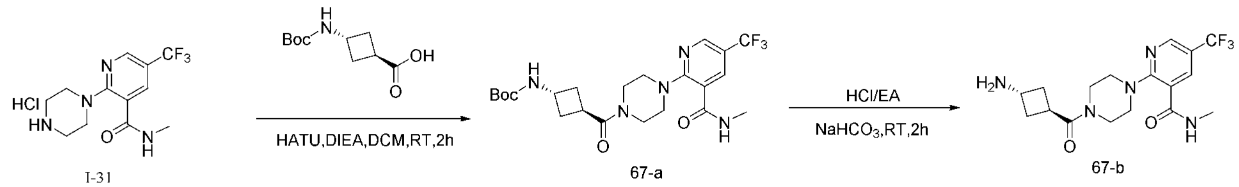


I-31 67-a 67-b

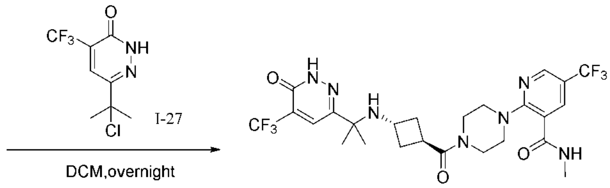


67

**Step 1: Preparation of tert-butyl ((1*R*,3*R*)-3-(4-(3-(methylcarbamoyl)-5-(trifluoromethyl)pyridin-2-yl)piperazine-1-carbonyl)cyclobutyl)carbamate (67-a)**

(1*R*,3*R*)-3-((tert-butoxycarbonyl)amino)cyclobutane-1-carboxylic acid (67 mg, 0.31 mmol) was dissolved in dichloromethane (6 mL), and *O*-(7-azabenzotriazol-1-yl)-*N,N,N',N'*-tetramethyluronium hexafluorophosphate (118 mg, 0.31 mmol) was added and stirred for 10 minutes. Then, *N,N*-diisopropylethylamine (162 μL, 0.93 mmol) and *N*-methyl-2-(piperazin-1-yl)-5-(trifluoromethyl)nicotinamide hydrochloride (I-31, 100mg, 0.31 mmol) were added, and the mixture was reacted at room temperature overnight. The reaction mixture was concentrated under reduced pressure and purified by Flash column chromatography (10g silica gel column, petroleum ether:ethyl acetate = 1:2) to obtain the target product tert-butyl ((1*R*,3*R*)-3-(4-(3-(methylcarbamoyl)-5-(trifluoromethyl)pyridin-2-yl)piperazine-1-carbonyl)cyclobutyl) carbamate (67-a, 165 mg, yield 109.78%). ESI $[M+H]^+$=486.0

**Step 2: Preparation of 2-(4-((1*R*,3*R*)-3-aminocyclobutane-1-carbonyl)piperazin-1-yl)-*N*-methyl-5-(trifluoromethyl)nicotinamide (67-b)**

tert-butyl ((1*R*,3*R*)-3-(4-(3-(methylcarbamoyl)-5-(trifluoromethyl)pyridin-2-yl)piperazine-1-carbonyl)cyclobutyl) carbamate (67-a, 165 mg, 0.34 mmol) was dissolved in 4M hydrogen chloride in ethyl acetate (3 mL) and reacted at room temperature for 2 hours, then concentrated under reduced pressure. Water (1 mL) was added, the pH was adjusted to 8 with $NaHCO_3$, and the mixture was extracted with (dichloromethane:methanol = 10:1). The organic phase was dried over anhydrous sodium sulfate and filtered, and the filtrate was concentrated under reduced pressure to obtain the target product 2-(4-((1*R*,3*R*)-3-aminocyclobutane-1-carbonyl)piperazin-1-yl)-*N*-methyl-5-(trifluoromethyl)nicotinamide (67-b, 130 mg, yield 99.24%). ESI $[M+H]^+$=386.0

**Step 3: Preparation of *N*-methyl-2-(4-((1*R*,3*R*)-3-((2-(6-oxo-5-(trifluoromethyl)-1,6-dihydropyridazin-3-yl)propan-2-yl)amino)cyclobutane-1-carbonyl)piperazin-1-yl)-5-(trifluoromethyl)nicotinamide (67)**

6-(2-chloropropan-2-yl)-4-(trifluoromethyl)pyridazin-3(*H*)-one (I-27, 28 mg, 0.117 mmol) was dissolved in dichloromethane (5 mL), and 2-(4-((1R,3R)-3-aminocyclobutane-1-carbonyl)piperazin-1-yl)-*N*-methyl-5-(trifluoromethyl) nicotinamide (67-b, 45 mg, 0.117 mmol) was added. After reacting at room temperature for 3 hours, the reaction mixture was concentrated under reduced pressure, and the crude product was purified by Flash column chromatography (dichloromethane:methanol = 20:1) to obtain the target product *N*-methyl-2-(4-((1*R*,3*R*)-3-((2-(6-oxo-5-(trifluoromethyl)-1,6-dihydropyridazin-3-yl)propan-2-yl)amino)cyclobutane-1-carbonyl)piperazin-1-yl)-5-(trifluoromethyl)nicotinamide (67, 4.42 mg, yield 6.42%). ESI $[M+H]^+$=590.2 $^1H$ NMR (600 MHz, DMSO-$d_6$) δ 13.44 (s, 1H), 8.49 (s, 1H), 8.47 (d, J = 4.3 Hz, 1H), 8.10 (s, 1H), 7.81 (s, 1H), 3.51 (s, 3H), 3.35 (s, 3H), 3.02 (d, J = 34.8 Hz, 3H), 2.75 (d, J = 4.3 Hz, 3H), 2.16 (s, 2H), 1.89 (d, J = 10.8 Hz, 2H), 1.27 (s, 6H).

**Embodiment 68: Preparation of 6-(2-(((1R,3R)-3-(4-(2-methyl-4-(trifluoromethyl)-1*H*-pyrrolo[2,3-*c*]pyridin-7-yl) piperazine-1-carbonyl)cyclobutyl)amino)propan-2-yl)-4-(trifluoromethyl)pyridazin-3(2*H*)-one (68)**

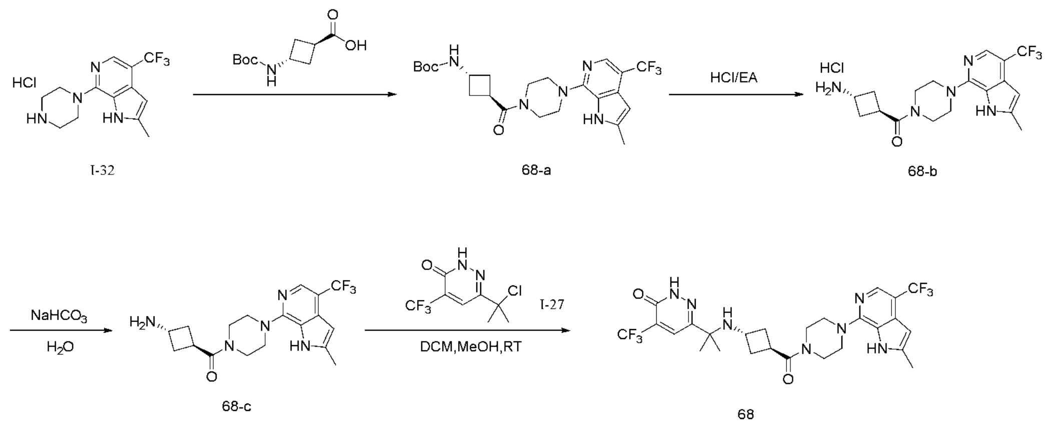


I-32 68-a 68-b 68-c 68

**Step 1: Preparation of tert-butyl ((1*R*,3*R*)-3-(4-(2-methyl-4-(trifluoromethyl)-1*H*-pyrrolo[2,3-*c*]pyridin-7-yl)piperazine-1-carbonyl)cyclobutyl)carbamate (68-a)**

(1*R*,3*R*)-3-((tert-butoxycarbonyl)amino)cyclobutane-1-carboxylic acid (68 mg, 0.33 mmol), *O*-(7-azabenzotriazol-1-yl)-*N,N,N',N'*-tetramethyluronium hexafluorophosphate (115 mg, 0.30 mmol), and *N,N*-diisopropylethylamine (160 μL, 0.95 mmol) were dissolved in dichloromethane (3 mL). The mixture was stirred for 0.5 hours, and then 2-methyl-7-(piperazin-1-yl)-4-(trifluoromethyl)-1*H*-pyrrolo[2,3-c]pyridine hydrochloride (I-32, 100 mg, 0.38 mmol) was added. The reaction was stirred at room temperature for 2 hours. After the reaction was complete, the reaction mixture was concentrated under reduced pressure, and the crude product was purified by Flash column chromatography (petroleum ether:ethyl acetate = 1:1) to obtain the target product tert-butyl ((1*R*,3*R*)-3-(4-(2-methyl-4-(trifluoromethyl)-1*H*-pyrrolo [2,3-*c*]pyridin-7-yl)piperazine-1-carbonyl)cyclobutyl)carbamate (68-a, 130 mg, yield 76.92%). ESI $[M+H]^+$=482.3

**Step 2: Preparation of ((1*R*,3*R*)-3-aminocyclobutyl)(4-(2-methyl-4-(trifluoromethyl)-1*H*-pyrrolo[2,3-*c*]pyridin-7-yl)piperazin-1-yl)methanone hydrochloride (68-b)**

tert-butyl ((1R,3R)-3-(4-(2-methyl-4-(trifluoromethyl)-1*H*-pyrrolo[2,3-c]pyridin-7-yl)piperazine-1-carbonyl)cyclobutyl)carbamate (68-a, 130 mg, 0.27 mmol) was dissolved in ethyl acetate (1 mL), and 4M hydrogen chloride in ethyl acetate solution (3 mL) was added. The mixture was reacted at room temperature for 2 hours. After the reaction was complete, the mixture was concentrated under reduced pressure, and dichloromethane solution was added and evaporated twice to obtain the target product ((1R,3R)-3-aminocyclobutyl)(4-(2-methyl-4-(trifluoromethyl)-1*H*-pyrrolo [2,3-*c*]pyridin-7-yl)piperazin-1-yl)methanone hydrochloride (68-b, 120 mg, crude product). ESI $[M+H]^+$=382.3

**Step 3: Preparation of ((1*R*,3*R*)-3-aminocyclobutyl)(4-(2-methyl-4-(trifluoromethyl)-1*H*-pyrrolo[2,3-*c*]pyridin-7-yl)piperazin-1-yl)methanone (68-c)**

((1*R*,3*R*)-3-aminocyclobutyl)(4-(2-methyl-4-(trifluoromethyl)-1*H*-pyrrolo[2,3-*c*]pyridin-7-yl)piperazin-1-yl) methanone hydrochloride (68-b, 116 mg, 0.30 mmol) was dissolved in water (5 mL), and solid sodium bicarbonate (46 mg, 0.55 mmol) was added. The mixture was concentrated under reduced pressure to obtain the target product ((1*R*,3*R*)-3-aminocyclobutyl)(4-(2-methyl-4-(trifluoromethyl)-1*H*-pyrrolo[2,3-*c*]pyridin-7-yl)piperazin-1-yl)methanone (68-c, 120 mg, crude product). ESI $[M+H]^+$=382.3

**Step 4: Preparation of 6-(2-((((1*R*,3*R*)-3-(4-(2-methyl-4-(trifluoromethyl)-1*H*-pyrrolo[2,3-*c*]pyridin-7-yl)piperazine-1-carbonyl)cyclobutyl)amino)propan-2-yl)-4-(trifluoromethyl)pyridazin-3(2*H*)-one (68)**

((1*R*,3*R*)-3-aminocyclobutyl)(4-(2-methyl-4-(trifluoromethyl)-1*H*-pyrrolo[2,3-*c*]pyridin-7-yl)piperazin-1-yl) methanone (68-c, 120 mg, 0.31 mmol) was dissolved in dichloromethane (4 mL), and 6-(2-chloropropan-2-yl)-4-(trifluoromethyl)pyridazin-3(2*H*)-one (I-27, 181 mg, purity 50%, 0.38 mmol) was added. The mixture was reacted at room temperature overnight. The reaction was filtered and concentrated under reduced pressure. The crude product was purified by preparative HPLC [Pack Column 30ID*250mm*10μm; A:$H_2O$(0.1%FA), B:ACN, B%: 10%-100% over 40 min] to obtain the target product 6-(2-((((1*R*,3*R*)-3-(4-(2-methyl-4-(trifluoromethyl)-1*H*-pyrrolo[2,3-*c*]pyridin-7-yl)piperazine-1-carbonyl)cyclobutyl)amino)propan-2-yl)-4-(trifluoromethyl)pyridazin-3(2H)-one (68, 6.18 mg, yield 3.35%). ESI $[M+H]^+$=586.1 $^1H$ NMR (600 MHz, DMSO-$d_6$) δ 11.53 (s, 1H), 8.34 (s, 2H), 8.12 (s, 1H), 7.99 (s, 1H), 6.25 (s, 1H), 3.64 (s, 4H), 3.13 - 3.06 (m, 2H), 3.01 (dt, *J* = 15.6, 7.8 Hz, 2H), 2.46 (s, 3H), 2.25 - 2.15 (m, 3H), 1.91 (dd, *J* = 19.3, 10.1 Hz,

3H), 1.28 (s, 6H).

**Embodiment 69: Preparation of 6-(2-((((1*R*,3*R*)-3-(4-(3-(1,2,4-oxadiazol-3-yl)-5-(trifluoromethyl)pyridin-2-yl)piperazine-1-carbonyl)cyclobutyl)amino)propan-2-yl)-4-(trifluoromethyl)pyridazin-3(2*H*)-one (69)**

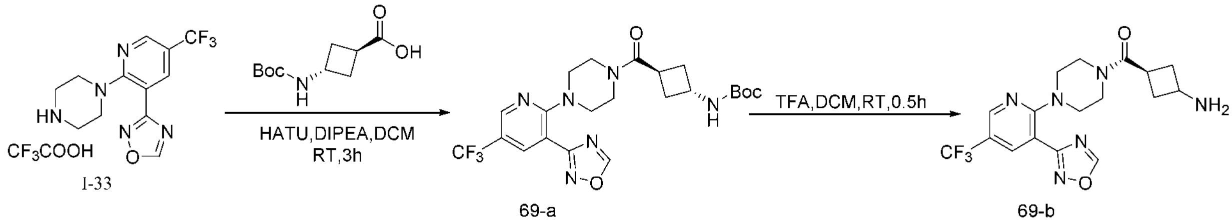


I-33 69-a 69-b

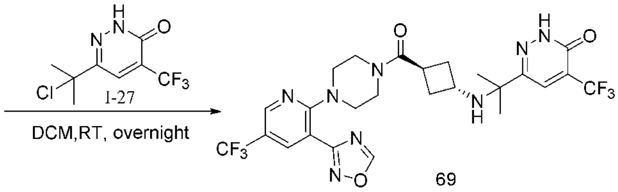


I-27 69

**Step 1: Preparation of tert-butyl ((1*R*,3*R*)-3-(4-(3-(1,2,4-oxadiazol-3-yl)-5-(trifluoromethyl)pyridin-2-yl)piperazine-1-carbonyl)cyclobutyl)carbamate (69-a)**

(1*R*,3*R*)-3-((tert-butoxycarbonyl)amino)cyclobutane-1-carboxylic acid (113 mg, 0.55 mmol) was dissolved in dichloromethane (5 mL), and *O*-(7-azabenzotriazol-1-yl)-*N,N,N',N'*-tetramethyluronium hexafluorophosphate (209 mg, 0.55 mmol) and *N,N*-diisopropylethylamine (319 μL, 1.84 mmol) were added. After stirring for 30 minutes, 3-(2-(piperazin-1-yl)-5-(trifluoromethyl)pyridin-3-yl)-1,2,4-oxadiazole trifluoroacetate (I-33, 110 mg, 0.37 mmol) was added, and the mixture was reacted at room temperature for 2.5 hours. After the reaction mixture was concentrated under reduced pressure, the crude product was purified by Flash column chromatography (10g silica gel column, dichloromethane:methanol = 0-7%) to obtain the target product tert-butyl ((1*R*,3*R*)-3-(4-(3-(1,2,4-oxadiazol-3-yl)-5-(trifluoromethyl)pyridin-2-yl)piperazine-1-carbonyl)cyclobutyl)carbamate (69-a, 110 mg, yield 59.9%). ESI $[M+H]^+$=497.3

**Step 2: Preparation of (4-(3-(1,2,4-oxadiazol-3-yl)-5-(trifluoromethyl)pyridin-2-yl)piperazin-1-yl)(3-aminocyclobutyl)methanone (69-b)**

tert-butyl ((1*R*,3*R*)-3-(4-(3-(1,2,4-oxadiazol-3-yl)-5-(trifluoromethyl)pyridin-2-yl)piperazine-1-carbonyl)cyclobutyl)carbamate (69-a, 110 mg, 0.22 mmol) was dissolved in dichloromethane (3 mL), and trifluoroacetic acid (0.6 mL) was added. After reacting at room temperature for 0.5 hours, the reaction mixture was concentrated under reduced pressure, the pH was adjusted to 8-9 with saturated sodium bicarbonate solution, and the mixture was concentrated under reduced pressure to obtain a crude product. The crude product was purified by Flash column chromatography (10g silica gel column, dichloromethane:methanol = 0~7%) to obtain the target product (4-(3-(1,2,4-oxadiazol-3-yl)-5-(trifluoromethyl)pyridin-2-yl)piperazin-1-yl)(3-aminocyclobutyl)methanone (69-b, 60 mg, yield 68.9%). ESI $[M+H]^+$=397.3

**Step 3: Preparation of 6-(2-((((1*R*,3*R*)-3-(4-(3-(1,2,4-oxadiazol-3-yl)-5-(trifluoromethyl)pyridin-2-yl)piperazine-1-carbonyl)cyclobutyl)amino)propan-2-yl)-4-(trifluoromethyl)pyridazin-3(2*H*)-one (69)**

(4-(3-(1,2,4-oxadiazol-3-yl)-5-(trifluoromethyl)pyridin-2-yl)piperazin-1-yl)(3-aminocyclobutyl)methanone (69-b, 60 mg, 0.15 mmol) was dissolved in dichloromethane (4 mL), and 6-(2-chloropropan-2-yl)-4-(trifluoromethyl)pyridazin-3(2*H*)-one (I-27, 72 mg, purity 50%, 0.15 mmol) and triethylamine (30 μL, 0.15 mmol) were added. The mixture was reacted at room temperature overnight. After the reaction mixture was distilled under reduced pressure, the crude product was purified by Flash column chromatography (10g silica gel column, dichloromethane:methanol = 0-10%), and then purified by preparative HPLC [Pack Column 30ID*250mm*10μm; A:$H_2O$(0.01%TFA), B:ACN, B%: 10%-100% over 45 min] to obtain the target product 6-(2-((((1*R*,3*R*)-3-(4-(3-(1,2,4-oxadiazol-3-yl)-5-(trifluoromethyl)pyridin-2-yl)piperazine-1-carbonyl)cyclobutyl)amino)propan-2-yl)-4-(trifluoromethyl)pyridazin-3(2H)-one (69, 5.92 mg, yield 6.5%). ESI

$[M+H]^+$=600.1 $^1H$ NMR (600 MHz, DMSO-$d_6$) δ 9.71 (s, 1H), 9.44 (s, 1H), 8.67 (d, J = 2.5 Hz, 1H), 8.25 (d, J = 2.4 Hz, 1H), 8.08 (s, 1H), 3.76 (t, J = 8.2 Hz, 4H), 3.53 (s, 2H), 3.37 - 3.35 (m, 2H), 3.31 (d, J = 5.3 Hz, 3H), 2.42 (dd, J = 29.9, 8.4 Hz, 4H), 1.57 (s, 6H).

**Test Example 1: PARP7 Inhibitory Activity Assay**

The *in vitro* activity of the embodiment compounds was demonstrated in the following assay (384-well plate) to show that PARP7 can catalyze NAD-dependent ADP-ribosylation:

Step one

1) Dilute histone (Active Motif, 81126) with PBS to 2 diluted to 6e, 25 μL per well, overnight at 4°C;
2) Wash 3 times with 100 μL PBST (1x PBS, 0.05% Tween-20) per well, and pat dry;
3) Add 75 μL of blocking buffer (Thermo Scientific™, 37535) per well, and incubate at room temperature for 1 hour;
4) Wash 3 times with 100 μL PBST per well, and pat dry.

Step two

1) Thaw PARP7 enzyme (BPS, 80527) on ice, and dilute to 1.94 ng/mM with buffer (20 mM Hepes, 100 mM NaCl, 0.1% BSA, 0.002% Tween20, 0.3 mM EDTA, 1 mM DTT);
2) Add 8 μL of PARP7 enzyme per well for the positive control and test compound groups, and 8 μL of buffer per well for the blank control group;
3) Add 2 μL of test compound or positive control per well, and incubate at room temperature for 15 min;
4) Dilute 250 liquid n positive control, incubate at room temperature icBS, 0.05% (BPS, 80610) to 5 dilution with buffer, add 10 μL per well, and incubate at room temperature for 2 h;
5) Wash 3 times with 100 μL PBST buffer, and pat dry.

Step three

1) Dilute Streptavidin-HRP (BPS, 80611) 1:50 with blocking buffer, add 20 μL per well, and incubate at room temperature for 30 min;
2) Wash 3 times with 100 μL PBST buffer, and pat dry;
3) Mix ELISA ECL Substrate A + ELISA ECL Substrate B (BPS, 79670) on ice, and add 40 μL per well;
4) Read the plate on a Spark 10M multi-function microplate reader (Tecan) to test enzyme activity, fit the curve using the 4-parameter method in GraphPad Prism software, and calculate the $IC_{50}$ value.

Inhibition rate (%) calculation formula:

$$\text{Inhibition rate (\%)} = (1 - (\text{Lu sample group} - \text{Lu blank control}) / (\text{Lu positive control} - \text{Lu blank control})) * 100\%.$$

The test results are shown in Table 1.

**Test Example 2: PARP2 Inhibitory Activity Assay**

The *in vitro* activity of the embodiment compounds was demonstrated in the following assay to show that PARP2 can catalyze NAD-dependent ADP-ribosylation. The PARP2 enzyme activity assay method used in the present disclosure employs reagents from the PARP2 Chemiluminescent Activity Assay Kit from BPS Bioscience, USA. The test was performed in a 384-well plate and was divided into three steps, briefly described as follows.

Step one:

1) Dilute the 5x histone mixture 1:5 with PBS, add 20 μL per well, and incubate overnight at 4°C;
2) Wash 3 times with 100 μL PBST (1x PBS, 0.05% Tween-20) per well, and pat dry on paper;
3) Add 80 μL of blocking buffer 3 per well, and incubate at room temperature for 90 minutes;
4) Wash 3 times with 100 μL PBST per well, and pat dry on paper.

Step two:

1) Dilute the 10x PARP test buffer 10-fold with water;
2) Thaw the PARP2 enzyme on ice. Add 8 μL of PARP2 enzyme per well for the positive control and test compound groups, and 8 μL of 1x PARP2 buffer per well for the blank control group;
3) Add 10 μL of substrate per well;
4) Add 2 μL of test compound or positive control;
5) Add 8 μL of 1X PARP2 buffer to the blank;
6) Add 8 μL of diluted PARP2 to the positive control and test wells, and incubate at room temperature for 1 hour;
7) After 1 hour, discard the reaction mixture, wash 3 times with 100 μL PBST buffer, and pat dry.

Step three:

1) Dilute Streptavidin-HRP 1:50 with blocking buffer 3, add 20 μL per well, and incubate at room temperature for 30 minutes;
2) Wash 3 times with 100 μL PBST buffer, and pat dry;
3) Mix ELISA ECL Substrate A + ELISA ECL Substrate B on ice, and add 40 μL per well;
4) Read the plate on a Spark 10M multi-function microplate reader (Tecan) to test enzyme activity, fit the curve using the 4-parameter method in GraphPad Prism software, and calculate the $IC_{50}$ value.

Inhibition rate (%) calculation formula:

$$\text{Inhibition rate (\%)} = (1 - (\text{Lu sample group} - \text{Lu blank control}) / (\text{Lu positive control} - \text{Lu blank control})) * 100\%.$$

The test results are shown in Table 1. /" indicates not tested.
For the $IC_{50}$ value, "++++" indicates an $IC_{50}$ between 0 nM and 10 nM; "+++" indicates an $IC_{50}$ between 10 nM and 100 nM (inclusive of 10 nM); "++" indicates an $IC_{50}$ between 100 nM and 1 μM (inclusive of 100 nM); "+" indicates an $IC_{50}$ between 1 μM and 10 μM (inclusive of 1 μM).

Table 1. *In vitro* biological activity $IC_{50}$ values (nM) of the embodiment compounds

| Embodiments | PARP7 $IC_{50}$(nM) | PARP2 $IC_{50}$(nM) |
|---|---|---|
| 1 | + | / |
| 2 | +++ | / |
| 3 | +++ | / |
| 4 | +++ | / |
| 5 | +++ | / |
| 6 | +++ | / |
| 7 | ++ | / |
| 8 | +++ | / |
| 9 | + | / |
| 10 | +++ | / |
| 11 | ++ | / |
| 12 | +++ | / |
| 13 | +++ | / |
| 14 | +++ | / |
| 16 | + | / |

(continued)

| Embodiments | PARP7 $IC_{50}$(nM) | PARP2 $IC_{50}$(nM) |
|---|---|---|
| 17 | ++ | / |
| 18 | ++ | / |
| 19 | +++ | / |
| 20 | ++ | / |
| 21 | + | / |
| 22 | +++ | / |
| 23 | ++++ | / |
| 24 | +++ | / |
| 25 | ++ | / |
| 26 | +++ | / |
| 27 | +++ | / |
| 28 | +++ | / |
| 29 | +++ | / |
| 30 | +++ | / |
| 31 | +++ | / |
| 32 | +++ | / |
| 33 | + | / |
| 34 | +++ | / |
| 35 | +++ | / |
| 36 | +++ | / |
| 37 | +++ | / |
| 38 | +++ | / |
| 39 | +++ | / |
| 40 | +++ | / |
| 41 | +++ | / |
| 42 | +++ | / |
| 43 | +++ | / |
| 44 | +++ | / |
| 45 | +++ | / |
| 46 | ++++ | +++ |
| 47 | +++ | / |
| 48 | ++++ | +++ |
| 49 | +++ | / |
| 50 | +++ | / |
| 51 | ++ | / |
| 52 | +++ | / |
| 53 | +++ | / |
| 54 | ++ | / |
| 55 | + | / |

(continued)

| Embodiments | PARP7 $IC_{50}$(nM) | PARP2 $IC_{50}$(nM) |
|---|---|---|
| 56 | +++ | / |
| 57 | ++++ | +++ |
| 58 | +++ | / |
| 59 | +++ | / |
| 60 | ++++ | ++ |
| 61 | ++++ | ++ |
| 62 | ++++ | +++ |
| 63 | ++++ | ++ |
| 64 | ++++ | ++ |
| 65 | ++++ | ++ |
| 66 | +++ | / |
| 67 | ++++ | ++ |
| 68 | ++++ | / |

**Test Example 3: Liver Microsomal Stability Study**

The above compounds were diluted to a 1.5 μM intermediate working solution with 0.1 M potassium phosphate buffer containing 0.75 mg/mL liver microsomes, and then 30 μL of the intermediate working solution was dispensed into reaction plates for different time points (0, 5, 15, 30, 45, 60 min). 150 μL of methanol solution containing internal standard was added to the 0 min plate wells. For the other time points (5, 15, 30, 45, 60 min), the sample plates were pre-incubated at 37°C for 5 min, after which the reaction was initiated with 15 μL of 6 mM NADPH solution and timing was started. 15 μL of 6 mM NADPH solution was added to the 0 min samples to balance the system. At 5 min, 15 min, 30 min, 45 min, and 60 min, respectively, 150 μL of methanol solution containing internal standard was added to the reaction wells to terminate the reaction. After the reaction was complete, the plates were shaken on a shaker at 600 rpm for 10 min, then centrifuged at 4000 rpm for 15 min. 80 μL of supernatant from each well was transferred to a 96-well sample plate containing 150 μL of ultrapure water, mixed, and then analyzed by LC/MS to calculate $T_{1/2}$. The results are shown in Table 2.

For the $T_{1/2}$ value, "+++" indicates a $T_{1/2}$ between 0 min and 30 min (inclusive of 30 min); "++" indicates a $T_{1/2}$ between 30 min and 60 min (inclusive of 60 min); "+" indicates a $T_{1/2}$ greater than 60 min.

The embodiment compounds have a long half-life ($T_{1/2}$) and good stability in liver microsomes of different species, especially in human liver microsomes. For some compounds in human liver microsomes, the $T_{1/2}$ is between 30 min and 60 min (inclusive of 60 min), and for some compounds, the $T_{1/2}$ is even greater than 60 min.

Table 2. Liver Microsomal $T_{1/2}$ (minute) of the Embodiment Compounds

| Compound | Liver Microsome Species | $T_{1/2}$ (minute) |
|---|---|---|
| Embodiment 3 | Human | + |
| | Rat | + |
| | Mouse | + |
| Embodiment 4 | Human | ++ |
| | Rat | + |
| | Mouse | + |
| Embodiment 5 | Human | + |
| | Rat | + |
| | Mouse | + |

(continued)

| Compound | Liver Microsome Species | $T_{1/2}$ (minute) |
|---|---|---|
| Embodiment 12 | Human | + |
| | Rat | + |
| | Mouse | + |
| Embodiment 13 | Human | ++ |
| | Rat | + |
| | Mouse | + |
| Embodiment 14 | Human | + |
| | Rat | + |
| | Mouse | + |
| Embodiment 61 | Human | + |
| | Rat | + |
| | Mouse | + |
| Embodiment 63 | Human | + |
| | Rat | + |
| | Mouse | + |

**Test Example 4: Mouse Pharmacokinetic Study**

(1) Study design: Female healthy Balb/c mice (specific pathogen-free (SPF) grade) were used as test animals, divided into 2 dose groups with 3 mice in each group, for single intravenous injection and single oral gavage administration, respectively. The vehicle formulations were 5% dimethyl sulfoxide (DMSO)+10% Solutol+85% Saline and 0.5% methylcellulose (MC), respectively.

(2) Plasma sample collection and processing: Blood was collected from the orbital venous plexus of the mice. After intravenous (IV) bolus administration, samples were collected at 0.083 h, 0.25 h, 0.5 h, 1 h, 2 h, 4 h, 8 h, and 24 h. After oral (PO) administration, samples were collected at 0.25 h, 0.5 h, 1 h, 2 h, 4 h, 6 h, 8 h, and 24 h. An appropriate volume of whole blood samples was collected with EDTA-K2 anticoagulation, placed on wet ice after collection, and plasma was separated by centrifugation within 30 min (centrifugation conditions: 4000 rpm, 10 minutes, 2-8°C). The collected plasma samples were stored in a -70°C freezer before analysis. After analysis, the remaining plasma samples continued to be stored in a -70°C freezer.

(3) Analytical batch processing and pharmacokinetic parameter calculation: The concentration of the test substance in the plasma samples was determined. The accuracy of quality control samples was evaluated concurrently with the analysis of the samples, with the requirement that more than 66.7% of the quality control samples have an accuracy between 80-120%. Using the plasma concentration data at different time points, pharmacokinetic parameters such as Co (IV), Area Under the Curve $(AUC)_{(0-t)}$, $T_{1/2}$, $C_{max}$, $T_{max}$, and Mean Residence Time (MRT) were calculated using WinNonlin. The pharmacokinetic parameters of the drugs in mice after intravenous injection and oral administration are shown in Table 3.

able 3. Pharmacokinetic parameters of representative compounds of the present invention in mice

| Embodiments | Species | Route of Administration | Dose | Vehicle Formulation | $C_{max}$ | $AUC_{(0-t)}$ | $T_{1/2}$ | $T_{max}$ | F |
|---|---|---|---|---|---|---|---|---|---|
| | | | mg/kg | | ng/mL | h*ng/mL | h | h | % |
| Embodiment 5 | BALB/c | IV | 1 | 5%DMA+10%solutol+ 85%Saline | 4208.88 | 7899.92 | 1.28 | 0.08 | - |
| | | PO | 10 | 0.5%MC | 21993.49 | 52136.41 | 0.98 | 0.33 | 66.00 |
| Embodiment 12 | BALB/c | IV | 1 | 5%DMSO+10%solutol +85%Saline | 1199.93 | 1792.72 | 0.89 | 0.08 | - |
| | | PO | 10 | 0.5%MC | 7060.19 | 10657.54 | 0.81 | 0.25 | 59.45 |

The foregoing description of specific exemplary embodiments of the present invention is for the purpose of illustration and exemplification. These descriptions are not intended to limit the present invention to the precise forms disclosed, and it is apparent that many modifications and variations are possible in light of the above teachings. The exemplary embodiments are chosen and described in order to explain the particular principles of the present invention and their practical application, thereby enabling those skilled in the art to realize and utilize the various exemplary embodiments of the present invention and various choices and modifications. The scope of the present invention is intended to be limited by the claims and their equivalents.

## Claims

1. A compound represented by formula (I), a pharmaceutically acceptable salt, stereoisomer, solvate, or prodrug thereof,

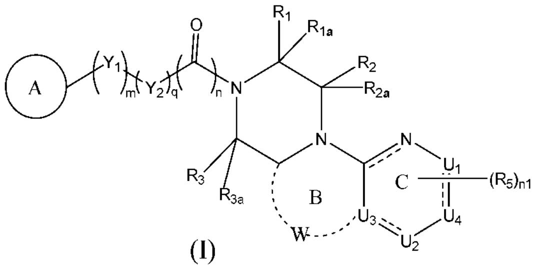

(I)

wherein, in formula (I),
Ring A is selected from

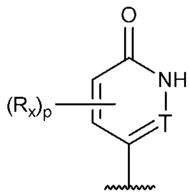
,
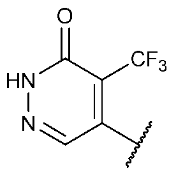
, or
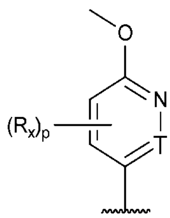
;

T is selected from CH or N;
$R_x$ is selected from hydrogen, deuterium, halogen, cyano, $C_{1-6}$ alkyl, $C_{3-7}$ cycloalkyl, $C_{1-6}$ alkoxy, $C_{3-7}$ cycloalkoxy, $C_{2-6}$ alkenyl, $C_{2-6}$ alkynyl, $C_{1-6}$ haloalkyl, $-NO_2$, $-NH_2$, wherein the $C_{1-6}$ alkyl, $C_{3-7}$ cycloalkyl, $C_{1-6}$ alkoxy, $C_{3-7}$ cycloalkoxy, $C_{2-6}$ alkenyl, $C_{2-6}$ alkynyl, $C_{1-6}$ haloalkyl are optionally substituted with one or more hydrogen, deuterium, methyl, ethyl, propyl, or isopropyl;
p is selected from 0, 1, or 2;
Ring A is selected from

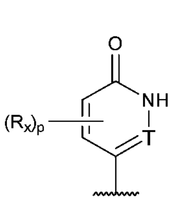
or
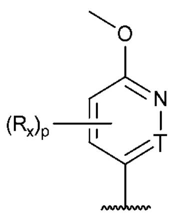
,

$Y_1$ is selected from $-C(R_6)(R_7)-$, m is selected from 1, 2, or 3, q is selected from 1, n is selected from 1, wherein $R_6$, $R_7$ are each independently selected from hydrogen, deuterium, $C_{1-3}$ alkyl, or $R_6$, $R_7$ together with the carbon atom to which they are attached form a 3-6 membered cycloalkyl;
or Ring A is selected from

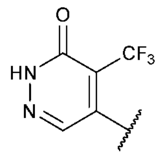

,

$Y_1$ is selected from $-N(R_6)-C_{1-3}$ alkyl-, m is selected from 1, q is selected from 1, n is selected from 1, wherein $R_6$ is selected from hydrogen or deuterium, any two of $R_1$, $R_2$, $R_3$, $R_4$ are optionally connected to each other to form $-(CH_2)_{n2}-$, wherein $n_2$ is selected from 1, 2, or 3;

$Y_2$ is selected from $-N(R_a)-C_{3-7}$ cycloalkyl-, $-N(R_a)-C_{6-10}$ aryl-, a 3-10 membered nitrogen-containing heterocycloalkyl containing at least one nitrogen atom, or a 7-10 membered nitrogen-containing spirocycloalkyl containing at least one nitrogen atom, wherein $R_a$ is selected from hydrogen, deuterium, or $C_{1-3}$ alkyl, and wherein the $-N(R_a)-C_{3-7}$ cycloalkyl-, $-N(R_a)-C_{6-10}$ aryl-, 3-10 membered nitrogen-containing heterocycloalkyl containing at least one nitrogen atom, 7-10 membered nitrogen-containing spirocycloalkyl containing at least one nitrogen atom, and $C_{1-3}$ alkyl are optionally substituted with one or more hydrogen, deuterium, methyl, ethyl, propyl, or isopropyl;

Ring B is absent, and the group

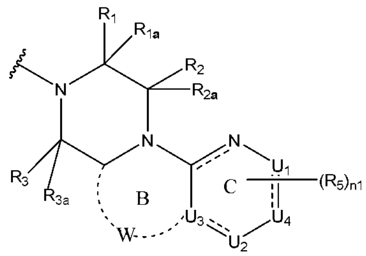

is

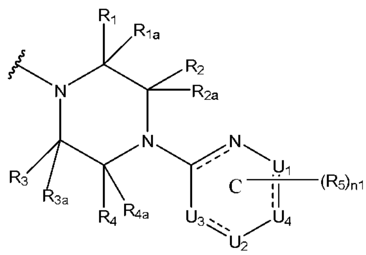

;

or Ring B is a 5-8 membered saturated or unsaturated heterocycle, wherein the Ring B is optionally substituted with one or more halogen, hydroxyl, $C_{1-6}$ alkyl, or $C_{1-6}$ alkoxy, and wherein the $C_{1-6}$ alkyl and $C_{1-6}$ alkoxy are optionally substituted with one or more hydrogen, deuterium, methyl, ethyl, propyl, or isopropyl;

W is selected from C, N, O, or S;

$U_1$, $U_2$, $U_3$, and $U_4$ are each independently selected from CH, $CH_2$, N, or NH;

the halogen is selected from fluorine, chlorine, or bromine;

$R_1$, $R_{1a}$, $R_2$, $R_{2a}$, $R_3$, $R_{3a}$, $R_4$, $R_{4a}$ are each independently selected from hydrogen, deuterium, halogen, hydroxyl, amino, nitro, cyano, $C_{1-6}$ alkyl, wherein the $C_{1-6}$ alkyl is optionally substituted with one or more halogen, hydroxyl, cyano, carboxyl, or $C_{1-3}$ alkyl, or any two of $R_1$, $R_2$, $R_3$, $R_4$ are optionally connected to each other to form $-(CH_2)_{n2}-$, wherein $n_2$ is selected from 1, 2, or 3, or $R_1$ and $R_{1a}$ together form an oxo, or $R_2$ and $R_{2a}$ together form an oxo, or $R_3$ and $R_{3a}$ together form an oxo, or $R_4$ and $R_{4a}$ together form an oxo;

$n_1$ $R_5$ are the same or different from each other, $n_1$ is selected from 0, 1, 2, or 3, $R_5$ is selected from hydrogen, deuterium, halogen, cyano, $C_{1-6}$ alkyl, $C_{1-6}$ haloalkyl, $C_{3-7}$ cycloalkyl, $C_{1-6}$ alkoxy, $C_{3-7}$ cycloalkoxy, $C_{2-6}$ alkenyl, $C_{2-6}$ alkynyl, $C_{1-3}$ amido, aminoacyl, 4-6 membered cycloaminoacyl, 4-6 membered saturated or unsaturated heterocyclic group, sulfonamido, 4-6 membered heterocycloamino, or two $R_5$ substituents on adjacent carbon atoms of Ring C, together with the carbon atoms to which they are attached, form a 4-6 membered saturated or unsaturated heterocyclic group, wherein the $C_{1-6}$ alkyl, $C_{1-6}$ haloalkyl, $C_{3-7}$ cycloalkyl, $C_{1-6}$ alkoxy, $C_{3-7}$ cycloalkoxy, $C_{2-6}$ alkenyl, $C_{2-6}$ alkynyl, $C_{1-3}$ amido, aminoacyl, 4-6 membered cycloaminoacyl, 4-6 membered saturated or unsaturated heterocyclic group, sulfonamido, and 4-6 membered heterocycloamino are optionally

substituted with one or more halogen, hydroxyl, or $C_{2-6}$ ester group.

**2.** A compound represented by formula (I'), a pharmaceutically acceptable salt, stereoisomer, solvate, or prodrug thereof,

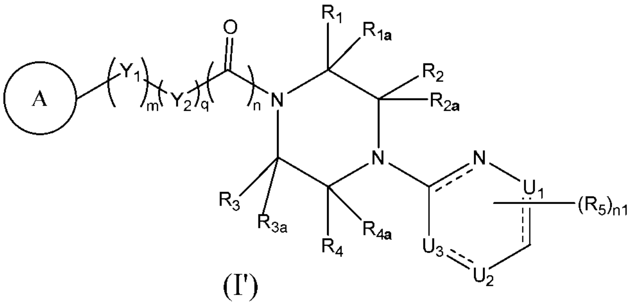

(I')

wherein, in formula (I'),
Ring A is selected from

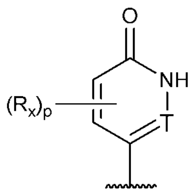

or

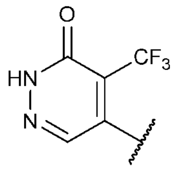

;

T is selected from CH or N;
$R_x$ is selected from hydrogen, deuterium, halogen, cyano, $C_{1-6}$ alkyl, $C_{1-6}$ alkoxy, $C_{2-6}$ alkenyl, $C_{2-6}$ alkynyl, $C_{1-6}$ haloalkyl, $-NO_2$, or $-NH_2$;
p is selected from 0, 1, or 2;
$Y_2$ is selected from $-N(R_a)-C_{3-7}$ cycloalkyl-, $-N(R_a)-C_{6-10}$ aryl-, a 3-10 membered nitrogen-containing heterocycloalkyl containing at least one nitrogen atom, or a 7-10 membered nitrogen-containing spirocycloalkyl containing at least one nitrogen atom, wherein $R_a$ is selected from hydrogen or deuterium;
$U_1$, $U_2$, and $U_3$ are each independently selected from CH, $CH_2$, N, or NH; the halogen is selected from fluorine, chlorine, or bromine;
$R_1$, $R_{1a}$, $R_2$, $R_{2a}$, $R_3$, $R_{3a}$, $R_4$, $R_{4a}$ are each independently selected from hydrogen, deuterium, halogen, hydroxyl, amino, nitro, cyano, $C_{1-6}$ alkyl, wherein the $C_{1-6}$ alkyl is optionally substituted with one or more halogen, hydroxyl, cyano, carboxyl, or $C_{1-3}$ alkyl, or any two of $R_1$, $R_2$, $R_3$, $R_4$ are optionally connected to each other to form $-(CH_2)_{n2}-$, wherein $n_2$ is selected from 1, 2, or 3, or $R_1$ and $R_{1a}$ together form an oxo, or $R_2$ and $R_{2a}$ together form an oxo, or $R_3$ and $R_{3a}$ together form an oxo, or $R_4$ and $R_{4a}$ together form an oxo;
$n_1$ $R_5$ are the same or different from each other, $n_1$ is selected from 0, 1, 2, or 3, $R_5$ is selected from hydrogen, deuterium, halogen, cyano, $C_{1-6}$ alkyl, $C_{1-6}$ alkoxy, $C_{2-6}$ alkenyl, or $C_{2-6}$ alkynyl, wherein the $C_{1-6}$ alkyl, $C_{1-6}$ alkoxy, $C_{2-6}$ alkenyl, and $C_{2-6}$ alkynyl are optionally substituted with one or more halogen, hydroxyl, $C_{2-6}$ ester group, $C_{1-3}$ amido, or sulfonamido;
the $C_{1-6}$ alkyl, $C_{1-6}$ alkoxy, $C_{2-6}$ alkenyl, $C_{2-6}$ alkynyl, $C_{1-6}$ haloalkyl, $C_{1-3}$ alkyl, $C_{3-7}$ cycloalkyl, $C_{6-10}$ aryl, 3-10 membered nitrogen-containing heterocycloalkyl containing at least one nitrogen atom, 7-10 membered nitrogen-containing spirocycloalkyl containing at least one nitrogen atom, $C_{2-6}$ ester group, and $C_{1-3}$ amido are optionally substituted with one or more hydrogen, deuterium, methyl, ethyl, propyl, or isopropyl;
when Ring A is selected from

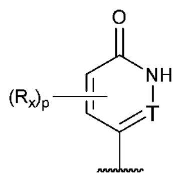

,

$Y_1$ is selected from $-C(R_6)(R_7)-$, m is selected from 0, 1, 2, or 3, q is selected from 0 or 1, n is selected from 0 or 1, and m, n, and q are not 0 at the same time, wherein $R_6$, $R_7$ are each independently selected from hydrogen or deuterium;

when Ring A is selected from

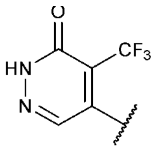,

$Y_1$ is selected from $-N(R_6)-C_{1-3}$ alkyl-, m is selected from 1, q is selected from 1, n is selected from 1, wherein $R_6$ is selected from hydrogen or deuterium, and any two of $R_1$, $R_2$, $R_3$, $R_4$ are optionally connected to each other to form $-(CH_2)_{n2}-$, wherein $n_2$ is selected from 1, 2, or 3.

**3.** The compound, pharmaceutically acceptable salt, stereoisomer, solvate, or prodrug thereof according to claim 1, wherein,

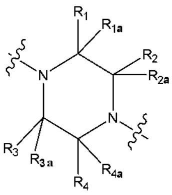

is selected from

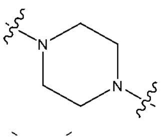, 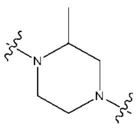, 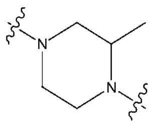, 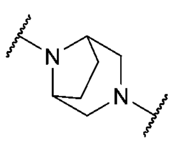, 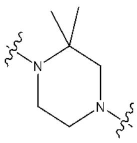,

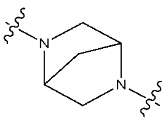, 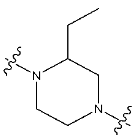, 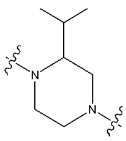, 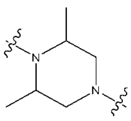, 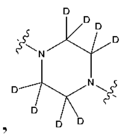, 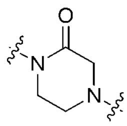, 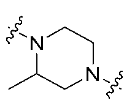, 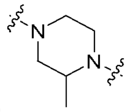,

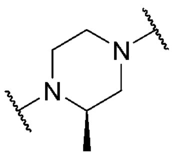 or 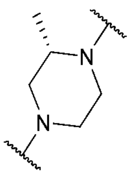;

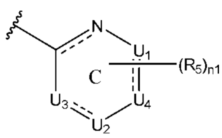

is selected from

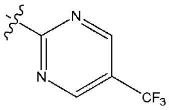, 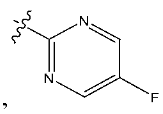, 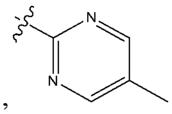, 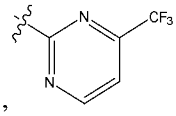

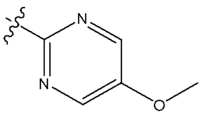, 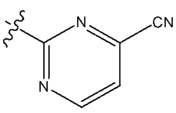, 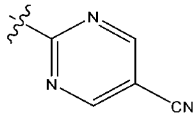, 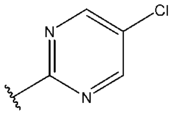, 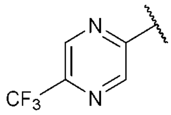, 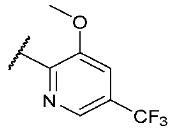,

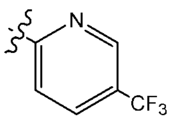 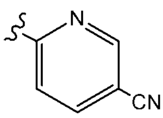 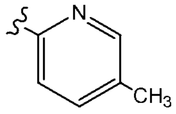, 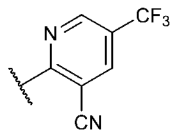, 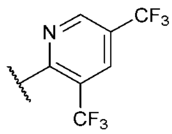, 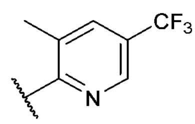,

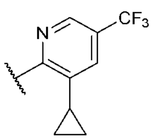, 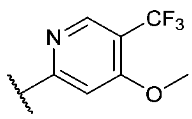, 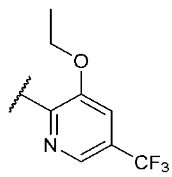, 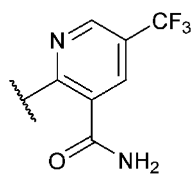, 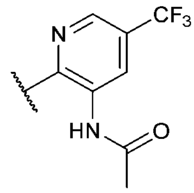,

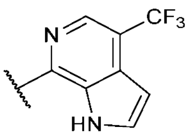, 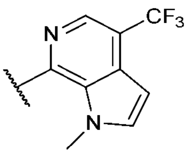, 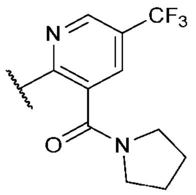, 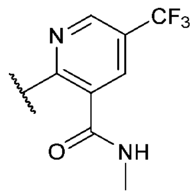, 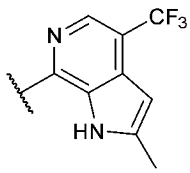,

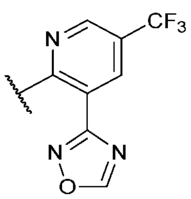, 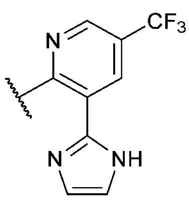, 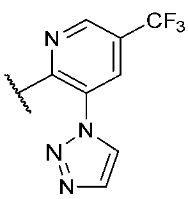, 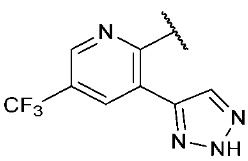, 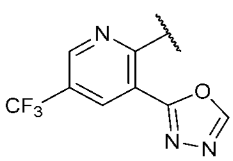,

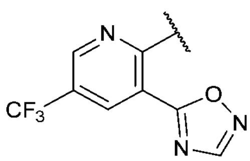, 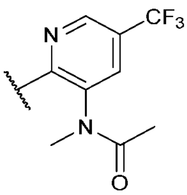, 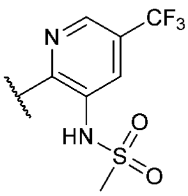, 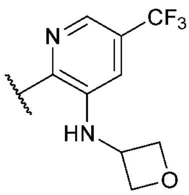, or 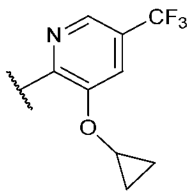;

Ring B is present, and

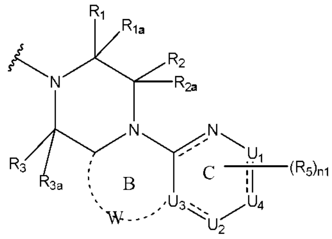

is selected from

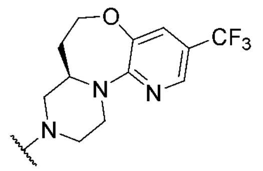,

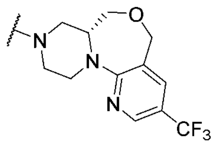, 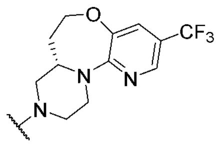, 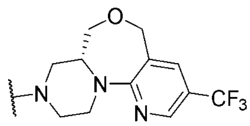, 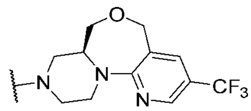, or

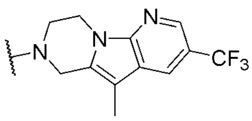;

W is selected from C or O.

**4.** The compound, pharmaceutically acceptable salt, stereoisomer, solvate, or prodrug thereof according to any one of claims 1-3, wherein,

Ring A is selected from

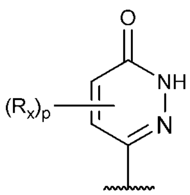, 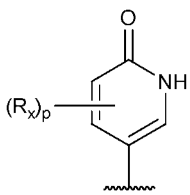, 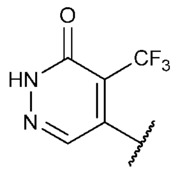, or 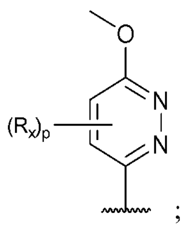;

$R_x$ is selected from hydrogen, deuterium, $C_{1-6}$ alkoxy, $C_{3-7}$ cycloalkoxy, $C_{1-6}$ haloalkyl, wherein the $C_{1-6}$ alkoxy, $C_{3-7}$ cycloalkoxy, $C_{2-6}$ alkenyl, $C_{2-6}$ alkynyl, and $C_{1-6}$ haloalkyl are optionally substituted with one or more hydrogen, deuterium, methyl, ethyl, propyl, or isopropyl;
p is selected from 1 or 2;
Ring A is selected from

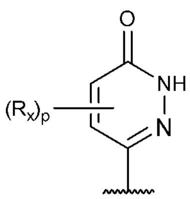, 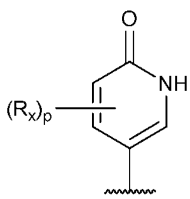, or 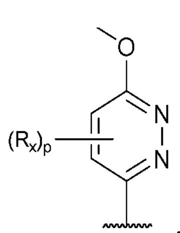, $Y_1$ is selected from $-C(R_6)(R_7)-$, m is selected from 1, q is selected from 1, n is selected from 1, wherein $R_6$, $R_7$ are each independently selected from hydrogen, deuterium, methyl, or $R_6$, $R_7$ together with the carbon atom to which they are attached form a 3-6 membered cycloalkyl;
or Ring A is selected from

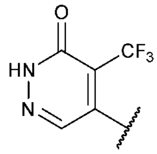,

$Y_1$ is selected from $-N(R_6)-C_{1-3}$ alkyl-, m is selected from 1, q is selected from 1, n is selected from 1, wherein $R_6$ is selected from hydrogen or deuterium, and any two of $R_1$, $R_2$, $R_3$, $R_4$ are optionally connected to each other to form $-(CH_2)_{n2}-$, wherein $n_2$ is selected from 1 or 2;
$Y_2$ is selected from $-N(R_a)-C_{3-7}$ cycloalkyl-, $-N(R_a)-C_{6-10}$ aryl-, a 3-10 membered nitrogen-containing heterocycloalkyl containing at least one nitrogen atom, or a 7-10 membered nitrogen-containing spirocycloalkyl containing at least one nitrogen atom, wherein $R_a$ is selected from hydrogen, deuterium, or $C_{1-3}$ alkyl, and wherein the $-N(R_a)-C_{3-7}$ cycloalkyl-, $-N(R_a)-C_{6-10}$ aryl-, 3-10 membered nitrogen-containing heterocycloalkyl containing at least one nitrogen atom, 7-10 membered nitrogen-containing spirocycloalkyl containing at least one nitrogen atom, and $C_{1-3}$ alkyl are optionally substituted with one or more hydrogen, deuterium, methyl, ethyl, propyl, or isopropyl;
$U_1$, $U_2$, $U_3$, and $U_4$ are each independently selected from CH, $CH_2$, N, or NH;
the halogen is selected from fluorine or chlorine;
$R_1$, $R_{1a}$, $R_2$, $R_{2a}$, $R_3$, $R_{3a}$, $R_4$, $R_{4a}$ are each independently selected from hydrogen, deuterium, $C_{1-6}$ alkyl, or any two of $R_1$, $R_2$, $R_3$, $R_4$ are optionally connected to each other to form $-(CH_2)_{n2}-$, wherein $n_2$ is selected from 1 or 2;
$n_1$ $R_5$ are the same or different from each other, $n_1$ is selected from 0, 1, 2, or 3, $R_5$ is selected from hydrogen, deuterium, halogen, cyano, $C_{1-6}$ alkyl, $C_{1-6}$ haloalkyl, $C_{3-7}$ cycloalkyl, $C_{1-6}$ alkoxy, $C_{3-7}$ cycloalkoxy, $C_{1-3}$ amido, aminoacyl, 4-6 membered cycloaminoacyl, 4-6 membered saturated or unsaturated heterocyclic group, sulfonamido, 4-6 membered heterocycloamino, or two $R_5$ substituents on adjacent carbon atoms of Ring C, together with the carbon atoms to which they are attached, form a 4-6 membered saturated or unsaturated heterocyclic group, wherein the $C_{1-6}$ alkyl, $C_{1-6}$ haloalkyl, $C_{3-7}$ cycloalkyl, $C_{1-6}$ alkoxy, $C_{3-7}$ cycloalkoxy, $C_{1-3}$ amido, aminoacyl, 4-6 membered cycloaminoacyl, 4-6 membered saturated or unsaturated heterocyclic group, sulfonamido, and 4-6 membered heterocycloamino are optionally substituted with one or more halogen or hydroxyl.

**5.** The compound, pharmaceutically acceptable salt, stereoisomer, solvate, or prodrug thereof according to any one of claims 1-4, wherein,

Ring A is selected from

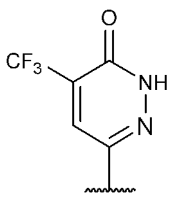, 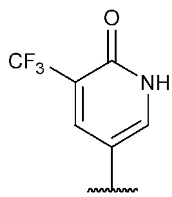, 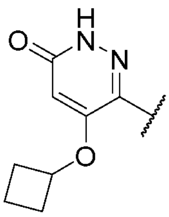, or 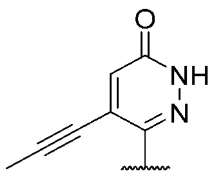,

and $Y_1$ is selected from $-CH_2-$, $-CH(CH_3)-$, $-C(CH_3)_2-$, or

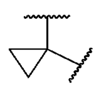;

or Ring A is selected from

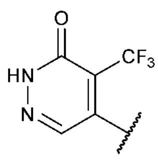,

$Y_1$ is selected from $-NH-CH(CH_3)\,CH_2-$, and any two of $R_1$, $R_2$, $R_3$, $R_4$ are optionally connected to each other to form $-(CH_2)_{n2}-$, wherein $n_2$ is selected from 1 or 2;
$Y_2$ is selected from

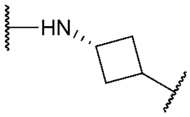, 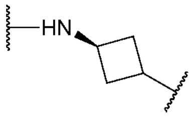, 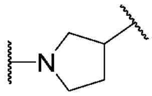, 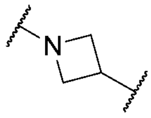, 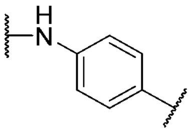,

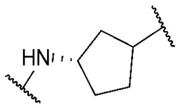, 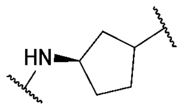, 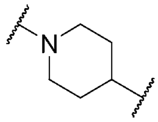, 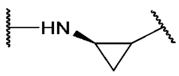, 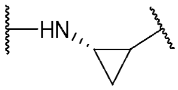, 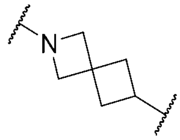,

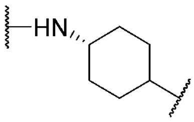, 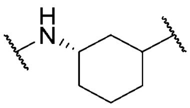, 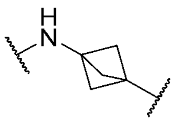, 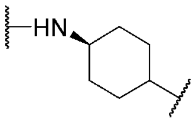, 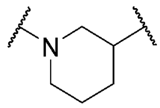, or

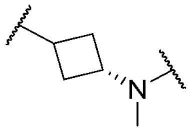;

Ring B is absent, and the group

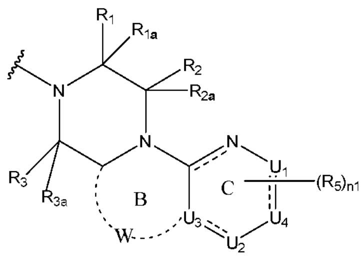

is selected from

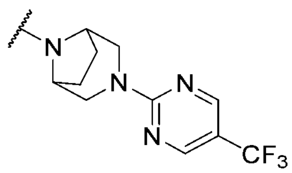,

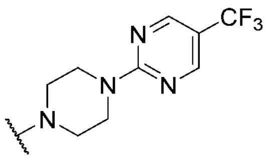, 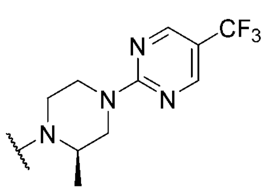, 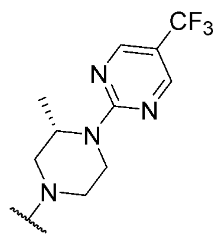, 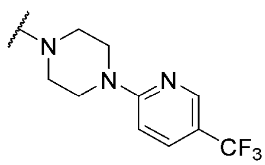,

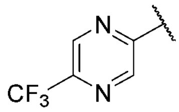 , 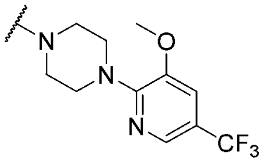 , 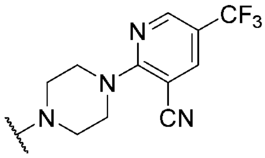 , 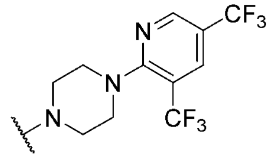 ,

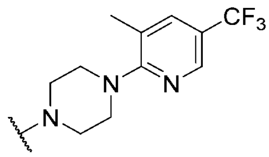 , 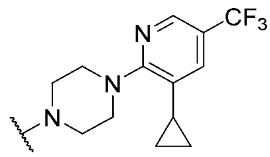 , 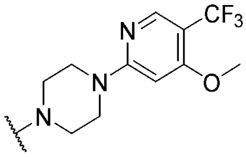 , 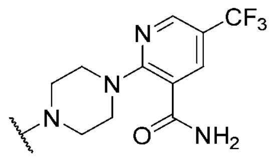 ,

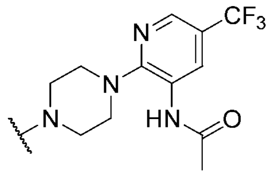 , 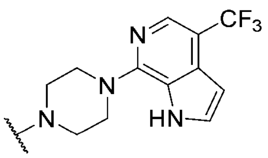 , 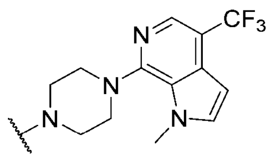 , 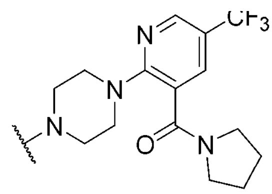 ,

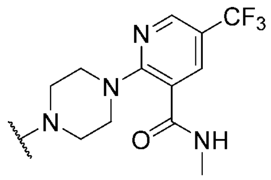 , 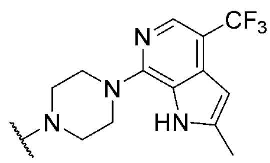 , 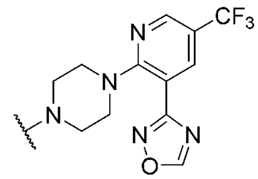 , 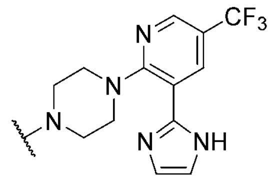 ,

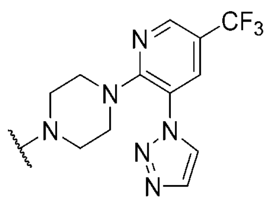 , 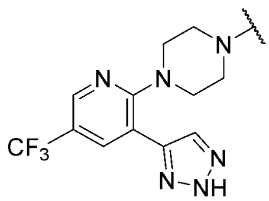 , 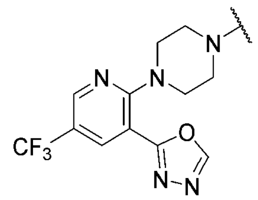 , 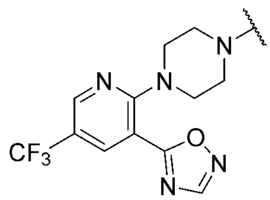 ,

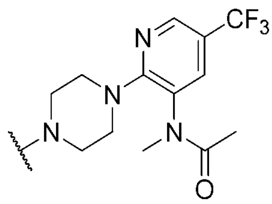 , 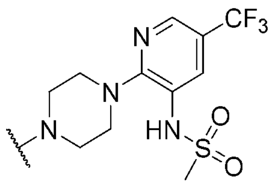 , 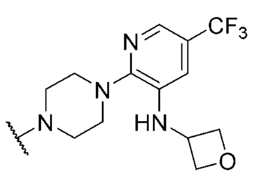 , or 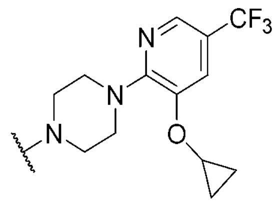 ;

or Ring B is a 5-8 membered saturated or unsaturated heterocycle, and the group

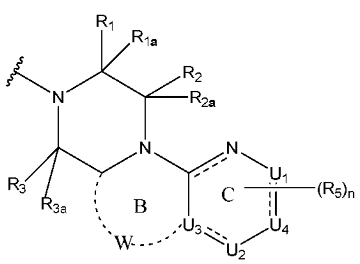

is selected from

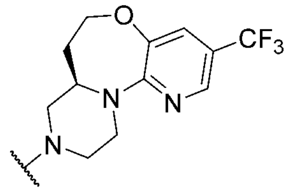 , 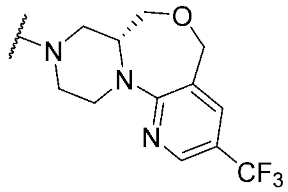 ,

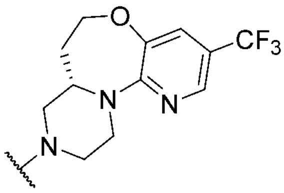 , 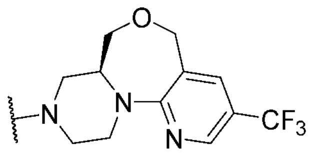 , or 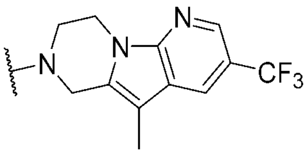 .

6. The compound, pharmaceutically acceptable salt, stereoisomer, solvate, or prodrug thereof according to any one of claims 1-5, which is a compound represented by formula (II), formula (III), formula (IV), or formula (V):

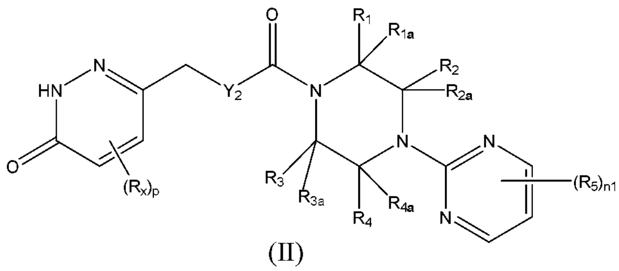

(II)

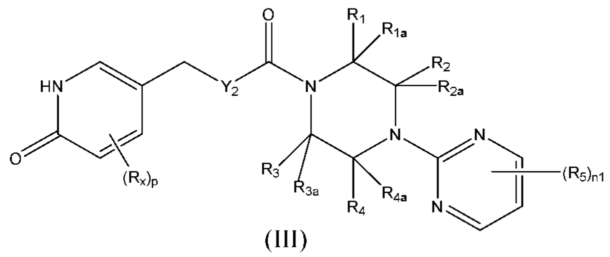

(III)

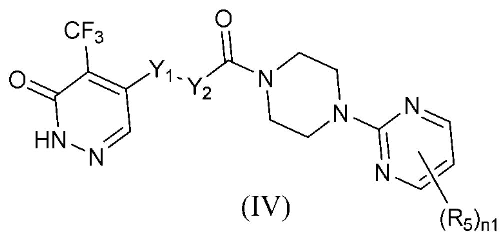

(IV)

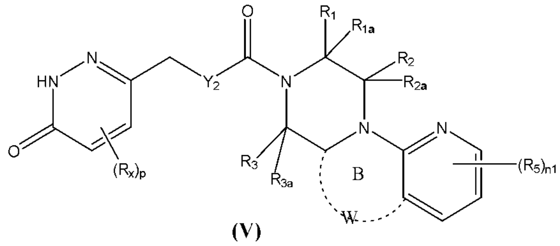

(V)

wherein $R_x$, p, $Y_1$, $Y_2$, $n_1$, $R_1$, $R_{1a}$, $R_2$, $R_{2a}$, $R_3$, $R_{3a}$, $R_4$, $R_{4a}$, $R_5$, W, and Ring B are as defined in any one of claims 1-5.

**7.** The following compounds, a pharmaceutically acceptable salt, stereoisomer, solvate, or prodrug thereof,

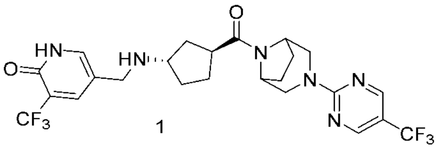
1

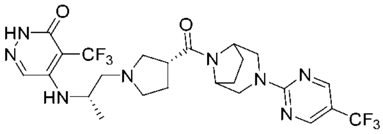
2

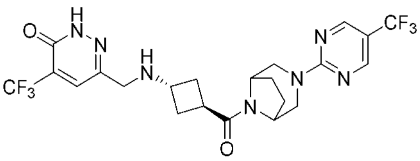
3

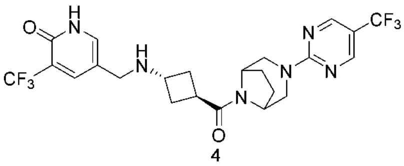
4

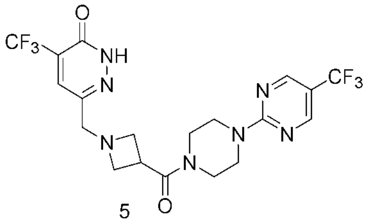
5

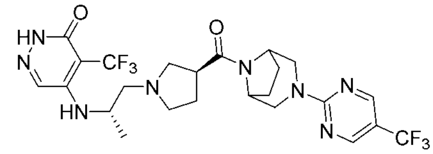
6

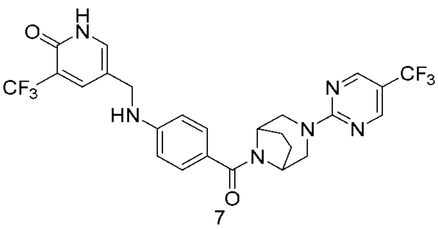
7

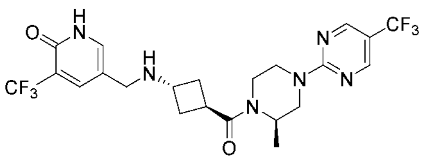
8

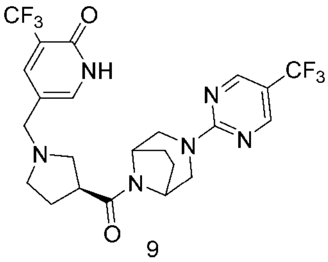
9

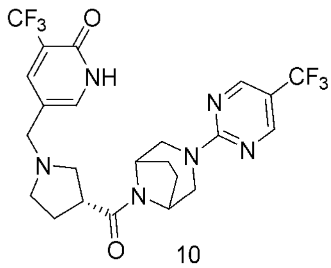
10

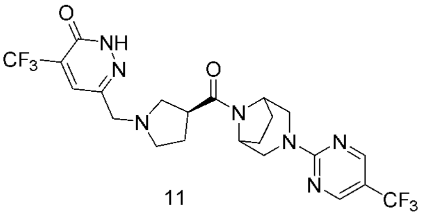
11

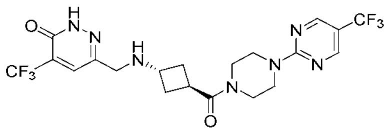
12

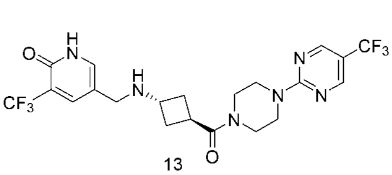
13

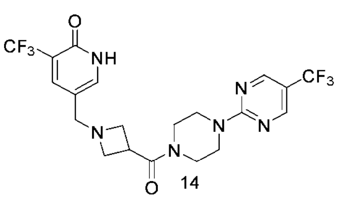
14

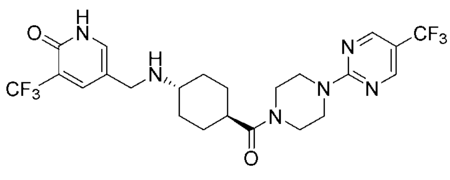
15

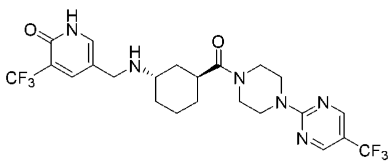

16

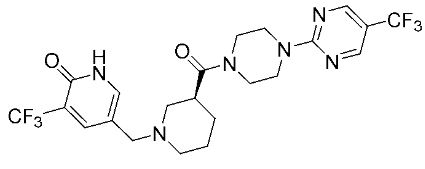

17

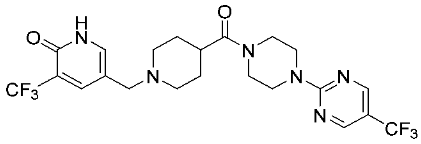

18

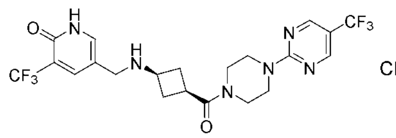

19

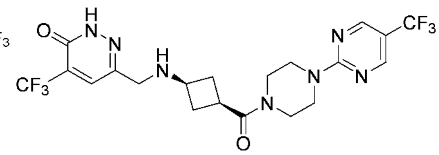

20

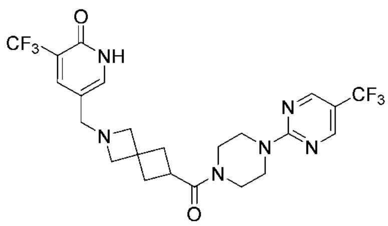

21

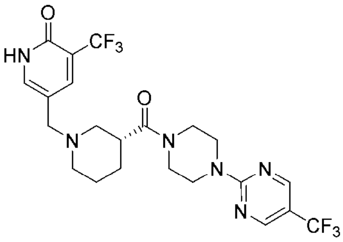

22

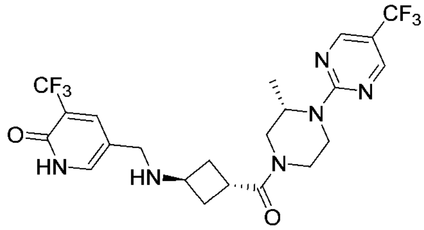

23

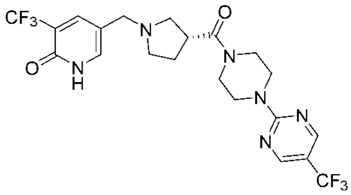

24

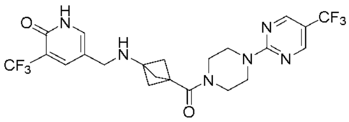

25

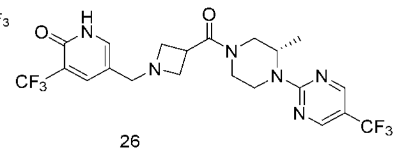

26

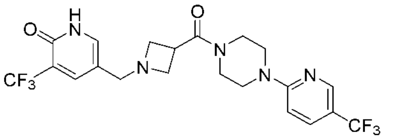

27

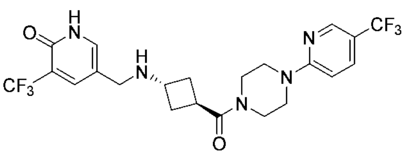

28

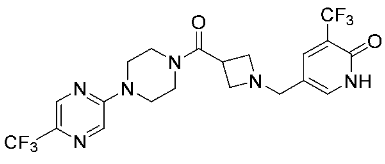

29

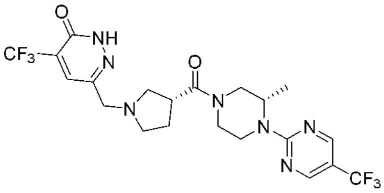

30

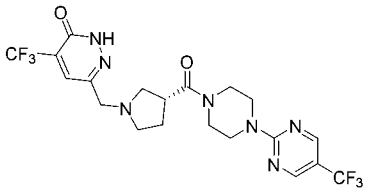
31

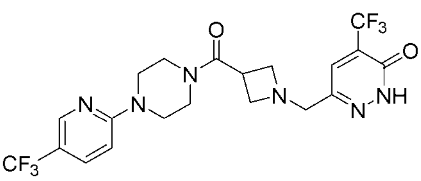
32

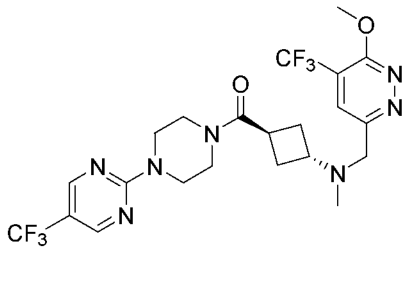
33

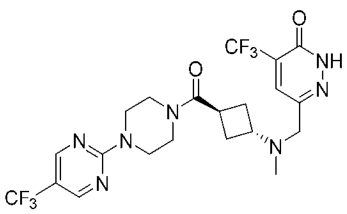
34

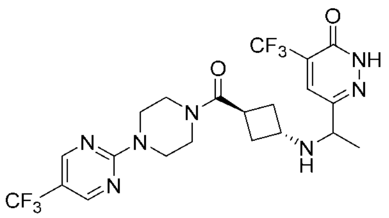
35-P1

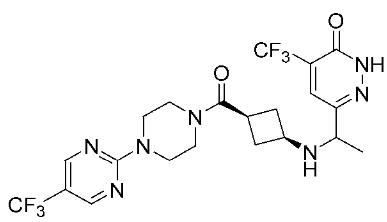
35-P2

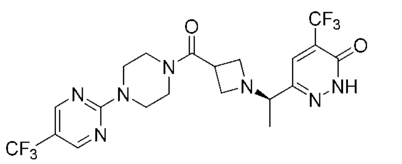
36-P1

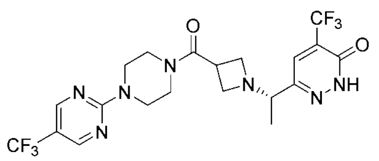
36-P2

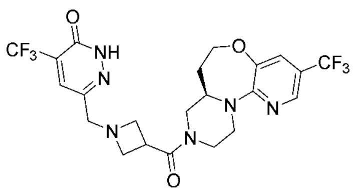
37

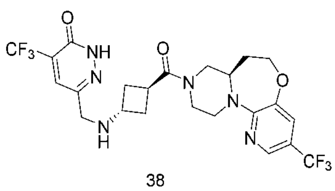
38

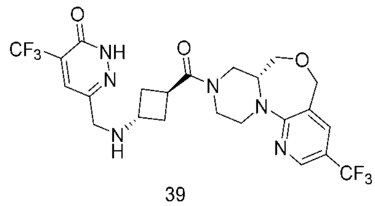
39

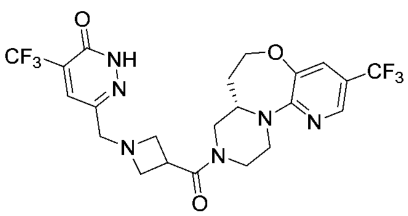
40

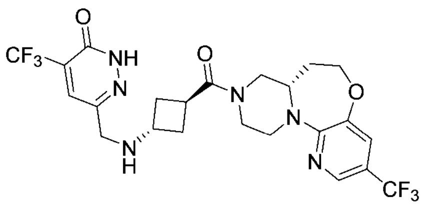
41

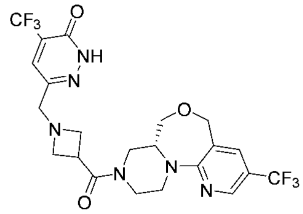
42

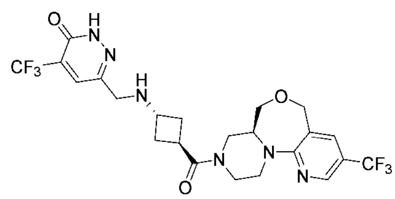
43

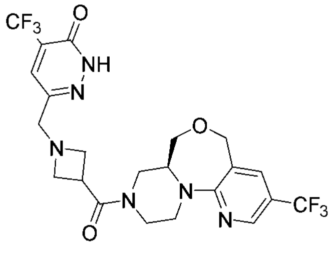

44

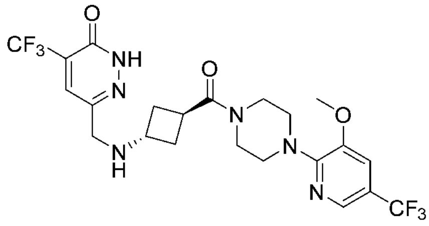

45

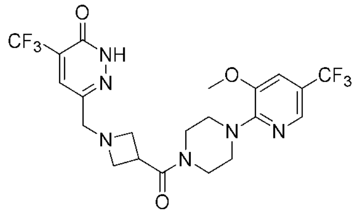

46

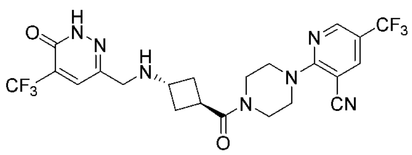

47

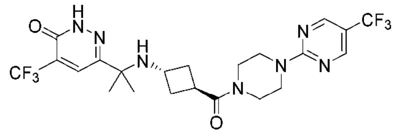

48

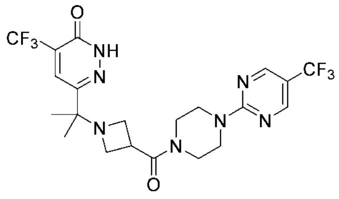

49

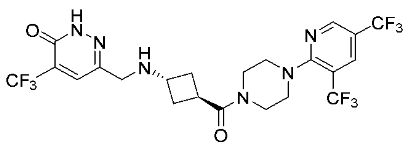

50

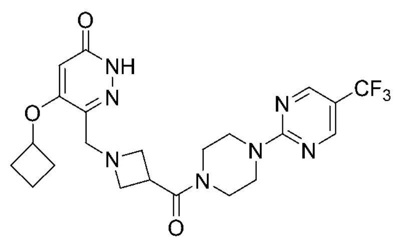

51

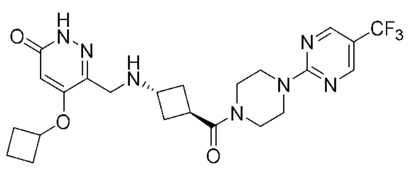

52

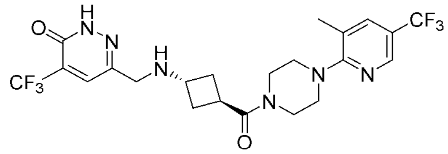

53

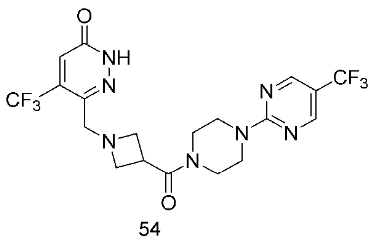

54

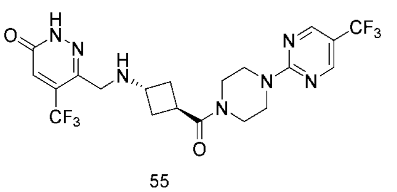

55

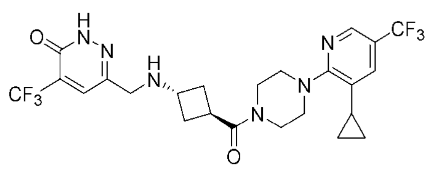

56

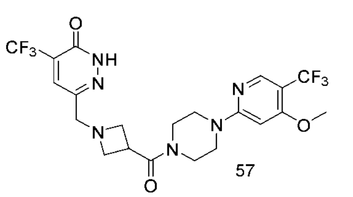

57

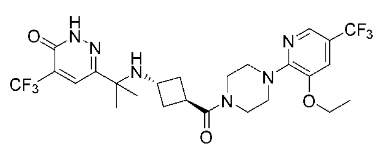

58

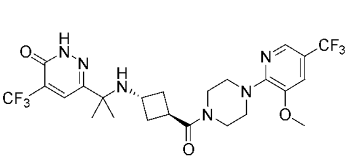

59

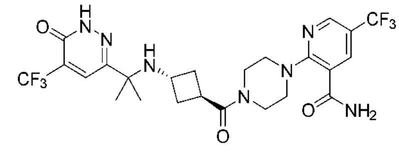

60

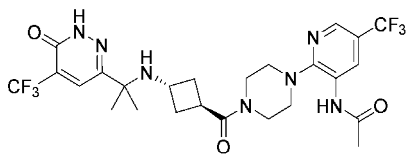

61

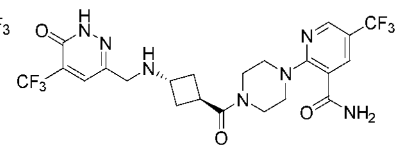

62

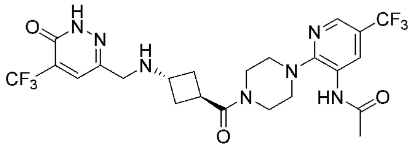

63

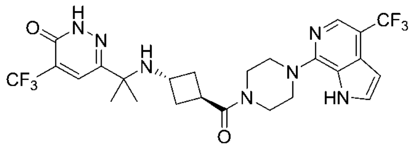

64

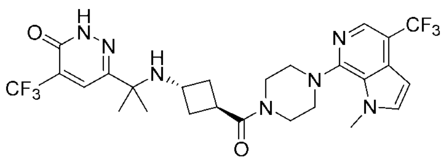

65

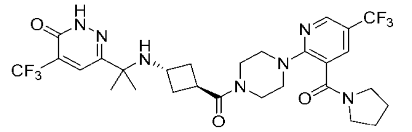

66

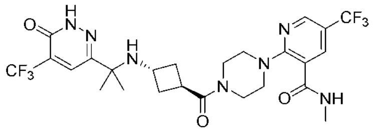

67

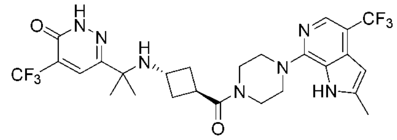

68

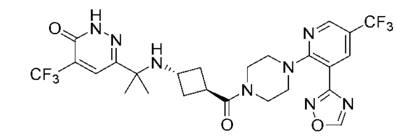

69

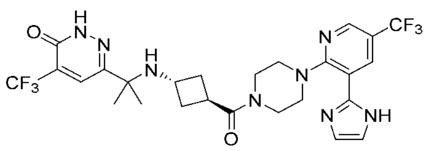

70

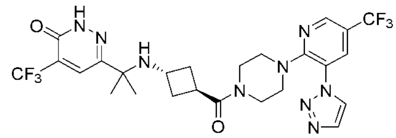

71

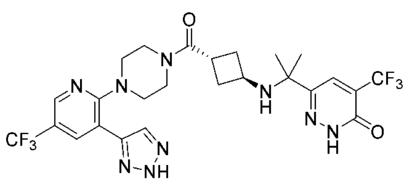

72

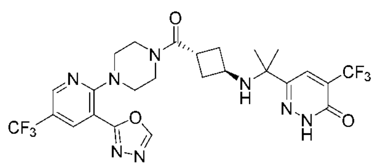

73

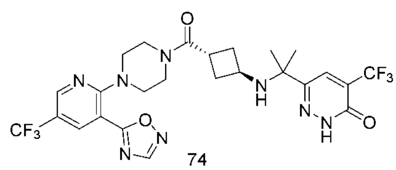

74

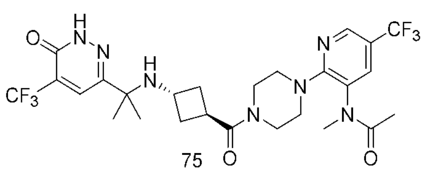

75

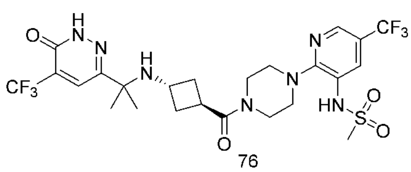

76

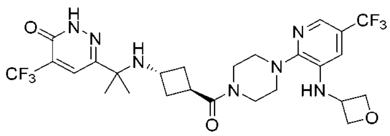
77

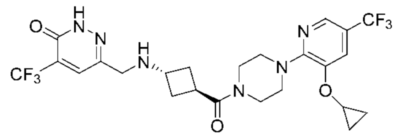
78

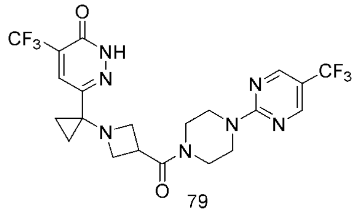
79

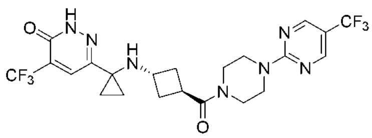
80

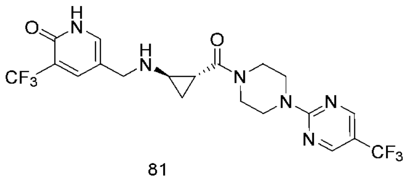
81

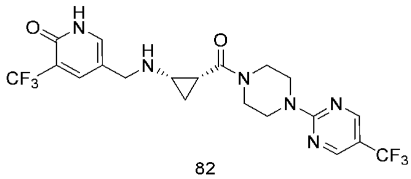
82

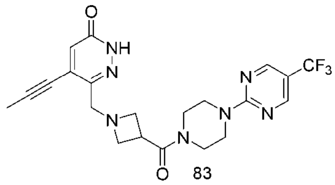
83

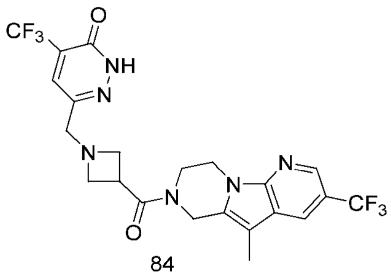
84

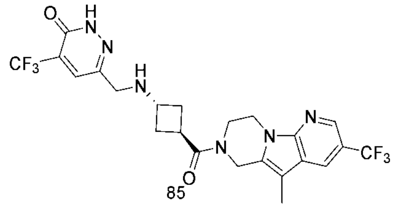
85

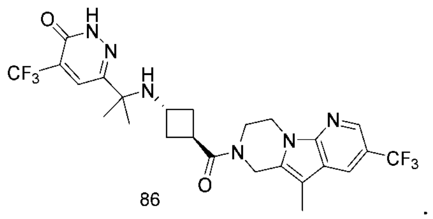
86

.

8. A pharmaceutical composition, wherein the pharmaceutical composition comprises the compound, pharmaceutically acceptable salt, stereoisomer, solvate, or prodrug thereof according to any one of claims 1-7, and at least one pharmaceutically acceptable carrier.

9. Use of the compound, pharmaceutically acceptable salt, stereoisomer, solvate, or prodrug thereof according to any one of claims 1-7, or the pharmaceutical composition of claim 8, in the manufacture of a PARP7 inhibitor or for use in treating a PARP7-mediated disease;

preferably, the PARP7-mediated disease includes cancer, immune diseases, inflammation, and viral infection;
preferably, the cancer includes PARP7-amplified solid tumors, small cell lung cancer, squamous cell lung cancer, or advanced non-small cell lung squamous carcinoma;
preferably, the PARP7-amplified solid tumors include esophageal cancer and head and neck cancer.

10. A method of inhibiting PARP7 in a patient in need thereof, comprising administering to the patient the compound, pharmaceutically acceptable salt, stereoisomer, solvate, or prodrug thereof according to any one of claims 1-7, or the pharmaceutical composition of claim 8.

11. A method of inhibiting PARP7 in a biological sample, comprising contacting the biological sample with the compound, pharmaceutically acceptable salt, stereoisomer, solvate, or prodrug thereof according to any one of claims 1-7, or the pharmaceutical composition of claim 8.

12. A method for treating a PARP7-mediated condition in a patient in need thereof, comprising administering to the patient the compound, pharmaceutically acceptable salt, stereoisomer, solvate, or prodrug thereof according to any one of claims 1-7, or the pharmaceutical composition of claim 8;

preferably, the PARP7-mediated disease includes cancer, immune diseases, inflammation, and viral infection;
preferably, the cancer includes PARP7-amplified solid tumors, small cell lung cancer, squamous cell lung cancer, or advanced non-small cell lung squamous carcinoma;
preferably, the PARP7-amplified solid tumors include esophageal cancer and head and neck cancer.

## INTERNATIONAL SEARCH REPORT

International application No.
**PCT/CN2024/116240**

**A. CLASSIFICATION OF SUBJECT MATTER**

C07D487/08(2006.01)i; C07D487/04(2006.01)i; C07D403/14(2006.01)i; C07D403/12(2006.01)i; C07D403/08(2006.01)i; C07D413/14(2006.01)i; C07D401/14(2006.01)i; C07D498/14(2006.01)i; C07D405/14(2006.01)i; A61K31/506(2006.01)i; A61K31/501(2006.01)i; A61K31/502(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT; CNKI; VEN; WOTXT; EPTXT; USTXT; STN; 万方, WANFANG: 华森英诺, 刘晓辉, 张伟, 王英, 张奇, 蒋诗忆, 冯学蓉, 田世杰, 杨霞, 哒嗪酮, 哌嗪, 三环, 抑制剂, pyridazinone, piperazine, tricycl+, PARP, inhibitor, 结构式, structural formula

**C. DOCUMENTS CONSIDERED TO BE RELEVANT**

| Category* | Citation of document, with indication, where appropriate, of the relevant passages | Relevant to claim No. |
|---|---|---|
| X | GU, Hongfeng et al. "Discovery of the Potent and Highly Selective PARP7 Inhibitor as a Novel Immunotherapeutic Agent for Tumors" *Journal of Medicinal Chemistry,* 28 December 2022 (2022-12-28), ISSN: 0022-2623, tables 1-5 | 1-9, 11 |
| X | CN 116375688 A (CHINA PHARMACEUTICAL UNIVERSITY) 04 July 2023 (2023-07-04) claims 1-8 and 10-12 | 1-9, 11 |
| X | WO 2023020479 A1 (CHONGQING PHARSCIN PHARMACEUTICAL CO., LTD.) 23 February 2023 (2023-02-23) claims 1-16 | 1-9, 11 |
| X | WO 2022184103 A1 (WUHAN YUXIANG PHARMACEUTICAL TECHNOLOGY CO., LTD.) 09 September 2022 (2022-09-09) claims 14-16 | 1, 4, 6, 8, 9 |
| X | WO 2023076983 A1 (GILEAD SCIENCES, INC.) 04 May 2023 (2023-05-04) description, pages 176-178, and claims 1-42 | 1-9, 11 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 October 2024** | **11 December 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/116240**

**C. DOCUMENTS CONSIDERED TO BE RELEVANT**

| Category* | Citation of document, with indication, where appropriate, of the relevant passages | Relevant to claim No. |
|---|---|---|
| PX | WO 2024073133 A1 (AZKARRA THERAPEUTICS, INC. et al.) 04 April 2024 (2024-04-04)<br>description, tables 2 and 8 | 1-4, 6, 8, 9 |
| Y | GU, Hongfeng et al. "Discovery of the Potent and Highly Selective PARP7 Inhibitor as a Novel Immunotherapeutic Agent for Tumors"<br>*Journal of Medicinal Chemistry,* 28 December 2022 (2022-12-28),<br>ISSN: 0022-2623,<br>tables 1-5 | 1-9, 11 |
| Y | WO 2023076983 A1 (GILEAD SCIENCES, INC.) 04 May 2023 (2023-05-04)<br>description, pages 176-178, and claims 1-42 | 1-9, 11 |
| Y | WO 2022170974 A1 (JACOBIO PHARMACEUTICALS CO., LTD.) 18 August 2022 (2022-08-18)<br>claims 1-62 | 1-9, 11 |

Form PCT/ISA/210 (second sheet) (July 2022)

## INTERNATIONAL SEARCH REPORT

International application No. **PCT/CN2024/116240**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **9 (in part), 10-12**
because they relate to subject matter not required to be searched by this Authority, namely:

Part of the technical solution of claim 9 relates to "the use in the treatment of PARP7-mediated diseases", claim 10 sets forth a method for inhibiting PARP7 in a patient in need thereof, claim 11 sets forth a method for inhibiting PARP7 in a biological sample, and claim 12 sets forth a method for treating a PARP7-mediated disease in a patient in need thereof, which all fall within the category of methods for treatment of a living human or animal body, that is, fall within the cases set out in PCT Rule 39.1(iv) for which no international search is required.

Nevertheless, a search is performed for claim 9 on the basis of the technical subject matter "the use for preparing a drug for treating PARP7-mediated diseases"; and a search is performed for claim 11 on the basis of the technical subject matter "the use for preparing a drug for inhibiting PARP7 in a biological sample".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/116240**

| Patent document cited in search report | | | Publication date (day/month/year) | Patent family member(s) | | | Publication date (day/month/year) |
|---|---|---|---|---|---|---|---|
| CN | 116375688 | A | 04 July 2023 | WO | 2023116824 | A1 | 29 June 2023 |
| WO | 2023020479 | A1 | 23 February 2023 | CN | 117377665 | A | 09 January 2024 |
| WO | 2022184103 | A1 | 09 September 2022 | CN | 115028648 | A | 09 September 2022 |
| | | | | CN | 115028648 | B | 30 August 2024 |
| WO | 2023076983 | A1 | 04 May 2023 | KR | 20240091056 | A | 21 June 2024 |
| | | | | US | 2023183216 | A1 | 15 June 2023 |
| | | | | AR | 127497 | A1 | 31 January 2024 |
| | | | | AU | 2022375782 | A1 | 02 May 2024 |
| | | | | JP | 2024539252 | A | 28 October 2024 |
| | | | | CA | 3234909 | A1 | 04 May 2023 |
| | | | | EP | 4423078 | A1 | 04 September 2024 |
| | | | | TW | 202330504 | A | 01 August 2023 |
| | | | | CN | 118139858 | A | 04 June 2024 |
| WO | 2024073133 | A1 | 04 April 2024 | None | | | |
| WO | 2022170974 | A1 | 18 August 2022 | US | 2024336629 | A1 | 10 October 2024 |
| | | | | EP | 4291560 | A1 | 20 December 2023 |
| | | | | AU | 2022220924 | A1 | 21 September 2023 |
| | | | | BR | 112023015721 | A2 | 07 November 2023 |
| | | | | TW | 202246266 | A | 01 December 2022 |
| | | | | JP | 2024506612 | A | 14 February 2024 |
| | | | | WO | 2022170974 | A9 | 30 March 2023 |
| | | | | CA | 3210885 | A1 | 18 August 2022 |
| | | | | KR | 20230167755 | A | 11 December 2023 |
| | | | | AR | 124698 | A1 | 26 April 2023 |
| | | | | CN | 116848114 | A | 03 October 2023 |
| | | | | IN | 202317055962 | A | 08 December 2023 |
| | | | | HK | 40094116 | A0 | 12 January 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311125694
- CN 202311423018


### Non-patent literature cited in the description


- **REMINGTON**. The Science and Practice of Pharmacy